# EUROPEAN PATENT APPLICATION

(11) **EP 3 845 061 A1**
(43) Date of publication of application: **07.07.2021**
(21) Application number: 20207396.1
(22) Date of filing: 17.11.2016
(51) Int. Cl.: A01H 1/08, C07K 14/415, C12N 15/82, C12N 9/18

(54) **HAPLOID INDUCTION COMPOSITIONS AND METHODS FOR USE THEREFOR**

(30) Priority: 18.11.2015 US 201562256902 P; 26.02.2016 US 201662300507 P
(62) Divisional of application: 16867143.6
(71) Applicant: Syngenta Participations AG, 4058 Basel (CH)
(72) Inventor: KELLIHER, Timothy, Joseph, Durham, NC 27709-2257 (US); DELZER, Brent, Janesville, WI 53546 (US); CHINTAMANANI, Satya, Slater, IA 50244 (US); SKIBBE, David, Stewart, Durham, NC 27709 (US); CHEN, Zhongying, Durham, NC 27709 (US); STARR, Dakota, Durham, NC 27709 (US); WENDEBORN, Sebastian, Volker, 4332 Stein (CH)
(74) Representative: SYNGENTA IP

(57) **Abstract**

Provided here are methods of using a mutated patatin-like phospholipase IIα ("pPLAIIα," renamed here MATRILINEAL) to induce haploid induction in plants, cloning a pPLAIIα to induce haploid induction in plants, and genetically engineering a plant to contain a mutated pPLAIIα. Also provided are methods of applying topical and spray chemicals, lipids, and RNAi molecules to plants during pollination in order to induce haploid production. Further provided are methods of chemically treating plants during pollination to induce haploids while also reducing embryo abortion and increasing seed set.

SEQUENCE LISTING

<110>  Syngenta Participations, AG
       Kelliher, Tim
       Starr, Dakota
       Wendeborn, Sebastian
       Delzer, Brent
       Chintamanani, Satya
       Skibbe, David
       Chen, Zhongying

<120>  HAPLOID INDUCTION COMPOSITIONS AND METHODS FOR USE THEREFOR

<130>  80906-WO-REG-ORG-P-1

<150>  US 62/256,902
<151>  2015-11-15

<150>  US 62/300,507
<151>  2016-02-16

<160>  81

<170>  PatentIn version 3.5

<210>  1
<211>  1371
<212>  DNA
<213>  Zea mays

<400>  1
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacca cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gctctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaagccgt cgaactgccg caggttcctg gtgctgtcca tcgggacggg gtcgacgtcc     840

gagcagggcc tctacacggc gcggcagtgc tcccggtggg gtatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 2
<211> 401
<212> PRT
<213> Zea mays

<400> 2

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr

165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210>  3
<211>  1375
<212>  DNA
<213>  Zea mays

<400>  3
agttcatcac taatcacact tattgtgccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctggaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacta cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gcgctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaacccgt cgaactgccg caggttcctg gtgctgtcca tcgggacggg gtcgacgtcc     840

gagcagggcc tctacacggc gcggcagtgc tcccggtggg gcatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct acgcatccag    1020

gacaactcgc tccgtggcgc cgcggcaacc gtggacgcgg cgacgccgga gaacatgcgg    1080

acgctcgtcg ggatcgggga gcggatgctg gcacagcggg tgtccagggt caacgtggag    1140

acagggagcg aggtacgaac cggtgaccgg agaaggaagc aatgccgatg ccctcggtgg    1200

gctcgctagg cagctctccg aggagaggag aacaaggctc gcgcgccgcg tctctgccat    1260

caaccccaga agctctagat gtgcgcccta cgatatctaa gacaagtggc tttactgtca    1320

atcacatgct tgtaaataag tagactttat tttaataaaa tataaaaata tatat         1375


<210>  4
<211>  372
<212>  PRT
<213>  Zea mays

<400>  4

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
        20                  25                  30

Val Thr Val Leu Thr Val Asp Gly Gly Gly Val Arg Gly Leu Ile Pro
    35                  40                  45

Gly Thr Ile Leu Ala Phe Leu Glu Ala Arg Leu Gln Glu Leu Asp Gly
50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn Tyr Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Asn Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Ser Glu Val Arg Thr Gly Asp Arg Arg Arg Lys Gln Cys Arg Cys Pro
        355                 360                 365

Arg Trp Ala Arg
    370

<210>  5
<211>  18
<212>  DNA
<213>  Artificial Sequence

<220>
<223>  GRMZM2G471240_nil.F1 primer

<400>  5
gtacgccgtg cgctaaca                                                    18

<210>  6
<211>  20
<212>  DNA
<213>  Artificial Sequence

<220>
<223>  GRMZM2G471240_nil.R1 primer

<400>  6
tcgtacctcc ctgtctccac                                                  20

<210>  7
<211>  19
<212>  DNA
<213>  Artificial Sequence

<220>
<223>  GRMZM2G471240_rwk.F1 primer

<400>  7

tacgccgtgc gctaacata                                                    19

<210>  8
<211>  20
<212>  DNA
<213>  Artificial Sequence

<220>
<223>  GRMZM2G471240_rwk.R1 primer

<400>  8
gtacctcgct ccctgtctcc                                                   20

<210>  9
<211>  1358
<212>  DNA
<213>  Artificial Sequence

<220>
<223>  TALEN-induced MTL mutation in Event 39A individual ID 22808-3954
       allele 1

<400>  9
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacca cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gctctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaagccgt cgaactgccg caggttcctg gtgctgtcca tcgggacggg gtcgacgtcc     840

gagcagggcc tctacacggc gcggcagtgc tcccggtggg gtatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct gcgcatccag    1020

gacaactcgc tccgtggcgc cgcggccacc gtggacgcgg cgacgccgga gaacatgcgg    1080

acgctcgtcg ggatcgggga gcggatgctg gcacagaggg tgtccagggt caacgtggag    1140

aaccggtgac tggcgaagga agcaatgccg atgccctcgg tgggctcgct aggcagctct 1200

ccgaggagag gagaacaagg ctcgcgcgcc gcgtctctgc catcaaccca agaggctcta 1260

gatgtgcgtc gtacgatatc taagacaagt ggctttactg tcagtcacat gcttgtaaat 1320

aagtagactt tattttaata aaacataaaa atatatat 1358

<210> 10
<211> 1366
<212> DNA
<213> Artificial Sequence

<220>
<223> TALEN-induced MTL mutation in Event 23A individual ID 22808-3924
      allele 1

<400> 10
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggagaa ccggtgactg gcgaaggaag caatgccgat gccctcggtg ggctcgctag 1200

gcagctctcc gaggagagga gaacaaggct cgcgcgccgc gtctctgcca tcaacccaag 1260

aggctctaga tgtgcgtcgt acgatatcta agacaagtgg ctttactgtc agtcacatgc 1320

ttgtaaataa gtagacttta ttttaataaa acataaaaat atatat 1366

<210> 11
<211> 1358
<212> DNA
<213> Artificial Sequence

<220>
<223> TALEN-induced MTL mutation in Event 81A

<400> 11
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

aaccggtgac tggcgaagga agcaatgccg atgccctcgg tgggctcgct aggcagctct 1200

ccgaggagag gagaacaagg ctcgcgcgcc gcgtctctgc catcaaccca agaggctcta 1260

gatgtgcgtc gtacgatatc taagacaagt ggctttactg tcagtcacat gcttgtaaat 1320

aagtagactt tattttaata aaacataaaa atatatat 1358

<210> 12
<211> 1343
<212> DNA
<213> Artificial Sequence

<220>
<223>  TALEN-induced MTL mutation in Event 39A individual ID 22808-3954
      allele 2

<400>  12
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacca cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gctctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaagccgt cgaactgccg caggttcctg gtgctgtcca tcgggacggg gtcgacgtcc     840

gagcagggcc tctacacggc gcggcagtgc tcccggtggg gtatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct gcgcatccag    1020

gacaactcgc tccgtggcgc cgcggccacc gtggacgcgg cgacgccgga gaacatgcgg    1080

acgctcgtcg ggatcgggga gcggatgctg gcacagaggg tgtccagggt caacgtggag    1140

acggaagcaa tgccgatgcc ctcggtgggc tcgctaggca gctctccgag gagaggagaa    1200

caaggctcgc gcgccgcgtc tctgccatca acccaagagg ctctagatgt gcgtcgtacg    1260

atatctaaga caagtggctt tactgtcagt cacatgcttg taaataagta gactttattt    1320

taataaaaca taaaaatata tat                                            1343


<210>  13
<211>  1363
<212>  DNA
<213>  Artificial Sequence

<220>
<223>  TALEN-induced MTL mutation in Event 23A ID 22808-3924 allele 2

<400>  13
agttcatcac taatcacact tattgttccc tcgacgagta tctagctagc tcattaatcg      60

atcaatcggg gtgtgcggtc gaaggcggca atggcgagct actcgtcgcg gcgtccatgc      120

aatacctgta gcacgaaggc gatggccggg agcgtggtcg gcgagcccgt cgtgctgggg      180

cagagggtga cggtgctgac ggtggacggc ggcggcgtcc ggggtctcat cccgggaacc      240

atcctcgcct tcctcgaggc caggctgcag gagctggacg gaccggaggc gaggctggcg      300

gactacttcg actacatcgc cggaaccagc accggcggtc tcatcaccgc catgctcacc      360

gcgcccggca aggacaagcg gcctctctac gctgccaagg acatcaacca cttttacatg      420

gagaactgcc cgcgcatctt ccctcagaag agcaggcttg cggccgccat gtccgcgctg      480

aggaagccaa agtacaacgg caagtgcatg cgcagcctga ttaggagcat cctcggcgag      540

acgagggcca agagcacgcc tctgaagaac gctctgctct cggacgtgtg cattggcacg      600

tccgccgcgc cgacctacct cccggcgcac tacttccaga ctgaagacgc caacggcaag      660

gagcgcgaat acaacctcat cgacggcggt gtggcggcca acaacccgac gatggttgcg      720

atgacgcaga tcaccaaaaa gatgcttgcc agcaaggaca aggccgagga gctgtaccca      780

gtgaagccgt cgaactgccg caggttcctg gtgctgtcca tcgggacggg gtcgacgtcc      840

gagcagggcc tctacacggc gcggcagtgc tcccggtggg gtatctgccg gtggctccgc      900

aacaacggca tggcccccat catcgacatc ttcatggcgg ccagctcgga cctggtggac      960

atccacgtcg ccgcgatgtt ccagtcgctc cacagcgacg gcgactacct gcgcatccag     1020

gacaactcgc tccgtggcgc cgcggccacc gtggacgcgg cgacgccgga gaacatgcgg     1080

acgctcgtcg ggatcgggga gcggatgctg gcacagaggg tgtccagggt caacgtggag     1140

acaggaaccg gtgactggcg aaggaagcaa tgccgatgcc ctcggtgggc tcgctaggca     1200

gctctccgag gagaggagaa caaggctcgc gcgccgcgtc tctgccatca acccaagagg     1260

ctctagatgt gcgtcgtacg atatctaaga caagtggctt tactgtcagt cacatgcttg     1320

taaataagta gactttattt taataaaaca taaaaatata tat                       1363

<210>  14
<211>  1360
<212>  DNA
<213>  Artificial Sequence

<220>
<223>  TALEN-induced MTL mutation in Event 38A individual ID 22808-4108
       allele 1

<400>  14
agttcatcac taatcacact tattgttccc tcgacgagta tctagctagc tcattaatcg       60

atcaatcggg gtgtgcggtc gaaggcggca atggcgagct actcgtcgcg gcgtccatgc      120

aatacctgta gcacgaaggc gatggccggg agcgtggtcg gcgagcccgt cgtgctgggg      180

cagagggtga cggtgctgac ggtggacggc ggcggcgtcc ggggtctcat cccgggaacc      240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgaggccca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaaccggtg actggcgaag gaagcaatgc cgatgccctc ggtgggctcg ctaggcagct 1200

ctccgaggag aggagaacaa ggctcgcgcg ccgcgtctct gccatcaacc caagaggctc 1260

tagatgtgcg tcgtacgata tctaagacaa gtggctttac tgtcagtcac atgcttgtaa 1320

ataagtagac tttattttaa taaaacataa aaatatatat 1360

<210> 15
<211> 1363
<212> DNA
<213> Artificial Sequence

<220>
<223> CRISPR-induced MTL mutation in Event 18A ID 22807-4016

<400> 15
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagaggtgga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaggaaccg gtgactggcg aaggaagcaa tgccgatgcc ctcggtgggc tcgctaggca 1200

gctctccgag gagaggagaa caaggctcgc gcgccgcgtc tctgccatca acccaagagg 1260

ctctagatgt gcgtcgtacg atatctaaga caagtggctt tactgtcagt cacatgcttg 1320

taaataagta gactttattt taataaaaca taaaaatata tat 1363

<210> 16
<211> 1372
<212> DNA
<213> Artificial Sequence

<220>
<223> CRISPR-induced MTL mutation in Event 27A individual ID 22807-4801
 allele 1

<400> 16
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaagggagg tacgaaccgg tgactggcga aggaagcaat gccgatgccc tcggtgggct 1200

cgctaggcag ctctccgagg agaggagaac aaggctcgcg cgccgcgtct ctgccatcaa 1260

cccaagaggc tctagatgtg cgtcgtacga tatctaagac aagtggcttt actgtcagtc 1320

acatgcttgt aaataagtag actttatttt aataaaacat aaaaatatat at 1372

<210> 17
<211> 1372
<212> DNA
<213> Artificial Sequence

<220>
<223> CRISPR-induced mutation in Event 27A individual ID 22807-4081
      allele 1

<400> 17
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgaggccaa agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaagggagg tacgaaccgg tgactggcga aggaagcaat gccgatgccc tcggtgggct 1200

cgctaggcag ctctccgagg agaggagaac aaggctcgcg cgccgcgtct ctgccatcaa 1260

cccaagaggc tctagatgtg cgtcgtacga tatctaagac aagtggcttt actgtcagtc 1320

acatgcttgt aaataagtag actttatttt aataaaacat aaaaatatat at 1372

<210> 18
<211> 1373
<212> DNA
<213> Artificial Sequence

<220>
<223> CRISPR-induced MTL mutation in Event 76A individual ID 22873-3999

<400> 18
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaaagggag gtacgaaccg gtgactggcg aaggaagcaa tgccgatgcc ctcggtgggc 1200

tcgctaggca gctctccgag gagaggagaa caaggctcgc gcgccgcgtc tctgccatca 1260

acccaagagg ctctagatgt gcgtcgtacg atatctaaga caagtggctt tactgtcagt 1320

cacatgcttg taaataagta gactttattt taataaaaca taaaaatata tat 1373

<210> 19
<211> 1370
<212> DNA
<213> Artificial Sequence

<220>
<223> CRISPR-induced mutation in Event 32A individual ID 22873-3991

<400> 19
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgaggccaa agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acgggaggta cgaaccggtg actggcgaag gaagcaatgc cgatgccctc ggtgggctcg 1200

ctaggcagct ctccgaggag aggagaacaa ggctcgcgcg ccgcgtgtct gccatcaacc 1260

caagaggctc tagatgtgcg tcgtacgata tctaagacaa gtggctttac tgtcagtcac 1320

atgcttgtaa ataagtagac tttattttaa taaaacataa aaatatatat 1370

<210> 20
<211> 18
<212> DNA
<213> Artificial Sequence

<220>
<223> nucleotide sequence encoding guide RNA

<400> 20
gtcaacgtgg agacaggg 18

<210> 21
<211> 1823
<212> DNA
<213> Oryza sativa

<220>
<221> 5'UTR
<222> (1)..(78)

<220>
<221> exon
<222> (79)..(444)
<223> exon 1

<220>
<221> Intron
<222> (445)..(536)
<223> intron 1

<220>
<221> exon
<222> (537)..(717)
<223> exon 2

<220>
<221> Intron
<222> (718)..(803)
<223> intron 2

<220>
<221> exon
<222> (804)..(967)
<223> exon 3

<220>
<221> Intron
<222> (968)..(1074)
<223> intron 3

<220>
<221> exon
<222> (1075)..(1662)
<223> exon 4

<220>
<221> 3'UTR
<222> (1663)..(1823)

<400> 21
acagtgacta gtgacaaacg atcgatcgat ccctccatcc acaaaccctc ctcgatctca 60

tcttccttcg tctcgtca atg gcg gcg agc tac tcg tgc cgg cgg aca tgc 111
Met Ala Ala Ser Tyr Ser Cys Arg Arg Thr Cys
1               5                   10

gag gcg tgc agc acg agg gcg atg gcc ggg tgc gtg gtg ggc gag ccg 159
Glu Ala Cys Ser Thr Arg Ala Met Ala Gly Cys Val Val Gly Glu Pro
            15                  20                  25

gcg tcg gcg ccg ggg cag cgg gtg acg ttg ctg gcg atc gac ggc ggc 207
Ala Ser Ala Pro Gly Gln Arg Val Thr Leu Leu Ala Ile Asp Gly Gly
        30                  35                  40

ggc atc agg ggc ctc atc ccg ggc acc atc ctc gcc ttc ctc gag gcc 255
Gly Ile Arg Gly Leu Ile Pro Gly Thr Ile Leu Ala Phe Leu Glu Ala
    45                  50                  55

agg ctg cag gag ctg gat ggc ccc gac gcg cgc ctc gcc gat tac ttc 303
Arg Leu Gln Glu Leu Asp Gly Pro Asp Ala Arg Leu Ala Asp Tyr Phe
60                  65                  70                  75

gac tgc atc gcc ggg acc agc acc ggc ggc ctc atc acc gcc atg ctc 351
Asp Cys Ile Ala Gly Thr Ser Thr Gly Gly Leu Ile Thr Ala Met Leu
                80                  85                  90

gcc gcg ccc ggc gac cac ggc cgc ccg ctc ttc gcc gcc agc gac atc 399
Ala Ala Pro Gly Asp His Gly Arg Pro Leu Phe Ala Ala Ser Asp Ile
            95                  100                 105

aac cgc ttc tac ctc gac aac ggc cca ctc atc ttc cca caa aag 444
Asn Arg Phe Tyr Leu Asp Asn Gly Pro Leu Ile Phe Pro Gln Lys
        110                 115                 120

taactgatca cctcgaattc gatctcctct cttcgatctc tgcattattt gatttgattg 504

gggattgtgg gcggcgtggc gtggcgtcca gg agg tgc ggc atg gcg gcg gcc 557
Arg Cys Gly Met Ala Ala Ala
        125

atg gcg gcg ctg acg agg ccg agg tac aac ggc aag tac ctg cag ggg 605
Met Ala Ala Leu Thr Arg Pro Arg Tyr Asn Gly Lys Tyr Leu Gln Gly
130                 135                 140                 145

aag atc agg aag atg ctg ggc gag acg agg gtg cgc gac acg ctg acg 653
Lys Ile Arg Lys Met Leu Gly Glu Thr Arg Val Arg Asp Thr Leu Thr
                150                 155                 160

aac gtc gtc atc ccc acg ttc gac gtc agg ctg ctc cag cca acc atc 701
Asn Val Val Ile Pro Thr Phe Asp Val Arg Leu Leu Gln Pro Thr Ile
            165                 170                 175

ttc tcc aca tac gac g tgcgtgcgtt gattccatcc gcattggcgt tggaatcagc 757
Phe Ser Thr Tyr Asp
        180

tgattgtttg attgatcgaa caattgatcg gttaaaattt tgcagg cg aag agc 811

Ala Lys Ser
        185

atg ccg ctc aag aac gcg ctc ctc tcc gac atc tgc atc agc aca tcc 859
Met Pro Leu Lys Asn Ala Leu Leu Ser Asp Ile Cys Ile Ser Thr Ser
                190                 195                 200

gcg gcg ccg acc tac ctc ccc gcg cac tgc ttc cag acc acc gac gac 907
Ala Ala Pro Thr Tyr Leu Pro Ala His Cys Phe Gln Thr Thr Asp Asp
            205                 210                 215

gcc acc ggc aag gtc cgc gag ttc gac ctc atc gac ggc ggc gtc gcc 955
Ala Thr Gly Lys Val Arg Glu Phe Asp Leu Ile Asp Gly Gly Val Ala
        220                 225                 230

gcc aac aac ccg gtaactaatc aatcaagcaa tccatcaaac gaagatccac 1007
Ala Asn Asn Pro
    235

atgtgcattc ctgtggtaca aatgctgatc gatcgatgga tggatcgatt ttcgcgagaa 1067

cgtacag acg atg gtg gcc atg acg cag atc acc aag aag ata atg gtg 1116
Thr Met Val Ala Met Thr Gln Ile Thr Lys Lys Ile Met Val
        240                 245                 250

aag gac aag gag gag ctg tac ccg gta aag ccg tcg gac tgc ggt aag 1164
Lys Asp Lys Glu Glu Leu Tyr Pro Val Lys Pro Ser Asp Cys Gly Lys
            255                 260                 265

ttc ctg gtg ctg tcc gtg ggc acc ggg tcg acg tcg gac cag ggg atg 1212
Phe Leu Val Leu Ser Val Gly Thr Gly Ser Thr Ser Asp Gln Gly Met
        270                 275                 280

tac acg gcg agg cag tgc tcg cgg tgg ggg atc gtc cgg tgg ctg cgc 1260
Tyr Thr Ala Arg Gln Cys Ser Arg Trp Gly Ile Val Arg Trp Leu Arg
    285                 290                 295

aac aag ggg atg gcg ccc atc atc gac atc ttc atg gcg gcc agc tcc 1308
Asn Lys Gly Met Ala Pro Ile Ile Asp Ile Phe Met Ala Ala Ser Ser
300                 305                 310                 315

gac ctc gtc gac atc cac gcc gcc gtc atg ttc cag tcg ctg cac agc 1356
Asp Leu Val Asp Ile His Ala Ala Val Met Phe Gln Ser Leu His Ser
                320                 325                 330

gac ggc gac tac ctc cgc atc cag gac aac acg ctc cac ggc gac gcc 1404
Asp Gly Asp Tyr Leu Arg Ile Gln Asp Asn Thr Leu His Gly Asp Ala
            335                 340                 345

gcc acg gtg gac gcc gcc acc agg gac aac atg cgg gcg ctc gtc ggg 1452
Ala Thr Val Asp Ala Ala Thr Arg Asp Asn Met Arg Ala Leu Val Gly
        350                 355                 360

atc ggc gag cgg atg ctg gcg cag cgg gtg tcg agg gtc aac gtc gag 1500
Ile Gly Glu Arg Met Leu Ala Gln Arg Val Ser Arg Val Asn Val Glu
    365                 370                 375

acc ggc agg tac gtc gag gtg ccc ggc gcc ggc agc aac gcc gac gcg 1548
Thr Gly Arg Tyr Val Glu Val Pro Gly Ala Gly Ser Asn Ala Asp Ala
380                 385                 390                 395

ctg agg ggc ttc gcc agg cag ctc tcc gag gag agg agg gcg agg cta 1596
Leu Arg Gly Phe Ala Arg Gln Leu Ser Glu Glu Arg Arg Ala Arg Leu

400                 405                 410

ggt cgg cga aac gcc tgc ggc ggc ggc ggc gaa gga gag ccc agc ggc      1644
Gly Arg Arg Asn Ala Cys Gly Gly Gly Gly Glu Gly Glu Pro Ser Gly
            415                 420                 425

gtg gcg tgc aag cgt tag taactgtaca cgcatcatgc tgacgcgatc             1692
Val Ala Cys Lys Arg
        430

tttttatatt ttcttttttt ttttttacct ttctagcgga catggggaat aacaagacgt    1752

gacagtagtg caatcggttt gtaacgtgcg tataccaaca ttgatccatt tcttcatcac    1812

agtttcagtt c                                                         1823


<210>  22
<211>  1299
<212>  DNA
<213>  Oryza sativa

<400>  22
atggcggcga gctactcgtg ccggcggaca tgcgaggcgt gcagcacgag ggcgatggcc      60

gggtgcgtgg tgggcgagcc ggcgtcggcg ccggggcagc gggtgacgtt gctggcgatc     120

gacggcggcg gcatcagggg cctcatcccg ggcaccatcc tcgccttcct cgaggccagg     180

ctgcaggagc tggatggccc cgacgcgcgc ctcgccgatt acttcgactg catcgccggg     240

accagcaccg gcggcctcat caccgccatg ctcgccgcgc ccggcgacca cggccgcccg     300

ctcttcgccg ccagcgacat caaccgcttc tacctcgaca acggcccact catcttccca     360

caaaagaggt gcggcatggc ggcggccatg gcggcgctga cgaggccgag gtacaacggc     420

aagtacctgc aggggaagat caggaagatg ctgggcgaga cgagggtgcg cgacacgctg     480

acgaacgtcg tcatccccac gttcgacgtc aggctgctcc agccaaccat cttctccaca     540

tacgacgcga agagcatgcc gctcaagaac gcgctcctct ccgacatctg catcagcaca     600

tccgcggcgc cgacctacct ccccgcgcac tgcttccaga ccaccgacga cgccaccggc     660

aaggtccgcg agttcgacct catcgacggc ggcgtcgccg ccaacaaccc gacgatggtg     720

gccatgacgc agatcaccaa gaagataatg gtgaaggaca aggaggagct gtacccggta     780

aagccgtcgg actgcggtaa gttcctggtg ctgtccgtgg gcaccgggtc gacgtcggac     840

caggggatgt acacggcgag gcagtgctcg cggtggggga tcgtccggtg gctgcgcaac     900

aaggggatgg cgcccatcat cgacatcttc atggcggcca gctccgacct cgtcgacatc     960

cacgccgccg tcatgttcca gtcgctgcac agcgacggcg actacctccg catccaggac    1020

aacacgctcc acggcgacgc cgccacggtg gacgccgcca ccagggacaa catgcgggcg    1080

ctcgtcggga tcggcgagcg gatgctggcg cagcgggtgt cgagggtcaa cgtcgagacc    1140

ggcaggtacg tcgaggtgcc cggcgccggc agcaacgccg acgcgctgag gggcttcgcc    1200

aggcagctct ccgaggagag gagggcgagg ctaggtcggc gaaacgcctg cggcggcggc 1260

ggcgaaggag agcccagcgg cgtggcgtgc aagcgttag 1299

<210> 23
<211> 432
<212> PRT
<213> Oryza sativa

<400> 23

Met Ala Ala Ser Tyr Ser Cys Arg Arg Thr Cys Glu Ala Cys Ser Thr
1               5                   10                  15

Arg Ala Met Ala Gly Cys Val Val Gly Glu Pro Ala Ser Ala Pro Gly
            20                  25                  30

Gln Arg Val Thr Leu Leu Ala Ile Asp Gly Gly Gly Ile Arg Gly Leu
        35                  40                  45

Ile Pro Gly Thr Ile Leu Ala Phe Leu Glu Ala Arg Leu Gln Glu Leu
    50                  55                  60

Asp Gly Pro Asp Ala Arg Leu Ala Asp Tyr Phe Asp Cys Ile Ala Gly
65                  70                  75                  80

Thr Ser Thr Gly Gly Leu Ile Thr Ala Met Leu Ala Ala Pro Gly Asp
                85                  90                  95

His Gly Arg Pro Leu Phe Ala Ala Ser Asp Ile Asn Arg Phe Tyr Leu
            100                 105                 110

Asp Asn Gly Pro Leu Ile Phe Pro Gln Lys Arg Cys Gly Met Ala Ala
        115                 120                 125

Ala Met Ala Ala Leu Thr Arg Pro Arg Tyr Asn Gly Lys Tyr Leu Gln
    130                 135                 140

Gly Lys Ile Arg Lys Met Leu Gly Glu Thr Arg Val Arg Asp Thr Leu
145                 150                 155                 160

Thr Asn Val Val Ile Pro Thr Phe Asp Val Arg Leu Leu Gln Pro Thr
                165                 170                 175

Ile Phe Ser Thr Tyr Asp Ala Lys Ser Met Pro Leu Lys Asn Ala Leu
            180                 185                 190

Leu Ser Asp Ile Cys Ile Ser Thr Ser Ala Ala Pro Thr Tyr Leu Pro
        195                 200                 205

Ala His Cys Phe Gln Thr Thr Asp Asp Ala Thr Gly Lys Val Arg Glu
    210                 215                 220

Phe Asp Leu Ile Asp Gly Gly Val Ala Ala Asn Asn Pro Thr Met Val
225                 230                 235                 240

Ala Met Thr Gln Ile Thr Lys Lys Ile Met Val Lys Asp Lys Glu Glu
                245                 250                 255

Leu Tyr Pro Val Lys Pro Ser Asp Cys Gly Lys Phe Leu Val Leu Ser
            260                 265                 270

Val Gly Thr Gly Ser Thr Ser Asp Gln Gly Met Tyr Thr Ala Arg Gln
        275                 280                 285

Cys Ser Arg Trp Gly Ile Val Arg Trp Leu Arg Asn Lys Gly Met Ala
    290                 295                 300

Pro Ile Ile Asp Ile Phe Met Ala Ala Ser Ser Asp Leu Val Asp Ile
305                 310                 315                 320

His Ala Ala Val Met Phe Gln Ser Leu His Ser Asp Gly Asp Tyr Leu
                325                 330                 335

Arg Ile Gln Asp Asn Thr Leu His Gly Asp Ala Ala Thr Val Asp Ala
            340                 345                 350

Ala Thr Arg Asp Asn Met Arg Ala Leu Val Gly Ile Gly Glu Arg Met
        355                 360                 365

Leu Ala Gln Arg Val Ser Arg Val Asn Val Glu Thr Gly Arg Tyr Val
    370                 375                 380

Glu Val Pro Gly Ala Gly Ser Asn Ala Asp Ala Leu Arg Gly Phe Ala
385                 390                 395                 400

Arg Gln Leu Ser Glu Glu Arg Arg Ala Arg Leu Gly Arg Arg Asn Ala
                405                 410                 415

Cys Gly Gly Gly Gly Glu Gly Glu Pro Ser Gly Val Ala Cys Lys Arg
            420                 425                 430

<210>  24
<211>  1371
<212>  DNA
<213>  Zea mays

<400>  24

agttcatcac taatcacact tattgtgccc tcgacgagta tctatagcta gctcattaat 60

cgattcgggg gtgtgttgtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctggaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

cagaactgcc cgcgcatctt tcctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtctc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 25
<211> 1375
<212> DNA
<213> Zea mays

<400> 25
agttcatcac taatcacact tattgtgccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctggaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacta cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gcgctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaacccgt cgaactgccg caggttcctg gtgctgtcca tcgggacggg gtcgacgtcc 840

gagcagggcc tctacacggc gcggcagtgc tcccggtggg gcatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct acgcatccag 1020

gacaactcgc tccgtggcgc cgcggcaacc gtggacgcgg cgacgccgga gaacatgcgg 1080

acgctcgtcg ggatcgggga gcggatgctg gcacagcggg tgtccagggt caacgtggag 1140

acagggagcg aggtacgaac cggtgaccgg agaaggaagc aatgccgatg ccctcggtgg 1200

gctcgctagg cagctctccg aggagaggag aacaaggctc gcgcgccgcg tctctgccat 1260

caaccccaga agctctagat gtgcgcccta cgatatctaa gacaagtggc tttactgtca 1320

atcacatgct tgtaaataag tagactttat tttaataaaa tataaaaata tatat 1375

<210> 26
<211> 1375
<212> DNA
<213> Zea mays

<400> 26
agttcatcac taatcacact tattgtgccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctggaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacta cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gcgctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaacccgt cgaactgccg caggttcctg gtgctgtcca tcgggacggg gtcgacgtcc 840

gagcagggcc tctacacggc gcggcagtgc tcccggtggg gcatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct acgcatccag 1020

gacaactcgc tccgtggcgc cgcggcaacc gtggacgcgg cgacgccgga gaacatgcgg 1080

acgctcgtcg ggatcgggga gcggatgctg gcacagcggg tgtccagggt caacgtggag 1140

acagggagcg aggtacgaac cggtgaccgg agaaggaagc aatgccgatg ccctcggtgg 1200

gctcgctagg cagctctccg aggagaggag aacaaggctc gcgcgccgcg tctctgccat 1260

caaccccaga agctctagat gtgcgcccta cgatatctaa gacaagtggc tttactgtca 1320

atcacatgct tgtaaataag tagactttat tttaataaaa tataaaaata tatat 1375

<210> 27
<211> 401
<212> PRT
<213> Zea mays

<400> 27

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser

115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210>  28
<211>  372
<212>  PRT
<213>  Zea mays

<400>  28

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn Tyr Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Asn Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Ser Glu Val Arg Thr Gly Asp Arg Arg Arg Lys Gln Cys Arg Cys Pro
        355                 360                 365

Arg Trp Ala Arg
    370

<210>  29
<211>  372
<212>  PRT
<213>  Zea mays

<400>  29

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn Tyr Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Asn Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Ser Glu Val Arg Thr Gly Asp Arg Arg Arg Lys Gln Cys Arg Cys Pro
        355                 360                 365

Arg Trp Ala Arg
    370

<210>  30
<211>  15463
<212>  DNA
<213>  Artificial Sequence

<220>
<223>  Expression cassette of construct 22466

<220>
<221>  promoter
<222>  (214)..(2339)

<220>
<221>  gene
<222>  (2341)..(3018)

<220>
<221>  terminator
<222>  (3024)..(4036)

<220>
<221>  enhancer
<222>  (4044)..(4237)
<223>  eFMV

<220>
<221>  enhancer
<222>  (4244)..(4536)
<223>  e35S

<220>
<221>  promoter
<222>  (4544)..(8537)

<223>  Ubi

<220>
<221>  terminator
<222>  (10009)..(11008)
<223>  Ubi

<220>
<221>  promoter
<222>  (11028)..(12618)
<223>  pPLA2

<220>
<221>  exon
<222>  (12619)..(12977)
<223>  PLA2 exon1

<220>
<221>  Intron
<222>  (12978)..(13096)
<223>  PLA2 intron1

<220>
<221>  exon
<222>  (13097)..(13193)
<223>  PLA2 exon2

<220>
<221>  Intron
<222>  (13194)..(13388)
<223>  PLA2 intron2

<220>
<221>  exon
<222>  (13389)..(13550)
<223>  PLA2 exon3

<220>
<221>  Intron
<222>  (13551)..(13660)
<223>  PLA2 intron3

<220>
<221>  exon
<222>  (13661)..(14248)
<223>  PLA2 exon4

<220>
<221>  terminator
<222>  (14249)..(15244)
<223>  tPLA2

<400>  30
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga cccgaaagta gcaaacaaca ggttcatgtg     240

cactataaaa agacaaaatt ctcgagtttc atcttttatt ccacataagc cttatatttt     300

ccattttcat atgattttta gtttaagttt gtgtcttaac tttttcgtta atacgtaatt 360

ctatgcatta tggatgcgtg aagtattttt gtttaaaaaa atgaaatgtc aaaatacgtt 420

ttgtgatcta tttccatgtt ttcacctaac aggtggtttt tactatatat tctgccataa 480

ctctagcctt agatgtaaat cgaaaaaaaa tgagagatga gctggagata gccttagatg 540

aagcgtctga aatataaaag aaagagtaat gttgaacgca gtaggtgtag cagctgtagt 600

tccatctcta ggaaagggaa ctgcaatccg ggctccgggc ctcgcgcaat ctggcctgtc 660

gtgtagatgc agccctgtcc atgacggccc aagcaacgcc cgcggctctc gatccaccac 720

ggaacccact ccgacacaca ctgacacaca catgctggat gtggatgtgc tgtccaatta 780

ttagtagcaa ttcggtaggc acaggcacgt actggccggt gttttagctg taagtaccga 840

accaatcacg gttaagaacc gattaatccg tgcccagccg ccgagtgcgt tcgtacgtgc 900

atcggatgca ctgcatgaat tgagagcatc atcatatcat acgcaggagt agtacgacgc 960

cgctgctgtc ttgtccggct aatgctttgc tcacagatta gtccatcgcc cacggtcggt 1020

gtggtgtgga tcgctgatgc cactgctttt tgtttggttt ttattcccct gataatcctc 1080

cgcgtccctg aatgtatcta tttattttca ttccgaaatc cctttcacga aaaagaaaac 1140

gaataaaaag agagttacga atacgcttcc ggcggcccac atcaccttcc agcgaacatc 1200

gcgccgcgct gacgtgtcgc ccatcgcggc cgtccatatc gccatccgac gaccgtggaa 1260

gctggcagcg gccgctccgt tccgtcgaag gggcaggtca gtcaggtcac ccacacggcc 1320

acacccgcgc gggggatacg cggtggaaaa cccggcgacc acatcaaaac acgaggcgtc 1380

tcccgcagga ctggtcactc ggcacgcagg cagaggcagc acagcagcag ccagctccat 1440

ccatcctctt tcccctcctc gcttcgcttc ctcggcggat tcctcctccc tcggccgtcc 1500

ccgtcccctt cttcgccgcg ccagctcgcc cgagttggta cgcccatcct ctgctgtact 1560

ccccccgttc ctgctgtgcg aaaccgagca cccaaaccct agcctaagct attcgcatcg 1620

caaactctat tgagcgacgg atcgcgaaac gcgcgccgcc gcattcggac gagacctcgt 1680

ggcccgtacc ttcctactct accgtcgtcg ccggagacca gctcaccggt tagggtttcg 1740

ggattagggg cttggggctt ggattcccgg gggactccat acccggtggc cttagaaggg 1800

gaagggggcc cagcggtggt ggtggttcga ctgtcagcga gcgagacggc ggggcaccgc 1860

cgatcgggcg tcgctggaca tttgattgag ctgggcaggc gaaggcgtgg agcttgctct 1920

tcgattggga ttagaggagg gcggaggtgg tatggtgggc ggctcggctg gctcaatgga 1980

gccgctggcc ggtggtgggg caggcgagat cacgctctct catagagcgt aatgggttgc 2040

gtaacacact cctgtttgta tttcgatccg atctaataat taaagtttat ttacgtggtg 2100

cacatgtaga attttcccat gcccactgct aacttggatt ggatttttgc caaaaaaacg 2160

ggtgtgcacc caatcactta tagtgaaacc gttgtgtgtt tgatgtgctt ctaaccaagt 2220

acaaatccag tgaaaacaca cctaccattc aacaatccac attttggttg caattatcag 2280

cattctagaa aggtgcatgt gcccattgat acacttgcta ttggtgcagg caaacccacc 2340

atggcctcct ccgagaacgt catcaccgag ttcatgcgct tcaaggtgcg catggagggc 2400

accgtgaacg gccacgagtt cgagatcgag ggcgagggcg agggccgccc ctacgagggc 2460

cacaacaccg tgaagctgaa ggtgaccaag ggcggccccc tgcccttcgc ctgggacatc 2520

ctgtcccccc agttccagta cggctccaag gtgtacgtga agcaccccgc cgacatcccc 2580

gactacaaga agctgtcctt ccccgagggc ttcaagtggg agcgcgtgat gaacttcgag 2640

gacggcggcg tggcgaccgt gacccaggac tcctccctgc aggacggctg cttcatctac 2700

aaggtgaagt tcatcggcgt gaacttcccc tccgacggcc ccgtgatgca gaagaagacc 2760

atgggctggg aggcctccac cgagcgcctg tacccccgcg acggcgtgct gaagggcgag 2820

acccacaagg ccctgaagct gaaggacggc ggccactacc tggtggagtt caagtccatc 2880

tacatggcca agaagcccgt gcagctgccc ggctactact acgtggacgc caagctggac 2940

atcacctccc acaacgagga ctacaccatc gtggagcagt acgagcgcac cgagggccgc 3000

caccacctgt tcctgtagga gctcgcatca tgatcatgca tcatggactc ggcctactac 3060

tgtggatttg tatgccatta tagacttggt gctgtgaaag actgcttgat gatttgcggg 3120

tttgttgctg tgtaaaaaaa ggtcccttgg ctcccagaag accatgaagg ttcggatcta 3180

tcatgtaatt ccttgttatc tgccaattat gtatggacta tggacatgtg ttgcgctgtt 3240

caacttacta ctacaaataa gtaatcgata tgttcccttc ccatgtctcg gtgacaattg 3300

tctggagaag cttaggggtc gtttgtttgg gattatgtct ggagaaactt attttaaact 3360

aagtgtgagt tcaagttaag ttagattata taatctaggc agattataat tccaagcgaa 3420

caggtcctta gtgtttttgg aaaatcctag gtgttctttt ggctacattg ttgtgtgtgc 3480

agatcccttg ttggtctgta agcgtgggga agtaagaatc gtccgtttct actgaagacc 3540

tgctcgagtt aggcaccgag gatgccggta accaaacaga gcaatagtgt ctctgtgggc 3600

acagtggagt gtgaatctgt gtgatgcaaa tccgtcattt gtttagcaaa atttccagcg 3660

ttgcatgatg cagtttcttt aacacggact taagggaagg gaaaaaaatg ttgagccagg 3720

agatccttca atgtgttaga ctgacgtgat agccaactaa accacgacgc aatgttgtcg 3780

ttaatgacaa aaaaactatt tgttcctaaa tccttggcga cattgcatgg ctgtctcatg 3840

agataatggt ctcatctctt atttatctct tatttatagc cggaagtggt agtgacccct 3900

gcttgattgc tcgtatgcca tctcaagttc tcaaccgtgt cgagcagcca ttttcccatc 3960

tcaagcgcat catcgtttcg tttgacctca tctgctatcc tgctcctagt gcaaatcaca 4020

tgcgacagaa agtgtgcgga cccagctgct tgtggggacc agacaaaaaa ggaatggtgc 4080

agaattgtta ggcgcaccta ccaaaagcaa ctttgccttt attgcaaaga taaagcagat 4140

tcctctagta caagtgggga acaaaataac gtggaaaaga gctgtcctga cagcccactc 4200

actattgcgt ttgacgaacg cagtgacgac cacaaaactc gagacttttc aacaaagggt 4260

attatccgga aacctcctcg gattccattg cccagctatc tgtcacttta ttgtgaagat 4320

agtggaaaag gaaggtggct cctacaaatg ccatcattgc gataaaggaa aggctatcgt 4380

tgaagatgcc tctgccgaca gtggtcccaa agatggaccc ccacccacga ggagcatcgt 4440

ggaaaaagaa gacgttccaa ccacgtcttc aaagcaagtg gattgatgtg atatctccac 4500

tgacgtaagg gttgacgaac aatcccacta tccttcaagc tttggaaaac caaaacaacg 4560

aataagcaaa ctgcaggaaa agtatgcagt ggaaaccaac ccagattcgg acgataggaa 4620

agtatcaagt gaatgatttg ccaggaaaag gagaggggta aaaaggggcg aagatttaga 4680

agatctaaag cacaagaacc agagattaga ttgaacaata gggaacttgg agcatccttt 4740

ttttcttcag ggaaaaactg aaaatccaaa ccatgttgag caaaaccgag tgggattgga 4800

aaccaaaaaa cccgagataa agaaactcga gaaaaagcat gaaatcgaaa ccaacttcag 4860

taaaacaaaa ggaggacaga aaagaaagtc ggaagctata aagaatacat taacattcag 4920

tgaaacagca tgctgtcttc ttcttttttt atgcacaaca gagcatacat atataccttc 4980

ccaggctgag gacttggcgg aggagagccg cggataggtt ggcggtgcag acggtctgga 5040

cgggcccgaa gacggagacg aacagcgggc ccttcctgcc caggcaccac gcttggaacg 5100

ccaagcacgc gccaaccgcg gccccgccga ggacgacgat ccccgcgaca agcgtggcgt 5160

cgatcctggg cgaccccaag ccgaggaacc tcccttccag gacgagccgt aggacggcgg 5220

tgagagaggc acccgtcgcg gaggtggcgc agcacaaggt gagcagcgcg gggaaggcgg 5280

cgagcgtggc ggcctgcagg acggtgacga gcgcgaagac ggtgacgccg gcgacgaggc 5340

agcagcagcc gaggatccag tcgtaggagg aagccggacc aaaccgggca atgcaacctg 5400

cagatgcact agacggaggt aacgaggagg aggagaaaac agagcaagag caggcggaga 5460

gaagatagag caaaacacga gtgaggcaca gcgtaagcac tcggtagaag tctccagagg 5520

cgaggtgcgc acaggagaac agatgagtaa agtcagccaa ggatccacga tccaacggct 5580

acgaattttt ggagtgacgt ggataggctc aaaggcgcca tttccatccg gctttatagt 5640

attttaaaaa aattcatttt cctccctcta gtgtgtgcgg aggcgtgagc ccgtttaacg 5700

gcgttgagaa gtctaacgga caccaaccac aaccaggaac cagcgccggc cgcgccgccg 5760

agtgaagcag actgcatacg gcacggcgcg gcatctctct ggctgcctct cgagagttcc 5820

gcccccacct tcccgcggta gcgtggtggt ttcgctttcc gctgtcggca tccggaagtt 5880

gcgtggcaga gtggacggag acgaggccgg gtcctccagc tcctctcaaa cgtcacggca 5940

ccggcatccg gcagccagcg cggtccttcc caaccactcg ttcccaaccc atcccccttc 6000

ctcgcccgcc gtcataaata gccagcccca tccccagctt ctttccccaa cctcatcttc 6060

tctccttttg ctctgaacgc acacaccgcc cggtctccga tctccgatcc ccgatcccct 6120

cgtcgatcct aggtacggcg accatcctac cccccccccc cccccctctc tctctgcctt 6180

ctctagatcg gcgatccgat ccatgcttac ttggttaggg cctgctaact atgttcatgt 6240

ttgcgttaga tccgtgcatg gacgcgatct gtacacacca gacgcgttct gattgctagc 6300

taactcgcca gtacctggga atcctgggat ggctgtagcc ggccccgcac gcagacggga 6360

ccgatttcat gattctctat ttttttcttt gtttcgttgc ctagggtttc gttcgatcga 6420

tccgcgttat tctttatttc catatattct ggtacgatgt tgatacggtt cgaccgtgct 6480

gcttacgttc tgtgcgcttg tttgccgggt catttttacc ttgccttttt tgtatggttt 6540

ggttgtggcg atgtggtctg gtcgggctgt cgttctagat cggagtagag tgctgtttca 6600

aactgtctag cggatctatt agatttggat ctgcatgtgt gacatatatc ttcgtagtta 6660

agatgatgca tctgtatgtg tgacatgcgg atctattaga tttggatctg tatgtgtgac 6720

atatatcttc gtagttgaga tgatgcatct gtatgtgtga catatatctt cgtagttaag 6780

attatgcatg gaaatatcaa tcctttagat aaggacgggt atacttgttg ctgtgggttt 6840

tactggtact tcgatagatg catatacatg atctaacatg cttagataca tgaagtaaca 6900

tgctgctacg gtttaataat tcttgagttg atttttactg gtacttagat agatgtatat 6960

acatgcttag atacatgaag taacatgctc ctacagttcc tttaatcatt attgagtacc 7020

tatatattct aataaatcag tatgttttaa attattttga ttttactggt acttagatag 7080

atgtatatat acatgctcaa acatgcttag atacatgaag taacatgctg ctacggttta 7140

gtcattattg agtgcctata tattctaata aatcagtatg ttttaaatta ttttgatttt 7200

actggtactt agatagatgt atatatacat gctcaaacat gcttagatac atgaagtaat 7260

atgctactac ggtttaattg ttcttgagta cctatatatt ctaataaatc agtatgtttt 7320

aaattatttc gattttactg gtacttagat agatgtatat atacatgctt agatacatga 7380

agtaacatgc tactacggtt taattgttct tgaataccta tatattctaa taaatcagta 7440

tgttttaaat tatttcgatt ttactggtac ttagatagat gtatatatac atgctcgaac 7500

atgcttagat acatgaagta acatgctaca tatatattat aataaatcag tatgtcttaa 7560

attattttga ttttactggt acttagatag atgtatatac atgctcaaac atgcttagat 7620

acatgaagta acatgctact acggtttaat cattattgag tacctatata ttctaataaa 7680

tcagtatgtt ttcaattgtt ttgattttac tggtacttag atatatgtat atatacatgc 7740

tcgaacatgc ttagatacgt gaagtaacat gctactatgg ttaattgttc ttgagtacct 7800

atatattcta ataaatcagt atgttttaaa ttatttcgat tttactggta cttagataga 7860

tgtatatata catgctcgaa catgcttaga tacatgaagt aacatgctac tacggtttaa 7920

tcgttcttga gtacctatat attctaataa atcagtatgt cttaaattat cttgatttta 7980

ctggtactta gatagatgta tatacatgct tagatacatg aagtaacatg ctactatgat 8040

ttaatcgttc ttgagtacct atatattcta ataaatcagt atgtttttaa ttattttgat 8100

tttactggta cttagataga tgtatatata catgctcgaa catgcttaga tacatgaagt 8160

aacatgctac tacggtttaa tcattcttga gtacctatat attctaataa atcagtatgt 8220

ttttaattat tttgatatta ctggtactta acatgtttag atacatcata tagcatgcac 8280

atgctgctac tgtttaatca ttcgtgaata cctatatatt ctaatatatc agtatgtctt 8340

ctaattatta tgattttgat gtacttgtat ggtggcatat gctgcagcta tgtgtagatt 8400

ttgaataccc agtgtgatga gcatgcatgg cgccttcata gttcatatgc tgtttatttc 8460

ctttgagact gttctttttt gttgatagtc accctgttgt ttggtgattc ttatgcagat 8520

ccagatcttc gtaaaccatg gctctggtgg ctaggccagt gctgtctgct agagtggctg 8580

cttctaggcc aagggtggcc gctagaaagg ctgtgagggt gagcgctgcc agtcacggcg 8640

ctagctctag gccagccacc gctaggaagt ccagcggcct gagcggcacc gtgcgcatcc 8700

caggcgacaa gagcatcagt caccgcagct tcatgttcgg cggcctggcc agcggcgaga 8760

ctaggatcac cggcctgctc gagggcgagg acgtgatcaa caccggcaag gccatgcagg 8820

ctatgggcgc tcgcatccgc aaggagggcg acacctggat catcgacggc gtgggtaatg 8880

gcggcctgct ggccccagag gccccactgg acttcggcaa cgccgccacc ggctgcaggc 8940

tgactatggg cctggtgggc gtgtacgact tcgacagcac ctttatcggc gacgccagcc 9000

tgaccaagag gccgatgggc agggtgctga acccgctgcg cgagatgggc gtgcaggtca 9060

agagcgagga cggcgacagg ctgccggtga ccctgagggg cccaaagacc ccgaccccga 9120

tcacctaccg cgtgccaatg gccagcgccc aggtgaagtc agccgtgctg ctggccggcc 9180

tgaacacccc aggcatcacc accgtgatcg agccgatcat gacccgcgat cacaccgaga 9240

agatgctgca gggcttcggc gccaacctga ccgtcgagac tgacgctgac ggcgtgagga 9300

ccatcaggct cgagggcagg ggcaagctga ccggccaggt gatcgacgtg ccgggcgacc 9360

caagcagcac cgccttccca ctggtggccg ccctgctggt gccaggctct gacgtgacca 9420

tcctgaacgt gctgatgaac ccgagccgca ccggcctgat cctgaccctg caggagatgg 9480

gcgccgatat cgaggtgatc aacccgaggc tggctggcgg cgaggacgtc gccgacctga 9540

gggtgaggtc cagcaccctg aagggcgtga ccgtgccaga ggacagggcc ccgagtatga 9600

ttgacgagta cccgatcctg gccgtggccg ctgccttcgc cgagggcgcc accgtgatga 9660

acggcctcga ggagctgcgc gtgaaggaga gcgacaggct gagcgctgtg gccaacggcc 9720

tgaagctgaa cggcgtggac tgcgacgagg gcgagactag cctggtggtg aggggcaggc 9780

cagacggcaa gggcctgggc aacgcttccg gcgctgccgt ggccacccac ctggatcaca 9840

ggatcgccat gtcgttcctc gtgatgggcc tcgtgagcga gaacccggtg accgtggacg 9900

acgccaccat gatcgccacc agcttccccg agttcatgga cctgatggcc ggcctgggcg 9960

ctaagatcga gctgagcgac accaaggccg cctgatcatc tagagctcgc caaggttcaa 10020

ttaagctgct gctgtacctg ggtatctgcg tcgtctggtg ccctctggtg tacctctata 10080

tggatgtcgt cgtctaataa acatctgtgg tttgtgtgtc atgaatcgtg gttgtggctt 10140

cgttggttta atggacctgt tgtgtcctct gtgttgtacc caaaactctt ctgcagcagt 10200

atggcttgaa tccttatgaa gtttgatatt tgaacttaaa agtctgctca ttatgttttt 10260

ttctggttat atctcctaat taactgcctg ggatcaaatt tgattcgctg gtgtttattg 10320

gacccctccc aggttcttgc tttctaccgt ttcttgctga atgttaactt gattctgtca 10380

ggctcagttt cccactatgg cttacagctt aacgtgtttg gtttgttgaa tgttaacttg 10440

gttttgtcaa gctcagtttt ttactctggc ttacagcata acatgtttga cttttggttt 10500

tgctgctttg ttattgggtt ctgggtagtt cttgatgaat ccaaaagatc atgtgcacag 10560

ccatattatc tatttaagcg atccaggtta ttactatgaa aggatgcctt ctagctaagg 10620

agtagttagg ttttttcttc aaggttaaat tttctcgatg ctctagtgtt cctgtgacca 10680

taatcataat aattcctttg aaagctctat ggtccctgga agcagggcat acaatgcaag 10740

acagcaactt gatcacatca actgaagtat acagggttct cttaactctt ggtgacttcg 10800

gtttaatgga ccggttgtac tcgtgttcta tccgtaaccg ttgtgatgtc ttgtgtgttt 10860

ggttgcggga tagctgggac cacgacgttt ccgtctaatt ctgatggata gctatagacg 10920

gcactgagat ggttatatta taacctctga tcctgaactc tacgagatcg tctcatccgt 10980

cattgccacc aaatacacca ttaaattact aattagctaa cggaccctat ttgtactcat 11040

tccatgtctc ataaactttg ggcaccatcc atccaacaca tccaatctaa acacaccaaa 11100

cgatggggaa tggaaagagc agtattcgat tcaacaatgg caaacaaata tcactgaatt 11160

agaccaagaa taaacctaat tagacaacga cctcccaacc atcattcgtc aggctgtaaa 11220

gaagataaag ctgccatggg gcatggatca agcagaacac cagagatgaa tccaaacaca 11280

cagaaaatca cgcgcgctgt ctacaatgac aacaagcccc acatttcatt gcagtacact 11340

gggctacaaa ggcacgtaca acaaagagct agggaaacat tgcggagggc acgagagagc 11400

agctaacttg acaatatagc agactgagct tgcactgtta gcaggcgagg aagggaatca 11460

tggggacgga gaatggggtc catgcccgcg aaggagaagg cggacgccgc cacggtggca 11520

ccggcgcacg cgcacacagg gaacccgcac aggcagccat ggatgctgcc tcgccattgc 11580

gccggtcgtc tctgccacgc tcctctctct ctcccgctgc atcgccgtgg atggggcaag 11640

cagagagcag ggactgcgac gatctgggcg gaggactcgc cttggagagc gcggacgcag 11700

acgggattct agggagagag cgaagacggg gcgcgcgcgg cgctcgcgcg gcgtggtggc 11760

ggcgagatta gcgggggtgg ggggagggcg gagccgtggt gagggtgtgg acgccctcct 11820

taccctctta agtagtagta gagatataat ccgttccaaa atatccatcc gttcaattta 11880

tatttcgttt gatcttttta ccctaaattt gattgactca tcttattaaa aaagttcata 11940

actattatta atctttattg agatatcatt tagcatataa tatactttaa gtgtggtttt 12000

agattttttt taaaaaaaaa aattcgcaaa aattaaatga aacgacccaa tcaaacttga 12060

aaagtaaaac taattataaa tttgaacgga aggagtaaga ggatgtttga atgtactaga 12120

gctaatagtt ggttgcttta aaatttgcta gtagaattag ctagctaata aatatctaga 12180

taactattag ctaatttgct aaaacagcta atagttgaac tattagctag attgtttgga 12240

tgtattcggc taattttaat ggctaactat tagctatagt acaatattca aacacctcct 12300

aattaaaatg gacaaatatc tcttcttttg gtcccttgcg ttagattttt catatctcct 12360

tatttagtat aaaagaatca tcaaaaagtg gacaacccct agtggaacac cattttagta 12420

gtggttgcat gaaacctttc gcgcaccagt ttctatgtgt cactctaaaa atgggacagc 12480

atgtacgtag tgcctatata tatacaagtc atctatcgtt gcctcctcag ttcatcacta 12540

atcacactta ttgtgccctc gacgagtatc tatagctagc tcattaatcg attcgggggt 12600

gtgttgtcga aggcggca atg gcg agc tac tcg tcg cgg cgt cca tgc aat 12651
Met Ala Ser Tyr Ser Ser Arg Arg Pro Cys Asn
1               5                   10

acc tgt agc acg aag gcg atg gcc ggg agc gtg gtc ggc gag ccc gtc 12699
Thr Cys Ser Thr Lys Ala Met Ala Gly Ser Val Val Gly Glu Pro Val
            15                  20                  25

gtg ctg ggg cag agg gtg acg gtg ctg acg gtg gac ggc ggc ggc gtc 12747
Val Leu Gly Gln Arg Val Thr Val Leu Thr Val Asp Gly Gly Gly Val
        30                  35                  40

cgg ggt ctc atc ccg gga acc atc ctc gcc ttc ctg gag gcc agg ctg 12795
Arg Gly Leu Ile Pro Gly Thr Ile Leu Ala Phe Leu Glu Ala Arg Leu
    45                  50                  55

cag gag ctg gac ggg ccg gag gcg agg ctg gcg gac tac ttc gac tac 12843
Gln Glu Leu Asp Gly Pro Glu Ala Arg Leu Ala Asp Tyr Phe Asp Tyr
60                  65                  70                  75

atc gcc gga acc agc acc ggc ggt ctc atc acc gcc atg ctc acc gcg 12891
Ile Ala Gly Thr Ser Thr Gly Gly Leu Ile Thr Ala Met Leu Thr Ala
                80                  85                  90

ccc ggc aag gac aag cgg cct ctc tac gct gcc aag gac atc aac cac 12939
Pro Gly Lys Asp Lys Arg Pro Leu Tyr Ala Ala Lys Asp Ile Asn His
            95                  100                 105

ttt tac atg cag aac tgc ccg cgc atc ttt cct cag aa gtgagtccga 12987
Phe Tyr Met Gln Asn Cys Pro Arg Ile Phe Pro Gln Lys
        110                 115                 120

tgctgccgcc attgttcttg catccatcca gcatcgtacg tacgtcctct atacatctgc 13047

ggatcatcat gtgcgcatgt ttgtggcatg catgcatgca tgtgagcag g agc agg 13103
Ser Arg

ctt gcg gcc gcc atg tcc gcg ctg agg aag cca aag tac aac ggc aag 13151
Leu Ala Ala Ala Met Ser Ala Leu Arg Lys Pro Lys Tyr Asn Gly Lys
        125                 130                 135

tgc atg cgc agc ctg att agg agc atc ctc ggc gag acg agg 13193
Cys Met Arg Ser Leu Ile Arg Ser Ile Leu Gly Glu Thr Arg
    140                 145                 150

gtaagcgaga cgctgaccaa cgtcatcatc cctgccttcg acatcaggct gctgcagcct 13253

atcatcttct ctacctacga cgtacgtacg tcgtcacgaa tgattcatct gtacgtcgtc 13313

gcatgcgaat ggctgcctac gtacgccgtg cgctaacata ctcagctctt tcctatctgc 13373

tgcgccaatt tgcag gcc aag agc acg cct ctg aag aac gct ctg ctc tcg 13424
Ala Lys Ser Thr Pro Leu Lys Asn Ala Leu Leu Ser
        155                 160

gac gtg tgc att ggc acg tcc gcc gcg ccg acc tac ctc ccg gcg cac 13472
Asp Val Cys Ile Gly Thr Ser Ala Ala Pro Thr Tyr Leu Pro Ala His
165                 170                 175                 180

tac ttc cag act gaa gac gcc aac ggc aag gag cgc gaa tac aac ctc 13520
Tyr Phe Gln Thr Glu Asp Ala Asn Gly Lys Glu Arg Glu Tyr Asn Leu
                185                 190                 195

atc gac ggc ggt gtg gcg gcc aac aac ccg gtaactgact agctaactgg 13570
Ile Asp Gly Gly Val Ala Ala Asn Asn Pro
            200                 205

aaaacggacg cacagactcc atgtccatgg cggcccacaa ggtcgatgct aattgttgct 13630

tatgtatgtc gcccgattgc acatgcgtag acg atg gtt gcg atg acg cag atc 13684
Thr Met Val Ala Met Thr Gln Ile
            210

acc aaa aag atg ctt gcc agc aag gac aag gcc gag gag ctg tac cca 13732
Thr Lys Lys Met Leu Ala Ser Lys Asp Lys Ala Glu Glu Leu Tyr Pro
215                 220                 225                 230

gtg aag ccg tcg aac tgc cgc agg ttc ctg gtg ctg tcc atc ggg acg 13780
Val Lys Pro Ser Asn Cys Arg Arg Phe Leu Val Leu Ser Ile Gly Thr
                235                 240                 245

ggg tcg acg tcc gag cag ggc ctc tac acg gcg cgg cag tgc tcc cgg 13828
Gly Ser Thr Ser Glu Gln Gly Leu Tyr Thr Ala Arg Gln Cys Ser Arg
            250                 255                 260

tgg ggt atc tgc cgg tgg ctc cgc aac aac ggc atg gcc ccc atc atc 13876
Trp Gly Ile Cys Arg Trp Leu Arg Asn Asn Gly Met Ala Pro Ile Ile
        265                 270                 275

gac atc ttc atg gcg gcc agc tcg gac ctg gtg gac atc cac gtc gcc 13924
Asp Ile Phe Met Ala Ala Ser Ser Asp Leu Val Asp Ile His Val Ala
    280                 285                 290

gcg atg ttc cag tcg ctc cac agc gac ggc gac tac ctg cgc atc cag     13972
Ala Met Phe Gln Ser Leu His Ser Asp Gly Asp Tyr Leu Arg Ile Gln
295                 300                 305                 310

gac aac tcg ctc cgt ggc gcc gcg gcc acc gtg gac gcg gcg acg ccg     14020
Asp Asn Ser Leu Arg Gly Ala Ala Ala Thr Val Asp Ala Ala Thr Pro
                315                 320                 325

gag aac atg cgg acg ctc gtc ggg atc ggg gag cgg atg ctg gca cag     14068
Glu Asn Met Arg Thr Leu Val Gly Ile Gly Glu Arg Met Leu Ala Gln
            330                 335                 340

agg gtg tcc agg gtc aac gtg gag aca ggg agg tac gaa ccg gtg act     14116
Arg Val Ser Arg Val Asn Val Glu Thr Gly Arg Tyr Glu Pro Val Thr
        345                 350                 355

ggc gaa gga agc aat gcc gat gcc ctc ggt ggg ctc gct agg cag ctc     14164
Gly Glu Gly Ser Asn Ala Asp Ala Leu Gly Gly Leu Ala Arg Gln Leu
    360                 365                 370

tcc gag gag agg aga aca agg ctc gcg cgc cgc gtc tct gcc atc aac     14212
Ser Glu Glu Arg Arg Thr Arg Leu Ala Arg Arg Val Ser Ala Ile Asn
375                 380                 385                 390

cca aga ggc tct aga tgt gcg tcg tac gat atc taa gacaagtggc     14258
Pro Arg Gly Ser Arg Cys Ala Ser Tyr Asp Ile
                395                 400

tttactgtca gtcacatgct tgtaaataag tagactttat tttaataaaa cataaaaata     14318

tatatatgtt cttgaatata aaattgataa ccaaattaaa attcgaacca tcacttatac     14378

ataattttac tttatttttt ataaaacgtg aacgggaagg actaccgtga atgactatag     14438

aaccaatcat actagtataa aatatatgat gacactacgg gagagacaaa ctttgtctgg     14498

cgctaaatat tttgccgagt gtgaattcac gggcactagg caaagatctt ctttgccgag     14558

tgttacgctg ggcaaagtaa gacactaggt aaatcagtca tttgccgagt gtccgccact     14618

aggcaaagca aaacactggc aaatcaaaag tttacctagt gccagacact aggcaaaaaa     14678

aaaacgctcg gcaaatcgga agtttcccta gtgccagaca ctagacaaag aaaaacactt     14738

gataaactag cgtcgtcagc taacaccatc caccaaccgt taacgttgcc gagtatctga     14798

cttcgacact cggcaaagaa ggtctctttg cctagtgtcg gtctggaaca ctaggcaaag     14858

aggcacttta cctagtgtcg tattttgaca ctcagtaaaa taattttttt tctttctgct     14918

tccaaacttt ttatgatgtg ttcctatagc acctagaact acatgtcaag ttttggtaaa     14978

atttttgaag tttttgctat atttacttaa tttattttat ttaattgaat ttcttttgat     15038

aattcaaatt tgaactcggc aaggtaagaa gcgagggtag cctggaaaca cactttgcct     15098

agtgttacac tcggtacagg agcctcccct gcctagtgct gcactcgaca aaagattcgc     15158

ctttgcctag cgctgcactc ggcacaggag tcgcctttgc ctagtgctgc actaggcaaa     15218

gcctccgtta ccgtgccttc catcgtcgga ccgcctgcag gcccgggggc gcgccctaat     15278

tagctaacgg ccaggatcgc cgcgtgagcc tttagcaact agctagatta attaacgcaa     15338

tctgttatta agttgtctaa gcgtcaattt gtttacacca caatatatcc tgccaccagc    15398

cagccaacag ctccccgacc ggcagctcgg cacaaaatca ccactcgata caggcagccc    15458

atcag                                                                15463

<210>  31
<211>  15757
<212>  DNA
<213>  Artificial Sequence

<220>
<223>  Expression cassette of construct 22467

<220>
<221>  promoter
<222>  (214)..(2339)

<220>
<221>  gene
<222>  (2341)..(3018)

<220>
<221>  terminator
<222>  (3024)..(4036)

<220>
<221>  enhancer
<222>  (4044)..(4237)
<223>  eFMV

<220>
<221>  enhancer
<222>  (4244)..(4536)
<223>  e35S

<220>
<221>  promoter
<222>  (4544)..(8537)
<223>  Ubi

<220>
<221>  gene
<222>  (8538)..(9995)
<223>  EPSPS

<220>
<221>  terminator
<222>  (10009)..(11008)
<223>  Ubi

<220>
<221>  gene
<222>  (11028)..(15538)
<223>  GRMZM2G062320

<220>
<221>  promoter
<222>  (11028)..(12596)
<223>  pPGM

<220>
<221>  exon
<222>  (12597)..(12834)
<223>  PGM exon1

<220>
<221>  Intron
<222>  (12835)..(12974)
<223>  PGM intron1

<220>
<221>  exon
<222>  (12975)..(13198)
<223>  PGM exon2

<220>
<221>  Intron
<222>  (13199)..(13295)
<223>  PGM intron2

<220>
<221>  exon
<222>  (13296)..(13382)
<223>  PGM exon3

<220>
<221>  Intron
<222>  (13383)..(13718)
<223>  PGM intron3

<220>
<221>  exon
<222>  (13719)..(13787)
<223>  PGM exon4

<220>
<221>  Intron
<222>  (13788)..(14102)
<223>  PGM intron4

<220>
<221>  exon
<222>  (14103)..(14204)
<223>  PGM exon5

<220>
<221>  Intron
<222>  (14205)..(14304)
<223>  PGM intron5

<220>
<221>  exon
<222>  (14305)..(14424)
<223>  PGM exon6

<220>
<221>  Intron
<222>  (14425)..(14492)
<223>  PGM intron6

<220>
<221>  exon

<222>   (14493)..(14540)
<223>   PGM exon7

<220>
<221>   terminator
<222>   (14541)..(15538)
<223>   tPGM

<400>   31
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga cccgaaagta gcaaacaaca ggttcatgtg     240

cactataaaa agacaaaatt ctcgagtttc atcttttatt ccacataagc cttatatttt     300

ccattttcat atgattttta gtttaagttt gtgtcttaac tttttcgtta atacgtaatt     360

ctatgcatta tggatgcgtg aagtattttt gtttaaaaaa atgaaatgtc aaaatacgtt     420

ttgtgatcta tttccatgtt ttcacctaac aggtggtttt tactatatat tctgccataa     480

ctctagcctt agatgtaaat cgaaaaaaaa tgagagatga gctggagata gccttagatg     540

aagcgtctga aatataaaag aaagagtaat gttgaacgca gtaggtgtag cagctgtagt     600

tccatctcta ggaaagggaa ctgcaatccg ggctccgggc ctcgcgcaat ctggcctgtc     660

gtgtagatgc agccctgtcc atgacggccc aagcaacgcc cgcggctctc gatccaccac     720

ggaacccact ccgacacaca ctgacacaca catgctggat gtggatgtgc tgtccaatta     780

ttagtagcaa ttcggtaggc acaggcacgt actggccggt gttttagctg taagtaccga     840

accaatcacg gttaagaacc gattaatccg tgcccagccg ccgagtgcgt tcgtacgtgc     900

atcggatgca ctgcatgaat tgagagcatc atcatatcat acgcaggagt agtacgacgc     960

cgctgctgtc ttgtccggct aatgctttgc tcacagatta gtccatcgcc cacggtcggt    1020

gtggtgtgga tcgctgatgc cactgctttt tgtttggttt ttattcccct gataatcctc    1080

cgcgtccctg aatgtatcta tttattttca ttccgaaatc cctttcacga aaaagaaaac    1140

gaataaaaag agagttacga atacgcttcc ggcggcccac atcaccttcc agcgaacatc    1200

gcgccgcgct gacgtgtcgc ccatcgcggc cgtccatatc gccatccgac gaccgtggaa    1260

gctggcagcg gccgctccgt tccgtcgaag gggcaggtca gtcaggtcac ccacacggcc    1320

acacccgcgc gggggatacg cggtggaaaa cccggcgacc acatcaaaac acgaggcgtc    1380

tcccgcagga ctggtcactc ggcacgcagg cagaggcagc acagcagcag ccagctccat    1440

ccatcctctt tcccctcctc gcttcgcttc ctcggcggat tcctcctccc tcggccgtcc    1500

ccgtcccctt cttcgccgcg ccagctcgcc cgagttggta cgcccatcct ctgctgtact    1560

ccccccgttc ctgctgtgcg aaaccgagca cccaaaccct agcctaagct attcgcatcg    1620

caaactctat tgagcgacgg atcgcgaaac gcgcgccgcc gcattcggac gagacctcgt 1680

ggcccgtacc ttcctactct accgtcgtcg ccggagacca gctcaccggt tagggtttcg 1740

ggattagggg cttggggctt ggattcccgg gggactccat acccggtggc cttagaaggg 1800

gaagggggcc cagcggtggt ggtggttcga ctgtcagcga gcgagacggc ggggcaccgc 1860

cgatcgggcg tcgctggaca tttgattgag ctgggcaggc gaaggcgtgg agcttgctct 1920

tcgattggga ttagaggagg gcggaggtgg tatggtgggc ggctcggctg gctcaatgga 1980

gccgctggcc ggtggtgggg caggcgagat cacgctctct catagagcgt aatgggttgc 2040

gtaacacact cctgtttgta tttcgatccg atctaataat taaagtttat ttacgtggtg 2100

cacatgtaga attttcccat gcccactgct aacttggatt ggatttttgc caaaaaaacg 2160

ggtgtgcacc caatcactta tagtgaaacc gttgtgtgtt tgatgtgctt ctaaccaagt 2220

acaaatccag tgaaaacaca cctaccattc aacaatccac attttggttg caattatcag 2280

cattctagaa aggtgcatgt gcccattgat acacttgcta ttggtgcagg caaacccacc 2340

atggcctcct ccgagaacgt catcaccgag ttcatgcgct tcaaggtgcg catggagggc 2400

accgtgaacg gccacgagtt cgagatcgag ggcgagggcg agggccgccc ctacgagggc 2460

cacaacaccg tgaagctgaa ggtgaccaag ggcggccccc tgcccttcgc ctgggacatc 2520

ctgtcccccc agttccagta cggctccaag gtgtacgtga agcaccccgc cgacatcccc 2580

gactacaaga agctgtcctt ccccgagggc ttcaagtggg agcgcgtgat gaacttcgag 2640

gacggcggcg tggcgaccgt gacccaggac tcctccctgc aggacggctg cttcatctac 2700

aaggtgaagt tcatcggcgt gaacttcccc tccgacggcc ccgtgatgca gaagaagacc 2760

atgggctggg aggcctccac cgagcgcctg tacccccgcg acggcgtgct gaagggcgag 2820

acccacaagg ccctgaagct gaaggacggc ggccactacc tggtggagtt caagtccatc 2880

tacatggcca agaagcccgt gcagctgccc ggctactact acgtggacgc caagctggac 2940

atcacctccc acaacgagga ctacaccatc gtggagcagt acgagcgcac cgagggccgc 3000

caccacctgt tcctgtagga gctcgcatca tgatcatgca tcatggactc ggcctactac 3060

tgtggatttg tatgccatta tagacttggt gctgtgaaag actgcttgat gatttgcggg 3120

tttgttgctg tgtaaaaaaa ggtcccttgg ctcccagaag accatgaagg ttcggatcta 3180

tcatgtaatt ccttgttatc tgccaattat gtatggacta tggacatgtg ttgcgctgtt 3240

caacttacta ctacaaataa gtaatcgata tgttcccttc ccatgtctcg gtgacaattg 3300

tctggagaag cttaggggtc gtttgtttgg gattatgtct ggagaaactt attttaaact 3360

aagtgtgagt tcaagttaag ttagattata taatctaggc agattataat tccaagcgaa 3420

caggtcctta gtgtttttgg aaaatcctag gtgttctttt ggctacattg ttgtgtgtgc 3480

agatcccttg ttggtctgta agcgtgggga agtaagaatc gtccgtttct actgaagacc 3540

tgctcgagtt aggcaccgag gatgccggta accaaacaga gcaatagtgt ctctgtgggc 3600

acagtggagt gtgaatctgt gtgatgcaaa tccgtcattt gtttagcaaa atttccagcg 3660

ttgcatgatg cagtttcttt aacacggact taagggaagg gaaaaaaatg ttgagccagg 3720

agatccttca atgtgttaga ctgacgtgat agccaactaa accacgacgc aatgttgtcg 3780

ttaatgacaa aaaaactatt tgttcctaaa tccttggcga cattgcatgg ctgtctcatg 3840

agataatggt ctcatctctt atttatctct tatttatagc cggaagtggt agtgacccct 3900

gcttgattgc tcgtatgcca tctcaagttc tcaaccgtgt cgagcagcca ttttcccatc 3960

tcaagcgcat catcgtttcg tttgacctca tctgctatcc tgctcctagt gcaaatcaca 4020

tgcgacagaa agtgtgcgga cccagctgct tgtggggacc agacaaaaaa ggaatggtgc 4080

agaattgtta ggcgcaccta ccaaaagcaa ctttgccttt attgcaaaga taaagcagat 4140

tcctctagta caagtgggga acaaaataac gtggaaaaga gctgtcctga cagcccactc 4200

actattgcgt ttgacgaacg cagtgacgac cacaaaactc gagacttttc aacaaagggt 4260

attatccgga aacctcctcg gattccattg cccagctatc tgtcacttta ttgtgaagat 4320

agtggaaaag gaaggtggct cctacaaatg ccatcattgc gataaaggaa aggctatcgt 4380

tgaagatgcc tctgccgaca gtggtcccaa agatggaccc ccacccacga ggagcatcgt 4440

ggaaaaagaa gacgttccaa ccacgtcttc aaagcaagtg gattgatgtg atatctccac 4500

tgacgtaagg gttgacgaac aatcccacta tccttcaagc tttggaaaac caaaacaacg 4560

aataagcaaa ctgcaggaaa agtatgcagt ggaaaccaac ccagattcgg acgataggaa 4620

agtatcaagt gaatgatttg ccaggaaaag gagaggggta aaaaggggcg aagatttaga 4680

agatctaaag cacaagaacc agagattaga ttgaacaata gggaacttgg agcatccttt 4740

ttttcttcag ggaaaaactg aaaatccaaa ccatgttgag caaaaccgag tgggattgga 4800

aaccaaaaaa cccgagataa agaaactcga gaaaaagcat gaaatcgaaa ccaacttcag 4860

taaaacaaaa ggaggacaga aaagaaagtc ggaagctata aagaatacat taacattcag 4920

tgaaacagca tgctgtcttc ttcttttttt atgcacaaca gagcatacat atataccttc 4980

ccaggctgag gacttggcgg aggagagccg cggataggtt ggcggtgcag acggtctgga 5040

cgggcccgaa gacggagacg aacagcgggc ccttcctgcc caggcaccac gcttggaacg 5100

ccaagcacgc gccaaccgcg gccccgccga ggacgacgat ccccgcgaca agcgtggcgt 5160

cgatcctggg cgaccccaag ccgaggaacc tcccttccag gacgagccgt aggacggcgg 5220

tgagagaggc acccgtcgcg gaggtggcgc agcacaaggt gagcagcgcg gggaaggcgg 5280

cgagcgtggc ggcctgcagg acggtgacga gcgcgaagac ggtgacgccg gcgacgaggc 5340

agcagcagcc gaggatccag tcgtaggagg aagccggacc aaaccgggca atgcaacctg 5400

cagatgcact agacggaggt aacgaggagg aggagaaaac agagcaagag caggcggaga 5460

gaagatagag caaaacacga gtgaggcaca gcgtaagcac tcggtagaag tctccagagg 5520

cgaggtgcgc acaggagaac agatgagtaa agtcagccaa ggatccacga tccaacggct 5580

acgaattttt ggagtgacgt ggataggctc aaaggcgcca tttccatccg gctttatagt 5640

attttaaaaa aattcatttt cctccctcta gtgtgtgcgg aggcgtgagc ccgtttaacg 5700

gcgttgagaa gtctaacgga caccaaccac aaccaggaac cagcgccggc cgcgccgccg 5760

agtgaagcag actgcatacg gcacggcgcg gcatctctct ggctgcctct cgagagttcc 5820

gcccccacct tcccgcggta gcgtggtggt ttcgctttcc gctgtcggca tccggaagtt 5880

gcgtggcaga gtggacggag acgaggccgg gtcctccagc tcctctcaaa cgtcacggca 5940

ccggcatccg gcagccagcg cggtccttcc caaccactcg ttcccaaccc atcccccttc 6000

ctcgcccgcc gtcataaata gccagcccca tccccagctt ctttccccaa cctcatcttc 6060

tctccttttg ctctgaacgc acacaccgcc cggtctccga tctccgatcc ccgatcccct 6120

cgtcgatcct aggtacggcg accatcctac cccccccccc cccccctctc tctctgcctt 6180

ctctagatcg gcgatccgat ccatgcttac ttggttaggg cctgctaact atgttcatgt 6240

ttgcgttaga tccgtgcatg gacgcgatct gtacacacca gacgcgttct gattgctagc 6300

taactcgcca gtacctggga atcctgggat ggctgtagcc ggccccgcac gcagacggga 6360

ccgatttcat gattctctat ttttttcttt gtttcgttgc ctagggtttc gttcgatcga 6420

tccgcgttat tctttatttc catatattct ggtacgatgt tgatacggtt cgaccgtgct 6480

gcttacgttc tgtgcgcttg tttgccgggt catttttacc ttgccttttt tgtatggttt 6540

ggttgtggcg atgtggtctg gtcgggctgt cgttctagat cggagtagag tgctgtttca 6600

aactgtctag cggatctatt agatttggat ctgcatgtgt gacatatatc ttcgtagtta 6660

agatgatgca tctgtatgtg tgacatgcgg atctattaga tttggatctg tatgtgtgac 6720

atatatcttc gtagttgaga tgatgcatct gtatgtgtga catatatctt cgtagttaag 6780

attatgcatg gaaatatcaa tcctttagat aaggacgggt atacttgttg ctgtgggttt 6840

tactggtact tcgatagatg catatacatg atctaacatg cttagataca tgaagtaaca 6900

tgctgctacg gtttaataat tcttgagttg atttttactg gtacttagat agatgtatat 6960

acatgcttag atacatgaag taacatgctc ctacagttcc tttaatcatt attgagtacc 7020

tatatattct aataaatcag tatgttttaa attattttga ttttactggt acttagatag 7080

atgtatatat acatgctcaa acatgcttag atacatgaag taacatgctg ctacggttta 7140

gtcattattg agtgcctata tattctaata aatcagtatg ttttaaatta ttttgatttt 7200

actggtactt agatagatgt atatatacat gctcaaacat gcttagatac atgaagtaat 7260

atgctactac ggtttaattg ttcttgagta cctatatatt ctaataaatc agtatgtttt 7320

aaattatttc gattttactg gtacttagat agatgtatat atacatgctt agatacatga 7380

agtaacatgc tactacggtt taattgttct tgaataccta tatattctaa taaatcagta 7440

tgttttaaat tatttcgatt ttactggtac ttagatagat gtatatatac atgctcgaac 7500

atgcttagat acatgaagta acatgctaca tatatattat aataaatcag tatgtcttaa 7560

attattttga ttttactggt acttagatag atgtatatac atgctcaaac atgcttagat 7620

acatgaagta acatgctact acggtttaat cattattgag tacctatata ttctaataaa 7680

tcagtatgtt ttcaattgtt ttgattttac tggtacttag atatatgtat atatacatgc 7740

tcgaacatgc ttagatacgt gaagtaacat gctactatgg ttaattgttc ttgagtacct 7800

atatattcta ataaatcagt atgttttaaa ttatttcgat tttactggta cttagataga 7860

tgtatatata catgctcgaa catgcttaga tacatgaagt aacatgctac tacggtttaa 7920

tcgttcttga gtacctatat attctaataa atcagtatgt cttaaattat cttgatttta 7980

ctggtactta gatagatgta tatacatgct tagatacatg aagtaacatg ctactatgat 8040

ttaatcgttc ttgagtacct atatattcta ataaatcagt atgtttttaa ttattttgat 8100

tttactggta cttagataga tgtatatata catgctcgaa catgcttaga tacatgaagt 8160

aacatgctac tacggtttaa tcattcttga gtacctatat attctaataa atcagtatgt 8220

ttttaattat tttgatatta ctggtactta acatgtttag atacatcata tagcatgcac 8280

atgctgctac tgtttaatca ttcgtgaata cctatatatt ctaatatatc agtatgtctt 8340

ctaattatta tgattttgat gtacttgtat ggtggcatat gctgcagcta tgtgtagatt 8400

ttgaataccc agtgtgatga gcatgcatgg cgccttcata gttcatatgc tgtttatttc 8460

ctttgagact gttctttttt gttgatagtc accctgttgt ttggtgattc ttatgcagat 8520

ccagatcttc gtaaaccatg gctctggtgg ctaggccagt gctgtctgct agagtggctg 8580

cttctaggcc aagggtggcc gctagaaagg ctgtgagggt gagcgctgcc agtcacggcg 8640

ctagctctag gccagccacc gctaggaagt ccagcggcct gagcggcacc gtgcgcatcc 8700

caggcgacaa gagcatcagt caccgcagct tcatgttcgg cggcctggcc agcggcgaga 8760

ctaggatcac cggcctgctc gagggcgagg acgtgatcaa caccggcaag gccatgcagg 8820

ctatgggcgc tcgcatccgc aaggagggcg acacctggat catcgacggc gtgggtaatg 8880

gcggcctgct ggccccagag gccccactgg acttcggcaa cgccgccacc ggctgcaggc 8940

tgactatggg cctggtgggc gtgtacgact tcgacagcac ctttatcggc gacgccagcc 9000

tgaccaagag gccgatgggc agggtgctga acccgctgcg cgagatgggc gtgcaggtca 9060

agagcgagga cggcgacagg ctgccggtga ccctgagggg cccaaagacc ccgaccccga 9120

tcacctaccg cgtgccaatg gccagcgccc aggtgaagtc agccgtgctg ctggccggcc 9180

tgaacacccc aggcatcacc accgtgatcg agccgatcat gacccgcgat cacaccgaga 9240

agatgctgca gggcttcggc gccaacctga ccgtcgagac tgacgctgac ggcgtgagga 9300

ccatcaggct cgagggcagg ggcaagctga ccggccaggt gatcgacgtg ccgggcgacc 9360

caagcagcac cgccttccca ctggtggccg ccctgctggt gccaggctct gacgtgacca 9420

tcctgaacgt gctgatgaac ccgagccgca ccggcctgat cctgaccctg caggagatgg 9480

gcgccgatat cgaggtgatc aacccgaggc tggctggcgg cgaggacgtc gccgacctga 9540

gggtgaggtc cagcaccctg aagggcgtga ccgtgccaga ggacagggcc ccgagtatga 9600

ttgacgagta cccgatcctg gccgtggccg ctgccttcgc cgagggcgcc accgtgatga 9660

acggcctcga ggagctgcgc gtgaaggaga gcgacaggct gagcgctgtg gccaacggcc 9720

tgaagctgaa cggcgtggac tgcgacgagg gcgagactag cctggtggtg aggggcaggc 9780

cagacggcaa gggcctgggc aacgcttccg gcgctgccgt ggccacccac ctggatcaca 9840

ggatcgccat gtcgttcctc gtgatgggcc tcgtgagcga gaacccggtg accgtggacg 9900

acgccaccat gatcgccacc agcttccccg agttcatgga cctgatggcc ggcctgggcg 9960

ctaagatcga gctgagcgac accaaggccg cctgatcatc tagagctcgc caaggttcaa 10020

ttaagctgct gctgtacctg ggtatctgcg tcgtctggtg ccctctggtg tacctctata 10080

tggatgtcgt cgtctaataa acatctgtgg tttgtgtgtc atgaatcgtg gttgtggctt 10140

cgttggttta atggacctgt tgtgtcctct gtgttgtacc caaaactctt ctgcagcagt 10200

atggcttgaa tccttatgaa gtttgatatt tgaacttaaa agtctgctca ttatgttttt 10260

ttctggttat atctcctaat taactgcctg ggatcaaatt tgattcgctg gtgtttattg 10320

gacccctccc aggttcttgc tttctaccgt ttcttgctga atgttaactt gattctgtca 10380

ggctcagttt cccactatgg cttacagctt aacgtgtttg gtttgttgaa tgttaacttg 10440

gttttgtcaa gctcagtttt ttactctggc ttacagcata acatgtttga cttttggttt 10500

tgctgctttg ttattgggtt ctgggtagtt cttgatgaat ccaaaagatc atgtgcacag 10560

ccatattatc tatttaagcg atccaggtta ttactatgaa aggatgcctt ctagctaagg 10620

agtagttagg ttttttcttc aaggttaaat tttctcgatg ctctagtgtt cctgtgacca 10680

taatcataat aattcctttg aaagctctat ggtccctgga agcagggcat acaatgcaag 10740

acagcaactt gatcacatca actgaagtat acagggttct cttaactctt ggtgacttcg 10800

gtttaatgga ccggttgtac tcgtgttcta tccgtaaccg ttgtgatgtc ttgtgtgttt 10860

ggttgcggga tagctgggac cacgacgttt ccgtctaatt ctgatggata gctatagacg 10920

gcactgagat ggttatatta taacctctga tcctgaactc tacgagatcg tctcatccgt 10980

cattgccacc aaatacacca ttaaattact aattagctaa cggacccctt gcctcggtta 11040

caattgagtt tgtcataaga aagaatgaga aagaaaagaa gcaatccaag cgcaagagct 11100

caaaggaaca caagtcactc tctcactagt cactattgat tggaattgaa ctagggactt 11160

gggagaggat tttatctctt tggtgtgtct tgtattgaat gctatagctc ttgtaatgtg 11220

tagaatgttg gaaacttgga tgccattgaa tgtggggtgg ttggggtatt tatagcccca 11280

accaccacaa tgtggtcgtt ggaagtctgc tgtcgcatgg cgcaccggat agtccgatgc 11340

gccaccggac actgtccggt gcgccagcca cgtcagccag ccgttggggt tcgaccgttg 11400

gagctctgac ttgtggggcc tctgggctgt ccggtggtgc accggacagg tcctgtagac 11460

tgtccggtgc accaactgcg cgtgctctga cctctgcgcg cgcaggcgcg cattaaatgc 11520

gttgcagtcg accgttgcgc ttgaagtagt cgttgctccg ctggcacacc ggacagtgtc 11580

cggtgaatta tagcggagcg gcctccattt tcccgaaggt agcgagttca gcgtcgagtt 11640

ccctggtgca ccggacactg tccggtggca caccggacag tccggtgcgc cagaccaggg 11700

cacacttcgg ttgtctttgg ctctctttgt ttgaaccctt tcttggtctt tttattggtc 11760

tattgtgaac ctttggcacc tgtaaaactt ataatctaga gtaaactagt tagtccaatt 11820

atttgtgttg ggcaattcaa ccaccaaaat caattaggaa aaggtgtaag cctatttccc 11880

tttcaggagg cgtacgtgag agggagaggt gaaaaggaac aacgcgtata ccagataagg 11940

tcccacagcc taagtaggta gccttctaat atctctacta actattaagg agagagtgta 12000

gactgccccc gctccctacc caacgccccc cgctacctgt tcaccgcgcg ccagcgaaac 12060

ctccgcacgc ccactgccca tctgttcccc gtgcgccagc gaaacatccg cacgcccgcg 12120

gcccgcctgt tccccgcgca tcccgctgca cgacttctgc taccgcaacg gccacccacg 12180

cacgcccgcc tgttcaccgc gcatcccgct gacctcccct tcacgctcgc acacgctccg 12240

ttcccccacc ccaccgcaat ccccgacgct ataagagcgg taaccaactc catctccctg 12300

gtgccacgca ttgttgagtt cttaaggtgc gtttcgttga ggacttgttc atttttgttg 12360

gtcatgtatt ccatttttact gctctaccat tttgtggaat aaaggaggaa atgttttcac 12420

tagaagagtt catcaatctt atgttggttt cttggatcag ttttgctcta tggctaaatg 12480

gtcgaattga gcctatttca ttataaagtt agcgagcgaa taattgttca gcctcttcct 12540

agaactcatt accagtagaa tcagttacta actgcttttc tttttcttgg attaga atg 12599
Met
1

gct ggg gct atc tct cac cat gcg cta gca ttt tca caa tcc cac tgg 12647
Ala Gly Ala Ile Ser His His Ala Leu Ala Phe Ser Gln Ser His Trp
            5                   10                  15

tgc agt gcg aag aac tct aga ttc gga aag agg acg ggc aat gct cgc 12695
Cys Ser Ala Lys Asn Ser Arg Phe Gly Lys Arg Thr Gly Asn Ala Arg
        20                  25                  30

ctg gtt tat cta aaa gga aga tgt ggt tca ggc agc aga aaa ctg ggt 12743
Leu Val Tyr Leu Lys Gly Arg Cys Gly Ser Gly Ser Arg Lys Leu Gly

    35                  40                  45

ttg atg tgg gcc tcg agc tcg cag tct tct gtc atg gag ccg acg cac 12791
Leu Met Trp Ala Ser Ser Ser Gln Ser Ser Val Met Glu Pro Thr His
50                  55                  60                  65

cta cca tct gat ggc aac agc agc cac acc cca aaa aaa tca a 12834
Leu Pro Ser Asp Gly Asn Ser Ser His Thr Pro Lys Lys Ser
                70                  75

gtaattttaa cgacctccta tggtggttat ttgtttttaa tttgagaaaa ctatccattt 12894

gacacattta actttgggct tctcagaatt tgggggcata taataagatc tgctaatctg 12954

ttatctctat gtcgttgtag gt gaa agc gct ctt ata ttg att tgg cat ggt 13006
Ser Glu Ser Ala Leu Ile Leu Ile Trp His Gly
80                  85                  90

gaa tcc ctg tgg aac gag aaa aat cta ttt cct ggc tgc atc gat gta 13054
Glu Ser Leu Trp Asn Glu Lys Asn Leu Phe Pro Gly Cys Ile Asp Val
                95                  100                 105

ccc ctg aca ccg aag ggt gtt gag gag gcc att gag gca ggt aaa agg 13102
Pro Leu Thr Pro Lys Gly Val Glu Glu Ala Ile Glu Ala Gly Lys Arg
            110                 115                 120

ata tgc aat atc cca atc gat gtg ata tat act tca tca ctg att tgt 13150
Ile Cys Asn Ile Pro Ile Asp Val Ile Tyr Thr Ser Ser Leu Ile Cys
        125                 130                 135

gct cag atg acc gca atg ctt gcc atg atg cag cat cga cgc aag aag 13198
Ala Gln Met Thr Ala Met Leu Ala Met Met Gln His Arg Arg Lys Lys
    140                 145                 150

gtttgtgtct ttcctttgaa attccagtaa tttcttctag catttgtatg aacttgccgg 13258

agaaatcatg ctttgctggt gatatatgta tttatag atc cta gtt atc acg cat 13313
Ile Leu Val Ile Thr His
155                 160

aat gag agt gaa caa gct cac agg tgg agt cag ata tac agt gag gag 13361
Asn Glu Ser Glu Gln Ala His Arg Trp Ser Gln Ile Tyr Ser Glu Glu
                165                 170                 175

aca atg aaa cag tcc att cct gtcatcacag cttggcaatt gaatgaacgg 13412
Thr Met Lys Gln Ser Ile Pro
            180

atgtaatact ttctccatac tctttgattt gctaattact ccctctgtct caaaatagta 13472

ttaattttag ctcttgattt ttatgtctat attcaaatag atgatgataa atctagattc 13532

tagacacaaa tataaaacat atacatcaag tattatatga atctattaat ttactaagac 13592

caattttaat ttgggacaga gggagtatac gattataata gttgtttgac tgtgcttctc 13652

tttaaatatc ccttgacatt tctaggtatg gtgagctaca aggccttaac aagcaagaaa 13712

ctgtag atc gat ttg gca aag aac aag ttc atg agt ggc gcc gca gtt 13760
Ile Asp Leu Ala Lys Asn Lys Phe Met Ser Gly Ala Ala Val
    185                 190                 195

atg ata ttc ctc cgc caa atg gag aaa gtctagagaa gtgtgctgag 13807

Met Ile Phe Leu Arg Gln Met Glu Lys
        200                 205

agagctgttg cttatttcaa agatcaggca catctagcaa ggccacttta cactaattga    13867

aagatacact ttttacttgg gttattggtc ttgctgcagt attggtatgc atgctaaagg    13927

ttattcttga atcgatgaat tcctctacta tgggatgcag aaatgcatgt gcttagtttt    13987

ctttctattg tgctagctca tatcaaattt ataacctgaa ttttttattt atgttcgact    14047

ctaaaaaaca gttttttcta gctcgatttg acctatagta atttttccgt aatag att    14105
                                                            Ile

att cca caa ctt gtg gct gga aaa cat gtg atg gtt gct gca cat ggg    14153
Ile Pro Gln Leu Val Ala Gly Lys His Val Met Val Ala Ala His Gly
        210                 215                 220

aat tca ctt cgt tca att ata atg cat ctg gac aaa tta act tct cag    14201
Asn Ser Leu Arg Ser Ile Ile Met His Leu Asp Lys Leu Thr Ser Gln
    225                 230                 235

aag gtaattcact gtcgtttttg tctttccatc aaaaaggact cggctaaaca    14254
Lys
240

gaacatgtag cattatgtta agtttgggag tgagcctttc gtcccttcag gta ata    14310
                                                      Val Ile

agc ctt gag ctg tct act ggc att ccc atg ctt tac ata ttc aaa gag    14358
Ser Leu Glu Leu Ser Thr Gly Ile Pro Met Leu Tyr Ile Phe Lys Glu
        245                 250                 255

gga aag ttt att cga cgt ggg act cca gta gga cct tcg gag gcc agt    14406
Gly Lys Phe Ile Arg Arg Gly Thr Pro Val Gly Pro Ser Glu Ala Ser
    260                 265                 270

gtt tat gct tat acc agg gtaagattct ttcccccaca tgttctacca    14454
Val Tyr Ala Tyr Thr Arg
275                 280

taggacgata ctccagttta caaaccttat ctgtacag acc aaa cga ttt gct gag    14510
                                         Thr Lys Arg Phe Ala Glu
                                                         285

cac att aca ttt cag aac aaa ttg gcc tag aagataaggg gtgtttggtt    14560
His Ile Thr Phe Gln Asn Lys Leu Ala
            290                 295

tgagaaatca ctctattcaa aatgagatgg tgtatcatgg gtccatttct caaatttggt    14620

gggatgaccc tattcctcat attagtacta actaggtgag tgtccgtgcg ttgcaacggg    14680

aacatataat aacatgataa cttatataca aaatgtgtct tatattgtta taagaaaatg    14740

tttcataatc catttgtaat cctggccata cataaatttt gttattttaa tttagctgtt    14800

tcactactac attgcaacca tcagtatcat gcagacttcg atatatgtca cgatttgcat    14860

agtcttatca ttgaagagca cgtgtcacac ctaccggtag aagttccctc gtacattgtc    14920

agggggacct catggtcgtc gctaaagccg gcgactctct ggttgttctg ggcaccgcat     14980

ccgacgacaa caccgtcacg ctgtccagct catcatccac ctgaagatca acctgccacg     15040

taagtcggta gtgaccggcc atgaattctt gtgcgctggg gcaacgggcg ttgcattccg     15100

ttgtgtgagt gttttagaac aagatgtatg taaaggagta gcacatccgg tggtgcaatg     15160

gctaggtgta ctacctcgct gatgagctcg cggtgcactt cgtttagcag cccagccaag     15220

agtcatcggt acttgccatg tcgtgcgtgt tcgacgacta ctatatcaag gactgtggcg     15280

tcctcttggc gccagaggtg aggagtaccg acagcaatga cctctcgcca tcgtcggggt     15340

agcttttgtg ctggcccgcc tttaattctc ttcgtaaaca catacacttg ccttttttgt     15400

ttaagcaagt gagtatagtg gtgcgtacct gatgactgac aatgtacgag gaaacttcta     15460

ctggcaggtg tggcacgtgc tcttcaatga tgagaccatg taaatcgtga catatatcga     15520

agtctgcatg atactgatcg gaccgcctgc aggcccgggg gcgcgcccta attagctaac     15580

ggccaggatc gccgcgtgag cctttagcaa ctagctagat taattaacgc aatctgttat     15640

taagttgtct aagcgtcaat ttgtttacac cacaatatat cctgccacca gccagccaac     15700

agctccccga ccggcagctc ggcacaaaat caccactcga tacaggcagc ccatcag     15757

<210>  32
<211>  15355
<212>  DNA
<213>  Artificial Sequence

<220>
<223>  Expression cassett of construct 22503


<220>
<221>  enhancer
<222>  (214)..(407)
<223>  eFMV

<220>
<221>  enhancer
<222>  (414)..(706)
<223>  e35S

<220>
<221>  promoter
<222>  (714)..(4707)
<223>  Ubi

<220>
<221>  gene
<222>  (4708)..(6165)
<223>  EPSPS

<220>
<221>  terminator
<222>  (6179)..(7178)
<223>  Ubi

<220>
<221>  promoter
<222>  (7198)..(9285)
<223>  pPLA2m

<220>
<221>  stem_loop
<222>  (9298)..(10212)
<223>  targeting exon2 of PLA2

<220>
<221>  terminator
<222>  (10223)..(11218)
<223>  tPLA2

<220>
<221>  promoter
<222>  (11226)..(13349)

<220>
<221>  gene
<222>  (13352)..(14131)

<220>
<221>  misc_feature
<222>  (14039)..(14128)

<220>
<221>  terminator
<222>  (14138)..(15136)

<400>  32
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga cccagctgct tgtggggacc agacaaaaaa     240

ggaatggtgc agaattgtta ggcgcaccta ccaaaagcaa ctttgccttt attgcaaaga     300

taaagcagat tcctctagta caagtgggga acaaaataac gtggaaaaga gctgtcctga     360

cagcccactc actattgcgt ttgacgaacg cagtgacgac cacaaaactc gagacttttc     420

aacaaagggt attatccgga aacctcctcg gattccattg cccagctatc tgtcacttta     480

ttgtgaagat agtggaaaag gaaggtggct cctacaaatg ccatcattgc gataaaggaa     540

aggctatcgt tgaagatgcc tctgccgaca gtggtcccaa agatggaccc ccacccacga     600

ggagcatcgt ggaaaaagaa gacgttccaa ccacgtcttc aaagcaagtg gattgatgtg     660

atatctccac tgacgtaagg gttgacgaac aatcccacta tccttcaagc tttggaaaac     720

caaaacaacg aataagcaaa ctgcaggaaa agtatgcagt ggaaaccaac ccagattcgg     780

acgataggaa agtatcaagt gaatgatttg ccaggaaaag gagaggggta aaaaggggcg     840

aagatttaga agatctaaag cacaagaacc agagattaga ttgaacaata gggaacttgg     900

agcatccttt ttttcttcag ggaaaaactg aaaatccaaa ccatgttgag caaaaccgag     960

tgggattgga aaccaaaaaa cccgagataa agaaactcga gaaaaagcat gaaatcgaaa 1020

ccaacttcag taaaacaaaa ggaggacaga aaagaaagtc ggaagctata aagaatacat 1080

taacattcag tgaaacagca tgctgtcttc ttcttttttt atgcacaaca gagcatacat 1140

atataccttc ccaggctgag gacttggcgg aggagagccg cggataggtt ggcggtgcag 1200

acggtctgga cgggcccgaa gacggagacg aacagcgggc ccttcctgcc caggcaccac 1260

gcttggaacg ccaagcacgc gccaaccgcg gccccgccga ggacgacgat ccccgcgaca 1320

agcgtggcgt cgatcctggg cgaccccaag ccgaggaacc tcccttccag gacgagccgt 1380

aggacggcgg tgagagaggc acccgtcgcg gaggtggcgc agcacaaggt gagcagcgcg 1440

gggaaggcgg cgagcgtggc ggcctgcagg acggtgacga gcgcgaagac ggtgacgccg 1500

gcgacgaggc agcagcagcc gaggatccag tcgtaggagg aagccggacc aaaccgggca 1560

atgcaacctg cagatgcact agacggaggt aacgaggagg aggagaaaac agagcaagag 1620

caggcggaga gaagatagag caaaacacga gtgaggcaca gcgtaagcac tcggtagaag 1680

tctccagagg cgaggtgcgc acaggagaac agatgagtaa agtcagccaa ggatccacga 1740

tccaacggct acgaattttt ggagtgacgt ggataggctc aaaggcgcca tttccatccg 1800

gctttatagt attttaaaaa aattcatttt cctccctcta gtgtgtgcgg aggcgtgagc 1860

ccgtttaacg gcgttgagaa gtctaacgga caccaaccac aaccaggaac cagcgccggc 1920

cgcgccgccg agtgaagcag actgcatacg gcacggcgcg gcatctctct ggctgcctct 1980

cgagagttcc gcccccacct tcccgcggta gcgtggtggt ttcgctttcc gctgtcggca 2040

tccggaagtt gcgtggcaga gtggacggag acgaggccgg gtcctccagc tcctctcaaa 2100

cgtcacggca ccggcatccg gcagccagcg cggtccttcc caaccactcg ttcccaaccc 2160

atcccccttc ctcgcccgcc gtcataaata gccagcccca tccccagctt ctttccccaa 2220

cctcatcttc tctccttttg ctctgaacgc acacaccgcc cggtctccga tctccgatcc 2280

ccgatcccct cgtcgatcct aggtacggcg accatcctac cccccccccc cccccctctc 2340

tctctgcctt ctctagatcg gcgatccgat ccatgcttac ttggttaggg cctgctaact 2400

atgttcatgt ttgcgttaga tccgtgcatg gacgcgatct gtacacacca gacgcgttct 2460

gattgctagc taactcgcca gtacctggga atcctgggat ggctgtagcc ggccccgcac 2520

gcagacggga ccgatttcat gattctctat ttttttcttt gtttcgttgc ctagggtttc 2580

gttcgatcga tccgcgttat tctttatttc catatattct ggtacgatgt tgatacggtt 2640

cgaccgtgct gcttacgttc tgtgcgcttg tttgccgggt catttttacc ttgccttttt 2700

tgtatggttt ggttgtggcg atgtggtctg gtcgggctgt cgttctagat cggagtagag 2760

tgctgtttca aactgtctag cggatctatt agatttggat ctgcatgtgt gacatatatc 2820

ttcgtagtta agatgatgca tctgtatgtg tgacatgcgg atctattaga tttggatctg 2880

tatgtgtgac atatatcttc gtagttgaga tgatgcatct gtatgtgtga catatatctt 2940

cgtagttaag attatgcatg gaaatatcaa tcctttagat aaggacgggt atacttgttg 3000

ctgtgggttt tactggtact tcgatagatg catatacatg atctaacatg cttagataca 3060

tgaagtaaca tgctgctacg gtttaataat tcttgagttg atttttactg gtacttagat 3120

agatgtatat acatgcttag atacatgaag taacatgctc ctacagttcc tttaatcatt 3180

attgagtacc tatatattct aataaatcag tatgttttaa attattttga ttttactggt 3240

acttagatag atgtatatat acatgctcaa acatgcttag atacatgaag taacatgctg 3300

ctacggttta gtcattattg agtgcctata tattctaata aatcagtatg ttttaaatta 3360

ttttgatttt actggtactt agatagatgt atatatacat gctcaaacat gcttagatac 3420

atgaagtaat atgctactac ggtttaattg ttcttgagta cctatatatt ctaataaatc 3480

agtatgtttt aaattatttc gattttactg gtacttagat agatgtatat atacatgctt 3540

agatacatga agtaacatgc tactacggtt taattgttct tgaataccta tatattctaa 3600

taaatcagta tgttttaaat tatttcgatt ttactggtac ttagatagat gtatatatac 3660

atgctcgaac atgcttagat acatgaagta acatgctaca tatatattat aataaatcag 3720

tatgtcttaa attattttga ttttactggt acttagatag atgtatatac atgctcaaac 3780

atgcttagat acatgaagta acatgctact acggtttaat cattattgag tacctatata 3840

ttctaataaa tcagtatgtt ttcaattgtt ttgattttac tggtacttag atatatgtat 3900

atatacatgc tcgaacatgc ttagatacgt gaagtaacat gctactatgg ttaattgttc 3960

ttgagtacct atatattcta ataaatcagt atgttttaaa ttatttcgat tttactggta 4020

cttagataga tgtatatata catgctcgaa catgcttaga tacatgaagt aacatgctac 4080

tacggtttaa tcgttcttga gtacctatat attctaataa atcagtatgt cttaaattat 4140

cttgatttta ctggtactta gatagatgta tatacatgct tagatacatg aagtaacatg 4200

ctactatgat ttaatcgttc ttgagtacct atatattcta ataaatcagt atgtttttaa 4260

ttattttgat tttactggta cttagataga tgtatatata catgctcgaa catgcttaga 4320

tacatgaagt aacatgctac tacggtttaa tcattcttga gtacctatat attctaataa 4380

atcagtatgt ttttaattat tttgatatta ctggtactta acatgtttag atacatcata 4440

tagcatgcac atgctgctac tgtttaatca ttcgtgaata cctatatatt ctaatatatc 4500

agtatgtctt ctaattatta tgattttgat gtacttgtat ggtggcatat gctgcagcta 4560

tgtgtagatt ttgaataccc agtgtgatga gcatgcatgg cgccttcata gttcatatgc 4620

tgtttatttc ctttgagact gttctttttt gttgatagtc accctgttgt ttggtgattc 4680

ttatgcagat ccagatcttc gtaaaccatg gctctggtgg ctaggccagt gctgtctgct 4740

agagtggctg cttctaggcc aagggtggcc gctagaaagg ctgtgagggt gagcgctgcc 4800

agtcacggcg ctagctctag gccagccacc gctaggaagt ccagcggcct gagcggcacc 4860

gtgcgcatcc caggcgacaa gagcatcagt caccgcagct tcatgttcgg cggcctggcc 4920

agcggcgaga ctaggatcac cggcctgctc gagggcgagg acgtgatcaa caccggcaag 4980

gccatgcagg ctatgggcgc tcgcatccgc aaggagggcg acacctggat catcgacggc 5040

gtgggtaatg gcggcctgct ggccccagag gccccactgg acttcggcaa cgccgccacc 5100

ggctgcaggc tgactatggg cctggtgggc gtgtacgact tcgacagcac ctttatcggc 5160

gacgccagcc tgaccaagag gccgatgggc agggtgctga acccgctgcg cgagatgggc 5220

gtgcaggtca agagcgagga cggcgacagg ctgccggtga ccctgagggg cccaaagacc 5280

ccgaccccga tcacctaccg cgtgccaatg gccagcgccc aggtgaagtc agccgtgctg 5340

ctggccggcc tgaacacccc aggcatcacc accgtgatcg agccgatcat gacccgcgat 5400

cacaccgaga agatgctgca gggcttcggc gccaacctga ccgtcgagac tgacgctgac 5460

ggcgtgagga ccatcaggct cgagggcagg ggcaagctga ccggccaggt gatcgacgtg 5520

ccgggcgacc caagcagcac cgccttccca ctggtggccg ccctgctggt gccaggctct 5580

gacgtgacca tcctgaacgt gctgatgaac ccgagccgca ccggcctgat cctgaccctg 5640

caggagatgg gcgccgatat cgaggtgatc aacccgaggc tggctggcgg cgaggacgtc 5700

gccgacctga gggtgaggtc cagcaccctg aagggcgtga ccgtgccaga ggacagggcc 5760

ccgagtatga ttgacgagta cccgatcctg gccgtggccg ctgccttcgc cgagggcgcc 5820

accgtgatga acggcctcga ggagctgcgc gtgaaggaga gcgacaggct gagcgctgtg 5880

gccaacggcc tgaagctgaa cggcgtggac tgcgacgagg gcgagactag cctggtggtg 5940

aggggcaggc cagacggcaa gggcctgggc aacgcttccg gcgctgccgt ggccacccac 6000

ctggatcaca ggatcgccat gtcgttcctc gtgatgggcc tcgtgagcga gaacccggtg 6060

accgtggacg acgccaccat gatcgccacc agcttccccg agttcatgga cctgatggcc 6120

ggcctgggcg ctaagatcga gctgagcgac accaaggccg cctgatcatc tagagctcgc 6180

caaggttcaa ttaagctgct gctgtacctg ggtatctgcg tcgtctggtg ccctctggtg 6240

tacctctata tggatgtcgt cgtctaataa acatctgtgg tttgtgtgtc atgaatcgtg 6300

gttgtggctt cgttggttta atggacctgt tgtgtcctct gtgttgtacc caaaactctt 6360

ctgcagcagt atggcttgaa tccttatgaa gtttgatatt tgaacttaaa agtctgctca 6420

ttatgttttt ttctggttat atctcctaat taactgcctg ggatcaaatt tgattcgctg 6480

gtgtttattg gacccctccc aggttcttgc tttctaccgt ttcttgctga atgttaactt 6540

gattctgtca ggctcagttt cccactatgg cttacagctt aacgtgtttg gtttgttgaa 6600

tgttaacttg gttttgtcaa gctcagtttt ttactctggc ttacagcata acatgtttga 6660

cttttggttt tgctgctttg ttattgggtt ctgggtagtt cttgatgaat ccaaaagatc 6720

atgtgcacag ccatattatc tatttaagcg atccaggtta ttactatgaa aggatgcctt 6780

ctagctaagg agtagttagg ttttttcttc aaggttaaat tttctcgatg ctctagtgtt 6840

cctgtgacca taatcataat aattcctttg aaagctctat ggtccctgga agcagggcat 6900

acaatgcaag acagcaactt gatcacatca actgaagtat acagggttct cttaactctt 6960

ggtgacttcg gtttaatgga ccggttgtac tcgtgttcta tccgtaaccg ttgtgatgtc 7020

ttgtgtgttt ggttgcggga tagctgggac cacgacgttt ccgtctaatt ctgatggata 7080

gctatagacg gcactgagat ggttatatta taacctctga tcctgaactc tacgagatcg 7140

tctcatccgt cattgccacc aaatacacca ttaaattact aattagctaa cggaccctat 7200

ttgtactcat tccatgtctc ataaactttg ggcaccatcc atccaacaca tccaatctaa 7260

acacaccaaa cgatggggaa tggaaagagc agtattcgat tcaacaatgg caaacaaata 7320

tcactgaatt agaccaagaa taaacctaat tagacaacga cctcccaacc atcattcgtc 7380

aggctgtaaa gaagataaag ctgccttggg gcatggatca agcagaacac cagagatgaa 7440

tccaaacaca cagaaaatca cgcgcgctgt ctacaatgac aacaagcccc acatttcatt 7500

gcagtacact gggctacaaa ggcacgtaca acaaagagct agggaaacat tgcggagggc 7560

acgagagagc agctaacttg acaatatagc agactgagct tgcactgtta gcaggcgagg 7620

aagggaatca tggggacgga gaatggggtc catgcccgcg aaggagaagg cggacgccgc 7680

cacggtggca ccggcgcacg cgcacacagg gaacccgcac aggcagccaa ggatgctgcc 7740

tcgccattgc gccggtcgtc tctgccacgc tcctctctct ctcccgctgc atcgccgtgg 7800

atggggcaag cagagagcag ggactgcgac gatctgggcg gaggactcgc cttggagagc 7860

gcggacgcag acgggattct agggagagag cgaagacggg gcgcgcgcgg cgctcgcgcg 7920

gcgtggtggc ggcgagatta gcgggggtgg ggggagggcg gagccgtggt gagggtgtgg 7980

acgccctcct taccctctta agtagtagta gagatataat ccgttccaaa atatccatcc 8040

gttcaattta tatttcgttt gatcttttta ccctaaattt gattgactca tcttattaaa 8100

aaagttcata actattatta atctttattg agatatcatt tagcatataa tatactttaa 8160

gtgtggtttt agattttttt taaaaaaaaa aattcgcaaa aattaaatga aacgacccaa 8220

tcaaacttga aaagtaaaac taattataaa tttgaacgga aggagtaaga ggatgtttga 8280

atgtactaga gctaatagtt ggttgcttta aaatttgcta gtagaattag ctagctaata 8340

aatatctaga taactattag ctaatttgct aaaacagcta atagttgaac tattagctag 8400

attgtttgga tgtattcggc taattttaat ggctaactat tagctatagt acaatattca 8460

aacacctcct aattaaaatg gacaaatatc tcttcttttg gtcccttgcg ttagattttt 8520

catatctcct tatttagtat aaaagaatca tcaaaaagtg gacaacccct agtggaacac 8580

cattttagta gtggttgcat gaaacctttc gcgcaccagt ttctatgtgt cactctaaaa 8640

atgggacagc atgtacgtag tgcctatata tatacaagtc atctatcgtt gcctcctcag 8700

ttcatcacta atcacactta ttgtgccctc gacgagtatc tatagctagc tcattaatcg 8760

attcgggggt gtgttgtcga aggcggcatt ggcgagctac tcgtcgcggc gtccaagcaa 8820

tacctgtagc acgaaggcga tcgccgggag cgtggtcggc gagcccgtcg tgctggggca 8880

gagggtgacg gtgctgacgg tggacggcgg cggcgtccgg ggtctcatcc cgggaaccat 8940

cctcgccttc ctggaggcca ggctgcagga gctggacgca ccggaggcga ggctggcgga 9000

ctacttcgac tacatcgccg gaaccagcac cggcggtctc atcaccgcct tgctgaccgc 9060

gcccggcaag gacaagcggc ctctctaggc tgccaaggac atcaaccact tttacatcca 9120

taactgcccg cgcatctttc ctcagaagtg agtccgatgc tgccgccatt gttcttgcat 9180

ccatccagca tcgtacgtac gtcctctata catctgcgga tcatcatgtg cgcatgtttg 9240

tggcatgcat gcatgcatgt gagcaggagc aggcttgcga aaaccccatg gagatctgaa 9300

caggcttgcg gccgccgtgt ccgcgctgag gaagccaaag tacaacggca agtgcatgcg 9360

cagcctgatt aggagcatcc tcggcgagac gaggggtcga ctcgggagca tgccctttgt 9420

gcttatgcct ccgttctgcc ttctgacgaa tttggtactg gaagcagatg agttttggtt 9480

cactatcatt ctgaatttac acctgcgctt gctgtcagac taggcaacca agtgactttt 9540

gtgactttga tcatgttcag tgtgtttcca agtcctaatc aatcaaaaag aaaaacagtt 9600

tgttaacgat tgtttgccat gtctatataa taaagttgct tttatagtag cttagaattc 9660

aatcggccaa ctttatctcg tacgctgaca gtaaaggtac atttaaaagg tgacaatgga 9720

tagtctaata cttgaactga caatagagac acattacatg tcagttgatt aagtttgtaa 9780

cagaaaaata aacaatacta cataattgca aagtttcttt gatgtctttc tttcaagaaa 9840

cacaaatata tcaatgctac agtattgctg atgaatttat ccatgttgag atgtttttct 9900

ggtttctgat ctgatcagtc tcaattggtg tgctgtttca ttttcatttg ctgatgatcg 9960

tccgagtagt taattcttac taatatttag ataatttggc atacaagcga atcacgtaga 10020

acatgatact tttgaatgaa tttatcaaag ttttatcact tggtgagttg tttcatggtt 10080

ttcctactga tgtctcttct tcagatttct cgagccctcg tctcgccgag gatgctccta 10140

atcaggctgc gcatgcactt gccgttgtac tttggcttcc tcagcgcgga catggcggcc 10200

gcaagcctgc tcggatccat gggacaagtg gctttactgt cagtcacatg cttgtaaata 10260

agtagacttt attttaataa aacataaaaa tatatatatg ttcttgaata taaaattgat 10320

aaccaaatta aaattcgaac catcacttat acataatttt actttatttt ttataaaacg 10380

tgaacgggaa ggactaccgt gaatgactat agaaccaatc atactagtat aaaatatatg 10440

atgacactac gggagagaca aactttgtct ggcgctaaat attttgccga gtgtgaattc 10500

acgggcacta ggcaaagatc ttctttgccg agtgttacgc tgggcaaagt aagacactag 10560

gtaaatcagt catttgccga gtgtccgcca ctaggcaaag caaaacactg gcaaatcaaa 10620

agtttaccta gtgccagaca ctaggcaaaa aaaaaacgct cggcaaatcg gaagtttccc 10680

tagtgccaga cactagacaa agaaaaacac ttgataaact agcgtcgtca gctaacacca 10740

tccaccaacc gttaacgttg ccgagtatct gacttcgaca ctcggcaaag aaggtctctt 10800

tgcctagtgt cggtctggaa cactaggcaa agaggcactt tacctagtgt cgtattttga 10860

cactcagtaa aataattttt tttctttctg cttccaaact ttttatgatg tgttcctata 10920

gcacctagaa ctacatgtca agttttggta aaatttttga agtttttgct atatttactt 10980

aatttatttt atttaattga atttcttttg ataattcaaa tttgaactcg gcaaggtaag 11040

aagcgagggt agcctggaaa cacactttgc ctagtgttac actcggtaca ggagcctccc 11100

ctgcctagtg ctgcactcga caaaagattc gcctttgcct agcgctgcac tcggcacagg 11160

agtcgccttt gcctagtgct gcactaggca aagcctccgt taccgtgcct tccatcgtcg 11220

gaccctggag aggcatgaac ccatcatgta tactcctgga cttggaagag gaaatgtcaa 11280

ccaaagtgaa aggttttctt ctagttgctg ctaagagata aattgaatac tagatctctc 11340

ctaagacatc ggggcattcg tctggactac tacacatgcg aaaattgcat cttacagtgg 11400

gaagaaacta tatctcacct cttcttgcgg tgtaactttg cccgaagatg ttggctcact 11460

gttggaatca ctccgccccg aacgttggat ctagcgcttg cagtgctaca tattagagca 11520

agactgacat tgtcgtggag aatggaaggt attataacca tgcatgtcat ggtacatatg 11580

gaaatgtcaa aataactgga tattcgaaaa cataccgcca acggtggcgg tctgcaagga 11640

aatgttcaag attgaaatga actacatatg caaccaggtt aagcttgaga caggagataa 11700

aagtagaaac tggatacaac actttgtaac atagcgacac tctctccttc ctttctttta 11760

ccttagaact atacatagaa tctacattca ataaaaatac agtaggtacg ccgagagatt 11820

taaaatgagt aagctaacat accaactaag gccctgtttg tttcggatta taatctctcc 11880

agattatata atccagcgta aataattcag cagataaaca aacacctaaa ttatatgttc 11940

agattatata atctatagcg gagattatga taatctcgta atctcctaag agtagcttat 12000

ttgagatttt tttggcaaaa gacccactac ccaaggttat gtaaatagaa ttacaatata 12060

tgacatcctt ctttcttcac ctcaaataaa caaacaaggg tactgttgtc tttatgaata 12120

atctacattt atataatcta gactaacaaa caactacata tagattataa tatgtctaga 12180

ttataatcta gattatataa tttaaattat agtccatatt atataatcta taagctaaaa 12240

caaataggcc ctaattatta gctattagtt gttagctatt taaatctaag ctaaaaccaa 12300

ctaatagctt attagttgaa ttacaattag ctcaacggaa ttctctgttt ttctaaaaaa 12360

aaactgcccc tctcttacag caaattgtcc gctgcccgtc gtccagatac aatgaacgta 12420

cctagtagga actcttttac acgctcggtc gctcgccgcg gatcggagtc cccggaacac 12480

gacaccactg tggaacacga caaagtctgc tcagaggcgg ccacaccctg gcgtgcaccg 12540

agccggagcc cggataagca cggtaaggag agtacggcgg gacgtggcga cccgtgtgtc 12600

tgctgccacg cagccttcct ccacgtagcc gcgcggccgc gccacgtacc agggcccggc 12660

gctggtataa cgcgccacct ccgctttagt tctgcataca gccaacccaa cacacacccg 12720

agcatatcac agtgacagac actacacggt gagcgccaga atcgttgtcc tcctcgccac 12780

cctcctatcc gctgccgccg cagtcgcgtc gtcctgggag gacgacaacc tccaccacca 12840

cgggggccac aagtccgggc gtagcgtgcg gcggtgcgag gaccggccct ggcaccagcg 12900

cccccggtgc ctggagcagt gcagggagga ggagcgggag aagcggcaag agcggagcag 12960

gctagaggcc gacgaccgca gcggcaaggg ttcgtcggag gtagagcgcg agcgagagca 13020

ggaggaggag aagcagaagg accggcggcc gtacgtgttc gaccggcgca gcttccgtcg 13080

cgtggtccgg agcgagcagg ggtctctgag ggtgctccgg ccgttcgacg aggtgtccag 13140

gctcctccgc ggcatccggg actaccgcgt ggcggtccta gaggcgaacc cgcgctcgtt 13200

cgtggtgccc agccataccg tagcgcactg catctgctac gtggtggaag gtacgttcgt 13260

ccggtccctg agaccacata gatcgagggc gacgccggcc gtgcacgtta gctgatctgt 13320

tgttgcctcg tgactaggcg agggataaac catggcctcc tccgagaacg tcatcaccga 13380

gttcatgcgc ttcaaggtgc gcatggaggg caccgtgaac ggccacgagt tcgagatcga 13440

gggcgagggc gagggccgcc cctacgaggg ccacaacacc gtgaagctga aggtgaccaa 13500

gggcggcccc ctgcccttcg cctgggacat cctgtccccc cagttccagt acggctccaa 13560

ggtgtacgtg aagcaccccg ccgacatccc cgactacaag aagctgtcct tccccgaggg 13620

cttcaagtgg gagcgcgtga tgaacttcga ggacggcggc gtggcgaccg tgacccagga 13680

ctcctccctg caggacggct gcttcatcta caaggtgaag ttcatcggcg tgaacttccc 13740

ctccgacggc cccgtgatgc agaagaagac aatgggctgg gaggcctcca ccgagcgcct 13800

gtacccccgc gacggcgtgc tgaagggcga gacccacaag gccctgaagc tgaaggacgg 13860

cggccactac ctggtggagt tcaagtccat ctacatggcc aagaagcccg tgcagctgcc 13920

cggctactac tacgtggacg ccaagctgga catcacctcc cacaacgagg actacaccat 13980

cgtggagcag tacgagcgca ccgagggccg ccaccacctg ttcctgagat ctcgagctga 14040

tccaaaaaag aagagaaagg tagatccaaa aaagaagaga aaggtagatc caaaaaagaa 14100

gagaaaggta ggctccaccg gatctagata agagctcggc cgccgcgctc gccaaaacga 14160

gcaagaagca acaagagggt ggcgcgcgac cgacgtgcgt acgtagctga gcctgagtgg 14220

agacgttgga cgtgtatgta tatacctctc tgcgtgttaa ctatgtacgt aagcggcagg 14280

cagtgcaata agtgtggctc tgtagtatgt acgtgcggat acgatgctgt atgctactga 14340

ggcaagtccc ataaataaat aatgacacgt gcgtgttcta taatctcttc gcttcttcag 14400

tgtccccttg cggagtttgg catccattga tgccgttaca ctgagaacat aacggacaca 14460

gtagacgaac cacttgagtt cttgtatgaa attctgaccc ttttttttga aggatcagga 14520

gggggaaacc cctactggcc tggtcctttt acaatcaata aaaagaaaag aaaaagaaag 14580

aaaaaggaag agctatttct atatttgcca caaatttagc cagttcggga ttgaggtgtc 14640

gaattctccc cgtgctctgt gaatgattag tcgcaattct ctactaaatt cctgagtaca 14700

gcggagcacg gagggatctc tatttgaaaa caaccactcg tttcggcttt tccagatgct 14760

ccaagtcata agaatagtga tctccataga gaagggaacg ttgagctggt gtttgaggtt 14820

cacagatgcc tcttcaaggt tggaaattct gggtggggta atgccaattg agttccagca 14880

ggctttcgcg aagttgcatc tgaggaaaag atgataaagg gtctcctctc tttgccaaag 14940

gcagttttcg caagagtagg aatctagctc catgtttctc cttctcaaca tatttctggt 15000

gtttagcctg ttcttaagcc acagccaata gaagactcta tgcttaggct gacacttact 15060

tttccaaact agtttgtaga taggatgagt ttgcatctgg cccatgaaag atctgtaagc 15120

tttttgggac gagaatcgga ccgcctgcag gcccgggggc gcgccctaat tagctaacgg 15180

ccaggatcgc cgcgtgagcc tttagcaact agctagatta attaacgcaa tctgttatta 15240

agttgtctaa gcgtcaattt gtttacacca caatatatcc tgccaccagc cagccaacag 15300

ctccccgacc ggcagctcgg cacaaaatca ccactcgata caggcagccc atcag 15355

<210> 33
<211> 15441
<212> DNA
<213> Artificial Sequence

<220>
<223> Expression cassette of construct 22513

<220>
<221> enhancer
<222> (214)..(407)
<223> eFMV

<220>
<221> enhancer
<222> (414)..(706)
<223> e35S

<220>
<221> promoter
<222> (714)..(4707)

<223>  Ubi

<220>
<221>  gene
<222>  (4708)..(6165)
<223>  EPSPS

<220>
<221>  terminator
<222>  (6179)..(7178)
<223>  Ubi

<220>
<221>  promoter
<222>  (7198)..(9285)
<223>  pPLA2

<220>
<221>  stem_loop
<222>  (9292)..(10386)
<223>  targeting exon4 of PLA2

<220>
<221>  terminator
<222>  (10397)..(11392)
<223>  tPLA2

<220>
<221>  promoter
<222>  (11400)..(13526)

<220>
<221>  gene
<222>  (13527)..(14204)

<220>
<221>  terminator
<222>  (14210)..(15222)

<400>  33
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga cccagctgct tgtggggacc agacaaaaaa     240

ggaatggtgc agaattgtta ggcgcaccta ccaaaagcaa ctttgccttt attgcaaaga     300

taaagcagat tcctctagta caagtgggga acaaaataac gtggaaaaga gctgtcctga     360

cagcccactc actattgcgt ttgacgaacg cagtgacgac cacaaaactc gagacttttc     420

aacaaagggt attatccgga aacctcctcg gattccattg cccagctatc tgtcacttta     480

ttgtgaagat agtggaaaag gaaggtggct cctacaaatg ccatcattgc gataaaggaa     540

aggctatcgt tgaagatgcc tctgccgaca gtggtcccaa agatggaccc ccacccacga     600

ggagcatcgt ggaaaaagaa gacgttccaa ccacgtcttc aaagcaagtg gattgatgtg     660

atatctccac tgacgtaagg gttgacgaac aatcccacta tccttcaagc tttggaaaac     720

caaaacaacg aataagcaaa ctgcaggaaa agtatgcagt ggaaaccaac ccagattcgg 780

acgataggaa agtatcaagt gaatgatttg ccaggaaaag gagaggggta aaaaggggcg 840

aagatttaga agatctaaag cacaagaacc agagattaga ttgaacaata gggaacttgg 900

agcatccttt ttttcttcag ggaaaaactg aaaatccaaa ccatgttgag caaaaccgag 960

tgggattgga aaccaaaaaa cccgagataa agaaactcga gaaaaagcat gaaatcgaaa 1020

ccaacttcag taaaacaaaa ggaggacaga aaagaaagtc ggaagctata aagaatacat 1080

taacattcag tgaaacagca tgctgtcttc ttcttttttt atgcacaaca gagcatacat 1140

atataccttc ccaggctgag gacttggcgg aggagagccg cggataggtt ggcggtgcag 1200

acggtctgga cgggcccgaa gacggagacg aacagcgggc ccttcctgcc caggcaccac 1260

gcttggaacg ccaagcacgc gccaaccgcg gccccgccga ggacgacgat ccccgcgaca 1320

agcgtggcgt cgatcctggg cgaccccaag ccgaggaacc tcccttccag gacgagccgt 1380

aggacggcgg tgagagaggc acccgtcgcg gaggtggcgc agcacaaggt gagcagcgcg 1440

gggaaggcgg cgagcgtggc ggcctgcagg acggtgacga gcgcgaagac ggtgacgccg 1500

gcgacgaggc agcagcagcc gaggatccag tcgtaggagg aagccggacc aaaccgggca 1560

atgcaacctg cagatgcact agacggaggt aacgaggagg aggagaaaac agagcaagag 1620

caggcggaga gaagatagag caaaacacga gtgaggcaca gcgtaagcac tcggtagaag 1680

tctccagagg cgaggtgcgc acaggagaac agatgagtaa agtcagccaa ggatccacga 1740

tccaacggct acgaattttt ggagtgacgt ggataggctc aaaggcgcca tttccatccg 1800

gctttatagt attttaaaaa aattcatttt cctccctcta gtgtgtgcgg aggcgtgagc 1860

ccgtttaacg gcgttgagaa gtctaacgga caccaaccac aaccaggaac cagcgccggc 1920

cgcgccgccg agtgaagcag actgcatacg gcacggcgcg gcatctctct ggctgcctct 1980

cgagagttcc gcccccacct tcccgcggta gcgtggtggt ttcgctttcc gctgtcggca 2040

tccggaagtt gcgtggcaga gtggacggag acgaggccgg gtcctccagc tcctctcaaa 2100

cgtcacggca ccggcatccg gcagccagcg cggtccttcc caaccactcg ttcccaaccc 2160

atcccccttc ctcgcccgcc gtcataaata gccagcccca tccccagctt ctttccccaa 2220

cctcatcttc tctccttttg ctctgaacgc acacaccgcc cggtctccga tctccgatcc 2280

ccgatcccct cgtcgatcct aggtacggcg accatcctac cccccccccc cccccctctc 2340

tctctgcctt ctctagatcg gcgatccgat ccatgcttac ttggttaggg cctgctaact 2400

atgttcatgt ttgcgttaga tccgtgcatg gacgcgatct gtacacacca gacgcgttct 2460

gattgctagc taactcgcca gtacctggga atcctgggat ggctgtagcc ggccccgcac 2520

gcagacggga ccgatttcat gattctctat ttttttcttt gtttcgttgc ctagggtttc 2580

gttcgatcga tccgcgttat tctttatttc catatattct ggtacgatgt tgatacggtt 2640

cgaccgtgct gcttacgttc tgtgcgcttg tttgccgggt catttttacc ttgccttttt 2700

tgtatggttt ggttgtggcg atgtggtctg gtcgggctgt cgttctagat cggagtagag 2760

tgctgtttca aactgtctag cggatctatt agatttggat ctgcatgtgt gacatatatc 2820

ttcgtagtta agatgatgca tctgtatgtg tgacatgcgg atctattaga tttggatctg 2880

tatgtgtgac atatatcttc gtagttgaga tgatgcatct gtatgtgtga catatatctt 2940

cgtagttaag attatgcatg gaaatatcaa tcctttagat aaggacgggt atacttgttg 3000

ctgtgggttt tactggtact tcgatagatg catatacatg atctaacatg cttagataca 3060

tgaagtaaca tgctgctacg gtttaataat tcttgagttg atttttactg gtacttagat 3120

agatgtatat acatgcttag atacatgaag taacatgctc ctacagttcc tttaatcatt 3180

attgagtacc tatatattct aataaatcag tatgttttaa attattttga ttttactggt 3240

acttagatag atgtatatat acatgctcaa acatgcttag atacatgaag taacatgctg 3300

ctacggttta gtcattattg agtgcctata tattctaata aatcagtatg ttttaaatta 3360

ttttgatttt actggtactt agatagatgt atatatacat gctcaaacat gcttagatac 3420

atgaagtaat atgctactac ggtttaattg ttcttgagta cctatatatt ctaataaatc 3480

agtatgtttt aaattatttc gattttactg gtacttagat agatgtatat atacatgctt 3540

agatacatga agtaacatgc tactacggtt taattgttct tgaataccta tatattctaa 3600

taaatcagta tgttttaaat tatttcgatt ttactggtac ttagatagat gtatatatac 3660

atgctcgaac atgcttagat acatgaagta acatgctaca tatatattat aataaatcag 3720

tatgtcttaa attattttga ttttactggt acttagatag atgtatatac atgctcaaac 3780

atgcttagat acatgaagta acatgctact acggtttaat cattattgag tacctatata 3840

ttctaataaa tcagtatgtt ttcaattgtt ttgattttac tggtacttag atatatgtat 3900

atatacatgc tcgaacatgc ttagatacgt gaagtaacat gctactatgg ttaattgttc 3960

ttgagtacct atatattcta ataaatcagt atgttttaaa ttatttcgat tttactggta 4020

cttagataga tgtatatata catgctcgaa catgcttaga tacatgaagt aacatgctac 4080

tacggtttaa tcgttcttga gtacctatat attctaataa atcagtatgt cttaaattat 4140

cttgatttta ctggtactta gatagatgta tatacatgct tagatacatg aagtaacatg 4200

ctactatgat ttaatcgttc ttgagtacct atatattcta ataaatcagt atgtttttaa 4260

ttattttgat tttactggta cttagataga tgtatatata catgctcgaa catgcttaga 4320

tacatgaagt aacatgctac tacggtttaa tcattcttga gtacctatat attctaataa 4380

atcagtatgt ttttaattat tttgatatta ctggtactta acatgtttag atacatcata 4440

tagcatgcac atgctgctac tgtttaatca ttcgtgaata cctatatatt ctaatatatc 4500

agtatgtctt ctaattatta tgattttgat gtacttgtat ggtggcatat gctgcagcta 4560

tgtgtagatt ttgaataccc agtgtgatga gcatgcatgg cgccttcata gttcatatgc 4620

tgtttatttc ctttgagact gttctttttt gttgatagtc accctgttgt ttggtgattc 4680

ttatgcagat ccagatcttc gtaaaccatg gctctggtgg ctaggccagt gctgtctgct 4740

agagtggctg cttctaggcc aaggtggccc gctagaaagg ctgtgagggt gagcgctgcc 4800

agtcacggcg ctagctctag gccagccacc gctaggaagt ccagcggcct gagcggcacc 4860

gtgcgcatcc caggcgacaa gagcatcagt caccgcagct tcatgttcgg cggcctggcc 4920

agcggcgaga ctaggatcac cggcctgctc gagggcgagg acgtgatcaa caccggcaag 4980

gccatgcagg ctatgggcgc tcgcatccgc aaggagggcg acacctggat catcgacggc 5040

gtgggtaatg gcggcctgct ggccccagag gccccactgg acttcggcaa cgccgccacc 5100

ggctgcaggc tgactatggg cctggtgggc gtgtacgact tcgacagcac ctttatcggc 5160

gacgccagcc tgaccaagag gccgatgggc agggtgctga acccgctgcg cgagatgggc 5220

gtgcaggtca agagcgagga cggcgacagg ctgccggtga ccctgagggg cccaaagacc 5280

ccgaccccga tcacctaccg cgtgccaatg gccagcgccc aggtgaagtc agccgtgctg 5340

ctggccggcc tgaacacccc aggcatcacc accgtgatcg agccgatcat gacccgcgat 5400

cacaccgaga agatgctgca gggcttcggc gccaacctga ccgtcgagac tgacgctgac 5460

ggcgtgagga ccatcaggct cgagggcagg ggcaagctga ccggccaggt gatcgacgtg 5520

ccgggcgacc caagcagcac cgccttccca ctggtggccg ccctgctggt gccaggctct 5580

gacgtgacca tcctgaacgt gctgatgaac ccgagccgca ccggcctgat cctgaccctg 5640

caggagatgg gcgccgatat cgaggtgatc aacccgaggc tggctggcgg cgaggacgtc 5700

gccgacctga gggtgaggtc cagcaccctg aagggcgtga ccgtgccaga ggacagggcc 5760

ccgagtatga ttgacgagta cccgatcctg gccgtggccg ctgccttcgc cgagggcgcc 5820

accgtgatga acggcctcga ggagctgcgc gtgaaggaga gcgacaggct gagcgctgtg 5880

gccaacggcc tgaagctgaa cggcgtggac tgcgacgagg gcgagactag cctggtggtg 5940

aggggcaggc cagacggcaa gggcctgggc aacgcttccg gcgctgccgt ggccacccac 6000

ctggatcaca ggatcgccat gtcgttcctc gtgatgggcc tcgtgagcga gaacccggtg 6060

accgtggacg acgccaccat gatcgccacc agcttccccg agttcatgga cctgatggcc 6120

ggcctgggcg ctaagatcga gctgagcgac accaaggccg cctgatcatc tagagctcgc 6180

caaggttcaa ttaagctgct gctgtacctg ggtatctgcg tcgtctggtg ccctctggtg 6240

tacctctata tggatgtcgt cgtctaataa acatctgtgg tttgtgtgtc atgaatcgtg 6300

gttgtggctt cgttggttta atggacctgt tgtgtcctct gtgttgtacc caaaactctt 6360

ctgcagcagt atggcttgaa tccttatgaa gtttgatatt tgaacttaaa agtctgctca 6420

ttatgttttt ttctggttat atctcctaat taactgcctg ggatcaaatt tgattcgctg 6480

gtgtttattg gacccctccc aggttcttgc tttctaccgt ttcttgctga atgttaactt 6540

gattctgtca ggctcagttt cccactatgg cttacagctt aacgtgtttg gtttgttgaa 6600

tgttaacttg gttttgtcaa gctcagtttt ttactctggc ttacagcata acatgtttga 6660

cttttggttt tgctgctttg ttattgggtt ctgggtagtt cttgatgaat ccaaaagatc 6720

atgtgcacag ccatattatc tatttaagcg atccaggtta ttactatgaa aggatgcctt 6780

ctagctaagg agtagttagg ttttttcttc aaggttaaat tttctcgatg ctctagtgtt 6840

cctgtgacca taatcataat aattcctttg aaagctctat ggtccctgga agcagggcat 6900

acaatgcaag acagcaactt gatcacatca actgaagtat acagggttct cttaactctt 6960

ggtgacttcg gtttaatgga ccggttgtac tcgtgttcta tccgtaaccg ttgtgatgtc 7020

ttgtgtgttt ggttgcggga tagctgggac cacgacgttt ccgtctaatt ctgatggata 7080

gctatagacg gcactgagat ggttatatta taacctctga tcctgaactc tacgagatcg 7140

tctcatccgt cattgccacc aaatacacca ttaaattact aattagctaa cggaccctat 7200

ttgtactcat tccatgtctc ataaactttg ggcaccatcc atccaacaca tccaatctaa 7260

acacaccaaa cgatggggaa tggaaagagc agtattcgat tcaacaatgg caaacaaata 7320

tcactgaatt agaccaagaa taaacctaat tagacaacga cctcccaacc atcattcgtc 7380

aggctgtaaa gaagataaag ctgccttggg gcatggatca agcagaacac cagagatgaa 7440

tccaaacaca cagaaaatca cgcgcgctgt ctacaatgac aacaagcccc acatttcatt 7500

gcagtacact gggctacaaa ggcacgtaca acaaagagct agggaaacat tgcggagggc 7560

acgagagagc agctaacttg acaatatagc agactgagct tgcactgtta gcaggcgagg 7620

aagggaatca tggggacgga gaatggggtc catgcccgcg aaggagaagg cggacgccgc 7680

cacggtggca ccggcgcacg cgcacacagg gaacccgcac aggcagccaa ggatgctgcc 7740

tcgccattgc gccggtcgtc tctgccacgc tcctctctct ctcccgctgc atcgccgtgg 7800

atggggcaag cagagagcag ggactgcgac gatctgggcg gaggactcgc cttggagagc 7860

gcggacgcag acgggattct agggagagag cgaagacggg gcgcgcgcgg cgctcgcgcg 7920

gcgtggtggc ggcgagatta gcgggggtgg ggggagggcg gagccgtggt gagggtgtgg 7980

acgccctcct taccctctta agtagtagta gagatataat ccgttccaaa atatccatcc 8040

gttcaattta tatttcgttt gatcttttta ccctaaattt gattgactca tcttattaaa 8100

aaagttcata actattatta atctttattg agatatcatt tagcatataa tatactttaa 8160

gtgtggtttt agattttttt taaaaaaaaa aattcgcaaa aattaaatga aacgacccaa 8220

tcaaacttga aaagtaaaac taattataaa tttgaacgga aggagtaaga ggatgtttga 8280

atgtactaga gctaatagtt ggttgcttta aaatttgcta gtagaattag ctagctaata 8340

aatatctaga taactattag ctaatttgct aaaacagcta atagttgaac tattagctag 8400

attgtttgga tgtattcggc taattttaat ggctaactat tagctatagt acaatattca 8460

aacacctcct aattaaaatg gacaaatatc tcttcttttg gtcccttgcg ttagattttt 8520

catatctcct tatttagtat aaaagaatca tcaaaaagtg gacaacccct agtggaacac 8580

cattttagta gtggttgcat gaaacctttc gcgcaccagt ttctatgtgt cactctaaaa 8640

atgggacagc atgtacgtag tgcctatata tatacaagtc atctatcgtt gcctcctcag 8700

ttcatcacta atcacactta ttgtgccctc gacgagtatc tatagctagc tcattaatcg 8760

attcgggggt gtgttgtcga aggcggcatt ggcgagctac tcgtcgcggc gtccaagcaa 8820

tacctgtagc acgaaggcga tcgccgggag cgtggtcggc gagcccgtcg tgctggggca 8880

gagggtgacg gtgctgacgg tggacggcgg cggcgtccgg ggtctcatcc cgggaaccat 8940

cctcgccttc ctggaggcca ggctgcagga gctggacgca ccggaggcga ggctggcgga 9000

ctacttcgac tacatcgccg gaaccagcac cggcggtctc atcaccgcct tgctgaccgc 9060

gcccggcaag gacaagcggc ctctctaggc tgccaaggac atcaaccact tttacatcca 9120

taactgcccg cgcatctttc ctcagaagtg agtccgatgc tgccgccatt gttcttgcat 9180

ccatccagca tcgtacgtac gtcctctata catctgcgga tcatcatgtg cgcatgtttg 9240

tggcatgcat gcatgcatgt gagcaggagc aggcttgcga aaaccccatg gagatctttg 9300

cgatggcgca gatcaccaaa aagatgcttg ccaacaagga caaggccgag gagctgtacc 9360

cagtgaagcc gtcgaactgc cgcaggttcc tggtgctgtc catcgggacg gggtcgacgt 9420

ccgagcaggg cctctacacg gcgcggcagt gctcccggtg gggtatctgc cggtggctcc 9480

gcgtcgactc gggagcatgc cctttgtgct tatgcctccg ttctgccttc tgacgaattt 9540

ggtactggaa gcagatgagt tttggttcac tatcattctg aatttacacc tgcgcttgct 9600

gtcagactag gcaaccaagt gacttttgtg actttgatca tgttcagtgt gtttccaagt 9660

cctaatcaat caaaaagaaa aacagtttgt taacgattgt ttgccatgtc tatataataa 9720

agttgctttt atagtagctt agaattcaat cggccaactt tatctcgtac gctgacagta 9780

aaggtacatt taaaaggtga caatggatag tctaatactt gaactgacaa tagagacaca 9840

ttacatgtca gttgattaag tttgtaacag aaaaataaac aatactacat aattgcaaag 9900

tttctttgat gtctttcttt caagaaacac aaatatatca atgctacagt attgctgatg 9960

aatttatcca tgttgagatg tttttctggt ttctgatctg atcagtctca attggtgtgc 10020

tgtttcattt tcatttgctg atgatcgtcc gagtagttaa ttcttactaa tatttagata 10080

atttggcata caagcgaatc acgtagaaca tgatactttt gaatgaattt atcaaagttt 10140

tatcacttgg tgagttgttt catggttttc ctactgatgt ctcttcttca gatttctcga 10200

ggcggagcca ccggcagata ccccaccggg agcactgccg cgccgtgtag aggccctgct 10260

cggacgtcga ccccgtcccg atggacagca ccaggaacct gcggcagttc gacggcttca 10320

ctgggtacag ctcctcggcc ttgtccttgc tggcaagcat ctttttggtg atctgcgtca 10380

tcgcaaggat ccatgggaca agtggcttta ctgtcagtca catgcttgta aataagtaga 10440

ctttatttta ataaaacata aaaatatata tatgttcttg aatataaaat tgataaccaa 10500

attaaaattc gaaccatcac ttatacataa ttttacttta ttttttataa aacgtgaacg 10560

ggaaggacta ccgtgaatga ctatagaacc aatcatacta gtataaaata tatgatgaca 10620

ctacgggaga gacaaacttt gtctggcgct aaatattttg ccgagtgtga attcacgggc 10680

actaggcaaa gatcttcttt gccgagtgtt acgctgggca aagtaagaca ctaggtaaat 10740

cagtcatttg ccgagtgtcc gccactaggc aaagcaaaac actggcaaat caaaagttta 10800

cctagtgcca gacactaggc aaaaaaaaaa cgctcggcaa atcggaagtt tccctagtgc 10860

cagacactag acaaagaaaa acacttgata aactagcgtc gtcagctaac accatccacc 10920

aaccgttaac gttgccgagt atctgacttc gacactcggc aaagaaggtc tctttgccta 10980

gtgtcggtct ggaacactag gcaaagaggc actttaccta gtgtcgtatt ttgacactca 11040

gtaaaataat tttttttctt tctgcttcca aactttttat gatgtgttcc tatagcacct 11100

agaactacat gtcaagtttt ggtaaaattt ttgaagtttt tgctatattt acttaattta 11160

ttttatttaa ttgaatttct tttgataatt caaatttgaa ctcggcaagg taagaagcga 11220

gggtagcctg gaaacacact ttgcctagtg ttacactcgg tacaggagcc tcccctgcct 11280

agtgctgcac tcgacaaaag attcgccttt gcctagcgct gcactcggca caggagtcgc 11340

ctttgcctag tgctgcacta ggcaaagcct ccgttaccgt gccttccatc gtcggacccg 11400

aaagtagcaa acaacaggtt catgtgcact ataaaaagac aaaattctcg agtttcatct 11460

tttattccac ataagcctta tattttccat tttcatatga tttttagttt aagtttgtgt 11520

cttaactttt tcgttaatac gtaattctat gcattatgga tgcgtgaagt atttttgttt 11580

aaaaaaatga aatgtcaaaa tacgttttgt gatctatttc catgttttca cctaacaggt 11640

ggtttttact atatattctg ccataactct agccttagat gtaaatcgaa aaaaaatgag 11700

agatgagctg gagatagcct tagatgaagc gtctgaaata taaaagaaag agtaatgttg 11760

aacgcagtag gtgtagcagc tgtagttcca tctctaggaa agggaactgc aatccgggct 11820

ccgggcctcg cgcaatctgg cctgtcgtgt agatgcagcc ctgtccatga cggcccaagc 11880

aacgcccgcg gctctcgatc caccacggaa cccactccga cacacactga cacacacatg 11940

ctggatgtgg atgtgctgtc caattattag tagcaattcg gtaggcacag gcacgtactg 12000

gccggtgttt tagctgtaag taccgaacca atcacggtta agaaccgatt aatccgtgcc 12060

cagccgccga gtgcgttcgt acgtgcatcg gatgcactgc atgaattgag agcatcatca 12120

tatcatacgc aggagtagta cgacgccgct gctgtcttgt ccggctaatg ctttgctcac 12180

agattagtcc atcgcccacg gtcggtgtgg tgtggatcgc tgatgccact gctttttgtt 12240

tggtttttat tcccctgata atcctccgcg tccctgaatg tatctattta ttttcattcc 12300

gaaatccctt tcacgaaaaa gaaaacgaat aaaaagagag ttacgaatac gcttccggcg 12360

gcccacatca ccttccagcg aacatcgcgc cgcgctgacg tgtcgcccat cgcggccgtc 12420

catatcgcca tccgacgacc gtggaagctg gcagcggccg ctccgttccg tcgaaggggc 12480

aggtcagtca ggtcacccac acggccacac ccgcgcgggg gatacgcggt ggaaaacccg 12540

gcgaccacat caaaacacga ggcgtctccc gcaggactgg tcactcggca cgcaggcaga 12600

ggcagcacag cagcagccag ctccatccat cctctttccc ctcctcgctt cgcttcctcg 12660

gcggattcct cctccctcgg ccgtccccgt ccccttcttc gccgcgccag ctcgcccgag 12720

ttggtacgcc catcctctgc tgtactcccc ccgttcctgc tgtgcgaaac cgagcaccca 12780

aaccctagcc taagctattc gcatcgcaaa ctctattgag cgacggatcg cgaaacgcgc 12840

gccgccgcat tcggacgaga cctcgtggcc cgtaccttcc tactctaccg tcgtcgccgg 12900

agaccagctc accggttagg gtttcgggat taggggcttg gggcttggat tcccggggga 12960

ctccataccc ggtggcctta gaaggggaag ggggcccagc ggtggtggtg gttcgactgt 13020

cagcgagcga gacggcgggg caccgccgat cgggcgtcgc tggacatttg attgagctgg 13080

gcaggcgaag gcgtggagct tgctcttcga ttgggattag aggagggcgg aggtggtatg 13140

gtgggcggct cggctggctc aatggagccg ctggccggtg gtggggcagg cgagatcacg 13200

ctctctcata gagcgtaatg ggttgcgtaa cacactcctg tttgtatttc gatccgatct 13260

aataattaaa gtttatttac gtggtgcaca tgtagaattt tcccatgccc actgctaact 13320

tggattggat ttttgccaaa aaaacgggtg tgcacccaat cacttatagt gaaaccgttg 13380

tgtgtttgat gtgcttctaa ccaagtacaa atccagtgaa aacacaccta ccattcaaca 13440

atccacattt tggttgcaat tatcagcatt ctagaaaggt gcatgtgccc attgatacac 13500

ttgctattgg tgcaggcaaa cccaccatgg cctcctccga gaacgtcatc accgagttca 13560

tgcgcttcaa ggtgcgcatg gagggcaccg tgaacggcca cgagttcgag atcgagggcg 13620

agggcgaggg ccgcccctac gagggccaca acaccgtgaa gctgaaggtg accaagggcg 13680

gccccctgcc cttcgcctgg gacatcctgt ccccccagtt ccagtacggc tccaaggtgt 13740

acgtgaagca ccccgccgac atccccgact acaagaagct gtccttcccc gagggcttca 13800

agtgggagcg cgtgatgaac ttcgaggacg gcggcgtggc gaccgtgacc caggactcct 13860

ccctgcagga cggctgcttc atctacaagg tgaagttcat cggcgtgaac ttcccctccg 13920

acggccccgt gatgcagaag aagaccatgg gctgggaggc ctccaccgag cgcctgtacc 13980

cccgcgacgg cgtgctgaag ggcgagaccc acaaggccct gaagctgaag gacggcggcc 14040

actacctggt ggagttcaag tccatctaca tggccaagaa gcccgtgcag ctgcccggct 14100

actactacgt ggacgccaag ctggacatca cctcccacaa cgaggactac accatcgtgg 14160

agcagtacga gcgcaccgag ggccgccacc acctgttcct gtaggagctc gcatcatgat 14220

catgcatcat ggactcggcc tactactgtg gatttgtatg ccattataga cttggtgctg 14280

tgaaagactg cttgatgatt tgcgggtttg ttgctgtgta aaaaaaggtc ccttggctcc 14340

cagaagacca tgaaggttcg gatctatcat gtaattcctt gttatctgcc aattatgtat 14400

ggactatgga catgtgttgc gctgttcaac ttactactac aaataagtaa tcgatatgtt 14460

cccttcccat gtctcggtga caattgtctg gagaagctta ggggtcgttt gtttgggatt 14520

atgtctggag aaacttattt taaactaagt gtgagttcaa gttaagttag attatataat 14580

ctaggcagat tataattcca agcgaacagg tccttagtgt ttttggaaaa tcctaggtgt 14640

tcttttggct acattgttgt gtgtgcagat cccttgttgg tctgtaagcg tggggaagta 14700

agaatcgtcc gtttctactg aagacctgct cgagttaggc accgaggatg ccggtaacca 14760

aacagagcaa tagtgtctct gtgggcacag tggagtgtga atctgtgtga tgcaaatccg 14820

tcatttgttt agcaaaattt ccagcgttgc atgatgcagt ttctttaaca cggacttaag 14880

ggaagggaaa aaaatgttga gccaggagat ccttcaatgt gttagactga cgtgatagcc 14940

aactaaacca cgacgcaatg ttgtcgttaa tgacaaaaaa actatttgtt cctaaatcct 15000

tggcgacatt gcatggctgt ctcatgagat aatggtctca tctcttattt atctcttatt 15060

tatagccgga agtggtagtg acccctgctt gattgctcgt atgccatctc aagttctcaa 15120

ccgtgtcgag cagccatttt cccatctcaa gcgcatcatc gtttcgtttg acctcatctg 15180

ctatcctgct cctagtgcaa atcacatgcg acagaaagtg tgcggaccgc ctgcaggccc 15240

gggggcgcgc cctaattagc taacggccag gatcgccgcg tgagccttta gcaactagct 15300

agattaatta acgcaatctg ttattaagtt gtctaagcgt caatttgttt acaccacaat 15360

atatcctgcc accagccagc caacagctcc ccgaccggca gctcggcaca aaatcaccac 15420

tcgatacagg cagcccatca g 15441

<210> 34
<211> 10671
<212> DNA
<213> Artificial Sequence

<220>
<223> Expression cassette of construct 22807

<220>

<221>  enhancer
<222>  (165)..(256)
<223>  eNOS

<220>
<221>  promoter
<222>  (301)..(2097)
<223>  Ubi

<220>
<221>  gene
<222>  (2114)..(6283)
<223>  Cas9

<220>
<221>  misc_feature
<222>  (6218)..(6274)
<223>  NLS-TAG

<220>
<221>  terminator
<222>  (6289)..(6541)
<223>  tNOS

<220>
<221>  promoter
<222>  (6548)..(6922)
<223>  pU3

<220>
<221>  misc_feature
<222>  (6924)..(6941)

<220>
<221>  misc_feature
<222>  (6924)..(7026)
<223>  sgRNA

<220>
<221>  promoter
<222>  (7037)..(9028)
<223>  Ubi

<220>
<221>  gene
<222>  (9045)..(10223)
<223>  PMI

<220>
<221>  terminator
<222>  (10246)..(10498)
<223>  tNOS

<400>  34
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tgaaggcggg aaacgacaat ctgatcatga gcggagaatt     180

aagggagtca cgttatgacc cccgccgatg acgcgggaca agccgtttta cgtttggaac     240

tgacagaacc gcaacgctgc aggaattggc cgcagcggcc atttaaacaa agcttggtac     300

cattatgtgg tctaggtagg ttctatatat aagaaaactt gaaatgttct aaaaaaaaat 360

tcaagcccat gcatgattga agcaaacggt atagcaacgg tgttaacctg atctagtgat 420

ctcttgcaat ccttaacggc cacctaccgc aggtagcaaa cggcgtcccc ctcctcgata 480

tctccgcggc gacctctggc tttttccgcg gaattgcgcg gtggggacgg attccacgag 540

accgcgacgc aaccgcctct cgccgctggg ccccacaccg ctcggtgccg tagcctcacg 600

ggactctttc tccctcctcc cccgttataa attggcttca tcccctcctt gcctcatcca 660

tccaaatccc agtccccaat cccatccctt cgtaggagaa attcatcgaa gctaagcgaa 720

tcctcgcgat cctctcaagg tactgcgagt tttcgatccc cctctcgacc cctcgtatgt 780

ttgtgtttgt cgtagcgttt gattaggtat gctttccctg tttgtgttcg tcgtagcgtt 840

tgattaggta tgctttccct gttcgtgttc atcgtagtgt ttgattaggt cgtgtgaggc 900

gatggcctgc tcgcgtcctt cgatctgtag tcgatttgcg ggtcgtggtg tagatctgcg 960

ggctgtgatg aagttatttg gtgtgatctg ctcgcctgat tctgcgggtt ggctcgagta 1020

gatatgatgg ttggaccggt tggttcgttt accgcgctag ggttgggctg ggatgatgtt 1080

gcatgcgccg ttgcgcgtga tcccgcagca ggacttgcgt ttgattgcca gatctcgtta 1140

cgattatgtg atttggtttg gactttttag atctgtagct tctgcttatg tgccagatgc 1200

gcctactgct catatgcctg atgataatca taaatggctg tggaactaac tagttgattg 1260

cggagtcatg tatcagctac aggtgtaggg actagctaca ggtgtaggga cttgcgtcta 1320

attgtttggt cctttactca tgttgcaatt atgcaattta gtttagattg tttgttccac 1380

tcatctaggc tgtaaaaggg acactgctta gattgctgtt taatcttttt agtagattat 1440

attatattgg taacttatta cccctattac atgccatacg tgacttctgc tcatgcctga 1500

tgataatcat agatcactgt ggaattaatt agttgattgt tgaatcatgt ttcatgtaca 1560

taccacggca caattgctta gttccttaac aaatgcaaat tttactgatc catgtatgat 1620

ttgcgtggtt ctctaatgtg aaatactata gctacttgtt agtaagaatc aggttcgtat 1680

gcttaatgct gtatgtgcct tctgctcatg cctgatgata atcatatatc actggaatta 1740

attagttgat cgtttaatca tatatcaagt acataccatg ccacaatttt tagtcactta 1800

acccatgcag attgaactgg tccctgcatg ttttgctaaa ttgttctatt ctgattagac 1860

catatatcat gtattttttt ttggtaatgg ttctcttatt ttaaatgcta tatagttctg 1920

gtacttgtta gaaagatctg cttcatagtt tagttgccta tccctcgaat taggatgctg 1980

agcagctgat cctatagctt tgtttcatgt atcaattctt ttgtgttcaa cagtcagttt 2040

ttgttagatt cattgtaact tatggtcgct tactcttctg gtcctcaatg cttgcaggat 2100

cgcggccgct catatggaca agaagtacag catcggcctg gacatcggca ccaacagcgt 2160

gggctgggcc gtgatcaccg acgagtacaa ggtgccgagc aagaagttca aggtgctggg 2220

caacaccgac aggcacagca tcaagaagaa cctgatcggc gccctgctgt tcgacagcgg 2280

cgagaccgcc gaggccacca ggctgaagag gaccgccagg aggaggtaca ccaggaggaa 2340

gaacaggatc tgctacctgc aggagatctt cagcaacgag atggccaagg tggacgacag 2400

cttcttccac aggctggagg agagcttcct ggtggaggag gacaagaagc acgagaggca 2460

cccgatcttc ggcaacatcg tggacgaggt ggcctaccac gagaagtacc cgaccatcta 2520

ccacctgagg aagaagctgg tggacagcac cgacaaggcc gacctgaggc tgatctacct 2580

ggccctggcc cacatgatca agttcagggg ccacttcctg atcgagggcg acctgaaccc 2640

ggacaacagc gacgtggaca agctgttcat ccagctggtg cagacctaca accagctgtt 2700

cgaggagaac ccgatcaacg ccagcggcgt ggacgccaag gccatcctga gcgccaggct 2760

gagcaagagc aggaggctgg agaacctgat cgcccagctg ccgggcgaga agaagaacgg 2820

cctgttcggc aacctgatcg ccctgagcct gggcctgacc ccgaacttca agagcaactt 2880

cgacctggcc gaggacgcca agctgcagct gagcaaggac acctacgacg acgacctgga 2940

caacctgctg gcccagatcg gcgaccagta cgccgacctg ttcctggccg ccaagaacct 3000

gagcgacgcc atcctgctga gcgacatcct gagggtgaac accgagatca ccaaggcccc 3060

gctgagcgcc agcatgatca agaggtacga cgagcaccac caggacctga ccctgctgaa 3120

ggccctggtg aggcagcagc tgccggagaa gtacaaggag atcttcttcg accagagcaa 3180

gaacggctac gccggctaca tcgacggcgg cgccagccag gaggagttct acaagttcat 3240

caagccgatc ctggagaaga tggacggcac cgaggagctg ctggtgaagc tgaacaggga 3300

ggacctgctg aggaagcaga ggaccttcga caacggcagc atcccgcacc agatccacct 3360

gggcgagctg cacgccatcc tgaggaggca ggaggacttc tacccgttcc tgaaggacaa 3420

cagggagaag atcgagaaga tcctgacctt ccgcatcccg tactacgtgg gcccgctggc 3480

caggggcaac agcaggttcg cctggatgac caggaagagc gaggagacca tcaccccgtg 3540

gaacttcgag gaggtggtgg acaagggcgc cagcgcccag agcttcatcg agaggatgac 3600

caacttcgac aagaacctgc cgaacgagaa ggtgctgccg aagcacagcc tgctgtacga 3660

gtacttcacc gtgtacaacg agctgaccaa ggtgaagtac gtgaccgagg gcatgaggaa 3720

gccggccttc ctgagcggcg agcagaagaa ggccatcgtg gacctgctgt tcaagaccaa 3780

caggaaggtg accgtgaagc agctgaagga ggactacttc aagaagatcg agtgcttcga 3840

cagcgtggag atcagcggcg tggaggacag gttcaacgcc agcctgggca cctaccacga 3900

cctgctgaag atcatcaagg acaaggactt cctggacaac gaggagaacg aggacatcct 3960

ggaggacatc gtgctgaccc tgaccctgtt cgaggacagg gagatgatcg aggagaggct 4020

gaagacctac gcccacctgt tcgacgacaa ggtgatgaag cagctgaaga ggaggaggta 4080

caccggctgg ggcaggctga gcaggaagct gatcaacggc atcagggaca agcagagcgg 4140

caagaccatc ctggacttcc tgaagagcga cggcttcgcc aacaggaact tcatgcagct 4200

gatccacgac gacagcctga ccttcaagga ggacatccag aaggcccagg tgagcggcca 4260

gggcgacagc ctgcacgagc acatcgccaa cctggccggc agcccggcca tcaagaaggg 4320

catcctgcag accgtgaagg tggtggacga gctggtgaag gtgatgggca ggcacaagcc 4380

ggagaacatc gtgatcgaga tggccaggga gaaccagacc acccagaagg gccagaagaa 4440

cagcagggag aggatgaaga ggatcgagga gggcatcaag gagctgggca gccagatcct 4500

gaaggagcac ccggtggaga acacccagct gcagaacgag aagctgtacc tgtactacct 4560

gcagaacggc agggacatgt acgtggacca ggagctggac atcaacaggc tgagcgacta 4620

cgacgtggac cacatcgtgc cgcagagctt cctgaaggac gacagcatcg acaacaaggt 4680

gctgaccagg agcgacaaga acaggggcaa gagcgacaac gtgccgagcg aggaggtggt 4740

gaagaagatg aaaaactact ggaggcagct gctgaacgcc aagctgatca cccagaggaa 4800

gttcgacaac ctgaccaagg ccgagagggg cggcctgagc gagctggaca aggccggctt 4860

cattaaaagg cagctggtgg agaccaggca gatcaccaag cacgtggccc agatcctgga 4920

cagcaggatg aacaccaagt acgacgagaa cgacaagctg atcagggagg tgaaggtgat 4980

caccctgaag agcaagctgg tgagcgactt caggaaggac ttccagttct acaaggtgag 5040

ggagatcaat aattaccacc acgcccacga cgcctacctg aacgccgtgg tgggcaccgc 5100

cctgattaaa aagtacccga agctggagag cgagttcgtg tacggcgact acaaggtgta 5160

cgacgtgagg aagatgatcg ccaagagcga gcaggagatc ggcaaggcca ccgccaagta 5220

cttcttctac agcaacatca tgaacttctt caagaccgag atcaccctgg ccaacggcga 5280

gatcaggaag aggccgctga tcgagaccaa cggcgagacc ggcgagatcg tgtgggacaa 5340

gggcagggac ttcgccaccg tgaggaaggt gctgtccatg ccgcaggtga acatcgtgaa 5400

gaagaccgag gtgcagaccg gcggcttcag caaggagagc atcctgccga agaggaacag 5460

cgacaagctg atcgccagga agaaggactg ggacccgaag aagtacggcg gcttcgacag 5520

cccgaccgtg gcctacagcg tgctggtggt ggccaaggtg gagaagggca agagcaagaa 5580

gctgaagagc gtgaaggagc tggtgggcat caccatcatg gagaggagca gcttcgagaa 5640

gaacccagtg gacttcctgg aggccaaggg ctacaaggag gtgaagaagg acctgatcat 5700

taaactgccg aagtacagcc tgttcgagct ggagaacggc aggaagagga tgctggccag 5760

cgccggcgag ctgcagaagg gcaacgagct ggccctgccg agcaagtacg tgaacttcct 5820

gtacctggcc agccactacg agaagctgaa gggcagcccg gaggacaacg agcagaagca 5880

gctgttcgtg gagcagcaca agcactacct ggacgagatc atcgagcaga tcagcgagtt 5940

cagcaagagg gtgatcctgg ccgacgccaa cctggacaag gtgctgagcg cctacaacaa 6000

gcacagggac aagccgatca gggagcaggc cgagaacatc atccacctgt tcaccctgac 6060

caacctgggc gccccggccg ccttcaagta cttcgacacc accatcgaca ggaagaggta 6120

caccagcacc aaggaggtgc tggacgccac cctgatccac cagagcatca ccggcctgta 6180

cgagaccagg atcgacctga gccagctggg cggcgacagc agcccgccga agaagaagag 6240

gaaggtgagc tggaaggacg ccagcggctg gagcaggatg tgaagcttga tcgttcaaac 6300

atttggcaat aaagtttctt aagattgaat cctgttgccg gtcttgcgat gattatcata 6360

taatttctgt tgaattacgt taagcatgta ataattaaca tgtaatgcat gacgttattt 6420

atgagatggg tttttatgat tagagtcccg caattataca tttaatacgc gatagaaaac 6480

aaaatatagc gcgcaaacta ggataaatta tcgcgcgcgg tgtcatctat gttactagat 6540

cttcgaaggg atctttaaac atacgaacag atcacttaaa gttcttctga agcaacttaa 6600

agttatcagg catgcatgga tcttggagga atcagatgtg cagtcaggga ccatagcaca 6660

ggacaggcgt cttctactgg tgctaccagc aaatgctgga agccgggaac actgggtacg 6720

ttggaaacca cgtgatgtgg agtaagataa actgtaggag aaaagcattt cgtagtgggc 6780

catgaagcct ttcaggacat gtattgcagt atgggccggc ccattacgca attggacgac 6840

aacaaagact agtattagta ccacctcggc tatccacata gatcaaagct ggtttaaaag 6900

agttgtgcag atgatccgtg gcagtcaacg tggagacagg ggttttagag ctagaaatag 6960

caagttaaaa taaggctagt ccgttatcaa cttgaaaaag tggcaccgag tcggtgcttt 7020

ttttttcgga ccgcgcctgc agtgcagcgt gacccggtcg tgcccctctc tagagataat 7080

gagcattgca tgtctaagtt ataaaaaatt accacatatt ttttttgtca cacttgtttg 7140

aagtgcagtt tatctatctt tatacatata tttaaacttt actctacgaa taatataatc 7200

tatagtacta caataatatc agtgttttag agaatcatat aaatgaacag ttagacatgg 7260

tctaaaggac aattgagtat tttgacaaca ggactctaca gttttatctt tttagtgtgc 7320

atgtgttctc cttttttttt gcaaatagct tcacctatat aatacttcat ccattttatt 7380

agtacatcca tttagggttt agggttaatg gtttttatag actaattttt ttagtacatc 7440

tattttattc tattttagcc tctaaattaa gaaaactaaa actctatttt agttttttta 7500

tttaataatt tagatataaa atagaataaa ataaagtgac taaaaattaa acaaataccc 7560

tttaagaaat taaaaaaact aaggaaacat ttttcttgtt tcgagtagat aatgccagcc 7620

tgttaaacgc cgtcgacgag tctaacggac accaaccagc gaaccagcag cgtcgcgtcg 7680

ggccaagcga agcagacggc acggcatctc tgtcgctgcc tctggacccc tctcgagagt 7740

tccgctccac cgttggactt gctccgctgt cggcatccag aaattgcgtg gcggagcggc 7800

agacgtgagc cggcacggca ggcggcctcc tcctcctctc acggcaccgg cagctacggg 7860

ggattccttt cccaccgctc cttcgctttc ccttcctcgc ccgccgtaat aaatagacac 7920

cccctccaca ccctctttcc ccaacctcgt gttgttcgga gcgcacacac acacaaccag 7980

atctccccca aatccacccg tcggcacctc cgcttcaagg tacgccgctc gtcctccccc 8040

ccccccctct ctaccttctc tagatcggcg ttccggtcca tggttagggc ccggtagttc 8100

tacttctgtt catgtttgtg ttagatccgt gtttgtgtta gatccgtgct gctagcgttc 8160

gtacacggat gcgacctgta cgtcagacac gttctgattg ctaacttgcc agtgtttctc 8220

tttggggaat cctgggatgg ctctagccgt tccgcagacg ggatcgattt catgattttt 8280

tttgtttcgt tgcatagggt ttggtttgcc cttttccttt atttcaatat atgccgtgca 8340

cttgtttgtc gggtcatctt ttcatgcttt tttttgtctt ggttgtgatg atgtggtctg 8400

gttgggcggt cgttctagat cggagtagaa ttctgtttca aactacctgg tggatttatt 8460

aattttggat ctgtatgtgt gtgccataca tattcatagt tacgaattga agatgatgga 8520

tggaaatatc gatctaggat aggtatacat gttgatgcgg gttttactga tgcatataca 8580

gagatgcttt ttgttcgctt ggttgtgatg atgtggtgtg gttgggcggt cgttcattcg 8640

ttctagatcg gagtagaata ctgtttcaaa ctacctggtg tatttattaa ttttggaact 8700

gtatgtgtgt gtcatacatc ttcatagtta cgagtttaag atggatggaa atatcgatct 8760

aggataggta tacatgttga tgtgggtttt actgatgcat atacatgatg gcatatgcag 8820

catctattca tatgctctaa ccttgagtac ctatctatta taataaacaa gtatgtttta 8880

taattatttt gatcttgata tacttggatg atggcatatg cagcagctat atgtggattt 8940

ttttagccct gccttcatac gctatttatt tgcttggtac tgtttctttt gtcgatgctc 9000

accctgttgt ttggtgttac ttctgcaggg atccggcagc agccatgcag aagctgatca 9060

acagcgtgca gaactacgcc tggggcagca agaccgccct gaccgagctg tacggcatgg 9120

agaaccccag cagccagccc atggccgagc tgtggatggg cgcccacccc aagagcagca 9180

gccgcgtgca gaacgccgcc ggcgacatcg tgagcctgcg cgacgtgatc gagagcgaca 9240

agagcaccct gctgggcgag gccgtggcca agcgcttcgg cgagctgccc ttcctgttca 9300

aggtgctgtg cgccgcccag cccctgagca tccaggtgca ccccaacaag cacaacagcg 9360

agatcggctt cgccaaggag aacgccgccg gcatccccat ggacgccgcc gagcgcaact 9420

acaaggaccc caaccacaag cccgagctgg tgttcgccct gacccccttc ctggccatga 9480

acgccttccg cgagttcagc gagatcgtga gcctgctgca gcccgtggcc ggcgcccacc 9540

ccgccatcgc ccacttcctg cagcagcccg acgccgagcg cctgagcgag ctgttcgcca 9600

gcctgctgaa catgcagggc gaggagaaga gccgcgccct ggccatcctg aagagcgccc 9660

tggacagcca gcagggcgag ccctggcaga ccatccgcct gatcagcgag ttctaccccg 9720

aggacagcgg cctgttcagc cccctgctgc tgaacgtggt gaagctgaac cccggcgagg 9780

ccatgttcct gttcgccgag accccccacg cctacctgca gggcgtggcc ctggaggtga 9840

tggccaacag cgacaacgtg ctgcgcgccg gcctgacccc caagtacatc gacatccccg 9900

agctggtggc caacgtgaag ttcgaggcca agcccgccaa ccagctgctg acccagcccg 9960

tgaagcaggg cgccgagctg gacttcccca tccccgtgga cgacttcgcc ttcagcctgc 10020

acgacctgag cgacaaggag accaccatca gccagcagag cgccgccatc ctgttctgcg 10080

tggagggcga cgccaccctg tggaagggca gccagcagct gcagctgaag cccggcgaga 10140

gcgccttcat cgccgccaac gagagccccg tgaccgtgaa gggccacggc cgcctggccc 10200

gcgtgtacaa caagctgtga taggagctcg atccgtcgac ctgcagatcg ttcaaacatt 10260

tggcaataaa gtttcttaag attgaatcct gttgccggtc ttgcgatgat tatcatataa 10320

tttctgttga attacgttaa gcatgtaata attaacatgt aatgcatgac gttatttatg 10380

agatgggttt ttatgattag agtcccgcaa ttatacattt aatacgcgat agaaaacaaa 10440

atatagcgcg caaactagga taaattatcg cgcgcggtgt catctatgtt actagatcgg 10500

cgcgccgcaa ttgaagtttg ggcggccagc atggccgtat ccgcaatgtg ttattaagtt 10560

gtctaagcgt caatttgttt acaccacaat atatcctgcc accagccagc caacagctcc 10620

ccgaccggca gctcggcaca aaatcaccac tcgatacagg cagcccatca g 10671

<210> 35
<211> 14475
<212> DNA
<213> Artificial Sequence

<220>
<223> Expression cassette of construct 22808

<220>
<221> enhancer
<222> (165)..(256)
<223> eNOS

<220>
<221> promoter
<222> (301)..(2097)
<223> Ubi

<220>
<221> gene
<222> (2116)..(5190)
<223> TNFw

<220>
<221> terminator
<222> (2116)..(5190)
<223> tNOS

<220>

<221>  promoter
<222>  (5483)..(7475)
<223>  Ubi

<220>
<221>  gene
<222>  (7489)..(10563)
<223>  TNRv

<220>
<221>  terminator
<222>  (10570)..(10822)
<223>  tNOS

<220>
<221>  promoter
<222>  (10841)..(12832)
<223>  Ubi

<220>
<221>  gene
<222>  (12849)..(14027)
<223>  PMI

<220>
<221>  terminator
<222>  (14050)..(14302)
<223>  tNOS

<400>  35
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tgaaggcggg aaacgacaat ctgatcatga gcggagaatt     180

aagggagtca cgttatgacc cccgccgatg acgcgggaca agccgtttta cgtttggaac     240

tgacagaacc gcaacgctgc aggaattggc cgcagcggcc atttaaacaa agcttggtac     300

cattatgtgg tctaggtagg ttctatatat aagaaaactt gaaatgttct aaaaaaaaat     360

tcaagcccat gcatgattga agcaaacggt atagcaacgg tgttaacctg atctagtgat     420

ctcttgcaat ccttaacggc cacctaccgc aggtagcaaa cggcgtcccc ctcctcgata     480

tctccgcggc gacctctggc tttttccgcg gaattgcgcg gtggggacgg attccacgag     540

accgcgacgc aaccgcctct cgccgctggg ccccacaccg ctcggtgccg tagcctcacg     600

ggactctttc tccctcctcc cccgttataa attggcttca tcccctcctt gcctcatcca     660

tccaaatccc agtccccaat cccatccctt cgtaggagaa attcatcgaa gctaagcgaa     720

tcctcgcgat cctctcaagg tactgcgagt tttcgatccc cctctcgacc cctcgtatgt     780

ttgtgtttgt cgtagcgttt gattaggtat gctttccctg tttgtgttcg tcgtagcgtt     840

tgattaggta tgctttccct gttcgtgttc atcgtagtgt ttgattaggt cgtgtgaggc     900

gatggcctgc tcgcgtcctt cgatctgtag tcgatttgcg ggtcgtggtg tagatctgcg     960

ggctgtgatg aagttatttg gtgtgatctg ctcgcctgat tctgcgggtt ggctcgagta    1020

gatatgatgg ttggaccggt tggttcgttt accgcgctag ggttgggctg ggatgatgtt 1080

gcatgcgccg ttgcgcgtga tcccgcagca ggacttgcgt ttgattgcca gatctcgtta 1140

cgattatgtg atttggtttg gactttttag atctgtagct tctgcttatg tgccagatgc 1200

gcctactgct catatgcctg atgataatca taaatggctg tggaactaac tagttgattg 1260

cggagtcatg tatcagctac aggtgtaggg actagctaca ggtgtaggga cttgcgtcta 1320

attgtttggt cctttactca tgttgcaatt atgcaattta gtttagattg tttgttccac 1380

tcatctaggc tgtaaaaggg acactgctta gattgctgtt taatcttttt agtagattat 1440

attatattgg taacttatta cccctattac atgccatacg tgacttctgc tcatgcctga 1500

tgataatcat agatcactgt ggaattaatt agttgattgt tgaatcatgt ttcatgtaca 1560

taccacggca caattgctta gttccttaac aaatgcaaat tttactgatc catgtatgat 1620

ttgcgtggtt ctctaatgtg aaatactata gctacttgtt agtaagaatc aggttcgtat 1680

gcttaatgct gtatgtgcct tctgctcatg cctgatgata atcatatatc actggaatta 1740

attagttgat cgtttaatca tatatcaagt acataccatg ccacaatttt tagtcactta 1800

acccatgcag attgaactgg tccctgcatg ttttgctaaa ttgttctatt ctgattagac 1860

catatatcat gtattttttt ttggtaatgg ttctcttatt ttaaatgcta tatagttctg 1920

gtacttgtta gaaagatctg cttcatagtt tagttgccta tccctcgaat taggatgctg 1980

agcagctgat cctatagctt tgtttcatgt atcaattctt ttgtgttcaa cagtcagttt 2040

ttgttagatt cattgtaact tatggtcgct tactcttctg gtcctcaatg cttgcaggat 2100

cgcggccgcg ccaccatggg aaaacctatt cctaatcctc tgctgggcct ggattctacc 2160

ggaggcatgg cccctaagaa aaagcggaag gtggacggcg gagtggacct gagaacactg 2220

ggatattctc agcagcagca ggagaagatc aagcccaagg tgagatctac agtggcccag 2280

caccacgaag ccctggtggg acacggattt acacacgccc acattgtggc cctgtctcag 2340

caccctgccg ccctgggaac agtggccgtg aaatatcagg atatgattgc cgccctgcct 2400

gaggccacac acgaagccat tgtgggagtg ggaaaacagt ggtctggagc cagagccctg 2460

gaagccctgc tgacagtggc cggagaactg agaggacctc ctctgcagct ggatacagga 2520

cagctgctga agattgccaa aaggggcgga gtgaccgcgg tggaagccgt gcacgcctgg 2580

agaaatgccc tgacaggagc ccctctgaac ctgacccccg aacaggtggt ggccattgcc 2640

agccacgacg gcggcaagca ggccctggaa accgtgcaga gactgctgcc cgtgctgtgc 2700

caggcccatg gcctgacacc tgaacaggtg gtggctatcg cctctcacga cggaggaaaa 2760

caggctctgg aaacagtgca gcggctgctg cctgtgctgt gtcaggctca cggcttgact 2820

ccagaacagg tggtggctat tgcttccaat attgggggaa aacaggccct ggaaactgtg 2880

cagcgcctgc tgccagtgct gtgccaggct cacggactga cccccgaaca ggtggtggcc 2940

attgccagca acaacggcgg caagcaggcc ctggaaaccg tgcagagact gctgcccgtg 3000

ctgtgccagg cccatggcct gacacctgaa caggtggtgg ctatcgcctc taacaacgga 3060

ggaaaacagg ctctggaaac agtgcagcgg ctgctgcctg tgctgtgtca ggctcacggc 3120

ttgactccag aacaggtggt ggctattgct tccaacaacg gggggaaaca ggccctggaa 3180

actgtgcagc gcctgctgcc agtgctgtgc caggctcacg ggctgacccc cgaacaggtg 3240

gtggccattg ccagcaacgg cggcggcaag caggccctgg aaaccgtgca gagactgctg 3300

cccgtgctgt gccaggccca tggcctgaca cctgaacagg tggtggctat cgcctctcac 3360

gacggaggaa aacaggctct ggaaacagtg cagcggctgc tgcctgtgct gtgtcaggct 3420

cacggcttga ctccagaaca ggtggtggct attgcttcca atattggggg gaaacaggcc 3480

ctggaaactg tgcagcgcct gctgccagtg ctgtgccagg ctcacggcct cactcccgaa 3540

caggtggtgg ccattgccag caacatcggc ggcaagcagg ccctggaaac cgtgcagaga 3600

ctgctgcccg tgctgtgcca ggcccatggc ctgacacctg aacaggtggt ggctatcgcc 3660

tctcacgacg gaggaaaaca ggctctggaa acagtgcagc ggctgctgcc tgtgctgtgt 3720

caggctcacg gcttgactcc agaacaggtg gtggctattg cttccaacaa cggggggaaa 3780

caggccctgg aaactgtgca gcgcctgctg ccagtgctgt gccaggctca cggactgacc 3840

cccgaacagg tggtggccat tgccagcaac ggcggcggca agcaggccct ggaaaccgtg 3900

cagagactgc tgcccgtgct gtgccaggcc catggcctga cacctgaaca ggtggtggct 3960

atcgcctcta acaacggagg aaaacaagca ctcgagacag tgcagcggct gctgcctgtg 4020

ctgtgtcagg ctcacggctt gactccagaa caggtggtgg ctattgcttc caacaacggg 4080

gggaaacagg ccctggaaac tgtgcagcgc ctgctgccag tgctgtgcca ggctcacggg 4140

ctgacccccg aacaggtggt ggccattgcc agcaacatcg gcggcaagca ggccctggaa 4200

accgtgcaga gactgctgcc cgtgctgtgc caggcccatg gcctgacacc tgaacaggtg 4260

gtggctatcg cctctaacaa cggaggaaaa caggctctgg aaacagtgca gcggctgctg 4320

cctgtgctgt gtcaggctca cggcttgact ccacagcagg tcgtggcaat tgctagcaat 4380

atcggcggac ggcccgccct ggagagcatt gtggcccagc tgtctagacc tgatcctgcc 4440

ctggccgccc tgacaaatga tcacctggtg gccctggcct gtctgggagg cagacctgcc 4500

ctggatgccg tgaaaaaagg actgcctcac gcccctgccc tgattaaaag aacaaataga 4560

agaatccccg agcggacctc tcacagagtg gccggatccc agctggtgaa atctgagctg 4620

gaggagaaga agtctgagct gagacacaag ctgaagtacg tgcctcacga gtacatcgag 4680

ctgatcgaga tcgccagaaa tagcacccag gatagaatcc tggagatgaa ggtgatggag 4740

ttcttcatga aagtgtacgg ctacagagga aagcatctgg gaggaagcag aaaacctgac 4800

ggagccattt atacagtggg cagccctatc gattatggcg tgatcgtgga tacaaaggcc 4860

tacagcggag gctacaatct gcctattgga caggccgatg agatgcagag atacgtggag 4920

gagaaccaaa ccaggaacaa gcatatcaac cctaacgagt ggtggaaggt gtacccttct 4980

agcgtgaccg agttcaagtt cctgtttgtg agcggccact tcaagggcaa ttataaggcc 5040

cagctgacca ggctgaacca catcacaaat tgtaatggcg ccgtgctgtc tgtggaggaa 5100

ctgctgattg gaggagagat gattaaggcc ggaacactga cactggagga ggtgagaaga 5160

aagttcaaca acggcgagat caacttctga aagcttgatc gttcaaacat ttggcaataa 5220

agtttcttaa gattgaatcc tgttgccggt cttgcgatga ttatcatata atttctgttg 5280

aattacgtta agcatgtaat aattaacatg taatgcatga cgttatttat gagatgggtt 5340

tttatgatta gagtcccgca attatacatt taatacgcga tagaaaacaa aatatagcgc 5400

gcaaactagg ataaattatc gcgcgcggtg tcatctatgt tactagatct tcgaacccta 5460

gtcgaagaca accggtgcat gcctgcagtg cagcgtgacc cggtcgtgcc cctctctaga 5520

gataatgagc attgcatgtc taagttataa aaaattacca catatttttt ttgtcacact 5580

tgtttgaagt gcagtttatc tatctttata catatattta aactttactc tacgaataat 5640

ataatctata gtactacaat aatatcagtg ttttagagaa tcatataaat gaacagttag 5700

acatggtcta aaggacaatt gagtattttg acaacaggac tctacagttt tatcttttta 5760

gtgtgcatgt gttctccttt ttttttgcaa atagcttcac ctatataata cttcatccat 5820

tttattagta catccattta gggtttaggg ttaatggttt ttatagacta atttttttag 5880

tacatctatt ttattctatt ttagcctcta aattaagaaa actaaaactc tattttagtt 5940

tttttattta ataatttaga tataaaatag aataaaataa agtgactaaa aattaaacaa 6000

ataccettta agaaattaaa aaaactaagg aaacattttt cttgtttcga gtagataatg 6060

ccagcctgtt aaacgccgtc gacgagtcta acggacacca accagcgaac cagcagcgtc 6120

gcgtcgggcc aagcgaagca gacggcacgg catctctgtc gctgcctctg gacccctctc 6180

gagagttccg ctccaccgtt ggacttgctc cgctgtcggc atccagaaat tgcgtggcgg 6240

agcggcagac gtgagccggc acggcaggcg gcctcctcct cctctcacgg caccggcagc 6300

tacgggggat tcctttccca ccgctccttc gctttccctt cctcgcccgc cgtaataaat 6360

agacaccccc tccacaccct ctttccccaa cctcgtgttg ttcggagcgc acacacacac 6420

aaccagatct cccccaaatc cacccgtcgg cacctccgct tcaaggtacg ccgctcgtcc 6480

tccccccccc cccctctcta ccttctctag atcggcgttc cggtccatgg ttagggcccg 6540

gtagttctac ttctgttcat gtttgtgtta gatccgtgtt tgtgttagat ccgtgctgct 6600

agcgttcgta cacggatgcg acctgtacgt cagacacgtt ctgattgcta acttgccagt 6660

gtttctcttt ggggaatcct gggatggctc tagccgttcc gcagacggga tcgatttcat 6720
gatttttttt gtttcgttgc atagggtttg gtttgccctt ttcctttatt tcaatatatg 6780
ccgtgcactt gtttgtcggg tcatcttttc atgctttttt ttgtcttggt tgtgatgatg 6840
tggtctggtt gggcggtcgt tctagatcgg agtagaattc tgtttcaaac tacctggtgg 6900
atttattaat tttggatctg tatgtgtgtg ccatacatat tcatagttac gaattgaaga 6960
tgatggatgg aaatatcgat ctaggatagg tatacatgtt gatgcgggtt ttactgatgc 7020
atatacagag atgctttttg ttcgcttggt tgtgatgatg tggtgtggtt gggcggtcgt 7080
tcattcgttc tagatcggag tagaatactg tttcaaacta cctggtgtat ttattaattt 7140
tggaactgta tgtgtgtgtc atacatcttc atagttacga gtttaagatg gatggaaata 7200
tcgatctagg ataggtatac atgttgatgt gggttttact gatgcatata catgatggca 7260
tatgcagcat ctattcatat gctctaacct tgagtaccta tctattataa taaacaagta 7320
tgttttataa ttattttgat cttgatatac ttggatgatg gcatatgcag cagctatatg 7380
tggatttttt tagccctgcc ttcatacgct atttatttgc ttggtactgt ttcttttgtc 7440
gatgctcacc ctgttgtttg gtgttacttc tgcagcggcc gcgccaccat gggaaaacct 7500
attcctaatc ctctgctggg cctggattct accggaggca tggcccctaa gaaaaagcgg 7560
aaggtggacg gcggagtgga cctgagaaca ctgggatatt ctcagcagca gcaggagaag 7620
atcaagccca aggtgagatc tacagtggcc cagcaccacg aagccctggt gggacacgga 7680
tttacacacg cccacattgt ggccctgtct cagcaccctg ccgccctggg aacagtggcc 7740
gtgaaatatc aggatatgat tgccgccctg cctgaggcca cacacgaagc cattgtggga 7800
gtgggaaaac agtggtctgg agccagagcc ctggaagccc tgctgacagt ggccggagaa 7860
ctgagaggac ctcctctgca gctggataca ggacagctgc tgaagattgc caaaaggggc 7920
ggagtgaccg cggtggaagc cgtgcacgcc tggagaaatg ccctgacagg agcccctctg 7980
aacctgaccc ccgaacaggt ggtggccatt gccagcaaca acggcggcaa gcaggccctg 8040
gaaaccgtgc agagactgct gcccgtgctg tgccaggccc atggcctgac acctgaacag 8100
gtggtggcta tcgcctctca cgacggagga aaacaggctc tggaaacagt gcagcggctg 8160
ctgcctgtgc tgtgtcaggc tcacggcttg actccagaac aggtggtggc tattgcttcc 8220
aacggcgggg ggaaacaggc cctggaaact gtgcagcgcc tgctgccagt gctgtgccag 8280
gctcacggac tgacccccga acaggtggtg gccattgcca gcaacggcgg cggcaagcag 8340
gccctggaaa ccgtgcagag actgctgccc gtgctgtgcc aggcccatgg cctgacacct 8400
gaacaggtgg tggctatcgc ctctcacgac ggaggaaaac aggctctgga aacagtgcag 8460
cggctgctgc ctgtgctgtg tcaggctcac ggcttgactc cagaacaggt ggtggctatt 8520
gcttcccacg acggggggaa acaggccctg gaaactgtgc agcgcctgct gccagtgctg 8580

tgccaggctc acgggctgac ccccgaacag gtggtggcca ttgccagcaa cggcggcggc 8640

aagcaggccc tggaaaccgt gcagagactg ctgcccgtgc tgtgccaggc ccatggcctg 8700

acacctgaac aggtggtggc tatcgcctct aacggcggag gaaaacaggc tctggaaaca 8760

gtgcagcggc tgctgcctgt gctgtgtcag gctcacggct tgactccaga acaggtggtg 8820

gctattgctt cccacgacgg ggggaaacag gccctggaaa ctgtgcagcg cctgctgcca 8880

gtgctgtgcc aggctcacgg cctcactccc gaacaggtgg tggccattgc cagcaacaac 8940

ggcggcaagc aggccctgga aaccgtgcag agactgctgc ccgtgctgtg ccaggcccat 9000

ggcctgacac ctgaacaggt ggtggctatc gcctctcacg acggaggaaa acaggctctg 9060

gaaacagtgc agcggctgct gcctgtgctg tgtcaggctc acggcttgac tccagaacag 9120

gtggtggcta ttgcttccca cgacgggggg aaacaggccc tggaaactgt gcagcgcctg 9180

ctgccagtgc tgtgccaggc tcacggactg acccccgaac aggtggtggc cattgccagc 9240

aacatcggcg gcaagcaggc cctggaaacc gtgcagagac tgctgcccgt gctgtgccag 9300

gcccatggcc tgacacctga acaggtggtg gctatcgcct ctaacaacgg aggaaaacaa 9360

gcactcgaga cagtgcagcg gctgctgcct gtgctgtgtc aggctcacgg cttgactcca 9420

gaacaggtgg tggctattgc ttccaacggc ggggggaaac aggccctgga aactgtgcag 9480

cgcctgctgc cagtgctgtg ccaggctcac gggctgaccc ccgaacaggt ggtggccatt 9540

gccagccacg acggcggcaa gcaggccctg gaaaccgtgc agagactgct gcccgtgctg 9600

tgccaggccc atggcctgac acctgaacag gtggtggcta tcgcctctaa tatcggagga 9660

aaacaggctc tggaaacagt gcagcggctg ctgcctgtgc tgtgtcaggc tcacggcttg 9720

actccacagc aggtcgtggc aattgctagc cacgacggcg gacggcccgc cctggagagc 9780

attgtggccc agctgtctag acctgatcct gccctggccg ccctgacaaa tgatcacctg 9840

gtggccctgg cctgtctggg aggcagacct gccctggatg ccgtgaaaaa aggactgcct 9900

cacgcccctg ccctgattaa aagaacaaat agaagaatcc ccgagcggac ctctcacaga 9960

gtggccggat cccagctggt gaaatctgag ctggaggaga agaagtctga gctgagacac 10020

aagctgaagt acgtgcctca cgagtacatc gagctgatcg agatcgccag aaatagcacc 10080

caggatagaa tcctggagat gaaggtgatg gagttcttca tgaaagtgta cggctacaga 10140

ggaaagcatc tgggaggaag cagaaaacct gacggagcca tttatacagt gggcagccct 10200

atcgattatg gcgtgatcgt ggatacaaag gcctacagcg gaggctacaa tctgcctatt 10260

ggacaggccg atgagatgca gagatacgtg gaggagaacc aaaccaggaa caagcatatc 10320

aaccctaacg agtggtggaa ggtgtaccct tctagcgtga ccgagttcaa gttcctgttt 10380

gtgagcggcc acttcaaggg caattataag gcccagctga ccaggctgaa ccacatcaca 10440

aattgtaatg gcgccgtgct gtctgtggag gaactgctga ttggaggaga gatgattaag 10500

gccggaacac tgacactgga ggaggtgaga agaaagttca acaacggcga gatcaacttc 10560

tgaaagcttg atcgttcaaa catttggcaa taaagtttct taagattgaa tcctgttgcc 10620

ggtcttgcga tgattatcat ataatttctg ttgaattacg ttaagcatgt aataattaac 10680

atgtaatgca tgacgttatt tatgagatgg gtttttatga ttagagtccc gcaattatac 10740

atttaatacg cgatagaaaa caaaatatag cgcgcaaact aggataaatt atcgcgcgcg 10800

gtgtcatcta tgttactaga tcttcgaaga cggaccgcgc ctgcagtgca gcgtgacccg 10860

gtcgtgcccc tctctagaga taatgagcat tgcatgtcta agttataaaa aattaccaca 10920

tatttttttt gtcacacttg tttgaagtgc agtttatcta tctttataca tatatttaaa 10980

ctttactcta cgaataatat aatctatagt actacaataa tatcagtgtt ttagagaatc 11040

atataaatga acagttagac atggtctaaa ggacaattga gtattttgac aacaggactc 11100

tacagtttta tctttttagt gtgcatgtgt tctccttttt ttttgcaaat agcttcacct 11160

atataatact tcatccattt tattagtaca tccatttagg gtttagggtt aatggttttt 11220

atagactaat ttttttagta catctatttt attctatttt agcctctaaa ttaagaaaac 11280

taaaactcta ttttagtttt tttatttaat aatttagata taaaatagaa taaaataaag 11340

tgactaaaaa ttaaacaaat accctttaag aaattaaaaa aactaaggaa acatttttct 11400

tgtttcgagt agataatgcc agcctgttaa acgccgtcga cgagtctaac ggacaccaac 11460

cagcgaacca gcagcgtcgc gtcgggccaa gcgaagcaga cggcacggca tctctgtcgc 11520

tgcctctgga cccctctcga gagttccgct ccaccgttgg acttgctccg ctgtcggcat 11580

ccagaaattg cgtggcggag cggcagacgt gagccggcac ggcaggcggc ctcctcctcc 11640

tctcacggca ccggcagcta cgggggattc ctttcccacc gctccttcgc tttcccttcc 11700

tcgcccgccg taataaatag acaccccctc cacaccctct ttccccaacc tcgtgttgtt 11760

cggagcgcac acacacacaa ccagatctcc cccaaatcca cccgtcggca cctccgcttc 11820

aaggtacgcc gctcgtcctc cccccccccc ctctctacct tctctagatc ggcgttccgg 11880

tccatggtta gggcccggta gttctacttc tgttcatgtt tgtgttagat ccgtgtttgt 11940

gttagatccg tgctgctagc gttcgtacac ggatgcgacc tgtacgtcag acacgttctg 12000

attgctaact tgccagtgtt tctctttggg gaatcctggg atggctctag ccgttccgca 12060

gacgggatcg atttcatgat tttttttgtt tcgttgcata gggtttggtt tgcccttttc 12120

ctttatttca atatatgccg tgcacttgtt tgtcgggtca tcttttcatg cttttttttg 12180

tcttggttgt gatgatgtgg tctggttggg cggtcgttct agatcggagt agaattctgt 12240

ttcaaactac ctggtggatt tattaatttt ggatctgtat gtgtgtgcca tacatattca 12300

tagttacgaa ttgaagatga tggatggaaa tatcgatcta ggataggtat acatgttgat 12360

gcgggtttta ctgatgcata tacagagatg ctttttgttc gcttggttgt gatgatgtgg 12420

tgtggttggg cggtcgttca ttcgttctag atcggagtag aatactgttt caaactacct 12480

ggtgtattta ttaattttgg aactgtatgt gtgtgtcata catcttcata gttacgagtt 12540

taagatggat ggaaatatcg atctaggata ggtatacatg ttgatgtggg ttttactgat 12600

gcatatacat gatggcatat gcagcatcta ttcatatgct ctaaccttga gtacctatct 12660

attataataa acaagtatgt tttataatta ttttgatctt gatatacttg gatgatggca 12720

tatgcagcag ctatatgtgg atttttttag ccctgccttc atacgctatt tatttgcttg 12780

gtactgtttc ttttgtcgat gctcaccctg ttgtttggtg ttacttctgc agggatccgg 12840

cagcagccat gcagaagctg atcaacagcg tgcagaacta cgcctggggc agcaagaccg 12900

ccctgaccga gctgtacggc atggagaacc ccagcagcca gcccatggcc gagctgtgga 12960

tgggcgccca ccccaagagc agcagccgcg tgcagaacgc cgccggcgac atcgtgagcc 13020

tgcgcgacgt gatcgagagc gacaagagca ccctgctggg cgaggccgtg gccaagcgct 13080

tcggcgagct gcccttcctg ttcaaggtgc tgtgcgccgc ccagcccctg agcatccagg 13140

tgcaccccaa caagcacaac agcgagatcg gcttcgccaa ggagaacgcc gccggcatcc 13200

ccatggacgc cgccgagcgc aactacaagg accccaacca caagcccgag ctggtgttcg 13260

ccctgacccc cttcctggcc atgaacgcct tccgcgagtt cagcgagatc gtgagcctgc 13320

tgcagcccgt ggccggcgcc caccccgcca tcgcccactt cctgcagcag cccgacgccg 13380

agcgcctgag cgagctgttc gccagcctgc tgaacatgca gggcgaggag aagagccgcg 13440

ccctggccat cctgaagagc gccctggaca gccagcaggg cgagccctgg cagaccatcc 13500

gcctgatcag cgagttctac cccgaggaca gcggcctgtt cagccccctg ctgctgaacg 13560

tggtgaagct gaaccccggc gaggccatgt tcctgttcgc cgagaccccc cacgcctacc 13620

tgcagggcgt ggccctggag gtgatggcca acagcgacaa cgtgctgcgc gccggcctga 13680

cccccaagta catcgacatc cccgagctgg tggccaacgt gaagttcgag gccaagcccg 13740

ccaaccagct gctgacccag cccgtgaagc agggcgccga gctggacttc cccatccccg 13800

tggacgactt cgccttcagc ctgcacgacc tgagcgacaa ggagaccacc atcagccagc 13860

agagcgccgc catcctgttc tgcgtggagg gcgacgccac cctgtggaag ggcagccagc 13920

agctgcagct gaagcccggc gagagcgcct tcatcgccgc caacgagagc cccgtgaccg 13980

tgaagggcca cggccgcctg gcccgcgtgt acaacaagct gtgataggag ctcgatccgt 14040

cgacctgcag atcgttcaaa catttggcaa taaagtttct taagattgaa tcctgttgcc 14100

ggtcttgcga tgattatcat ataatttctg ttgaattacg ttaagcatgt aataattaac 14160

atgtaatgca tgacgttatt tatgagatgg gtttttatga ttagagtccc gcaattatac 14220

atttaatacg cgatagaaaa caaaatatag cgcgcaaact aggataaatt atcgcgcgcg     14280

gtgtcatcta tgttactaga tcggcgcgcc gcaattgaag tttgggcggc cagcatggcc     14340

gtatccgcaa tgtgttatta agttgtctaa gcgtcaattt gtttacacca caatatatcc     14400

tgccaccagc cagccaacag ctccccgacc ggcagctcgg cacaaaatca ccactcgata     14460

caggcagccc atcag                                                      14475


<210>  36
<211>  10668
<212>  DNA
<213>  Artificial Sequence

<220>
<223>  Expression cassette of construct 22873


<220>
<221>  enhancer
<222>  (165)..(256)
<223>  eNOS

<220>
<221>  promoter
<222>  (301)..(2097)
<223>  Ubi

<220>
<221>  gene
<222>  (2114)..(6280)
<223>  Cas9m

<220>
<221>  misc_feature
<222>  (6215)..(6271)
<223>  NLS-TAG

<220>
<221>  terminator
<222>  (6286)..(6538)
<223>  tNOS

<220>
<221>  promoter
<222>  (6545)..(6919)
<223>  pU3

<220>
<221>  misc_feature
<222>  (6921)..(6938)

<220>
<221>  misc_feature
<222>  (6921)..(7023)
<223>  sgRNA

<220>
<221>  promoter
<222>  (7034)..(9025)
<223>  Ubi

<220>
<221>  gene
<222>  (9042)..(10220)
<223>  PMI

<220>
<221>  terminator
<222>  (10243)..(10495)
<223>  tNOS

<400>  36
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tgaaggcggg aaacgacaat ctgatcatga gcggagaatt     180

aagggagtca cgttatgacc cccgccgatg acgcgggaca agccgtttta cgtttggaac     240

tgacagaacc gcaacgctgc aggaattggc cgcagcggcc atttaaacaa agcttggtac     300

cattatgtgg tctaggtagg ttctatatat aagaaaactt gaaatgttct aaaaaaaaat     360

tcaagcccat gcatgattga agcaaacggt atagcaacgg tgttaacctg atctagtgat     420

ctcttgcaat ccttaacggc cacctaccgc aggtagcaaa cggcgtcccc ctcctcgata     480

tctccgcggc gacctctggc tttttccgcg gaattgcgcg gtggggacgg attccacgag     540

accgcgacgc aaccgcctct cgccgctggg ccccacaccg ctcggtgccg tagcctcacg     600

ggactctttc tccctcctcc cccgttataa attggcttca tcccctcctt gcctcatcca     660

tccaaatccc agtccccaat cccatccctt cgtaggagaa attcatcgaa gctaagcgaa     720

tcctcgcgat cctctcaagg tactgcgagt tttcgatccc cctctcgacc cctcgtatgt     780

ttgtgtttgt cgtagcgttt gattaggtat gctttccctg tttgtgttcg tcgtagcgtt     840

tgattaggta tgctttccct gttcgtgttc atcgtagtgt ttgattaggt cgtgtgaggc     900

gatggcctgc tcgcgtcctt cgatctgtag tcgatttgcg ggtcgtggtg tagatctgcg     960

ggctgtgatg aagttatttg gtgtgatctg ctcgcctgat tctgcgggtt ggctcgagta    1020

gatatgatgg ttggaccggt tggttcgttt accgcgctag ggttgggctg ggatgatgtt    1080

gcatgcgccg ttgcgcgtga tcccgcagca ggacttgcgt ttgattgcca gatctcgtta    1140

cgattatgtg atttggtttg gactttttag atctgtagct tctgcttatg tgccagatgc    1200

gcctactgct catatgcctg atgataatca taaatggctg tggaactaac tagttgattg    1260

cggagtcatg tatcagctac aggtgtaggg actagctaca ggtgtaggga cttgcgtcta    1320

attgtttggt cctttactca tgttgcaatt atgcaattta gtttagattg tttgttccac    1380

tcatctaggc tgtaaaaggg acactgctta gattgctgtt taatcttttt agtagattat    1440

attatattgg taacttatta cccctattac atgccatacg tgacttctgc tcatgcctga    1500

tgataatcat agatcactgt ggaattaatt agttgattgt tgaatcatgt ttcatgtaca    1560

taccacggca caattgctta gttccttaac aaatgcaaat tttactgatc catgtatgat 1620

ttgcgtggtt ctctaatgtg aaatactata gctacttgtt agtaagaatc aggttcgtat 1680

gcttaatgct gtatgtgcct tctgctcatg cctgatgata atcatatatc actggaatta 1740

attagttgat cgtttaatca tatatcaagt acataccatg ccacaatttt tagtcactta 1800

acccatgcag attgaactgg tccctgcatg ttttgctaaa ttgttctatt ctgattagac 1860

catatatcat gtattttttt ttggtaatgg ttctcttatt ttaaatgcta tatagttctg 1920

gtacttgtta gaaagatctg cttcatagtt tagttgccta tccctcgaat taggatgctg 1980

agcagctgat cctatagctt tgtttcatgt atcaattctt ttgtgttcaa cagtcagttt 2040

ttgttagatt cattgtaact tatggtcgct tactcttctg gtcctcaatg cttgcaggat 2100

cgcggccgct aaaatggata agaagtactc tattggcctc gatattggaa ccaactctgt 2160

gggctgggcc gtgatcaccg atgagtacaa ggtgccatct aagaagttca aggtgctcgg 2220

caacaccgat aggcactcta tcaagaagaa cctcatcggt gccctcctct tcgattctgg 2280

cgagaccgcc gaggccacca ggctcaagag gaccgccagg aggaggtaca ccaggaggaa 2340

gaacaggatc tgctacctcc aggagatctt ctctaacgag atggccaagg tggatgattc 2400

tttcttccac aggctcgagg agtctttcct cgtggaggag gataagaagc acgagaggca 2460

cccaatcttc ggcaacatcg tggatgaggt ggcctaccac gagaagtacc caaccatcta 2520

ccacctcagg aagaagctcg tggattctac cgataaggcc gatctcaggc tcatctacct 2580

cgccctcgcc cacatgatca agttcagggg ccacttcctc atcgagggcg atctcaaccc 2640

agataactct gatgtggata agctcttcat ccagctcgtg cagacctaca accagctctt 2700

cgaggagaac ccaatcaacg cctctggcgt ggatgccaag gccatcctct ctgccaggct 2760

ctctaagtct aggaggctcg agaacctcat cgcccagctc ccaggcgaga agaagaacgg 2820

cctcttcggc aacctcatcg ccctctctct cggcctcacc ccaaacttca agtctaactt 2880

cgatctcgcc gaggatgcca agctccagct ctctaaggat acctacgatg atgatctcga 2940

taacctcctc gcccagatcg gcgatcagta cgccgatctc ttcctcgccg ccaagaacct 3000

ctctgatgcc atcctcctct ctgatatcct cagggtgaac accgagatca ccaaggcccc 3060

actctctgcc tctatgatca agaggtacga tgagcaccac caggatctca ccctcctcaa 3120

ggccctcgtg aggcagcagc tcccagagaa gtacaaggag atcttcttcg atcagtctaa 3180

gaacggctac gccggctaca tcgatggcgg cgcctctcag gaggagttct acaagttcat 3240

caagccaatc ctcgagaaga tggatggcac cgaggagctc ctcgtgaagc tcaacaggga 3300

ggatctcctc aggaagcaga ggaccttcga taacggctct atcccacacc agatccacct 3360

cggcgagctc cacgccatcc tcaggaggca ggaggatttc tacccattcc tcaaggataa 3420

cagggagaag atcgagaaga tcctcacctt ccgcatccca tactacgtgg gcccactcgc 3480

caggggcaac tctaggttcg cctggatgac caggaagtct gaggagacca tcaccccttg 3540

gaacttcgag gaggtggtgg ataagggcgc ctctgcccag tctttcatcg agaggatgac 3600

caacttcgat aagaacctcc caaacgagaa ggtgctccca aagcactctc tcctctacga 3660

gtacttcacc gtgtacaacg agctcaccaa ggtgaagtac gtgaccgagg gcatgaggaa 3720

gccagccttc ctctctggcg agcagaagaa ggccatcgtg gatctcctct tcaagaccaa 3780

caggaaggtg accgtgaagc agctcaagga ggattacttc aagaagatcg agtgcttcga 3840

ttctgtggag atctctggcg tggaggatag gttcaacgcc tctctcggca cctaccacga 3900

tctcctcaag atcatcaagg ataaggattt cctcgataac gaggagaacg aggatatcct 3960

cgaggatatc gtgctcaccc tcaccctctt cgaggatagg gagatgatcg aggagaggct 4020

caagacctac gcccacctct tcgatgataa ggtgatgaag cagctcaaga ggaggaggta 4080

caccggctgg ggcaggctct ctaggaagct catcaacggc atcagggata agcagtctgg 4140

caagaccatc ctcgatttcc tcaagtctga tggcttcgcc aacaggaact tcatgcagct 4200

catccacgat gattctctca ccttcaagga ggatatccag aaggctcagg tgtctggcca 4260

gggccactct ctccacgagc agatcgccaa cctcgccggc tctccagcca tcaagaaggg 4320

catcctccag accgtgaagg tggtggatga gctcgtgaag gtgatgggcc acaagccaga 4380

gaacatcgtg atcgagatgg ccagggagaa ccagaccacc cagaagggcc agaagaactc 4440

tagggagagg atgaagagga tcgaggaggg catcaaggag ctcggctctc agatcctcaa 4500

ggagcaccca gtggagaaca cccagctcca gaacgagaag ctctacctct actacctcca 4560

gaacggcagg gatatgtacg tggatcagga gctcgatatc aacaggctct ctgattacga 4620

tgtggatcac atcgtgccac agtctttcct caaggatgat tctatcgata acaaggtgct 4680

caccaggtct gataagaaca ggggcaagtc tgataacgtg ccatctgagg aggtggttaa 4740

gaagatgaag aactactgga ggcagctcct caacgccaag ctcatcaccc agaggaagtt 4800

cgataacctc accaaggccg agaggggcgg cctctctgag ctcgataagg ccggcttcat 4860

caagaggcag ctcgtggaga ccaggcagat cactaagcac gtggcccaga tcctcgattc 4920

taggatgaac accaagtacg atgagaacga taagctcatc agggaggtga aggtgatcac 4980

cctcaagtct aagctcgtgt ctgatttcag gaaggatttc cagttctaca aggtgaggga 5040

gatcaacaac taccaccacg cccacgatgc ctacctcaac gccgtggtgg gcaccgccct 5100

catcaagaag tacccaaaac tcgagtctga gttcgtgtac ggcgattaca aggtgtacga 5160

tgtgaggaag atgatcgcca agtctgagca ggagatcggc aaggccaccg ccaagtactt 5220

cttctactct aacatcatga acttcttcaa gaccgagatc accctcgcca acggcgagat 5280

caggaagagg ccactcatcg agaccaacgg cgagaccggc gagatcgtgt gggataaggg 5340

cagggatttc gccaccgtga ggaaggtgct ctctatgcca caggtgaaca tcgtgaagaa 5400

gaccgaggtg cagaccggcg gcttctctaa ggagtctatc ctcccaaaga ggaactctga 5460

taagctcatc gccaggaaga aggattggga cccgaagaag tacggcggct tcgacagccc 5520

gaccgtggcc tacagcgtgc tggtggtggc caaggtggag aagggcaaga gcaagaagct 5580

gaagagcgtg aaggagctgg tgggcatcac catcatggag aggagcagct tcgagaagaa 5640

cccagtggac ttcctggagg ccaagggcta caaggaggtg aagaaggacc tgatcattaa 5700

actgccgaag tacagcctgt tcgagctgga gaacggcagg aagaggatgc tggccagcgc 5760

cggcgagctg cagaagggca acgagctggc cctgccgagc aagtacgtga acttcctgta 5820

cctggccagc cactacgaga agctgaaggg cagcccggag gacaacgagc agaagcagct 5880

gttcgtggag cagcacaagc actacctgga cgagatcatc gagcagatca gcgagttcag 5940

caagagggtg atcctggccg acgccaacct ggacaaggtg ctgagcgcct acaacaagca 6000

cagggacaag ccgatcaggg agcaggccga gaacatcatc cacctgttca ccctgaccaa 6060

cctgggcgcc ccggccgcct tcaagtactt cgacaccacc atcgacagga agaggtacac 6120

cagcaccaag gaggtgctgg acgccaccct gatccaccag agcatcaccg gcctgtacga 6180

gaccaggatc gacctgagcc agctgggcgg cgacagcagc ccgccgaaga agaagaggaa 6240

ggtgagctgg aaggacgcca gcggctggag caggatgtga agcttgatcg ttcaaacatt 6300

tggcaataaa gtttcttaag attgaatcct gttgccggtc ttgcgatgat tatcatataa 6360

tttctgttga attacgttaa gcatgtaata attaacatgt aatgcatgac gttatttatg 6420

agatgggttt ttatgattag agtcccgcaa ttatacattt aatacgcgat agaaaacaaa 6480

atatagcgcg caaactagga taaattatcg cgcgcggtgt catctatgtt actagatctt 6540

cgaagggatc tttaaacata cgaacagatc acttaaagtt cttctgaagc aacttaaagt 6600

tatcaggcat gcatggatct tggaggaatc agatgtgcag tcagggacca tagcacagga 6660

caggcgtctt ctactggtgc taccagcaaa tgctggaagc cgggaacact gggtacgttg 6720

gaaaccacgt gatgtggagt aagataaact gtaggagaaa agcatttcgt agtgggccat 6780

gaagcctttc aggacatgta ttgcagtatg ggccggccca ttacgcaatt ggacgacaac 6840

aaagactagt attagtacca cctcggctat ccacatagat caaagctggt ttaaaagagt 6900

tgtgcagatg atccgtggca gtcaacgtgg agacaggggt tttagagcta gaaatagcaa 6960

gttaaaataa ggctagtccg ttatcaactt gaaaaagtgg caccgagtcg gtgctttttt 7020

tttcggaccg cgcctgcagt gcagcgtgac ccggtcgtgc ccctctctag agataatgag 7080

cattgcatgt ctaagttata aaaaattacc acatattttt tttgtcacac ttgtttgaag 7140

tgcagtttat ctatctttat acatatattt aaactttact ctacgaataa tataatctat 7200

agtactacaa taatatcagt gttttagaga atcatataaa tgaacagtta gacatggtct 7260

aaaggacaat tgagtatttt gacaacagga ctctacagtt ttatcttttt agtgtgcatg 7320

tgttctcctt tttttttgca aatagcttca cctatataat acttcatcca ttttattagt 7380

acatccattt agggtttagg gttaatggtt tttatagact aattttttta gtacatctat 7440

tttattctat tttagcctct aaattaagaa aactaaaact ctattttagt ttttttattt 7500

aataatttag atataaaata gaataaaata aagtgactaa aaattaaaca aatacccttt 7560

aagaaattaa aaaaactaag gaaacatttt tcttgtttcg agtagataat gccagcctgt 7620

taaacgccgt cgacgagtct aacggacacc aaccagcgaa ccagcagcgt cgcgtcgggc 7680

caagcgaagc agacggcacg gcatctctgt cgctgcctct ggacccctct cgagagttcc 7740

gctccaccgt tggacttgct ccgctgtcgg catccagaaa ttgcgtggcg gagcggcaga 7800

cgtgagccgg cacggcaggc ggcctcctcc tcctctcacg gcaccggcag ctacggggga 7860

ttcctttccc accgctcctt cgctttccct tcctcgcccg ccgtaataaa tagacacccc 7920

ctccacaccc tctttcccca acctcgtgtt gttcggagcg cacacacaca caaccagatc 7980

tcccccaaat ccacccgtcg gcacctccgc ttcaaggtac gccgctcgtc ctcccccccc 8040

cccctctcta ccttctctag atcggcgttc cggtccatgg ttagggcccg gtagttctac 8100

ttctgttcat gtttgtgtta gatccgtgtt tgtgttagat ccgtgctgct agcgttcgta 8160

cacggatgcg acctgtacgt cagacacgtt ctgattgcta acttgccagt gtttctcttt 8220

ggggaatcct gggatggctc tagccgttcc gcagacggga tcgatttcat gatttttttt 8280

gtttcgttgc atagggtttg gtttgccctt ttcctttatt tcaatatatg ccgtgcactt 8340

gtttgtcggg tcatcttttc atgctttttt ttgtcttggt tgtgatgatg tggtctggtt 8400

gggcggtcgt tctagatcgg agtagaattc tgtttcaaac tacctggtgg atttattaat 8460

tttggatctg tatgtgtgtg ccatacatat tcatagttac gaattgaaga tgatggatgg 8520

aaatatcgat ctaggatagg tatacatgtt gatgcgggtt ttactgatgc atatacagag 8580

atgctttttg ttcgcttggt tgtgatgatg tggtgtggtt gggcggtcgt tcattcgttc 8640

tagatcggag tagaatactg tttcaaacta cctggtgtat ttattaattt tggaactgta 8700

tgtgtgtgtc atacatcttc atagttacga gtttaagatg gatggaaata tcgatctagg 8760

ataggtatac atgttgatgt gggttttact gatgcatata catgatggca tatgcagcat 8820

ctattcatat gctctaacct tgagtaccta tctattataa taaacaagta tgttttataa 8880

ttattttgat cttgatatac ttggatgatg gcatatgcag cagctatatg tggatttttt 8940

tagccctgcc ttcatacgct atttatttgc ttggtactgt ttcttttgtc gatgctcacc 9000

ctgttgtttg gtgttacttc tgcagggatc cggcagcagc catgcagaag ctgatcaaca 9060

gcgtgcagaa ctacgcctgg ggcagcaaga ccgccctgac cgagctgtac ggcatggaga 9120

accccagcag ccagcccatg gccgagctgt ggatgggcgc ccaccccaag agcagcagcc 9180

gcgtgcagaa cgccgccggc gacatcgtga gcctgcgcga cgtgatcgag agcgacaaga 9240

gcaccctgct gggcgaggcc gtggccaagc gcttcggcga gctgcccttc ctgttcaagg 9300

tgctgtgcgc cgcccagccc ctgagcatcc aggtgcaccc caacaagcac aacagcgaga 9360

tcggcttcgc caaggagaac gccgccggca tccccatgga cgccgccgag cgcaactaca 9420

aggaccccaa ccacaagccc gagctggtgt tcgccctgac ccccttcctg gccatgaacg 9480

ccttccgcga gttcagcgag atcgtgagcc tgctgcagcc cgtggccggc gcccaccccg 9540

ccatcgccca cttcctgcag cagcccgacg ccgagcgcct gagcgagctg ttcgccagcc 9600

tgctgaacat gcagggcgag gagaagagcc gcgccctggc catcctgaag agcgccctgg 9660

acagccagca gggcgagccc tggcagacca tccgcctgat cagcgagttc taccccgagg 9720

acagcggcct gttcagcccc ctgctgctga acgtggtgaa gctgaacccc ggcgaggcca 9780

tgttcctgtt cgccgagacc ccccacgcct acctgcaggg cgtggccctg gaggtgatgg 9840

ccaacagcga caacgtgctg cgcgccggcc tgacccccaa gtacatcgac atccccgagc 9900

tggtggccaa cgtgaagttc gaggccaagc ccgccaacca gctgctgacc cagcccgtga 9960

agcagggcgc cgagctggac ttccccatcc ccgtggacga cttcgccttc agcctgcacg 10020

acctgagcga caaggagacc accatcagcc agcagagcgc cgccatcctg ttctgcgtgg 10080

agggcgacgc caccctgtgg aagggcagcc agcagctgca gctgaagccc ggcgagagcg 10140

ccttcatcgc cgccaacgag agccccgtga ccgtgaaggg ccacggccgc ctggcccgcg 10200

tgtacaacaa gctgtgatag gagctcgatc cgtcgacctg cagatcgttc aaacatttgg 10260

caataaagtt tcttaagatt gaatcctgtt gccggtcttg cgatgattat catataattt 10320

ctgttgaatt acgttaagca tgtaataatt aacatgtaat gcatgacgtt atttatgaga 10380

tgggttttta tgattagagt cccgcaatta tacatttaat acgcgataga aaacaaaata 10440

tagcgcgcaa actaggataa attatcgcgc gcggtgtcat ctatgttact agatcggcgc 10500

gccgcaattg aagtttgggc ggccagcatg gccgtatccg caatgtgtta ttaagttgtc 10560

taagcgtcaa tttgtttaca ccacaatata tcctgccacc agccagccaa cagctccccg 10620

accggcagct cggcacaaaa tcaccactcg atacaggcag cccatcag 10668

<210> 37
<211> 10673
<212> DNA
<213> Artificial Sequence

<220>
<223> Expression cassette of construct 23123

<220>
<221>  enhancer
<222>  (165)..(256)
<223>  eNOS

<220>
<221>  promoter
<222>  (301)..(2097)
<223>  Ubi

<220>
<221>  gene
<222>  (2114)..(6283)
<223>  Cas9

<220>
<221>  misc_feature
<222>  (6218)..(6274)
<223>  NLS-TAG

<220>
<221>  terminator
<222>  (6289)..(6541)
<223>  tNOS

<220>
<221>  promoter
<222>  (6548)..(6922)
<223>  pU3

<220>
<221>  misc_feature
<222>  (6924)..(6943)

<220>
<221>  misc_feature
<222>  (6924)..(7028)
<223>  sgRNA

<220>
<221>  promoter
<222>  (7039)..(9030)
<223>  Ubi

<220>
<221>  gene
<222>  (9047)..(10225)
<223>  PMI

<220>
<221>  terminator
<222>  (10248)..(10500)
<223>  tNOS

<400>  37
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tgaaggcggg aaacgacaat ctgatcatga gcggagaatt     180

aagggagtca cgttatgacc cccgccgatg acgcgggaca agccgtttta cgtttggaac     240

tgacagaacc gcaacgctgc aggaattggc cgcagcggcc atttaaacaa agcttggtac 300

cattatgtgg tctaggtagg ttctatatat aagaaaactt gaaatgttct aaaaaaaaat 360

tcaagcccat gcatgattga agcaaacggt atagcaacgg tgttaacctg atctagtgat 420

ctcttgcaat ccttaacggc cacctaccgc aggtagcaaa cggcgtcccc ctcctcgata 480

tctccgcggc gacctctggc tttttccgcg gaattgcgcg gtggggacgg attccacgag 540

accgcgacgc aaccgcctct cgccgctggg ccccacaccg ctcggtgccg tagcctcacg 600

ggactctttc tccctcctcc cccgttataa attggcttca tcccctcctt gcctcatcca 660

tccaaatccc agtccccaat cccatccctt cgtaggagaa attcatcgaa gctaagcgaa 720

tcctcgcgat cctctcaagg tactgcgagt tttcgatccc cctctcgacc cctcgtatgt 780

ttgtgtttgt cgtagcgttt gattaggtat gctttccctg tttgtgttcg tcgtagcgtt 840

tgattaggta tgctttccct gttcgtgttc atcgtagtgt ttgattaggt cgtgtgaggc 900

gatggcctgc tcgcgtcctt cgatctgtag tcgatttgcg ggtcgtggtg tagatctgcg 960

ggctgtgatg aagttatttg gtgtgatctg ctcgcctgat tctgcgggtt ggctcgagta 1020

gatatgatgg ttggaccggt tggttcgttt accgcgctag ggttgggctg ggatgatgtt 1080

gcatgcgccg ttgcgcgtga tcccgcagca ggacttgcgt ttgattgcca gatctcgtta 1140

cgattatgtg atttggtttg gactttttag atctgtagct tctgcttatg tgccagatgc 1200

gcctactgct catatgcctg atgataatca taaatggctg tggaactaac tagttgattg 1260

cggagtcatg tatcagctac aggtgtaggg actagctaca ggtgtaggga cttgcgtcta 1320

attgtttggt cctttactca tgttgcaatt atgcaattta gtttagattg tttgttccac 1380

tcatctaggc tgtaaaaggg acactgctta gattgctgtt taatcttttt agtagattat 1440

attatattgg taacttatta cccctattac atgccatacg tgacttctgc tcatgcctga 1500

tgataatcat agatcactgt ggaattaatt agttgattgt tgaatcatgt ttcatgtaca 1560

taccacggca caattgctta gttccttaac aaatgcaaat tttactgatc catgtatgat 1620

ttgcgtggtt ctctaatgtg aaatactata gctacttgtt agtaagaatc aggttcgtat 1680

gcttaatgct gtatgtgcct tctgctcatg cctgatgata atcatatatc actggaatta 1740

attagttgat cgtttaatca tatatcaagt acataccatg ccacaatttt tagtcactta 1800

acccatgcag attgaactgg tccctgcatg ttttgctaaa ttgttctatt ctgattagac 1860

catatatcat gtattttttt ttggtaatgg ttctcttatt ttaaatgcta tatagttctg 1920

gtacttgtta gaaagatctg cttcatagtt tagttgccta tccctcgaat taggatgctg 1980

agcagctgat cctatagctt tgtttcatgt atcaattctt ttgtgttcaa cagtcagttt 2040

ttgttagatt cattgtaact tatggtcgct tactcttctg gtcctcaatg cttgcaggat 2100

cgcggccgct catatggaca agaagtacag catcggcctg gacatcggca ccaacagcgt 2160

gggctgggcc gtgatcaccg acgagtacaa ggtgccgagc aagaagttca aggtgctggg 2220

caacaccgac aggcacagca tcaagaagaa cctgatcggc gccctgctgt tcgacagcgg 2280

cgagaccgcc gaggccacca ggctgaagag gaccgccagg aggaggtaca ccaggaggaa 2340

gaacaggatc tgctacctgc aggagatctt cagcaacgag atggccaagg tggacgacag 2400

cttcttccac aggctggagg agagcttcct ggtggaggag gacaagaagc acgagaggca 2460

cccgatcttc ggcaacatcg tggacgaggt ggcctaccac gagaagtacc cgaccatcta 2520

ccacctgagg aagaagctgg tggacagcac cgacaaggcc gacctgaggc tgatctacct 2580

ggccctggcc cacatgatca agttcagggg ccacttcctg atcgagggcg acctgaaccc 2640

ggacaacagc gacgtggaca agctgttcat ccagctggtg cagacctaca accagctgtt 2700

cgaggagaac ccgatcaacg ccagcggcgt ggacgccaag gccatcctga gcgccaggct 2760

gagcaagagc aggaggctgg agaacctgat cgcccagctg ccgggcgaga agaagaacgg 2820

cctgttcggc aacctgatcg ccctgagcct gggcctgacc ccgaacttca agagcaactt 2880

cgacctggcc gaggacgcca agctgcagct gagcaaggac acctacgacg acgacctgga 2940

caacctgctg gcccagatcg gcgaccagta cgccgacctg ttcctggccg ccaagaacct 3000

gagcgacgcc atcctgctga gcgacatcct gagggtgaac accgagatca ccaaggcccc 3060

gctgagcgcc agcatgatca agaggtacga cgagcaccac caggacctga ccctgctgaa 3120

ggccctggtg aggcagcagc tgccggagaa gtacaaggag atcttcttcg accagagcaa 3180

gaacggctac gccggctaca tcgacggcgg cgccagccag gaggagttct acaagttcat 3240

caagccgatc ctggagaaga tggacggcac cgaggagctg ctggtgaagc tgaacaggga 3300

ggacctgctg aggaagcaga ggaccttcga caacggcagc atcccgcacc agatccacct 3360

gggcgagctg cacgccatcc tgaggaggca ggaggacttc tacccgttcc tgaaggacaa 3420

cagggagaag atcgagaaga tcctgacctt ccgcatcccg tactacgtgg gcccgctggc 3480

caggggcaac agcaggttcg cctggatgac caggaagagc gaggagacca tcaccccgtg 3540

gaacttcgag gaggtggtgg acaagggcgc cagcgcccag agcttcatcg agaggatgac 3600

caacttcgac aagaacctgc cgaacgagaa ggtgctgccg aagcacagcc tgctgtacga 3660

gtacttcacc gtgtacaacg agctgaccaa ggtgaagtac gtgaccgagg gcatgaggaa 3720

gccggccttc ctgagcggcg agcagaagaa ggccatcgtg gacctgctgt tcaagaccaa 3780

caggaaggtg accgtgaagc agctgaagga ggactacttc aagaagatcg agtgcttcga 3840

cagcgtggag atcagcggcg tggaggacag gttcaacgcc agcctgggca cctaccacga 3900

cctgctgaag atcatcaagg acaaggactt cctggacaac gaggagaacg aggacatcct 3960

ggaggacatc gtgctgaccc tgaccctgtt cgaggacagg gagatgatcg aggagaggct 4020

gaagacctac gcccacctgt tcgacgacaa ggtgatgaag cagctgaaga ggaggaggta 4080

caccggctgg ggcaggctga gcaggaagct gatcaacggc atcagggaca agcagagcgg 4140

caagaccatc ctggacttcc tgaagagcga cggcttcgcc aacaggaact tcatgcagct 4200

gatccacgac gacagcctga ccttcaagga ggacatccag aaggcccagg tgagcggcca 4260

gggcgacagc ctgcacgagc acatcgccaa cctggccggc agcccggcca tcaagaaggg 4320

catcctgcag accgtgaagg tggtggacga gctggtgaag gtgatgggca ggcacaagcc 4380

ggagaacatc gtgatcgaga tggccaggga gaaccagacc acccagaagg gccagaagaa 4440

cagcagggag aggatgaaga ggatcgagga gggcatcaag gagctgggca gccagatcct 4500

gaaggagcac ccggtggaga acacccagct gcagaacgag aagctgtacc tgtactacct 4560

gcagaacggc agggacatgt acgtggacca ggagctggac atcaacaggc tgagcgacta 4620

cgacgtggac cacatcgtgc cgcagagctt cctgaaggac gacagcatcg acaacaaggt 4680

gctgaccagg agcgacaaga acaggggcaa gagcgacaac gtgccgagcg aggaggtggt 4740

gaagaagatg aaaaactact ggaggcagct gctgaacgcc aagctgatca cccagaggaa 4800

gttcgacaac ctgaccaagg ccgagagggg cggcctgagc gagctggaca aggccggctt 4860

cattaaaagg cagctggtgg agaccaggca gatcaccaag cacgtggccc agatcctgga 4920

cagcaggatg aacaccaagt acgacgagaa cgacaagctg atcagggagg tgaaggtgat 4980

caccctgaag agcaagctgg tgagcgactt caggaaggac ttccagttct acaaggtgag 5040

ggagatcaat aattaccacc acgcccacga cgcctacctg aacgccgtgg tgggcaccgc 5100

cctgattaaa aagtacccga agctggagag cgagttcgtg tacggcgact acaaggtgta 5160

cgacgtgagg aagatgatcg ccaagagcga gcaggagatc ggcaaggcca ccgccaagta 5220

cttcttctac agcaacatca tgaacttctt caagaccgag atcaccctgg ccaacggcga 5280

gatcaggaag aggccgctga tcgagaccaa cggcgagacc ggcgagatcg tgtgggacaa 5340

gggcagggac ttcgccaccg tgaggaaggt gctgtccatg ccgcaggtga acatcgtgaa 5400

gaagaccgag gtgcagaccg gcggcttcag caaggagagc atcctgccga agaggaacag 5460

cgacaagctg atcgccagga agaaggactg ggacccgaag aagtacggcg gcttcgacag 5520

cccgaccgtg gcctacagcg tgctggtggt ggccaaggtg gagaagggca agagcaagaa 5580

gctgaagagc gtgaaggagc tggtgggcat caccatcatg gagaggagca gcttcgagaa 5640

gaacccagtg gacttcctgg aggccaaggg ctacaaggag gtgaagaagg acctgatcat 5700

taaactgccg aagtacagcc tgttcgagct ggagaacggc aggaagagga tgctggccag 5760

cgccggcgag ctgcagaagg gcaacgagct ggccctgccg agcaagtacg tgaacttcct 5820

gtacctggcc agccactacg agaagctgaa gggcagcccg gaggacaacg agcagaagca 5880

gctgttcgtg gagcagcaca agcactacct ggacgagatc atcgagcaga tcagcgagtt 5940

cagcaagagg gtgatcctgg ccgacgccaa cctggacaag gtgctgagcg cctacaacaa 6000

gcacagggac aagccgatca gggagcaggc cgagaacatc atccacctgt tcaccctgac 6060

caacctgggc gccccggccg ccttcaagta cttcgacacc accatcgaca ggaagaggta 6120

caccagcacc aaggaggtgc tggacgccac cctgatccac cagagcatca ccggcctgta 6180

cgagaccagg atcgacctga gccagctggg cggcgacagc agcccgccga agaagaagag 6240

gaaggtgagc tggaaggacg ccagcggctg gagcaggatg tgaagcttga tcgttcaaac 6300

atttggcaat aaagtttctt aagattgaat cctgttgccg gtcttgcgat gattatcata 6360

taatttctgt tgaattacgt taagcatgta ataattaaca tgtaatgcat gacgttattt 6420

atgagatggg tttttatgat tagagtcccg caattataca tttaatacgc gatagaaaac 6480

aaaatatagc gcgcaaacta ggataaatta tcgcgcgcgg tgtcatctat gttactagat 6540

cttcgaaggg atctttaaac atacgaacag atcacttaaa gttcttctga agcaacttaa 6600

agttatcagg catgcatgga tcttggagga atcagatgtg cagtcaggga ccatagcaca 6660

ggacaggcgt cttctactgg tgctaccagc aaatgctgga agccgggaac actgggtacg 6720

ttggaaacca cgtgatgtgg agtaagataa actgtaggag aaaagcattt cgtagtgggc 6780

catgaagcct ttcaggacat gtattgcagt atgggccggc ccattacgca attggacgac 6840

aacaaagact agtattagta ccacctcggc tatccacata gatcaaagct ggtttaaaag 6900

agttgtgcag atgatccgtg gcagggtcaa cgtggagaca ggggttttag agctagaaat 6960

agcaagttaa aataaggcta gtccgttatc aacttgaaaa agtggcaccg agtcggtgct 7020

ttttttttcg gaccgcgcct gcagtgcagc gtgacccggt cgtgcccctc tctagagata 7080

atgagcattg catgtctaag ttataaaaaa ttaccacata ttttttttgt cacacttgtt 7140

tgaagtgcag tttatctatc tttatacata tatttaaact ttactctacg aataatataa 7200

tctatagtac tacaataata tcagtgtttt agagaatcat ataaatgaac agttagacat 7260

ggtctaaagg acaattgagt attttgacaa caggactcta cagttttatc tttttagtgt 7320

gcatgtgttc tccttttttt ttgcaaatag cttcacctat ataatacttc atccatttta 7380

ttagtacatc catttagggt ttagggttaa tggtttttat agactaattt ttttagtaca 7440

tctattttat tctattttag cctctaaatt aagaaaacta aaactctatt ttagtttttt 7500

tatttaataa tttagatata aaatagaata aaataaagtg actaaaaatt aaacaaatac 7560

cctttaagaa attaaaaaaa ctaaggaaac atttttcttg tttcgagtag ataatgccag 7620

cctgttaaac gccgtcgacg agtctaacgg acaccaacca gcgaaccagc agcgtcgcgt 7680

cgggccaagc gaagcagacg gcacggcatc tctgtcgctg cctctggacc cctctcgaga 7740

gttccgctcc accgttggac ttgctccgct gtcggcatcc agaaattgcg tggcggagcg 7800

gcagacgtga gccggcacgg caggcggcct cctcctcctc tcacggcacc ggcagctacg 7860

ggggattcct ttcccaccgc tccttcgctt tcccttcctc gcccgccgta ataaatagac 7920

accccctcca caccctcttt ccccaacctc gtgttgttcg gagcgcacac acacacaacc 7980

agatctcccc caaatccacc cgtcggcacc tccgcttcaa ggtacgccgc tcgtcctccc 8040

ccccccccct ctctaccttc tctagatcgg cgttccggtc catggttagg gcccggtagt 8100

tctacttctg ttcatgtttg tgttagatcc gtgtttgtgt tagatccgtg ctgctagcgt 8160

tcgtacacgg atgcgacctg tacgtcagac acgttctgat tgctaacttg ccagtgtttc 8220

tctttgggga atcctgggat ggctctagcc gttccgcaga cgggatcgat ttcatgattt 8280

tttttgtttc gttgcatagg gtttggtttg cccttttcct ttatttcaat atatgccgtg 8340

cacttgtttg tcgggtcatc ttttcatgct tttttttgtc ttggttgtga tgatgtggtc 8400

tggttgggcg gtcgttctag atcggagtag aattctgttt caaactacct ggtggattta 8460

ttaattttgg atctgtatgt gtgtgccata catattcata gttacgaatt gaagatgatg 8520

gatggaaata tcgatctagg ataggtatac atgttgatgc gggttttact gatgcatata 8580

cagagatgct ttttgttcgc ttggttgtga tgatgtggtg tggttgggcg gtcgttcatt 8640

cgttctagat cggagtagaa tactgtttca aactacctgg tgtatttatt aattttggaa 8700

ctgtatgtgt gtgtcataca tcttcatagt tacgagttta agatggatgg aaatatcgat 8760

ctaggatagg tatacatgtt gatgtgggtt ttactgatgc atatacatga tggcatatgc 8820

agcatctatt catatgctct aaccttgagt acctatctat tataataaac aagtatgttt 8880

tataattatt ttgatcttga tatacttgga tgatggcata tgcagcagct atatgtggat 8940

ttttttagcc ctgccttcat acgctattta tttgcttggt actgtttctt ttgtcgatgc 9000

tcaccctgtt gtttggtgtt acttctgcag ggatccggca gcagccatgc agaagctgat 9060

caacagcgtg cagaactacg cctggggcag caagaccgcc ctgaccgagc tgtacggcat 9120

ggagaacccc agcagccagc ccatggccga gctgtggatg ggcgcccacc ccaagagcag 9180

cagccgcgtg cagaacgccg ccggcgacat cgtgagcctg cgcgacgtga tcgagagcga 9240

caagagcacc ctgctgggcg aggccgtggc caagcgcttc ggcgagctgc ccttcctgtt 9300

caaggtgctg tgcgccgccc agcccctgag catccaggtg caccccaaca agcacaacag 9360

cgagatcggc ttcgccaagg agaacgccgc cggcatcccc atggacgccg ccgagcgcaa 9420

ctacaaggac cccaaccaca agcccgagct ggtgttcgcc ctgaccccct tcctggccat 9480

gaacgccttc cgcgagttca gcgagatcgt gagcctgctg cagcccgtgg ccggcgccca 9540

ccccgccatc gcccacttcc tgcagcagcc cgacgccgag cgcctgagcg agctgttcgc 9600

cagcctgctg aacatgcagg gcgaggagaa gagccgcgcc ctggccatcc tgaagagcgc 9660

cctggacagc cagcagggcg agccctggca gaccatccgc ctgatcagcg agttctaccc 9720

cgaggacagc ggcctgttca gccccctgct gctgaacgtg gtgaagctga accccggcga 9780

ggccatgttc ctgttcgccg agacccccca cgcctacctg cagggcgtgg ccctggaggt 9840

gatggccaac agcgacaacg tgctgcgcgc cggcctgacc cccaagtaca tcgacatccc 9900

cgagctggtg gccaacgtga agttcgaggc caagcccgcc aaccagctgc tgacccagcc 9960

cgtgaagcag ggcgccgagc tggacttccc catccccgtg gacgacttcg ccttcagcct 10020

gcacgacctg agcgacaagg agaccaccat cagccagcag agcgccgcca tcctgttctg 10080

cgtggagggc gacgccaccc tgtggaaggg cagccagcag ctgcagctga agcccggcga 10140

gagcgccttc atcgccgcca acgagagccc cgtgaccgtg aagggccacg gccgcctggc 10200

ccgcgtgtac aacaagctgt gataggagct cgatccgtcg acctgcagat cgttcaaaca 10260

tttggcaata aagtttctta agattgaatc ctgttgccgg tcttgcgatg attatcatat 10320

aatttctgtt gaattacgtt aagcatgtaa taattaacat gtaatgcatg acgttattta 10380

tgagatgggt ttttatgatt agagtcccgc aattatacat ttaatacgcg atagaaaaca 10440

aaatatagcg cgcaaactag gataaattat cgcgcgcggt gtcatctatg ttactagatc 10500

ggcgcgccgc aattgaagtt tgggcggcca gcatggccgt atccgcaatg tgttattaag 10560

ttgtctaagc gtcaatttgt ttacaccaca atatatcctg ccaccagcca gccaacagct 10620

ccccgaccgg cagctcggca caaaatcacc actcgataca ggcagcccat cag 10673

<210> 38
<211> 10693
<212> DNA
<213> Artificial Sequence

<220>
<223> Expression cassette of construct 23501, gRNA target 1 dual guides


<220>
<221> promoter
<222> (214)..(734)
<223> 35S

<220>
<221> gene
<222> (748)..(4917)
<223> Cas9

<220>
<221> misc_feature
<222> (4852)..(4908)
<223> NLS-TAG

<220>
<221> terminator
<222> (4923)..(5175)
<223> tNOS

<220>
<221>  promoter
<222>  (5196)..(5570)
<223>  pU3

<220>
<221>  misc_feature
<222>  (5572)..(5591)

<220>
<221>  misc_feature
<222>  (5592)..(5636)
<223>  crRNA

<220>
<221>  promoter
<222>  (5643)..(6158)
<223>  pU6

<220>
<221>  misc_feature
<222>  (6159)..(6246)
<223>  tracrRNA

<220>
<221>  promoter
<222>  (6253)..(8245)
<223>  Ubi

<220>
<221>  gene
<222>  (8257)..(9435)
<223>  PMI

<220>
<221>  terminator
<222>  (9440)..(10474)
<223>  Ubi

<400>  38
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga ccgagtcaaa gattcaaata gaggacctaa     240

cagaactcgc cgtaaagact ggcgaacagt tcatacagag tctcttacga ctcaatgaca     300

agaagaaaat cttcgtcaac ttggtggagc acgacacgct agtctactcc aaaaatatca     360

aagatacagt ctcagaagac caaagggcaa ttgagacttt tcaacaaagg gtaatatccg     420

gaaacctcct cggattccat tgcccagcta tctgtcactt aattgtgaag atagtggaaa     480

aggaaggtgg ctcctacaaa tgccatcatt gcgataaagg aaaggccatc gttgaagatg     540

cctctgccga cagtggtccc aaagatggac ccccacccac gaggagcatc gtggaaaaag     600

aagacgttcc aaccacgtct tcaaagcaag tggattgatg tgatatctcc actgacgtaa     660

gggatgacgc acaatcccac tatccttcgc aagacccttc ctctatataa ggaagttcat     720

ttcatttgga gaggataatt atccaccatg gacaagaagt acagcatcgg cctggacatc 780

ggcaccaaca gcgtgggctg ggccgtgatc accgacgagt acaaggtgcc gagcaagaag 840

ttcaaggtgc tgggcaacac cgacaggcac agcatcaaga agaacctgat cggcgccctg 900

ctgttcgaca gcggcgagac cgccgaggcc accaggctga agaggaccgc caggaggagg 960

tacaccagga ggaagaacag gatctgctac ctgcaggaga tcttcagcaa cgagatggcc 1020

aaggtggacg acagcttctt ccacaggctg gaggagagct tcctggtgga ggaggacaag 1080

aagcacgaga ggcacccgat cttcggcaac atcgtggacg aggtggccta ccacgagaag 1140

tacccgacca tctaccacct gaggaagaag ctggtggaca gcaccgacaa ggccgacctg 1200

aggctgatct acctggccct ggcccacatg atcaagttca ggggccactt cctgatcgag 1260

ggcgacctga acccggacaa cagcgacgtg gacaagctgt tcatccagct ggtgcagacc 1320

tacaaccagc tgttcgagga gaacccgatc aacgccagcg gcgtggacgc caaggccatc 1380

ctgagcgcca ggctgagcaa gagcaggagg ctggagaacc tgatcgccca gctgccgggc 1440

gagaagaaga acggcctgtt cggcaacctg atcgccctga gcctgggcct gaccccgaac 1500

ttcaagagca acttcgacct ggccgaggac gccaagctgc agctgagcaa ggacacctac 1560

gacgacgacc tggacaacct gctggcccag atcggcgacc agtacgccga cctgttcctg 1620

gccgccaaga acctgagcga cgccatcctg ctgagcgaca tcctgagggt gaacaccgag 1680

atcaccaagg ccccgctgag cgccagcatg atcaagaggt acgacgagca ccaccaggac 1740

ctgaccctgc tgaaggccct ggtgaggcag cagctgccgg agaagtacaa ggagatcttc 1800

ttcgaccaga gcaagaacgg ctacgccggc tacatcgacg gcggcgccag ccaggaggag 1860

ttctacaagt tcatcaagcc gatcctggag aagatggacg gcaccgagga gctgctggtg 1920

aagctgaaca gggaggacct gctgaggaag cagaggacct tcgacaacgg cagcatcccg 1980

caccagatcc acctgggcga gctgcacgcc atcctgagga ggcaggagga cttctacccg 2040

ttcctgaagg acaacaggga gaagatcgag aagatcctga ccttccgcat cccgtactac 2100

gtgggcccgc tggccagggg caacagcagg ttcgcctgga tgaccaggaa gagcgaggag 2160

accatcaccc cgtggaactt cgaggaggtg gtggacaagg gcgccagcgc ccagagcttc 2220

atcgagagga tgaccaactt cgacaagaac ctgccgaacg agaaggtgct gccgaagcac 2280

agcctgctgt acgagtactt caccgtgtac aacgagctga ccaaggtgaa gtacgtgacc 2340

gagggcatga ggaagccggc cttcctgagc ggcgagcaga agaaggccat cgtggacctg 2400

ctgttcaaga ccaacaggaa ggtgaccgtg aagcagctga aggaggacta cttcaagaag 2460

atcgagtgct tcgacagcgt ggagatcagc ggcgtggagg acaggttcaa cgccagcctg 2520

ggcacctacc acgacctgct gaagatcatc aaggacaagg acttcctgga caacgaggag 2580

aacgaggaca tcctggagga catcgtgctg accctgaccc tgttcgagga cagggagatg 2640

atcgaggaga ggctgaagac ctacgcccac ctgttcgacg acaaggtgat gaagcagctg 2700

aagaggagga ggtacaccgg ctggggcagg ctgagcagga agctgatcaa cggcatcagg 2760

gacaagcaga gcggcaagac catcctggac ttcctgaaga gcgacggctt cgccaacagg 2820

aacttcatgc agctgatcca cgacgacagc ctgaccttca aggaggacat ccagaaggcc 2880

caggtgagcg gccagggcga cagcctgcac gagcacatcg ccaacctggc cggcagcccg 2940

gccatcaaga agggcatcct gcagaccgtg aaggtggtgg acgagctggt gaaggtgatg 3000

ggcaggcaca agccggagaa catcgtgatc gagatggcca gggagaacca gaccacccag 3060

aagggccaga agaacagcag ggagaggatg aagaggatcg aggagggcat caaggagctg 3120

ggcagccaga tcctgaagga gcacccggtg gagaacaccc agctgcagaa cgagaagctg 3180

tacctgtact acctgcagaa cggcagggac atgtacgtgg accaggagct ggacatcaac 3240

aggctgagcg actacgacgt ggaccacatc gtgccgcaga gcttcctgaa ggacgacagc 3300

atcgacaaca aggtgctgac caggagcgac aagaacaggg gcaagagcga caacgtgccg 3360

agcgaggagg tggtgaagaa gatgaaaaac tactggaggc agctgctgaa cgccaagctg 3420

atcacccaga ggaagttcga caacctgacc aaggccgaga ggggcggcct gagcgagctg 3480

gacaaggccg gcttcattaa aaggcagctg gtggagacca ggcagatcac caagcacgtg 3540

gcccagatcc tggacagcag gatgaacacc aagtacgacg agaacgacaa gctgatcagg 3600

gaggtgaagg tgatcaccct gaagagcaag ctggtgagcg acttcaggaa ggacttccag 3660

ttctacaagg tgagggagat caataattac caccacgccc acgacgccta cctgaacgcc 3720

gtggtgggca ccgccctgat taaaaagtac ccgaagctgg agagcgagtt cgtgtacggc 3780

gactacaagg tgtacgacgt gaggaagatg atcgccaaga gcgagcagga gatcggcaag 3840

gccaccgcca agtacttctt ctacagcaac atcatgaact tcttcaagac cgagatcacc 3900

ctggccaacg gcgagatcag gaagaggccg ctgatcgaga ccaacggcga gaccggcgag 3960

atcgtgtggg acaagggcag ggacttcgcc accgtgagga aggtgctgtc catgccgcag 4020

gtgaacatcg tgaagaagac cgaggtgcag accggcggct tcagcaagga gagcatcctg 4080

ccgaagagga acagcgacaa gctgatcgcc aggaagaagg actgggatcc gaagaagtac 4140

ggcggcttcg acagcccgac cgtggcctac agcgtgctgg tggtggccaa ggtggagaag 4200

ggcaagagca agaagctgaa gagcgtgaag gagctggtgg gcatcaccat catggagagg 4260

agcagcttcg agaagaaccc agtggacttc ctggaggcca agggctacaa ggaggtgaag 4320

aaggacctga tcattaaact gccgaagtac agcctgttcg agctggagaa cggcaggaag 4380

aggatgctgg ccagcgccgg cgagctgcag aagggcaacg agctggccct gccgagcaag 4440

tacgtgaact tcctgtacct ggccagccac tacgagaagc tgaagggcag cccggaggac 4500

aacgagcaga agcagctgtt cgtggagcag cacaagcact acctggacga gatcatcgag 4560

cagatcagcg agttcagcaa gagggtgatc ctggccgacg ccaacctgga caaggtgctg 4620

agcgcctaca acaagcacag ggacaagccg atcagggagc aggccgagaa catcatccac 4680

ctgttcaccc tgaccaacct gggcgccccg gccgccttca agtacttcga caccaccatc 4740

gacaggaaga ggtacaccag caccaaggag gtgctggacg ccaccctgat ccaccagagc 4800

atcaccggcc tgtacgagac caggatcgac ctgagccagc tgggcggcga cagcagcccg 4860

ccgaagaaga agaggaaggt gagctggaag gacgccagcg gctggagcag gatgtgaagc 4920

ttgatcgttc aaacatttgg caataaagtt tcttaagatt gaatcctgtt gccggtcttg 4980

cgatgattat catataattt ctgttgaatt acgttaagca tgtaataatt aacatgtaat 5040

gcatgacgtt atttatgaga tgggttttta tgattagagt cccgcaatta tacatttaat 5100

acgcgataga aaacaaaata tagcgcgcaa actaggataa attatcgcgc gcggtgtcat 5160

ctatgttact agatcgggac cggaattcgg gacccgggat ctttaaacat acgaacagat 5220

cacttaaagt tcttctgaag caacttaaag ttatcaggca tgcatggatc ttggaggaat 5280

cagatgtgca gtcagggacc atagcacagg acaggcgtct tctactggtg ctaccagcaa 5340

atgctggaag ccgggaacac tgggtacgtt ggaaaccacg tgatgtggag taagataaac 5400

tgtaggagaa aagcatttcg tagtgggcca tgaagccttt caggacatgt attgcagtat 5460

gggccggccc attacgcaat tggacgacaa caaagactag tattagtacc acctcggcta 5520

tccacataga tcaaagctgg tttaaaagag ttgtgcagat gatccgtggc atgcagtcga 5580

agtaatcggc ggttttagag ctatgctgtt ttgaatggtc ccaaaacttt ttttttacta 5640

gttttgtgaa agttgaatta cggcatagcc gaaggaataa cagaatcgtt tcacactttc 5700

gtaacaaagg tcttcttatc atgtttcaga cgatggaggc aaggctgatc aaagtgatca 5760

agcacataaa cgcatttttt taccatgttt cactccataa gcgtctgaga ttatcacaag 5820

tcacgtctag tagtttgatg gtacactagt gacaatcagt tcgtgcagac agagctcata 5880

cttgactact tgagcgatta caggcgaaag tgtgaaacgc atgtgatgtg ggctgggagg 5940

aggagaatat atactaatgg gccgtatcct gatttgggct gcgtcggaag gtgcagccca 6000

cgcgcgccgt accgcgcggg tggcgctgct acccacttta gtccgttgga tggggatccg 6060

atggtttgcg cggtggcgtt gcgggggatg tttagtacca catcggaaac cgaaagacga 6120

tggaaccagc ttataaaccc gcgcgctgta gtcagcttgg aaccattcaa aacagcatag 6180

caagttaaaa taaggctagt ccgttatcaa cttgaaaaag tggcaccgag tcggtgcttt 6240

tttttcctta ggctgcagtg cagcgtgacc cggtcgtgcc cctctctaga gataatgagc 6300

attgcatgtc taagttataa aaaattacca catatttttt ttgtcacact tgtttgaagt 6360

gcagtttatc tatctttata catatattta aactttactc tacgaataat ataatctata 6420

gtactacaat aatatcagtg ttttagagaa tcatataaat gaacagttag acatggtcta 6480

aaggacaatt gagtattttg acaacaggac tctacagttt tatcttttta gtgtgcatgt 6540

gttctccttt ttttttgcaa atagcttcac ctatataata cttcatccat tttattagta 6600

catccattta gggtttaggg ttaatggttt ttatagacta atttttttag tacatctatt 6660

ttattctatt ttagcctcta aattaagaaa actaaaactc tattttagtt tttttattta 6720

ataatttaga tataaaatag aataaaataa agtgactaaa aattaaacaa atacccttta 6780

agaaattaaa aaaactaagg aaacattttt cttgtttcga gtagataatg ccagcctgtt 6840

aaacgccgtc gacgagtcta acggacacca accagcgaac cagcagcgtc gcgtcgggcc 6900

aagcgaagca gacggcacgg catctctgtc gctgcctctg gacccctctc gagagttccg 6960

ctccaccgtt ggacttgctc cgctgtcggc atccagaaat tgcgtggcgg agcggcagac 7020

gtgagccggc acggcaggcg gcctcctcct cctctcacgg caccggcagc tacgggggat 7080

tcctttccca ccgctccttc gctttccctt cctcgcccgc cgtaataaat agacaccccc 7140

tccacaccct ctttccccaa cctcgtgttg ttcggagcgc acacacacac aaccagatct 7200

cccccaaatc cacccgtcgg cacctccgct tcaaggtacg ccgctcgtcc tccccccccc 7260

cccctctcta ccttctctag atcggcgttc cggtccatgg ttagggcccg gtagttctac 7320

ttctgttcat gtttgtgtta gatccgtgtt tgtgttagat ccgtgctgct agcgttcgta 7380

cacggatgcg acctgtacgt cagacacgtt ctgattgcta acttgccagt gtttctcttt 7440

ggggaatcct gggatggctc tagccgttcc gcagacggga tcgatttcat gatttttttt 7500

gtttcgttgc atagggtttg gtttgccctt ttcctttatt tcaatatatg ccgtgcactt 7560

gtttgtcggg tcatcttttc atgctttttt ttgtcttggt tgtgatgatg tggtctggtt 7620

gggcggtcgt tctagatcgg agtagaattc tgtttcaaac tacctggtgg atttattaat 7680

tttggatctg tatgtgtgtg ccatacatat tcatagttac gaattgaaga tgatggatgg 7740

aaatatcgat ctaggatagg tatacatgtt gatgcgggtt ttactgatgc atatacagag 7800

atgctttttg ttcgcttggt tgtgatgatg tggtgtggtt gggcggtcgt tcattcgttc 7860

tagatcggag tagaatactg tttcaaacta cctggtgtat ttattaattt tggaactgta 7920

tgtgtgtgtc atacatcttc atagttacga gtttaagatg gatggaaata tcgatctagg 7980

ataggtatac atgttgatgt gggttttact gatgcatata catgatggca tatgcagcat 8040

ctattcatat gctctaacct tgagtaccta tctattataa taaacaagta tgttttataa 8100

ttattttgat cttgatatac ttggatgatg gcatatgcag cagctatatg tggatttttt 8160

tagccctgcc ttcatacgct atttatttgc ttggtactgt ttcttttgtc gatgctcacc 8220

ctgttgtttg gtgttacttc tgcagtgact aaatagatgc agaagctgat caacagcgtg 8280

cagaactacg cctggggcag caagaccgcc ctgaccgagc tgtacggcat ggagaacccc 8340

agcagccagc ccatggccga gctgtggatg ggcgcccacc ccaagagctc aagccgcgtg 8400

cagaacgccg ccggcgatat cgttagcctg cgcgacgtga tcgagagcga caagagcacc 8460

ctgctgggcg aggccgtggc caagcgcttc ggcgagctgc ccttcctgtt caaggtgctg 8520

tgcgccgctc agcccctgag catccaggtg caccctaaca agcacaacag cgagatcggc 8580

ttcgccaagg agaacgccgc cggcatcccc atggacgccg ccgagcgcaa ctacaaggac 8640

cccaaccaca agcccgagct ggtgttcgcc ctgaccccct tcctggccat gaacgccttc 8700

cgcgagttca gcgagatcgt tagcctgctg cagcccgtgg ccggcgccca ccccgctatc 8760

gcccacttcc ttcagcagcc cgacgccgag cgcctgagcg agctgttcgc cagcctgctg 8820

aacatgcagg gtgaggagaa gtcacgcgcc ctggccatcc tgaagagcgc cctggacagc 8880

cagcagggcg agccctggca gacaatccgc ctgatcagcg agttctaccc cgaggatagc 8940

ggcctgttca gccccctgct gctgaacgtg gtgaagctga accccggcga ggccatgttc 9000

ctgttcgccg agacccccca cgcctacctg cagggcgtgg ccctggaggt gatggccaac 9060

agcgacaacg tgctgcgcgc cggcctgacc cccaagtaca tcgacatccc cgagctggtg 9120

gccaacgtga agttcgaggc taagcccgcc aaccagctgc tgacccagcc cgtgaagcag 9180

ggcgccgagc tggacttccc tatccccgtt gacgacttcg ccttcagcct gcacgacctg 9240

agcgacaagg agaccactat cagccagcag agcgccgcga tcctgttctg cgtggagggc 9300

gacgccaccc tgtggaaggg cagccagcag ctgcagctga agcccggcga gagcgccttt 9360

atcgccgcca acgagagccc cgtgaccgtg aagggccacg gccgcctggc ccgcgtgtac 9420

aacaagctgt gatagctacg tcatgggtcg tttaagctgc cgatgtgcct gcgtcgtctg 9480

gtgccctctc tccatatgga ggttgtcaaa gtatctgctg ttcgtgtcat gagtcgtgtc 9540

agtgttggtt taataatgga ccggttgtgt tgtgtgtgcg tactacccag aactatgaca 9600

aatcatgaat aagtttgatg tttgaaatta aagcctgtgc tcattatgtt ctgtctttca 9660

gttgtctcct aatatttgcc tgcaggtact ggctatctac cgtttcttac ttaggaggtg 9720

tttgaatgca ctaaaactaa tagttagtgg ctaaaattag ttaaaacatc caaacaccat 9780

agctaatagt tgaactatta gctatttttg gaaaattagt taatagtgag gtagttattt 9840

gttagctagc taattcaact aacaattttt agccaactaa caattagttt cagtgcattc 9900

aaacaccccc ttaatgttaa cgtggttcta tctaccgtct cctaatatat ggttgattgt 9960

tcggtttgtt gctatgctat tgggttctga ttgctgctag ttcttgctga atccagaagt 10020

tctcgtagta tagctcagat tcatattatt tatttgagtg ataagtgatc caggttatta 10080

ctatgttagc taggtttttt ttacaaggat aaattatctg tgatcataat tcttatgaaa 10140

gctttatgtt tcctggaggc agtggcatgc aatgcatgac agcaacttga tcacaccagc 10200

tgaggtagat acggtaacaa ggttcttaaa tctgttcacc aaatcattgg agaacacaca 10260

tacacattct tgccagtctt ggttagagaa atttcatgac aaaatgccaa agctgtcttg 10320

actcttcact tttggccatg agtcgtgact tagtttggtt taatggaccg gttctcctag 10380

cttgttctac tcaaaactgt tgttgatgcg aataagttgt gatggttgat ctctggattt 10440

tgttttgctc tcaatagtgg acgagattag atagcggacc gcctgcaggc ccgggggcgc 10500

gccctaatta gctaacggcc aggatcgccg cgtgagcctt tagcaactag ctagattaat 10560

taacgcaatc tgttattaag ttgtctaagc gtcaatttgt ttacaccaca atatatcctg 10620

ccaccagcca gccaacagct ccccgaccgg cagctcggca caaaatcacc actcgataca 10680

ggcagcccat cag 10693

<210> 39
<211> 10264
<212> DNA
<213> Artificial Sequence

<220>
<223> Expression cassette of construct 23501, gRNA target 1 single
      guide

<220>
<221> promoter
<222> (214)..(734)
<223> 35S

<220>
<221> gene
<222> (748)..(4917)
<223> Cas9

<220>
<221> misc_feature
<222> (4852)..(4908)
<223> NLS-TAG

<220>
<221> terminator
<222> (4923)..(5175)
<223> tNOS

<220>
<221> promoter
<222> (5196)..(5711)
<223> pU6

<220>
<221> misc_feature
<222> (5712)..(5817)
<223> sgRNA

<220>
<221> misc_feature

<222>  (5713)..(5732)

<220>
<221>  promoter
<222>  (5824)..(7816)
<223>  Ubi promoter

<220>
<221>  gene
<222>  (7828)..(9006)
<223>  PMI

<220>
<221>  terminator
<222>  (9011)..(10045)
<223>  Ubi terminator

<400>  39
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga ccgagtcaaa gattcaaata gaggacctaa     240

cagaactcgc cgtaaagact ggcgaacagt tcatacagag tctcttacga ctcaatgaca     300

agaagaaaat cttcgtcaac ttggtggagc acgacacgct agtctactcc aaaaatatca     360

aagatacagt ctcagaagac caaagggcaa ttgagacttt tcaacaaagg gtaatatccg     420

gaaacctcct cggattccat tgcccagcta tctgtcactt aattgtgaag atagtggaaa     480

aggaaggtgg ctcctacaaa tgccatcatt gcgataaagg aaaggccatc gttgaagatg     540

cctctgccga cagtggtccc aaagatggac ccccacccac gaggagcatc gtggaaaaag     600

aagacgttcc aaccacgtct tcaaagcaag tggattgatg tgatatctcc actgacgtaa     660

gggatgacgc acaatcccac tatccttcgc aagacccttc ctctatataa ggaagttcat     720

ttcatttgga gaggataatt atccaccatg gacaagaagt acagcatcgg cctggacatc     780

ggcaccaaca gcgtgggctg ggccgtgatc accgacgagt acaaggtgcc gagcaagaag     840

ttcaaggtgc tgggcaacac cgacaggcac agcatcaaga agaacctgat cggcgccctg     900

ctgttcgaca gcggcgagac cgccgaggcc accaggctga agaggaccgc caggaggagg     960

tacaccagga ggaagaacag gatctgctac ctgcaggaga tcttcagcaa cgagatggcc    1020

aaggtggacg acagcttctt ccacaggctg gaggagagct tcctggtgga ggaggacaag    1080

aagcacgaga ggcacccgat cttcggcaac atcgtggacg aggtggccta ccacgagaag    1140

tacccgacca tctaccacct gaggaagaag ctggtggaca gcaccgacaa ggccgacctg    1200

aggctgatct acctggccct ggcccacatg atcaagttca ggggccactt cctgatcgag    1260

ggcgacctga acccggacaa cagcgacgtg gacaagctgt tcatccagct ggtgcagacc    1320

tacaaccagc tgttcgagga gaacccgatc aacgccagcg gcgtggacgc caaggccatc    1380

ctgagcgcca ggctgagcaa gagcaggagg ctggagaacc tgatcgccca gctgccgggc 1440

gagaagaaga acggcctgtt cggcaacctg atcgccctga gcctgggcct gacccccaac 1500

ttcaagagca acttcgacct ggccgaggac gccaagctgc agctgagcaa ggacacctac 1560

gacgacgacc tggacaacct gctggcccag atcggcgacc agtacgccga cctgttcctg 1620

gccgccaaga acctgagcga cgccatcctg ctgagcgaca tcctgagggt gaacaccgag 1680

atcaccaagg ccccgctgag cgccagcatg atcaagaggt acgacgagca ccaccaggac 1740

ctgaccctgc tgaaggccct ggtgaggcag cagctgccgg agaagtacaa ggagatcttc 1800

ttcgaccaga gcaagaacgg ctacgccggc tacatcgacg gcggcgccag ccaggaggag 1860

ttctacaagt tcatcaagcc gatcctggag aagatggacg gcaccgagga gctgctggtg 1920

aagctgaaca gggaggacct gctgaggaag cagaggacct tcgacaacgg cagcatcccg 1980

caccagatcc acctgggcga gctgcacgcc atcctgagga ggcaggagga cttctacccg 2040

ttcctgaagg acaacaggga gaagatcgag aagatcctga ccttccgcat cccgtactac 2100

gtgggcccgc tggccagggg caacagcagg ttcgcctgga tgaccaggaa gagcgaggag 2160

accatcaccc cgtggaactt cgaggaggtg gtggacaagg gcgccagcgc ccagagcttc 2220

atcgagagga tgaccaactt cgacaagaac ctgccgaacg agaaggtgct gccgaagcac 2280

agcctgctgt acgagtactt caccgtgtac aacgagctga ccaaggtgaa gtacgtgacc 2340

gagggcatga ggaagccggc cttcctgagc ggcgagcaga agaaggccat cgtggacctg 2400

ctgttcaaga ccaacaggaa ggtgaccgtg aagcagctga aggaggacta cttcaagaag 2460

atcgagtgct tcgacagcgt ggagatcagc ggcgtggagg acaggttcaa cgccagcctg 2520

ggcacctacc acgacctgct gaagatcatc aaggacaagg acttcctgga caacgaggag 2580

aacgaggaca tcctggagga catcgtgctg accctgaccc tgttcgagga cagggagatg 2640

atcgaggaga ggctgaagac ctacgcccac ctgttcgacg acaaggtgat gaagcagctg 2700

aagaggagga ggtacaccgg ctggggcagg ctgagcagga agctgatcaa cggcatcagg 2760

gacaagcaga gcggcaagac catcctggac ttcctgaaga gcgacggctt cgccaacagg 2820

aacttcatgc agctgatcca cgacgacagc ctgaccttca aggaggacat ccagaaggcc 2880

caggtgagcg gccagggcga cagcctgcac gagcacatcg ccaacctggc cggcagcccg 2940

gccatcaaga agggcatcct gcagaccgtg aaggtggtgg acgagctggt gaaggtgatg 3000

ggcaggcaca agccggagaa catcgtgatc gagatggcca gggagaacca gaccacccag 3060

aagggccaga agaacagcag ggagaggatg aagaggatcg aggagggcat caaggagctg 3120

ggcagccaga tcctgaagga gcacccggtg gagaacaccc agctgcagaa cgagaagctg 3180

tacctgtact acctgcagaa cggcagggac atgtacgtgg accaggagct ggacatcaac 3240

aggctgagcg actacgacgt ggaccacatc gtgccgcaga gcttcctgaa ggacgacagc 3300

atcgacaaca aggtgctgac caggagcgac aagaacaggg gcaagagcga caacgtgccg 3360

agcgaggagg tggtgaagaa gatgaaaaac tactggaggc agctgctgaa cgccaagctg 3420

atcacccaga ggaagttcga caacctgacc aaggccgaga ggggcggcct gagcgagctg 3480

gacaaggccg gcttcattaa aaggcagctg gtggagacca ggcagatcac caagcacgtg 3540

gcccagatcc tggacagcag gatgaacacc aagtacgacg agaacgacaa gctgatcagg 3600

gaggtgaagg tgatcaccct gaagagcaag ctggtgagcg acttcaggaa ggacttccag 3660

ttctacaagg tgagggagat caataattac caccacgccc acgacgccta cctgaacgcc 3720

gtggtgggca ccgccctgat taaaaagtac ccgaagctgg agagcgagtt cgtgtacggc 3780

gactacaagg tgtacgacgt gaggaagatg atcgccaaga gcgagcagga gatcggcaag 3840

gccaccgcca agtacttctt ctacagcaac atcatgaact tcttcaagac cgagatcacc 3900

ctggccaacg gcgagatcag gaagaggccg ctgatcgaga ccaacggcga gaccggcgag 3960

atcgtgtggg acaagggcag ggacttcgcc accgtgagga aggtgctgtc catgccgcag 4020

gtgaacatcg tgaagaagac cgaggtgcag accggcggct tcagcaagga gagcatcctg 4080

ccgaagagga acagcgacaa gctgatcgcc aggaagaagg actgggatcc gaagaagtac 4140

ggcggcttcg acagcccgac cgtggcctac agcgtgctgg tggtggccaa ggtggagaag 4200

ggcaagagca agaagctgaa gagcgtgaag gagctggtgg gcatcaccat catggagagg 4260

agcagcttcg agaagaaccc agtggacttc ctggaggcca agggctacaa ggaggtgaag 4320

aaggacctga tcattaaact gccgaagtac agcctgttcg agctggagaa cggcaggaag 4380

aggatgctgg ccagcgccgg cgagctgcag aagggcaacg agctggccct gccgagcaag 4440

tacgtgaact tcctgtacct ggccagccac tacgagaagc tgaagggcag cccggaggac 4500

aacgagcaga agcagctgtt cgtggagcag cacaagcact acctggacga gatcatcgag 4560

cagatcagcg agttcagcaa gagggtgatc ctggccgacg ccaacctgga caaggtgctg 4620

agcgcctaca acaagcacag ggacaagccg atcagggagc aggccgagaa catcatccac 4680

ctgttcaccc tgaccaacct gggcgccccg gccgccttca agtacttcga caccaccatc 4740

gacaggaaga ggtacaccag caccaaggag gtgctggacg ccaccctgat ccaccagagc 4800

atcaccggcc tgtacgagac caggatcgac ctgagccagc tgggcggcga cagcagcccg 4860

ccgaagaaga agaggaaggt gagctggaag gacgccagcg gctggagcag gatgtgaagc 4920

ttgatcgttc aaacatttgg caataaagtt tcttaagatt gaatcctgtt gccggtcttg 4980

cgatgattat catataattt ctgttgaatt acgttaagca tgtaataatt aacatgtaat 5040

gcatgacgtt atttatgaga tgggttttta tgattagagt cccgcaatta tacatttaat 5100

acgcgataga aaacaaaata tagcgcgcaa actaggataa attatcgcgc gcggtgtcat 5160

ctatgttact agatcgggac cggaattcgg gaccctttgt gaaagttgaa ttacggcata 5220

gccgaaggaa taacagaatc gtttcacact ttcgtaacaa aggtcttctt atcatgtttc 5280

agacgatgga ggcaaggctg atcaaagtga tcaagcacat aaacgcattt ttttaccatg 5340

tttcactcca taagcgtctg agattatcac aagtcacgtc tagtagtttg atggtacact 5400

agtgacaatc agttcgtgca gacagagctc atacttgact acttgagcga ttacaggcga 5460

aagtgtgaaa cgcatgtgat gtgggctggg aggaggagaa tatatactaa tgggccgtat 5520

cctgatttgg gctgcgtcgg aaggtgcagc ccacgcgcgc cgtaccgcgc gggtggcgct 5580

gctacccact ttagtccgtt ggatggggat ccgatggttt gcgcggtggc gttgcggggg 5640

atgtttagta ccacatcgga aaccgaaaga cgatggaacc agcttataaa cccgcgcgct 5700

gtagtcagct tgtgcagtcg aagtaatcgg cggttttaga gctagaaata gcaagttaaa 5760

ataaggctag tccgttatca acttgaaaaa gtggcaccga gtcggtgctt tttttttcct 5820

aggctgcagt gcagcgtgac ccggtcgtgc ccctctctag agataatgag cattgcatgt 5880

ctaagttata aaaaattacc acatattttt tttgtcacac ttgtttgaag tgcagtttat 5940

ctatctttat acatatattt aaactttact ctacgaataa tataatctat agtactacaa 6000

taatatcagt gttttagaga atcatataaa tgaacagtta gacatggtct aaaggacaat 6060

tgagtatttt gacaacagga ctctacagtt ttatcttttt agtgtgcatg tgttctcctt 6120

tttttttgca aatagcttca cctatataat acttcatcca ttttattagt acatccattt 6180

agggtttagg gttaatggtt tttatagact aattttttta gtacatctat tttattctat 6240

tttagcctct aaattaagaa aactaaaact ctattttagt ttttttattt aataatttag 6300

atataaaata gaataaaata aagtgactaa aaattaaaca aatacccttt aagaaattaa 6360

aaaaactaag gaaacatttt tcttgtttcg agtagataat gccagcctgt taaacgccgt 6420

cgacgagtct aacggacacc aaccagcgaa ccagcagcgt cgcgtcgggc caagcgaagc 6480

agacggcacg gcatctctgt cgctgcctct ggacccctct cgagagttcc gctccaccgt 6540

tggacttgct ccgctgtcgg catccagaaa ttgcgtggcg gagcggcaga cgtgagccgg 6600

cacggcaggc ggcctcctcc tcctctcacg gcaccggcag ctacggggga ttcctttccc 6660

accgctcctt cgctttccct tcctcgcccg ccgtaataaa tagacacccc ctccacaccc 6720

tctttcccca acctcgtgtt gttcggagcg cacacacaca caaccagatc tcccccaaat 6780

ccacccgtcg gcacctccgc ttcaaggtac gccgctcgtc ctcccccccc ccccctctct 6840

accttctcta gatcggcgtt ccggtccatg gttagggccc ggtagttcta cttctgttca 6900

tgtttgtgtt agatccgtgt ttgtgttaga tccgtgctgc tagcgttcgt acacggatgc 6960

gacctgtacg tcagacacgt tctgattgct aacttgccag tgtttctctt tggggaatcc 7020

tgggatggct ctagccgttc cgcagacggg atcgatttca tgattttttt tgtttcgttg 7080

catagggttt ggtttgccct tttcctttat ttcaatatat gccgtgcact tgtttgtcgg 7140

gtcatctttt catgcttttt tttgtcttgg ttgtgatgat gtggtctggt tgggcggtcg 7200

ttctagatcg gagtagaatt ctgtttcaaa ctacctggtg gatttattaa ttttggatct 7260

gtatgtgtgt gccatacata ttcatagtta cgaattgaag atgatggatg gaaatatcga 7320

tctaggatag gtatacatgt tgatgcgggt tttactgatg catatacaga gatgcttttt 7380

gttcgcttgg ttgtgatgat gtggtgtggt tgggcggtcg ttcattcgtt ctagatcgga 7440

gtagaatact gtttcaaact acctggtgta tttattaatt ttggaactgt atgtgtgtgt 7500

catacatctt catagttacg agtttaagat ggatggaaat atcgatctag gataggtata 7560

catgttgatg tgggttttac tgatgcatat acatgatggc atatgcagca tctattcata 7620

tgctctaacc ttgagtacct atctattata ataaacaagt atgttttata attattttga 7680

tcttgatata cttggatgat ggcatatgca gcagctatat gtggattttt ttagccctgc 7740

cttcatacgc tatttatttg cttggtactg tttcttttgt cgatgctcac cctgttgttt 7800

ggtgttactt ctgcagtgac taaatagatg cagaagctga tcaacagcgt gcagaactac 7860

gcctggggca gcaagaccgc cctgaccgag ctgtacggca tggagaaccc cagcagccag 7920

cccatggccg agctgtggat gggcgcccac cccaagagct caagccgcgt gcagaacgcc 7980

gccggcgata tcgttagcct gcgcgacgtg atcgagagcg acaagagcac cctgctgggc 8040

gaggccgtgg ccaagcgctt cggcgagctg cccttcctgt tcaaggtgct gtgcgccgct 8100

cagcccctga gcatccaggt gcaccctaac aagcacaaca gcgagatcgg cttcgccaag 8160

gagaacgccg ccggcatccc catggacgcc gccgagcgca actacaagga ccccaaccac 8220

aagcccgagc tggtgttcgc cctgaccccc ttcctggcca tgaacgcctt ccgcgagttc 8280

agcgagatcg ttagcctgct gcagcccgtg gccggcgccc accccgctat cgcccacttc 8340

cttcagcagc ccgacgccga gcgcctgagc gagctgttcg ccagcctgct gaacatgcag 8400

ggtgaggaga agtcacgcgc cctggccatc ctgaagagcg ccctggacag ccagcagggc 8460

gagccctggc agacaatccg cctgatcagc gagttctacc ccgaggatag cggcctgttc 8520

agccccctgc tgctgaacgt ggtgaagctg aaccccggcg aggccatgtt cctgttcgcc 8580

gagacccccc acgcctacct gcagggcgtg gccctggagg tgatggccaa cagcgacaac 8640

gtgctgcgcg ccggcctgac ccccaagtac atcgacatcc ccgagctggt ggccaacgtg 8700

aagttcgagg ctaagcccgc caaccagctg ctgacccagc ccgtgaagca gggcgccgag 8760

ctggacttcc ctatccccgt tgacgacttc gccttcagcc tgcacgacct gagcgacaag 8820

gagaccacta tcagccagca gagcgccgcg atcctgttct gcgtggaggg cgacgccacc 8880

ctgtggaagg gcagccagca gctgcagctg aagcccggcg agagcgcctt tatcgccgcc 8940

aacgagagcc ccgtgaccgt gaagggccac ggccgcctgg cccgcgtgta caacaagctg 9000

tgatagctac gtcatgggtc gtttaagctg ccgatgtgcc tgcgtcgtct ggtgccctct 9060

ctccatatgg aggttgtcaa agtatctgct gttcgtgtca tgagtcgtgt cagtgttggt 9120

ttaataatgg accggttgtg ttgtgtgtgc gtactaccca gaactatgac aaatcatgaa 9180

taagtttgat gtttgaaatt aaagcctgtg ctcattatgt tctgtctttc agttgtctcc 9240

taatatttgc ctgcaggtac tggctatcta ccgtttctta cttaggaggt gtttgaatgc 9300

actaaaacta atagttagtg gctaaaatta gttaaaacat ccaaacacca tagctaatag 9360

ttgaactatt agctattttt ggaaaattag ttaatagtga ggtagttatt tgttagctag 9420

ctaattcaac taacaatttt tagccaacta acaattagtt tcagtgcatt caaacacccc 9480

cttaatgtta acgtggttct atctaccgtc tcctaatata tggttgattg ttcggtttgt 9540

tgctatgcta ttgggttctg attgctgcta gttcttgctg aatccagaag ttctcgtagt 9600

atagctcaga ttcatattat ttatttgagt gataagtgat ccaggttatt actatgttag 9660

ctaggttttt tttacaagga taaattatct gtgatcataa ttcttatgaa agctttatgt 9720

ttcctggagg cagtggcatg caatgcatga cagcaacttg atcacaccag ctgaggtaga 9780

tacggtaaca aggttcttaa atctgttcac caaatcattg gagaacacac atacacattc 9840

ttgccagtct tggttagaga aatttcatga caaaatgcca aagctgtctt gactcttcac 9900

ttttggccat gagtcgtgac ttagtttggt ttaatggacc ggttctccta gcttgttcta 9960

ctcaaaactg ttgttgatgc gaataagttg tgatggttga tctctggatt ttgttttgct 10020

ctcaatagtg gacgagatta gatagcggac cgcctgcagg cccgggggcg cgccctaatt 10080

agctaacggc caggatcgcc gcgtgagcct ttagcaacta gctagattaa ttaacgcaat 10140

ctgttattaa gttgtctaag cgtcaatttg tttacaccac aatatatcct gccaccagcc 10200

agccaacagc tccccgaccg gcagctcggc acaaaatcac cactcgatac aggcagccca 10260

tcag 10264

<210> 40
<211> 10692
<212> DNA
<213> Artificial Sequence

<220>
<223> Expression cassette of construct 23501, gRNA target 2 dual guides


<220>
<221> promoter
<222> (214)..(734)
<223> 35S

<220>

<221>  gene
<222>  (748)..(4917)
<223>  Cas9

<220>
<221>  misc_feature
<222>  (4852)..(4908)
<223>  NLS-TAG

<220>
<221>  terminator
<222>  (4923)..(5175)
<223>  tNOS

<220>
<221>  promoter
<222>  (5196)..(5570)
<223>  pU3

<220>
<221>  misc_feature
<222>  (5572)..(5590)

<220>
<221>  misc_feature
<222>  (5591)..(5635)
<223>  crRNA

<220>
<221>  promoter
<222>  (5642)..(6157)
<223>  pU6

<220>
<221>  misc_feature
<222>  (6158)..(6245)
<223>  tracrRNA

<220>
<221>  promoter
<222>  (6252)..(8244)
<223>  Ubi promoter

<220>
<221>  gene
<222>  (8256)..(9434)
<223>  PMI

<220>
<221>  terminator
<222>  (9439)..(10473)
<223>  Ubi terminator

<400>  40
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga ccgagtcaaa gattcaaata gaggacctaa     240

cagaactcgc cgtaaagact ggcgaacagt tcatacagag tctcttacga ctcaatgaca     300

agaagaaaat cttcgtcaac ttggtggagc acgacacgct agtctactcc aaaaatatca 360

aagatacagt ctcagaagac caaagggcaa ttgagacttt tcaacaaagg gtaatatccg 420

gaaacctcct cggattccat tgcccagcta tctgtcactt aattgtgaag atagtggaaa 480

aggaaggtgg ctcctacaaa tgccatcatt gcgataaagg aaaggccatc gttgaagatg 540

cctctgccga cagtggtccc aaagatggac ccccacccac gaggagcatc gtggaaaaag 600

aagacgttcc aaccacgtct tcaaagcaag tggattgatg tgatatctcc actgacgtaa 660

gggatgacgc acaatcccac tatccttcgc aagacccttc ctctatataa ggaagttcat 720

ttcatttgga gaggataatt atccaccatg gacaagaagt acagcatcgg cctggacatc 780

ggcaccaaca gcgtgggctg ggccgtgatc accgacgagt acaaggtgcc gagcaagaag 840

ttcaaggtgc tgggcaacac cgacaggcac agcatcaaga agaacctgat cggcgccctg 900

ctgttcgaca gcggcgagac cgccgaggcc accaggctga agaggaccgc caggaggagg 960

tacaccagga ggaagaacag gatctgctac ctgcaggaga tcttcagcaa cgagatggcc 1020

aaggtggacg acagcttctt ccacaggctg gaggagagct tcctggtgga ggaggacaag 1080

aagcacgaga ggcacccgat cttcggcaac atcgtggacg aggtggccta ccacgagaag 1140

tacccgacca tctaccacct gaggaagaag ctggtggaca gcaccgacaa ggccgacctg 1200

aggctgatct acctggccct ggcccacatg atcaagttca ggggccactt cctgatcgag 1260

ggcgacctga acccggacaa cagcgacgtg gacaagctgt tcatccagct ggtgcagacc 1320

tacaaccagc tgttcgagga gaacccgatc aacgccagcg gcgtggacgc caaggccatc 1380

ctgagcgcca ggctgagcaa gagcaggagg ctggagaacc tgatcgccca gctgccgggc 1440

gagaagaaga acggcctgtt cggcaacctg atcgccctga gcctgggcct gaccccgaac 1500

ttcaagagca acttcgacct ggccgaggac gccaagctgc agctgagcaa ggacacctac 1560

gacgacgacc tggacaacct gctggcccag atcggcgacc agtacgccga cctgttcctg 1620

gccgccaaga acctgagcga cgccatcctg ctgagcgaca tcctgagggt gaacaccgag 1680

atcaccaagg ccccgctgag cgccagcatg atcaagaggt acgacgagca ccaccaggac 1740

ctgaccctgc tgaaggccct ggtgaggcag cagctgccgg agaagtacaa ggagatcttc 1800

ttcgaccaga gcaagaacgg ctacgccggc tacatcgacg gcggcgccag ccaggaggag 1860

ttctacaagt tcatcaagcc gatcctggag aagatggacg gcaccgagga gctgctggtg 1920

aagctgaaca gggaggacct gctgaggaag cagaggacct tcgacaacgg cagcatcccg 1980

caccagatcc acctgggcga gctgcacgcc atcctgagga ggcaggagga cttctacccg 2040

ttcctgaagg acaacaggga gaagatcgag aagatcctga ccttccgcat cccgtactac 2100

gtgggcccgc tggccagggg caacagcagg ttcgcctgga tgaccaggaa gagcgaggag 2160

accatcaccc cgtggaactt cgaggaggtg gtggacaagg gcgccagcgc ccagagcttc 2220

atcgagagga tgaccaactt cgacaagaac ctgccgaacg agaaggtgct gccgaagcac 2280

agcctgctgt acgagtactt caccgtgtac aacgagctga ccaaggtgaa gtacgtgacc 2340

gagggcatga ggaagccggc cttcctgagc ggcgagcaga agaaggccat cgtggacctg 2400

ctgttcaaga ccaacaggaa ggtgaccgtg aagcagctga aggaggacta cttcaagaag 2460

atcgagtgct tcgacagcgt ggagatcagc ggcgtggagg acaggttcaa cgccagcctg 2520

ggcacctacc acgacctgct gaagatcatc aaggacaagg acttcctgga caacgaggag 2580

aacgaggaca tcctggagga catcgtgctg accctgaccc tgttcgagga cagggagatg 2640

atcgaggaga ggctgaagac ctacgcccac ctgttcgacg acaaggtgat gaagcagctg 2700

aagaggagga ggtacaccgg ctggggcagg ctgagcagga agctgatcaa cggcatcagg 2760

gacaagcaga gcggcaagac catcctggac ttcctgaaga gcgacggctt cgccaacagg 2820

aacttcatgc agctgatcca cgacgacagc ctgaccttca aggaggacat ccagaaggcc 2880

caggtgagcg gccagggcga cagcctgcac gagcacatcg ccaacctggc cggcagcccg 2940

gccatcaaga agggcatcct gcagaccgtg aaggtggtgg acgagctggt gaaggtgatg 3000

ggcaggcaca agccggagaa catcgtgatc gagatggcca gggagaacca gaccacccag 3060

aagggccaga agaacagcag ggagaggatg aagaggatcg aggagggcat caaggagctg 3120

ggcagccaga tcctgaagga gcacccggtg gagaacaccc agctgcagaa cgagaagctg 3180

tacctgtact acctgcagaa cggcagggac atgtacgtgg accaggagct ggacatcaac 3240

aggctgagcg actacgacgt ggaccacatc gtgccgcaga gcttcctgaa ggacgacagc 3300

atcgacaaca aggtgctgac caggagcgac aagaacaggg gcaagagcga caacgtgccg 3360

agcgaggagg tggtgaagaa gatgaaaaac tactggaggc agctgctgaa cgccaagctg 3420

atcacccaga ggaagttcga caacctgacc aaggccgaga ggggcggcct gagcgagctg 3480

gacaaggccg gcttcattaa aaggcagctg gtggagacca ggcagatcac caagcacgtg 3540

gcccagatcc tggacagcag gatgaacacc aagtacgacg agaacgacaa gctgatcagg 3600

gaggtgaagg tgatcaccct gaagagcaag ctggtgagcg acttcaggaa ggacttccag 3660

ttctacaagg tgagggagat caataattac caccacgccc acgacgccta cctgaacgcc 3720

gtggtgggca ccgccctgat taaaaagtac ccgaagctgg agagcgagtt cgtgtacggc 3780

gactacaagg tgtacgacgt gaggaagatg atcgccaaga gcgagcagga gatcggcaag 3840

gccaccgcca agtacttctt ctacagcaac atcatgaact tcttcaagac cgagatcacc 3900

ctggccaacg gcgagatcag gaagaggccg ctgatcgaga ccaacggcga gaccggcgag 3960

atcgtgtggg acaagggcag ggacttcgcc accgtgagga aggtgctgtc catgccgcag 4020

gtgaacatcg tgaagaagac cgaggtgcag accggcggct tcagcaagga gagcatcctg 4080

ccgaagagga acagcgacaa gctgatcgcc aggaagaagg actgggatcc gaagaagtac 4140

ggcggcttcg acagcccgac cgtggcctac agcgtgctgg tggtggccaa ggtggagaag 4200

ggcaagagca agaagctgaa gagcgtgaag gagctggtgg gcatcaccat catggagagg 4260

agcagcttcg agaagaaccc agtggacttc ctggaggcca agggctacaa ggaggtgaag 4320

aaggacctga tcattaaact gccgaagtac agcctgttcg agctggagaa cggcaggaag 4380

aggatgctgg ccagcgccgg cgagctgcag aagggcaacg agctggccct gccgagcaag 4440

tacgtgaact tcctgtacct ggccagccac tacgagaagc tgaagggcag cccggaggac 4500

aacgagcaga agcagctgtt cgtggagcag cacaagcact acctggacga gatcatcgag 4560

cagatcagcg agttcagcaa gagggtgatc ctggccgacg ccaacctgga caaggtgctg 4620

agcgcctaca acaagcacag ggacaagccg atcagggagc aggccgagaa catcatccac 4680

ctgttcaccc tgaccaacct gggcgccccg gccgccttca agtacttcga caccaccatc 4740

gacaggaaga ggtacaccag caccaaggag gtgctggacg ccaccctgat ccaccagagc 4800

atcaccggcc tgtacgagac caggatcgac ctgagccagc tgggcggcga cagcagcccg 4860

ccgaagaaga agaggaaggt gagctggaag gacgccagcg gctggagcag gatgtgaagc 4920

ttgatcgttc aaacatttgg caataaagtt tcttaagatt gaatcctgtt gccggtcttg 4980

cgatgattat catataattt ctgttgaatt acgttaagca tgtaataatt aacatgtaat 5040

gcatgacgtt atttatgaga tgggttttta tgattagagt cccgcaatta tacatttaat 5100

acgcgataga aaacaaaata tagcgcgcaa actaggataa attatcgcgc gcggtgtcat 5160

ctatgttact agatcgggac cggaattcgg gacccgggat ctttaaacat acgaacagat 5220

cacttaaagt tcttctgaag caacttaaag ttatcaggca tgcatggatc ttggaggaat 5280

cagatgtgca gtcagggacc atagcacagg acaggcgtct tctactggtg ctaccagcaa 5340

atgctggaag ccgggaacac tgggtacgtt ggaaaccacg tgatgtggag taagataaac 5400

tgtaggagaa aagcatttcg tagtgggcca tgaagccttt caggacatgt attgcagtat 5460

gggccggccc attacgcaat tggacgacaa caaagactag tattagtacc acctcggcta 5520

tccacataga tcaaagctgg tttaaaagag ttgtgcagat gatccgtggc agagaccggc 5580

aggtacgtcg gttttagagc tatgctgttt tgaatggtcc caaaactttt tttttactag 5640

ttttgtgaaa gttgaattac ggcatagccg aaggaataac agaatcgttt cacactttcg 5700

taacaaaggt cttcttatca tgtttcagac gatggaggca aggctgatca aagtgatcaa 5760

gcacataaac gcattttttt accatgtttc actccataag cgtctgagat tatcacaagt 5820

cacgtctagt agtttgatgg tacactagtg acaatcagtt cgtgcagaca gagctcatac 5880

ttgactactt gagcgattac aggcgaaagt gtgaaacgca tgtgatgtgg gctgggagga 5940

ggagaatata tactaatggg ccgtatcctg atttgggctg cgtcggaagg tgcagcccac 6000

gcgcgccgta ccgcgcgggt ggcgctgcta cccactttag tccgttggat ggggatccga 6060

tggtttgcgc ggtggcgttg cgggggatgt ttagtaccac atcggaaacc gaaagacgat 6120

ggaaccagct tataaacccg cgcgctgtag tcagcttgga accattcaaa acagcatagc 6180

aagttaaaat aaggctagtc cgttatcaac ttgaaaaagt ggcaccgagt cggtgctttt 6240

tttttcctag gctgcagtgc agcgtgaccc ggtcgtgccc ctctctagag ataatgagca 6300

ttgcatgtct aagttataaa aaattaccac atattttttt tgtcacactt gtttgaagtg 6360

cagtttatct atctttatac atatatttaa actttactct acgaataata taatctatag 6420

tactacaata atatcagtgt tttagagaat catataaatg aacagttaga catggtctaa 6480

aggacaattg agtattttga caacaggact ctacagtttt atctttttag tgtgcatgtg 6540

ttctcctttt tttttgcaaa tagcttcacc tatataatac ttcatccatt ttattagtac 6600

atccatttag ggtttagggt taatggtttt tatagactaa tttttttagt acatctattt 6660

tattctattt tagcctctaa attaagaaaa ctaaaactct attttagttt ttttatttaa 6720

taatttagat ataaaataga ataaaataaa gtgactaaaa attaaacaaa taccctttaa 6780

gaaattaaaa aaactaagga aacatttttc ttgtttcgag tagataatgc cagcctgtta 6840

aacgccgtcg acgagtctaa cggacaccaa ccagcgaacc agcagcgtcg cgtcgggcca 6900

agcgaagcag acggcacggc atctctgtcg ctgcctctgg acccctctcg agagttccgc 6960

tccaccgttg gacttgctcc gctgtcggca tccagaaatt gcgtggcgga gcggcagacg 7020

tgagccggca cggcaggcgg cctcctcctc ctctcacggc accggcagct acgggggatt 7080

cctttcccac cgctccttcg ctttcccttc ctcgcccgcc gtaataaata gacaccccct 7140

ccacaccctc tttccccaac ctcgtgttgt tcggagcgca cacacacaca accagatctc 7200

ccccaaatcc acccgtcggc acctccgctt caaggtacgc cgctcgtcct cccccccccc 7260

ccctctctac cttctctaga tcggcgttcc ggtccatggt tagggcccgg tagttctact 7320

tctgttcatg tttgtgttag atccgtgttt gtgttagatc cgtgctgcta gcgttcgtac 7380

acggatgcga cctgtacgtc agacacgttc tgattgctaa cttgccagtg tttctctttg 7440

gggaatcctg ggatggctct agccgttccg cagacgggat cgatttcatg attttttttg 7500

tttcgttgca tagggtttgg tttgcccttt tcctttattt caatatatgc cgtgcacttg 7560

tttgtcgggt catcttttca tgcttttttt tgtcttggtt gtgatgatgt ggtctggttg 7620

ggcggtcgtt ctagatcgga gtagaattct gtttcaaact acctggtgga tttattaatt 7680

ttggatctgt atgtgtgtgc catacatatt catagttacg aattgaagat gatggatgga 7740

aatatcgatc taggataggt atacatgttg atgcgggttt tactgatgca tatacagaga 7800

tgctttttgt tcgcttggtt gtgatgatgt ggtgtggttg ggcggtcgtt cattcgttct 7860

agatcggagt agaatactgt ttcaaactac ctggtgtatt tattaatttt ggaactgtat 7920

gtgtgtgtca tacatcttca tagttacgag tttaagatgg atggaaatat cgatctagga 7980

taggtataca tgttgatgtg ggttttactg atgcatatac atgatggcat atgcagcatc 8040

tattcatatg ctctaacctt gagtacctat ctattataat aaacaagtat gttttataat 8100

tattttgatc ttgatatact tggatgatgg catatgcagc agctatatgt ggattttttt 8160

agccctgcct tcatacgcta tttatttgct tggtactgtt tcttttgtcg atgctcaccc 8220

tgttgtttgg tgttacttct gcagtgacta aatagatgca gaagctgatc aacagcgtgc 8280

agaactacgc ctggggcagc aagaccgccc tgaccgagct gtacggcatg gagaacccca 8340

gcagccagcc catggccgag ctgtggatgg gcgcccaccc caagagctca agccgcgtgc 8400

agaacgccgc cggcgatatc gttagcctgc gcgacgtgat cgagagcgac aagagcaccc 8460

tgctgggcga ggccgtggcc aagcgcttcg gcgagctgcc cttcctgttc aaggtgctgt 8520

gcgccgctca gcccctgagc atccaggtgc accctaacaa gcacaacagc gagatcggct 8580

tcgccaagga gaacgccgcc ggcatcccca tggacgccgc cgagcgcaac tacaaggacc 8640

ccaaccacaa gcccgagctg gtgttcgccc tgaccccctt cctggccatg aacgccttcc 8700

gcgagttcag cgagatcgtt agcctgctgc agcccgtggc cggcgcccac cccgctatcg 8760

cccacttcct tcagcagccc gacgccgagc gcctgagcga gctgttcgcc agcctgctga 8820

acatgcaggg tgaggagaag tcacgcgccc tggccatcct gaagagcgcc ctggacagcc 8880

agcagggcga gccctggcag acaatccgcc tgatcagcga gttctacccc gaggatagcg 8940

gcctgttcag ccccctgctg ctgaacgtgg tgaagctgaa ccccggcgag gccatgttcc 9000

tgttcgccga gaccccccac gcctacctgc agggcgtggc cctggaggtg atggccaaca 9060

gcgacaacgt gctgcgcgcc ggcctgaccc ccaagtacat cgacatcccc gagctggtgg 9120

ccaacgtgaa gttcgaggct aagcccgcca accagctgct gacccagccc gtgaagcagg 9180

gcgccgagct ggacttccct atccccgttg acgacttcgc cttcagcctg cacgacctga 9240

gcgacaagga gaccactatc agccagcaga gcgccgcgat cctgttctgc gtggagggcg 9300

acgccaccct gtggaagggc agccagcagc tgcagctgaa gcccggcgag agcgccttta 9360

tcgccgccaa cgagagcccc gtgaccgtga agggccacgg ccgcctggcc cgcgtgtaca 9420

acaagctgtg atagctacgt catgggtcgt ttaagctgcc gatgtgcctg cgtcgtctgg 9480

tgccctctct ccatatggag gttgtcaaag tatctgctgt tcgtgtcatg agtcgtgtca 9540

gtgttggttt aataatggac cggttgtgtt gtgtgtgcgt actacccaga actatgacaa 9600

atcatgaata agtttgatgt ttgaaattaa agcctgtgct cattatgttc tgtctttcag 9660

ttgtctccta atatttgcct gcaggtactg gctatctacc gtttcttact taggaggtgt 9720

ttgaatgcac taaaactaat agttagtggc taaaattagt taaaacatcc aaacaccata 9780

gctaatagtt gaactattag ctatttttgg aaaattagtt aatagtgagg tagttatttg 9840

ttagctagct aattcaacta acaattttta gccaactaac aattagtttc agtgcattca 9900

aacaccccct taatgttaac gtggttctat ctaccgtctc ctaatatatg gttgattgtt 9960

cggtttgttg ctatgctatt gggttctgat tgctgctagt tcttgctgaa tccagaagtt 10020

ctcgtagtat agctcagatt catattattt atttgagtga taagtgatcc aggttattac 10080

tatgttagct aggttttttt tacaaggata aattatctgt gatcataatt cttatgaaag 10140

ctttatgttt cctggaggca gtggcatgca atgcatgaca gcaacttgat cacaccagct 10200

gaggtagata cggtaacaag gttcttaaat ctgttcacca aatcattgga gaacacacat 10260

acacattctt gccagtcttg gttagagaaa tttcatgaca aaatgccaaa gctgtcttga 10320

ctcttcactt ttggccatga gtcgtgactt agtttggttt aatggaccgg ttctcctagc 10380

ttgttctact caaaactgtt gttgatgcga ataagttgtg atggttgatc tctggatttt 10440

gttttgctct caatagtgga cgagattaga tagcggaccg cctgcaggcc cgggggcgcg 10500

ccctaattag ctaacggcca ggatcgccgc gtgagccttt agcaactagc tagattaatt 10560

aacgcaatct gttattaagt tgtctaagcg tcaatttgtt tacaccacaa tatatcctgc 10620

caccagccag ccaacagctc cccgaccggc agctcggcac aaaatcacca ctcgatacag 10680

gcagcccatc ag 10692

<210> 41
<211> 10262
<212> DNA
<213> Artificial Sequence

<220>
<223> Expression cassette of construct 23501, gRNA target 2 single
      guide


<220>
<221> promoter
<222> (214)..(734)
<223> 35S promoter

<220>
<221> gene
<222> (748)..(4917)
<223> Cas9

<220>
<221> terminator
<222> (4923)..(5175)
<223> tNOS

<220>
<221> promoter
<222> (5196)..(5711)

<220>
<221>  misc_feature
<222>  (5712)..(5730)

<220>
<221>  misc_feature
<222>  (5712)..(5815)
<223>  sgRNA

<220>
<221>  promoter
<222>  (5822)..(7814)
<223>  Ubi promoter

<220>
<221>  gene
<222>  (7826)..(9004)
<223>  PMI

<220>
<221>  terminator
<222>  (9009)..(10043)
<223>  Ubi terminator

<400>  41
cctgtggttg gcatgcacat acaaatggac gaacggataa accttttcac gcccttttaa      60

atatccgatt attctaataa acgctctttt ctcttaggtt tacccgccaa tatatcctgt     120

caaacactga tagtttaaac tggcactagc ctaacggtgt tgactaacta ggccgcttcc     180

ctaattagct aacccggggg cgcgccggga ccgagtcaaa gattcaaata gaggacctaa     240

cagaactcgc cgtaaagact ggcgaacagt tcatacagag tctcttacga ctcaatgaca     300

agaagaaaat cttcgtcaac ttggtggagc acgacacgct agtctactcc aaaaatatca     360

aagatacagt ctcagaagac caaagggcaa ttgagacttt tcaacaaagg gtaatatccg     420

gaaacctcct cggattccat tgcccagcta tctgtcactt aattgtgaag atagtggaaa     480

aggaaggtgg ctcctacaaa tgccatcatt gcgataaagg aaaggccatc gttgaagatg     540

cctctgccga cagtggtccc aaagatggac ccccacccac gaggagcatc gtggaaaaag     600

aagacgttcc aaccacgtct tcaaagcaag tggattgatg tgatatctcc actgacgtaa     660

gggatgacgc acaatcccac tatccttcgc aagacccttc ctctatataa ggaagttcat     720

ttcatttgga gaggataatt atccaccatg gacaagaagt acagcatcgg cctggacatc     780

ggcaccaaca gcgtgggctg ggccgtgatc accgacgagt acaaggtgcc gagcaagaag     840

ttcaaggtgc tgggcaacac cgacaggcac agcatcaaga agaacctgat cggcgccctg     900

ctgttcgaca gcggcgagac cgccgaggcc accaggctga agaggaccgc caggaggagg     960

tacaccagga ggaagaacag gatctgctac ctgcaggaga tcttcagcaa cgagatggcc    1020

aaggtggacg acagcttctt ccacaggctg gaggagagct tcctggtgga ggaggacaag    1080

aagcacgaga ggcacccgat cttcggcaac atcgtggacg aggtggccta ccacgagaag    1140

tacccgacca tctaccacct gaggaagaag ctggtggaca gcaccgacaa ggccgacctg 1200

aggctgatct acctggccct ggcccacatg atcaagttca ggggccactt cctgatcgag 1260

ggcgacctga acccggacaa cagcgacgtg gacaagctgt tcatccagct ggtgcagacc 1320

tacaaccagc tgttcgagga gaacccgatc aacgccagcg gcgtggacgc caaggccatc 1380

ctgagcgcca ggctgagcaa gagcaggagg ctggagaacc tgatcgccca gctgccgggc 1440

gagaagaaga acggcctgtt cggcaacctg atcgccctga gcctgggcct gaccccgaac 1500

ttcaagagca acttcgacct ggccgaggac gccaagctgc agctgagcaa ggacacctac 1560

gacgacgacc tggacaacct gctggcccag atcggcgacc agtacgccga cctgttcctg 1620

gccgccaaga acctgagcga cgccatcctg ctgagcgaca tcctgagggt gaacaccgag 1680

atcaccaagg ccccgctgag cgccagcatg atcaagaggt acgacgagca ccaccaggac 1740

ctgaccctgc tgaaggccct ggtgaggcag cagctgccgg agaagtacaa ggagatcttc 1800

ttcgaccaga gcaagaacgg ctacgccggc tacatcgacg gcggcgccag ccaggaggag 1860

ttctacaagt tcatcaagcc gatcctggag aagatggacg gcaccgagga gctgctggtg 1920

aagctgaaca gggaggacct gctgaggaag cagaggacct tcgacaacgg cagcatcccg 1980

caccagatcc acctgggcga gctgcacgcc atcctgagga ggcaggagga cttctacccg 2040

ttcctgaagg acaacaggga gaagatcgag aagatcctga ccttccgcat cccgtactac 2100

gtgggcccgc tggccagggg caacagcagg ttcgcctgga tgaccaggaa gagcgaggag 2160

accatcaccc cgtggaactt cgaggaggtg gtggacaagg gcgccagcgc ccagagcttc 2220

atcgagagga tgaccaactt cgacaagaac ctgccgaacg agaaggtgct gccgaagcac 2280

agcctgctgt acgagtactt caccgtgtac aacgagctga ccaaggtgaa gtacgtgacc 2340

gagggcatga ggaagccggc cttcctgagc ggcgagcaga agaaggccat cgtggacctg 2400

ctgttcaaga ccaacaggaa ggtgaccgtg aagcagctga aggaggacta cttcaagaag 2460

atcgagtgct tcgacagcgt ggagatcagc ggcgtggagg acaggttcaa cgccagcctg 2520

ggcacctacc acgacctgct gaagatcatc aaggacaagg acttcctgga caacgaggag 2580

aacgaggaca tcctggagga catcgtgctg accctgaccc tgttcgagga cagggagatg 2640

atcgaggaga ggctgaagac ctacgcccac ctgttcgacg acaaggtgat gaagcagctg 2700

aagaggagga ggtacaccgg ctggggcagg ctgagcagga agctgatcaa cggcatcagg 2760

gacaagcaga gcggcaagac catcctggac ttcctgaaga gcgacggctt cgccaacagg 2820

aacttcatgc agctgatcca cgacgacagc ctgaccttca aggaggacat ccagaaggcc 2880

caggtgagcg gccagggcga cagcctgcac gagcacatcg ccaacctggc cggcagcccg 2940

gccatcaaga agggcatcct gcagaccgtg aaggtggtgg acgagctggt gaaggtgatg 3000

ggcaggcaca agccggagaa catcgtgatc gagatggcca gggagaacca gaccacccag 3060

aagggccaga agaacagcag ggagaggatg aagaggatcg aggagggcat caaggagctg 3120

ggcagccaga tcctgaagga gcacccggtg gagaacaccc agctgcagaa cgagaagctg 3180

tacctgtact acctgcagaa cggcagggac atgtacgtgg accaggagct ggacatcaac 3240

aggctgagcg actacgacgt ggaccacatc gtgccgcaga gcttcctgaa ggacgacagc 3300

atcgacaaca aggtgctgac caggagcgac aagaacaggg gcaagagcga caacgtgccg 3360

agcgaggagg tggtgaagaa gatgaaaaac tactggaggc agctgctgaa cgccaagctg 3420

atcacccaga ggaagttcga caacctgacc aaggccgaga ggggcggcct gagcgagctg 3480

gacaaggccg gcttcattaa aaggcagctg gtggagacca ggcagatcac caagcacgtg 3540

gcccagatcc tggacagcag gatgaacacc aagtacgacg agaacgacaa gctgatcagg 3600

gaggtgaagg tgatcaccct gaagagcaag ctggtgagcg acttcaggaa ggacttccag 3660

ttctacaagg tgagggagat caataattac caccacgccc acgacgccta cctgaacgcc 3720

gtggtgggca ccgccctgat taaaaagtac ccgaagctgg agagcgagtt cgtgtacggc 3780

gactacaagg tgtacgacgt gaggaagatg atcgccaaga gcgagcagga gatcggcaag 3840

gccaccgcca agtacttctt ctacagcaac atcatgaact tcttcaagac cgagatcacc 3900

ctggccaacg gcgagatcag gaagaggccg ctgatcgaga ccaacggcga gaccggcgag 3960

atcgtgtggg acaagggcag ggacttcgcc accgtgagga aggtgctgtc catgccgcag 4020

gtgaacatcg tgaagaagac cgaggtgcag accggcggct tcagcaagga gagcatcctg 4080

ccgaagagga acagcgacaa gctgatcgcc aggaagaagg actgggatcc gaagaagtac 4140

ggcggcttcg acagcccgac cgtggcctac agcgtgctgg tggtggccaa ggtggagaag 4200

ggcaagagca agaagctgaa gagcgtgaag gagctggtgg gcatcaccat catggagagg 4260

agcagcttcg agaagaaccc agtggacttc ctggaggcca agggctacaa ggaggtgaag 4320

aaggacctga tcattaaact gccgaagtac agcctgttcg agctggagaa cggcaggaag 4380

aggatgctgg ccagcgccgg cgagctgcag aagggcaacg agctggccct gccgagcaag 4440

tacgtgaact tcctgtacct ggccagccac tacgagaagc tgaagggcag cccggaggac 4500

aacgagcaga agcagctgtt cgtggagcag cacaagcact acctggacga gatcatcgag 4560

cagatcagcg agttcagcaa gagggtgatc ctggccgacg ccaacctgga caaggtgctg 4620

agcgcctaca acaagcacag ggacaagccg atcagggagc aggccgagaa catcatccac 4680

ctgttcaccc tgaccaacct gggcgccccg gccgccttca agtacttcga caccaccatc 4740

gacaggaaga ggtacaccag caccaaggag gtgctggacg ccaccctgat ccaccagagc 4800

atcaccggcc tgtacgagac caggatcgac ctgagccagc tgggcggcga cagcagcccg 4860

ccgaagaaga agaggaaggt gagctggaag gacgccagcg gctggagcag gatgtgaagc 4920

ttgatcgttc aaacatttgg caataaagtt tcttaagatt gaatcctgtt gccggtcttg 4980

cgatgattat catataattt ctgttgaatt acgttaagca tgtaataatt aacatgtaat 5040

gcatgacgtt atttatgaga tgggttttta tgattagagt cccgcaatta tacatttaat 5100

acgcgataga aaacaaaata tagcgcgcaa actaggataa attatcgcgc gcggtgtcat 5160

ctatgttact agatcgggac cggaattcgg gaccctttgt gaaagttgaa ttacggcata 5220

gccgaaggaa taacagaatc gtttcacact ttcgtaacaa aggtcttctt atcatgtttc 5280

agacgatgga ggcaaggctg atcaaagtga tcaagcacat aaacgcattt ttttaccatg 5340

tttcactcca taagcgtctg agattatcac aagtcacgtc tagtagtttg atggtacact 5400

agtgacaatc agttcgtgca gacagagctc atacttgact acttgagcga ttacaggcga 5460

aagtgtgaaa cgcatgtgat gtgggctggg aggaggagaa tatatactaa tgggccgtat 5520

cctgatttgg gctgcgtcgg aaggtgcagc ccacgcgcgc cgtaccgcgc gggtggcgct 5580

gctacccact ttagtccgtt ggatggggat ccgatggttt gcgcggtggc gttgcggggg 5640

atgtttagta ccacatcgga aaccgaaaga cgatggaacc agcttataaa cccgcgcgct 5700

gtagtcagct tgagaccggc aggtacgtcg gttttagagc tagaaatagc aagttaaaat 5760

aaggctagtc cgttatcaac ttgaaaaagt ggcaccgagt cggtgctttt tttttcctag 5820

gctgcagtgc agcgtgaccc ggtcgtgccc ctctctagag ataatgagca ttgcatgtct 5880

aagttataaa aaattaccac atattttttt tgtcacactt gtttgaagtg cagtttatct 5940

atctttatac atatatttaa actttactct acgaataata taatctatag tactacaata 6000

atatcagtgt tttagagaat catataaatg aacagttaga catggtctaa aggacaattg 6060

agtattttga caacaggact ctacagtttt atctttttag tgtgcatgtg ttctcctttt 6120

tttttgcaaa tagcttcacc tatataatac ttcatccatt ttattagtac atccatttag 6180

ggtttagggt taatggtttt tatagactaa tttttttagt acatctattt tattctattt 6240

tagcctctaa attaagaaaa ctaaaactct attttagttt ttttatttaa taatttagat 6300

ataaaataga ataaaataaa gtgactaaaa attaaacaaa taccctttaa gaaattaaaa 6360

aaactaagga aacatttttc ttgtttcgag tagataatgc cagcctgtta aacgccgtcg 6420

acgagtctaa cggacaccaa ccagcgaacc agcagcgtcg cgtcgggcca agcgaagcag 6480

acggcacggc atctctgtcg ctgcctctgg acccctctcg agagttccgc tccaccgttg 6540

gacttgctcc gctgtcggca tccagaaatt gcgtggcgga gcggcagacg tgagccggca 6600

cggcaggcgg cctcctcctc ctctcacggc accggcagct acgggggatt cctttcccac 6660

cgctccttcg ctttcccttc ctcgcccgcc gtaataaata gacaccccct ccacaccctc 6720

tttccccaac ctcgtgttgt tcggagcgca cacacacaca accagatctc ccccaaatcc 6780

acccgtcggc acctccgctt caaggtacgc cgctcgtcct cccccccccc ccctctctac 6840

cttctctaga tcggcgttcc ggtccatggt tagggcccgg tagttctact tctgttcatg 6900

tttgtgttag atccgtgttt gtgttagatc cgtgctgcta gcgttcgtac acggatgcga 6960

cctgtacgtc agacacgttc tgattgctaa cttgccagtg tttctctttg gggaatcctg 7020

ggatggctct agccgttccg cagacgggat cgatttcatg attttttttg tttcgttgca 7080

tagggtttgg tttgcccttt tcctttattt caatatatgc cgtgcacttg tttgtcgggt 7140

catcttttca tgcttttttt tgtcttggtt gtgatgatgt ggtctggttg ggcggtcgtt 7200

ctagatcgga gtagaattct gtttcaaact acctggtgga tttattaatt ttggatctgt 7260

atgtgtgtgc catacatatt catagttacg aattgaagat gatggatgga aatatcgatc 7320

taggataggt atacatgttg atgcgggttt tactgatgca tatacagaga tgctttttgt 7380

tcgcttggtt gtgatgatgt ggtgtggttg ggcggtcgtt cattcgttct agatcggagt 7440

agaatactgt ttcaaactac ctggtgtatt tattaatttt ggaactgtat gtgtgtgtca 7500

tacatcttca tagttacgag tttaagatgg atggaaatat cgatctagga taggtataca 7560

tgttgatgtg ggttttactg atgcatatac atgatggcat atgcagcatc tattcatatg 7620

ctctaacctt gagtacctat ctattataat aaacaagtat gttttataat tattttgatc 7680

ttgatatact tggatgatgg catatgcagc agctatatgt ggattttttt agccctgcct 7740

tcatacgcta tttatttgct tggtactgtt tcttttgtcg atgctcaccc tgttgtttgg 7800

tgttacttct gcagtgacta aatagatgca gaagctgatc aacagcgtgc agaactacgc 7860

ctggggcagc aagaccgccc tgaccgagct gtacggcatg gagaacccca gcagccagcc 7920

catggccgag ctgtggatgg gcgcccaccc caagagctca agccgcgtgc agaacgccgc 7980

cggcgatatc gttagcctgc gcgacgtgat cgagagcgac aagagcaccc tgctgggcga 8040

ggccgtggcc aagcgcttcg gcgagctgcc cttcctgttc aaggtgctgt gcgccgctca 8100

gcccctgagc atccaggtgc accctaacaa gcacaacagc gagatcggct tcgccaagga 8160

gaacgccgcc ggcatcccca tggacgccgc cgagcgcaac tacaaggacc ccaaccacaa 8220

gcccgagctg gtgttcgccc tgaccccctt cctggccatg aacgccttcc gcgagttcag 8280

cgagatcgtt agcctgctgc agcccgtggc cggcgcccac cccgctatcg cccacttcct 8340

tcagcagccc gacgccgagc gcctgagcga gctgttcgcc agcctgctga acatgcaggg 8400

tgaggagaag tcacgcgccc tggccatcct gaagagcgcc ctggacagcc agcagggcga 8460

gccctggcag acaatccgcc tgatcagcga gttctacccc gaggatagcg gcctgttcag 8520

ccccctgctg ctgaacgtgg tgaagctgaa ccccggcgag gccatgttcc tgttcgccga 8580

gaccccccac gcctacctgc agggcgtggc cctggaggtg atggccaaca gcgacaacgt 8640

gctgcgcgcc ggcctgaccc ccaagtacat cgacatcccc gagctggtgg ccaacgtgaa 8700

gttcgaggct aagcccgcca accagctgct gacccagccc gtgaagcagg gcgccgagct 8760

ggacttccct atccccgttg acgacttcgc cttcagcctg cacgacctga gcgacaagga 8820

gaccactatc agccagcaga gcgccgcgat cctgttctgc gtggagggcg acgccaccct 8880

gtggaagggc agccagcagc tgcagctgaa gcccggcgag agcgccttta tcgccgccaa 8940

cgagagcccc gtgaccgtga agggccacgg ccgcctggcc cgcgtgtaca acaagctgtg 9000

atagctacgt catgggtcgt ttaagctgcc gatgtgcctg cgtcgtctgg tgccctctct 9060

ccatatggag gttgtcaaag tatctgctgt tcgtgtcatg agtcgtgtca gtgttggttt 9120

aataatggac cggttgtgtt gtgtgtgcgt actacccaga actatgacaa atcatgaata 9180

agtttgatgt ttgaaattaa agcctgtgct cattatgttc tgtctttcag ttgtctccta 9240

atatttgcct gcaggtactg gctatctacc gtttcttact taggaggtgt ttgaatgcac 9300

taaaactaat agttagtggc taaaattagt taaaacatcc aaacaccata gctaatagtt 9360

gaactattag ctatttttgg aaaattagtt aatagtgagg tagttatttg ttagctagct 9420

aattcaacta acaattttta gccaactaac aattagtttc agtgcattca aacaccccct 9480

taatgttaac gtggttctat ctaccgtctc ctaatatatg gttgattgtt cggtttgttg 9540

ctatgctatt gggttctgat tgctgctagt tcttgctgaa tccagaagtt ctcgtagtat 9600

agctcagatt catattattt atttgagtga taagtgatcc aggttattac tatgttagct 9660

aggttttttt tacaaggata aattatctgt gatcataatt cttatgaaag ctttatgttt 9720

cctggaggca gtggcatgca atgcatgaca gcaacttgat cacaccagct gaggtagata 9780

cggtaacaag gttcttaaat ctgttcacca aatcattgga gaacacacat acacattctt 9840

gccagtcttg gttagagaaa tttcatgaca aaatgccaaa gctgtcttga ctcttcactt 9900

ttggccatga gtcgtgactt agtttggttt aatggaccgg ttctcctagc ttgttctact 9960

caaaactgtt gttgatgcga ataagttgtg atggttgatc tctggatttt gttttgctct 10020

caatagtgga cgagattaga tagcggaccg cctgcaggcc cgggggcgcg ccctaattag 10080

ctaacggcca ggatcgccgc gtgagccttt agcaactagc tagattaatt aacgcaatct 10140

gttattaagt tgtctaagcg tcaatttgtt tacaccacaa tatatcctgc caccagccag 10200

ccaacagctc cccgaccggc agctcggcac aaaatcacca ctcgatacag gcagcccatc 10260

ag 10262

<210> 42
<211> 1366
<212> DNA
<213> Artificial Sequence

<220>
<223> TALEN-induced mutation in Event 38A ID 22808-4108 allele 2

<400>  42
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacca cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gctctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaagccgt cgaactgccg caggttcctg gtgctgtcca tcgggacggg gtcgacgtcc     840

gagcagggcc tctacacggc gcggcagtgc tcccggtggg gtatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct gcgcatccag    1020

gacaactcgc tccgtggcgc cgcggccacc gtggacgcgg cgacgccgga gaacatgcgg    1080

acgctcgtcg ggatcgggga gcggatgctg gcacagaggg tgtccagggt caacgtggag    1140

acaggacgaa ccggtgactg gcgaaggaag caatgccgat gccctcggtg ggctcgctag    1200

gcagctctcc gaggagagga gaacaaggct cgcgcgccgc gtctctgcca tcaacccaag    1260

aggctctaga tgtgcgtcgt acgatatcta agacaagtgg ctttactgtc agtcacatgc    1320

ttgtaaataa gtagacttta ttttaataaa acataaaaat atatat                    1366


<210>  43
<211>  1366
<212>  DNA
<213>  Artificial Sequence

<220>
<223>  CRISPR-induced MTL mutation in Event 27A ID 22807-4073 allele 2

<400>  43
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggagaa ccggtgactg gcgaaggaag caatgccgat gccctcggtg ggctcgctag 1200

gcagctctcc gaggagagga gaacaaggct cgcgcgccgc gtctctgcca tcaacccaag 1260

aggctctaga tgtgcgtcgt acgatatcta agacaagtgg ctttactgtc agtcacatgc 1320

ttgtaaataa gtagacttta ttttaataaa acataaaaat atatat 1366

<210> 44
<211> 1363
<212> DNA
<213> Artificial Sequence

<220>
<223> CRISPR-induced MTL mutation in Event 27A ID 22807-4081 allele 2

<400> 44
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acaggaaccg gtgactggcg aaggaagcaa tgccgatgcc ctcggtgggc tcgctaggca 1200

gctctccgag gagaggagaa caaggctcgc gcgccgcgtc tctgccatca acccaagagg 1260

ctctagatgt gcgtcgtacg atatctaaga caagtggctt tactgtcagt cacatgcttg 1320

taaataagta gactttattt taataaaaca taaaaatata tat 1363

<210> 45
<211> 1371
<212> DNA
<213> Zea mays

<400> 45
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctata gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 46
<211> 1371
<212> DNA
<213> Zea mays

<400> 46
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagctcgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgaggccaa agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 47
<211> 1371
<212> DNA
<213> Zea mays

<400> 47
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc tgcgcatctt ccctcagaag agcaggcttg cggccgccat gtccgcgctg 480

aggaagccaa agtacaacgg caagtgcatg cgcagcctga ttaggagcat cctcggcgag 540

acgaggccaa agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 48
<211> 1371
<212> DNA
<213> Zea mays

<400> 48
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagagggtga cggtgctgac ggtggacggc ggcggcgtcc ggggtctcat cccgggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttacg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 49
<211> 1371
<212> DNA
<213> Zea mays

<400>  49
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc     360

gcgcccggca aggacaagcg gcctctctac gctgccaagg acatcaacca cttttacatg     420

gagaactgcc cgcgcatctt ccctcagaag agcaggcttg cggccgccat gtccgcgctg     480

aggaagccaa agtacaacgg caagtgcatg cgcagcctga ttaggagcat cctcggcgag     540

acgagggcca agagcacgcc tctgaagaac gctctgctct cggacgtgtg cattggcacg     600

tccgccgcgc cgacctacct cccggcgcac tacttccaga ctgaagacgc caacggcaag     660

gagcgcgaat acaacctcat cgacggcggt gtggcggcca acaacccgac gatggttgcg     720

atgacgcaga tcaccaaaaa gatgcttgcc agcaaggaca aggccgagga gctgtaccca     780

gtgaagccgt cgaactgccg caggttcctg gtgctgtcca tcgggacggg gtcgacgtcc     840

gagcagggcc tctacacggc gcggcagtgc tcccggtggg gtatctgccg gtggctccgc     900

aacaacggca tggcccccat catcgacatc ttcatggcgg ccagctcgga cctggtggac     960

atccacgtcg ccgcgatgtt ccagtcgctc cacagcgacg gcgactacct gcgcatccag    1020

gacaactcgc tccgtggcgc cgcggccacc gtggacgcgg cgacgccgga gaacatacgg    1080

acgctcgtcg ggatcgggga gcggatgctg gcacagaggg tgtccagggt caacgtggag    1140

acagggaggt acgaaccggt gactggcgaa ggaagcaatg ccgatgccct cggtgggctc    1200

gctaggcagc tctccgagga gaggagaaca aggctcgcgc gccgcgtgtc tgccatcaac    1260

ccaagaggct ctagatgtgc gtcgtacgat atctaagaca agtggcttta ctgtcagtca    1320

catgcttgta aataagtaga ctttatttta ataaaacata aaaatatata t              1371

<210>  50
<211>  1371
<212>  DNA
<213>  Zea mays

<400>  50
agttcatcac taatcacact tattgttccc tcgacgagta tctagctagc tcattaatcg      60

atcaatcggg gtgtgcggtc gaaggcggca atggcgagct actcgtcgcg gcgtccatgc     120

aatacctgta gcacgaaggc gatggccggg agcgtggtcg gcgagcccgt cgtgctgggg     180

cagagggtga cggtgctgac ggtggacggc ggcggcgtcc ggggtctcat cccgggaacc     240

atcctcgcct tcctcgaggc caggctgcag gagctggacg gaccggaggc gaggctggcg     300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gattggcgaa ggaagcaatg ccgatgccct cggtgggctc 1200

gctaggcagc tctccgagga gaggagaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaatatatat t 1371

<210> 51
<211> 1371
<212> DNA
<213> Zea mays

<400> 51
agttcatcac taatcacact tattgttccc tcgacgagta tctagctagc tcattaatcg 60

atcaatcggg gtgtgcggtc gaaggcggca atggcgagct actcgtcgcg gcgtccatgc 120

aatacctgta gcacgaaggc gatggccggg agcgtggtcg gcgagcccgt cgtgctgggg 180

cagaggtgga cggtgctgac ggtggacggc ggcggcgtcc ggggtctcat cccggaacc 240

atcctcgcct tcctcgaggc caggctgcag gagctggacg gaccggaggc gaggctggcg 300

gactacttcg actacatcgc cggaaccagc accggcggtc tcatcaccgc catgctcacc 360

gcgcccggca aggacaagcg gcctctctac gctgccaagg acatcaacca cttttacatg 420

gagaactgcc cgcgcatctt ccctcagaag agcaggcttg cggccgccat gtccgcgctg 480

aggaagccaa agtacaacgg caagtgcatg cgcagcctga ttaggagcat cctcggcgag 540

acgagggcca agagcacgcc tctgaagaac gctctgctct cggacgtgtg cattggcacg 600

tccgccgcgc cgacctacct cccggcgcac tacttccaga ctgaagacgc caacggcaag 660

gagcgcgaat acaacctcat cgacggcggt gtggcggcca acaacccgac gatggttgcg 720

atgacgcaga tcaccaaaaa gatgcttgcc agcaaggaca aggccgagga gctgtaccca 780

gtgaagccgt cgaactgccg caggttcctg gtgctgtcca tcgggacggg gtcgacgtcc 840

gagcagggcc tctacacggc gcggcagtgc tcccggtggg gtatctgccg gtggctccgc 900

aacaacggca tggcccccat catcgacatc ttcatggcgg ccagctcgga cctggtggac 960

atccacgtcg ccgcgatgtt ccagtcgctc cacagcgacg gcgactacct gcgcatccag 1020

gacaactcgc tccgtggcgc cgcggccacc gtggacgcgg cgacgccgga gaacatgcgg 1080

acgctcgtcg ggatcgggga gcggatgctg gcacagaggg tgtccagggt caacgtggag 1140

acagggaggt acgaaccggt gactggcgaa ggaagcaatg ccgatgccct cggtgggctc 1200

gctaggcagc tctccgagga gaggaaaaca aggctcgcgc gccgcgtgtc tgccatcaac 1260

ccaagaggct ctagatgtgc gtcgtacgat atctaagaca agtggcttta ctgtcagtca 1320

catgcttgta aataagtaga ctttatttta ataaaacata aaaatatata t 1371

<210> 52
<211> 401
<212> PRT
<213> Zea mays

<400> 52
   Met Ala Ser Tyr Ser Ser Arg Arg Pro Cys Asn Thr Tyr Ser Thr Lys
   1 5 10 15
   Ala Met Ala Gly Ser Val Val Gly Glu Pro Val Val Leu Gly Gln Arg
   20 25 30
   Val Thr Val Leu Thr Val Asp Gly Gly Gly Val Arg Gly Leu Ile Pro
   35 40 45
   Gly Thr Ile Leu Ala Phe Leu Glu Ala Arg Leu Gln Glu Leu Asp Gly
   50 55 60
   Pro Glu Ala Arg Leu Ala Asp Tyr Phe Asp Tyr Ile Ala Gly Thr Ser
   65 70 75 80
   Thr Gly Gly Leu Ile Thr Ala Met Leu Thr Ala Pro Gly Lys Asp Lys
   85 90 95
   Arg Pro Leu Tyr Ala Ala Lys Asp Ile Asn His Phe Tyr Met Glu Asn
   100 105 110
   Cys Pro Arg Ile Phe Pro Gln Lys Ser Arg Leu Ala Ala Ala Met Ser
           115                 120                 125
   Ala Leu Arg Lys Pro Lys Tyr Asn Gly Lys Cys Met Arg Ser Leu Ile
       130                 135                 140
   Arg Ser Ile Leu Gly Glu Thr Arg Ala Lys Ser Thr Pro Leu Lys Asn
   145                 150                 155                 160
   Ala Leu Leu Ser Asp Val Cys Ile Gly Thr Ser Ala Ala Pro Thr Tyr
                   165                 170                 175
   Leu Pro Ala His Tyr Phe Gln Thr Glu Asp Ala Asn Gly Lys Glu Arg
               180                 185                 190
   Glu Tyr Asn Leu Ile Asp Gly Gly Val Ala Ala Asn Asn Pro Thr Met
           195                 200                 205
   Val Ala Met Thr Gln Ile Thr Lys Lys Met Leu Ala Ser Lys Asp Lys
       210                 215                 220
   Ala Glu Glu Leu Tyr Pro Val Lys Pro Ser Asn Cys Arg Arg Phe Leu
   225                 230                 235                 240
   Val Leu Ser Ile Gly Thr Gly Ser Thr Ser Glu Gln Gly Leu Tyr Thr
                   245                 250                 255
   Ala Arg Gln Cys Ser Arg Trp Gly Ile Cys Arg Trp Leu Arg Asn Asn
               260                 265                 270
   Gly Met Ala Pro Ile Ile Asp Ile Phe Met Ala Ala Ser Ser Asp Leu
           275                 280                 285
   Val Asp Ile His Val Ala Ala Met Phe Gln Ser Leu His Ser Asp Gly
       290                 295                 300
   Asp Tyr Leu Arg Ile Gln Asp Asn Ser Leu Arg Gly Ala Ala Ala Thr
   305                 310                 315                 320
   Val Asp Ala Ala Thr Pro Glu Asn Met Arg Thr Leu Val Gly Ile Gly
                   325                 330                 335
   Glu Arg Met Leu Ala Gln Arg Val Ser Arg Val Asn Val Glu Thr Gly
               340                 345                 350
   Arg Tyr Glu Pro Val Thr Gly Glu Gly Ser Asn Ala Asp Ala Leu Gly
           355                 360                 365
   Gly Leu Ala Arg Gln Leu Ser Glu Glu Arg Arg Thr Arg Leu Ala Arg
       370                 375                 380
   Arg Val Ser Ala Ile Asn Pro Arg Gly Ser Arg Cys Ala Ser Tyr Asp
   385                 390                 395                 400
   Ile

<210>  53
<211>  401
<212>  PRT
<213>  Zea mays

<400>  53

Met Ala Ser Tyr Ser Ser Arg Arg Pro Cys Asn Thr Cys Ser Thr Lys
1               5                   10                  15

Ala Met Ala Gly Ser Val Val Gly Glu Leu Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210>  54
<211>  401

<212>  PRT
<213>  Zea mays

<400>  54

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn
            100                 105                 110

Cys Leu Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210> 55
<211> 401
<212> PRT
<213> Zea mays

<400> 55

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Thr Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly

290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210>  56
<211>  401
<212>  PRT
<213>  Zea mays

<400>  56

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn

            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Ile Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210> 57
<211> 401
<212> PRT
<213> Zea mays

<400> 57

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Ile Gly Glu Gly Ser Asn Ala Asp Ala Leu Gly
        355                 360                 365

Gly Leu Ala Arg Gln Leu Ser Glu Glu Arg Arg Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210> 58
<211> 401
<212> PRT
<213> Zea mays

<400> 58

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Glu Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Ala Lys Ser Thr Pro Leu Lys Asn
145                 150                 155                 160

Ala Leu Leu Ser Asp Val Cys Ile Gly Thr Ser Ala Ala Pro Thr Tyr
                165                 170                 175

Leu Pro Ala His Tyr Phe Gln Thr Glu Asp Ala Asn Gly Lys Glu Arg
            180                 185                 190

Glu Tyr Asn Leu Ile Asp Gly Gly Val Ala Ala Asn Asn Pro Thr Met
        195                 200                 205

Val Ala Met Thr Gln Ile Thr Lys Lys Met Leu Ala Ser Lys Asp Lys
    210                 215                 220

Ala Glu Glu Leu Tyr Pro Val Lys Pro Ser Asn Cys Arg Arg Phe Leu
225                 230                 235                 240

Val Leu Ser Ile Gly Thr Gly Ser Thr Ser Glu Gln Gly Leu Tyr Thr
                245                 250                 255

Ala Arg Gln Cys Ser Arg Trp Gly Ile Cys Arg Trp Leu Arg Asn Asn
            260                 265                 270

Gly Met Ala Pro Ile Ile Asp Ile Phe Met Ala Ala Ser Ser Asp Leu
        275                 280                 285

Val Asp Ile His Val Ala Ala Met Phe Gln Ser Leu His Ser Asp Gly
    290                 295                 300

Asp Tyr Leu Arg Ile Gln Asp Asn Ser Leu Arg Gly Ala Ala Ala Thr
305                 310                 315                 320

Val Asp Ala Ala Thr Pro Glu Asn Met Arg Thr Leu Val Gly Ile Gly
                325                 330                 335

Glu Arg Met Leu Ala Gln Arg Val Ser Arg Val Asn Val Glu Thr Gly
            340                 345                 350

Arg Tyr Glu Pro Val Thr Gly Glu Gly Ser Asn Ala Asp Ala Leu Gly
        355                 360                 365

Gly Leu Ala Arg Gln Leu Ser Glu Glu Arg Lys Thr Arg Leu Ala Arg
    370                 375                 380

Arg Val Ser Ala Ile Asn Pro Arg Gly Ser Arg Cys Ala Ser Tyr Asp
385                 390                 395                 400

Ile

<210>  59
<211>  379
<212>  PRT
<213>  Zea mays

<400>  59

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Asn Arg
    370                 375

<210>  60
<211>  396
<212>  PRT
<213>  Zea mays

<400>  60

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Arg Thr Gly Asp Trp

370                 375                 380

Arg Arg Lys Gln Cys Arg Cys Pro Arg Trp Ala Arg
385                 390                 395

<210>  61
<211>  379
<212>  PRT
<213>  Zea mays

<400>  61

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr

195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Asn Arg
    370                 375

<210>  62
<211>  394
<212>  PRT
<213>  Zea mays

<400>  62

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro

35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Thr Gly Asp Trp Arg Arg
    370                 375                 380

Lys Gln Cys Arg Cys Pro Arg Trp Ala Arg
385                 390

<210>  63
<211>  395
<212>  PRT
<213>  Zea mays

<400>  63
   Met Ala Ser Tyr Ser Ser Arg Arg Pro Cys Asn Thr Cys Ser Thr Lys
   1               5                   10                  15
   Ala Met Ala Gly Ser Val Val Gly Glu Pro Val Val Leu Gly Gln Arg
               20                  25                  30
   Val Thr Val Leu Thr Val Asp Gly Gly Gly Val Arg Gly Leu Ile Pro
           35                  40                  45
   Gly Thr Ile Leu Ala Phe Leu Glu Ala Arg Leu Gln Glu Leu Asp Gly
       50                  55                  60
   Pro Glu Ala Arg Leu Ala Asp Tyr Phe Asp Tyr Ile Ala Gly Thr Ser
   65                  70                  75                  80
   Thr Gly Gly Leu Ile Thr Ala Met Leu Thr Ala Pro Gly Lys Asp Lys
                   85                  90                  95
   Arg Pro Leu Tyr Ala Ala Lys Asp Ile Asn His Phe Tyr Met Gln Asn
               100                 105                 110
   Cys Pro Arg Ile Phe Pro Gln Lys Ser Arg Leu Ala Ala Ala Met Ser
           115                 120                 125
   Ala Leu Arg Lys Pro Lys Tyr Asn Gly Lys Cys Met Arg Ser Leu Ile
       130                 135                 140
   Arg Ser Ile Leu Gly Glu Thr Arg Val Ser Glu Thr Leu Thr Asn Val
   145                 150                 155                 160
   Ile Ile Pro Ala Phe Asp Ile Arg Leu Leu Gln Pro Ile Ile Phe Ser
                   165                 170                 175
   Thr Tyr Asp Ala Lys Ser Thr Pro Leu Lys Asn Ala Leu Leu Ser Asp
               180                 185                 190
   Val Cys Ile Gly Thr Ser Ala Ala Pro Thr Tyr Leu Pro Ala His Tyr
           195                 200                 205
   Phe Gln Thr Glu Asp Ala Asn Gly Lys Glu Arg Glu Tyr Asn Leu Ile
       210                 215                 220
   Asp Gly Gly Val Ala Ala Asn Asn Pro Thr Met Val Ala Met Thr Gln
   225                 230                 235                 240
   Ile Thr Lys Lys Met Leu Ala Ser Lys Asp Lys Ala Glu Glu Leu Tyr
                   245                 250                 255
   Pro Val Asn Pro Ser Asn Cys Arg Arg Phe Leu Val Leu Ser Ile Gly
               260                 265                 270
   Thr Gly Ser Thr Ser Glu Gln Gly Leu Tyr Thr Ala Arg Gln Cys Ser
           275                 280                 285
   Arg Trp Gly Ile Cys Arg Trp Leu Arg Asn Asn Gly Met Ala Pro Ile
       290                 295                 300
   Ile Asp Ile Phe Met Ala Ala Ser Ser Asp Leu Val Asp Ile His Val
   305                 310                 315                 320
   Ala Ala Met Phe Gln Ser Leu His Ser Asp Gly Asp Tyr Leu Arg Ile
                   325                 330                 335
   Gln Asp Asn Ser Leu Arg Gly Ala Ala Ala Thr Val Asp Ala Ala Thr
               340                 345                 350
   Pro Glu Asn Met Arg Thr Leu Val Gly Ile Gly Glu Arg Met Leu Ala
           355                 360                 365
   Gln Arg Val Ser Arg Val Asn Val Glu Thr Gly Thr Gly Asp Trp Arg
       370                 375                 380
   Arg Lys Gln Cys Arg Cys Pro Arg Trp Ala Arg
   385                 390                 395

<210>  64
<211>  393
<212>  PRT
<213>  Zea mays

<400>  64

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Thr Gly Asp Trp Arg Arg
    370                 375                 380

Lys Gln Cys Arg Cys Pro Arg Trp Ala
385                 390

<210> 65
<211> 395
<212> PRT
<213> Zea mays

<400> 65

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Thr Gly Asp Trp Arg
    370                 375                 380

Arg Lys Gln Cys Arg Cys Pro Arg Trp Ala Arg
385                 390                 395

<210>  66
<211>  398
<212>  PRT
<213>  Zea mays

<400>  66

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr

340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Arg Glu Val Arg Thr Gly
    370                 375                 380

Asp Trp Arg Arg Lys Gln Cys Arg Cys Pro Arg Trp Ala Arg
385                 390                 395

<210>  67
<211>  398
<212>  PRT
<213>  Zea mays

<400>  67

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser

165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Arg Glu Val Arg Thr Gly
    370                 375                 380

Asp Trp Arg Arg Lys Gln Cys Arg Cys Pro Arg Trp Ala Arg
385                 390                 395

<210>  68
<211>  384
<212>  PRT
<213>  Zea mays

<400>  68

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Lys Gly Gly Thr Asn Arg
    370                 375                 380

<210> 69
<211> 383
<212> PRT
<213> Zea mays

<400> 69

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Gly Thr Asn Arg
    370                 375                 380

<210>  70
<211>  396
<212>  PRT
<213>  Zea mays

<400>  70

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Arg Thr Gly Asp Trp
    370                 375                 380

Arg Arg Lys Gln Cys Arg Cys Pro Arg Trp Ala Arg
385                 390                 395

<210>  71
<211>  396
<212>  PRT
<213>  Zea mays

<400>  71

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile
                325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Arg Thr Gly Asp Trp
    370                 375                 380

Arg Arg Lys Gln Cys Arg Cys Pro Arg Trp Ala Arg
385                 390                 395

<210>  72
<211>  395
<212>  PRT
<213>  Zea mays

<400>  72

Met Ala Ser Tyr Ser Ser Arg Arg Pro Cys Asn Thr Cys Ser Thr Lys
1               5                   10                  15

Ala Met Ala Gly Ser Val Val Gly Glu Pro Val Val Leu Gly Gln Arg
            20                  25                  30

Val Thr Val Leu Thr Val Asp Gly Gly Gly Val Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Tyr Ile Ala Gly Thr Ser
65                  70                  75                  80

Thr Gly Gly Leu Ile Thr Ala Met Leu Thr Ala Pro Gly Lys Asp Lys
                85                  90                  95

Arg Pro Leu Tyr Ala Ala Lys Asp Ile Asn His Phe Tyr Met Gln Asn
            100                 105                 110

Cys Pro Arg Ile Phe Pro Gln Lys Ser Arg Leu Ala Ala Ala Met Ser
        115                 120                 125

Ala Leu Arg Lys Pro Lys Tyr Asn Gly Lys Cys Met Arg Ser Leu Ile
    130                 135                 140

Arg Ser Ile Leu Gly Glu Thr Arg Val Ser Glu Thr Leu Thr Asn Val
145                 150                 155                 160

Ile Ile Pro Ala Phe Asp Ile Arg Leu Leu Gln Pro Ile Ile Phe Ser
                165                 170                 175

Thr Tyr Asp Ala Lys Ser Thr Pro Leu Lys Asn Ala Leu Leu Ser Asp
            180                 185                 190

Val Cys Ile Gly Thr Ser Ala Ala Pro Thr Tyr Leu Pro Ala His Tyr
        195                 200                 205

Phe Gln Thr Glu Asp Ala Asn Gly Lys Glu Arg Glu Tyr Asn Leu Ile
    210                 215                 220

Asp Gly Gly Val Ala Ala Asn Asn Pro Thr Met Val Ala Met Thr Gln
225                 230                 235                 240

Ile Thr Lys Lys Met Leu Ala Ser Lys Asp Lys Ala Glu Glu Leu Tyr
                245                 250                 255

Pro Val Asn Pro Ser Asn Cys Arg Arg Phe Leu Val Leu Ser Ile Gly
            260                 265                 270

Thr Gly Ser Thr Ser Glu Gln Gly Leu Tyr Thr Ala Arg Gln Cys Ser
        275                 280                 285

Arg Trp Gly Ile Cys Arg Trp Leu Arg Asn Asn Gly Met Ala Pro Ile
    290                 295                 300

Ile Asp Ile Phe Met Ala Ala Ser Ser Asp Leu Val Asp Ile His Val
305                 310                 315                 320

Ala Ala Met Phe Gln Ser Leu His Ser Asp Gly Asp Tyr Leu Arg Ile

325                 330                 335

Gln Asp Asn Ser Leu Arg Gly Ala Ala Ala Thr Val Asp Ala Ala Thr
            340                 345                 350

Pro Glu Asn Met Arg Thr Leu Val Gly Ile Gly Glu Arg Met Leu Ala
        355                 360                 365

Gln Arg Val Ser Arg Val Asn Val Glu Thr Gly Thr Gly Asp Trp Arg
    370                 375                 380

Arg Lys Gln Cys Arg Cys Pro Arg Trp Ala Arg
385                 390                 395

<210>  73
<211>  437
<212>  PRT
<213>  Sorghum bicolor

<400>  73

Met Ala Thr Tyr Tyr Ser Ser Arg Arg Pro Cys Asn Ala Cys Ser Thr
1               5                   10                  15

Lys Ala Met Ala Gly Ser Val Val Gly Glu Pro Val Val Leu Gly Gln
            20                  25                  30

Arg Val Thr Val Leu Thr Val Asp Gly Gly Gly Ile Arg Gly Leu Ile
        35                  40                  45

Pro Gly Thr Ile Leu Ala Phe Leu Glu Ala Arg Leu Gln Glu Leu Asp
    50                  55                  60

Gly Pro Glu Val Arg Leu Ala Asp Tyr Phe Asp Tyr Ile Ala Gly Thr
65                  70                  75                  80

Ser Thr Gly Gly Leu Ile Thr Ala Met Leu Thr Ala Pro Gly Lys Asp
                85                  90                  95

Arg Arg Pro Leu Tyr Ala Ala Lys Asp Ile Asn Gln Phe Tyr Met Glu
            100                 105                 110

Asn Cys Pro Arg Ile Phe Pro Gln Lys Ser Ser Arg Leu Ala Ala Ala
        115                 120                 125

Met Ser Ala Leu Arg Lys Pro Arg Tyr Asn Gly Lys Cys Leu Arg Asn
    130                 135                 140

Leu Ile Met Ser Met Leu Gly Glu Thr Arg Val Ser Asp Thr Leu Thr

145                 150                 155                 160

Asn Val Ile Ile Pro Thr Phe Asp Val Arg Leu Leu Gln Pro Ile Ile
                165                 170                 175

Phe Ser Thr Tyr Asp Ala Lys Ser Met Pro Leu Lys Asn Ala Leu Leu
            180                 185                 190

Ser Asp Val Cys Ile Gly Thr Ser Ala Ala Pro Thr Tyr Leu Pro Ala
        195                 200                 205

His Tyr Phe Gln Thr Lys Asp Ala Gly Ser Gly Lys Glu Arg Glu Tyr
    210                 215                 220

Asn Leu Ile Asp Gly Gly Val Ala Ala Asn Asn Pro Thr Met Val Ala
225                 230                 235                 240

Met Thr Gln Ile Thr Lys Lys Met Leu Ala Ser Lys Glu Lys Ala Glu
                245                 250                 255

Glu Leu Tyr Pro Val Lys Pro Trp Asn Cys Arg Lys Phe Leu Val Leu
            260                 265                 270

Ser Ile Gly Thr Gly Ser Thr Ser Glu Gln Gly Leu Tyr Thr Ala Arg
        275                 280                 285

Gln Cys Ser Arg Trp Gly Ile Cys Arg Trp Ile Arg Asn Asn Gly Met
    290                 295                 300

Ala Pro Ile Ile Asp Ile Phe Met Ala Ala Ser Ser Asp Leu Val Asp
305                 310                 315                 320

Ile His Val Ala Ala Met Phe Gln Ser Leu His Ser Asp Gly Asp Tyr
                325                 330                 335

Leu Arg Ile Gln Asp Asn Ser Leu His Gly Ala Ala Ala Thr Val Asp
            340                 345                 350

Ala Ala Thr Pro Glu Asn Met Arg Thr Leu Val Gly Ile Gly Glu Arg
        355                 360                 365

Met Leu Ala Gln Arg Val Ser Arg Val Asn Val Glu Thr Gly Arg Tyr
    370                 375                 380

Glu Pro Val Pro Gly Glu Gly Ser Asn Ala Asp Ala Leu Ala Gly Ile
385                 390                 395                 400

Ala Arg Gln Leu Ser Glu Glu Arg Arg Thr Arg Leu Ala Arg Arg Thr
                405                 410                 415

Ser Ala Ile Val Ser Ser Gly Gly Ala Ser Arg Arg Thr Cys Ala Ser
            420                 425                 430

Lys Val Ser Asn Val
        435

<210>  74
<211>  421
<212>  PRT
<213>  Setaria italica

<400>  74

Met Ala Ser Tyr Ser Ser Arg Arg Pro Cys Asn Ala Cys Arg Thr Lys
1               5                   10                  15

Ala Met Ala Gly Ser Val Val Gly Glu Pro Val Val Pro Gly Gln Arg
            20                  25                  30

Val Thr Val Leu Thr Ile Asp Gly Gly Gly Ile Arg Gly Leu Ile Pro
        35                  40                  45

Gly Thr Ile Leu Ala Phe Leu Glu Ala Arg Leu Gln Glu Leu Asp Gly
    50                  55                  60

Pro Glu Ala Arg Leu Ala Asp Tyr Phe Asp Cys Ile Ala Gly Thr Ser
65                  70                  75                  80

Thr Gly Gly Leu Ile Thr Ala Met Ile Thr Thr Pro Gly Glu Asp Lys
                85                  90                  95

Arg Pro Leu Phe Ala Ala Arg Asp Ile Asn Arg Phe Tyr Phe Asp Asn
            100                 105                 110

Cys Pro Arg Ile Phe Pro Gln Ser Arg Ser Ser Leu Ala Ala Ala Met
        115                 120                 125

Ser Ala Leu Arg Lys Pro Arg Tyr Asn Gly Lys Tyr Leu Arg Ser Thr
    130                 135                 140

Ile Arg Ser Met Leu Gly Glu Thr Arg Val Ser Asp Ala Leu Thr Asn
145                 150                 155                 160

Val Val Ile Pro Thr Phe Asp Ile Lys Leu Ile Gln Pro Ile Ile Phe
                165                 170                 175

Ser Thr Tyr Asp Val Lys Asn Met Pro Leu Lys Asn Ala Leu Leu Ser
        180                 185                 190

Asp Val Cys Ile Ser Thr Ser Ala Ala Pro Thr Tyr Leu Pro Ala His
    195                 200                 205

Tyr Phe Gln Ile Gln Asp Ala Gly Gly Lys Thr Arg Glu Tyr Asn Leu
    210                 215                 220

Ile Asp Gly Gly Val Ala Ala Asn Asn Pro Thr Met Val Ala Met Thr
225                 230                 235                 240

Gln Ile Thr Lys Met Met Leu Ala Lys Asp Lys Glu Glu Leu Tyr Pro
                245                 250                 255

Val Lys Pro Glu Asp Cys Arg Lys Phe Leu Val Leu Ser Ile Gly Thr
            260                 265                 270

Gly Ser Thr Ser Asp Glu Gly Leu Phe Thr Ala Arg Gln Cys Ser Arg
        275                 280                 285

Trp Gly Val Val Arg Trp Leu Arg Asn Asn Gly Met Ala Pro Ile Ile
    290                 295                 300

Asp Ile Phe Met Ala Ala Ser Ser Asp Leu Val Asp Ile His Ala Ala
305                 310                 315                 320

Val Leu Phe Gln Ser Leu His Ser Asp Gly His Ser Leu Arg Gly Ala
                325                 330                 335

Ala Ala Thr Val Asp Ala Ala Thr Pro Glu Asn Met Arg Thr Leu Val
            340                 345                 350

Gly Ile Gly Glu Arg Met Leu Ala Gln Arg Val Ser Arg Val Asn Val
        355                 360                 365

Glu Thr Gly Arg Tyr Glu Pro Val Pro Gly Glu Gly Ser Asn Ala Asp
    370                 375                 380

Ala Leu Val Ala Leu Ala Arg Gln Leu Ser Asp Glu Arg Arg Ala Arg
385                 390                 395                 400

Ile Ala Arg Arg Ala Ala Ala Ala Cys Ala Gly Gly Ser Arg Cys Cys
                405                 410                 415

Ser Pro Val Lys Thr
            420

<210>  75
<211>  422
<212>  PRT
<213>  Hordeum vulgare

<400>  75

Met Ala Ser Tyr Trp Cys Arg Arg Pro Cys Glu Ser Cys Ser Thr Arg
1               5                   10                  15

Ala Met Ala Gly Ser Val Val Gly Gln Pro Val Ala Pro Gly Gln Arg
            20                  25                  30

Val Thr Val Leu Thr Ile Asp Gly Gly Gly Ile Arg Gly Leu Ile Pro
        35                  40                  45

Gly Thr Ile Leu Ala Phe Leu Glu Ala Arg Leu Gln Glu Leu Asp Gly
    50                  55                  60

Pro Asp Ala Arg Leu Ala Asp Tyr Phe Asp Cys Ile Ala Gly Thr Ser
65                  70                  75                  80

Thr Gly Gly Leu Ile Thr Ala Met Leu Thr Ala Pro Gly Gln Asp Gly
                85                  90                  95

Arg Pro Leu Phe Ala Ala Lys Asp Val Asn Arg Phe Tyr Leu Asp Asn
            100                 105                 110

Gly Pro Tyr Ile Phe Pro Gln Arg Arg Cys Ala Leu Ala Ala Val Thr
        115                 120                 125

Ala Ser Leu Arg Arg Pro Arg Tyr Ser Gly Lys Tyr Leu His Gly Lys
    130                 135                 140

Ile Arg Ser Met Leu Gly Glu Thr Arg Leu Cys Asp Ala Leu Thr Asp
145                 150                 155                 160

Val Val Ile Pro Thr Phe Asp Val Lys Leu Leu Gln Pro Ile Ile Phe
                165                 170                 175

Ser Thr Tyr Asp Ala Arg Asn Met Pro Leu Lys Asn Ala Arg Leu Ala
            180                 185                 190

Asp Ile Cys Ile Gly Thr Ser Ala Ala Pro Thr Tyr Leu Pro Ala His
        195                 200                 205

His Phe His Thr Gln Asp Asp Asn Gly Lys Glu Arg Glu Tyr Asn Leu
    210                 215                 220

Ile Asp Gly Gly Val Ala Ala Asn Asn Pro Thr Met Val Thr Met Thr
225                 230                 235                 240

Gln Ile Thr Lys Lys Met Met Val Lys Asp Arg Glu Glu Leu Tyr Pro
                245                 250                 255

Val Lys Pro Ser Asp Cys Gly Lys Phe Leu Val Leu Ser Ile Gly Thr
            260                 265                 270

Gly Ser Thr Ser Asp Gln Gly Leu Tyr Thr Ala Lys Gln Cys Ser Gln
        275                 280                 285

Trp Gly Ile Ile Arg Trp Leu Arg Asn Lys Gly Met Ala Pro Ile Ile
    290                 295                 300

Asp Ile Phe Met Ala Ala Ser Ser Asp Leu Val Asp Ile His Ala Ala
305                 310                 315                 320

Val Leu Phe Gln Ser Leu His Ser Asp Gly Asn Tyr Leu Arg Ile Gln
                325                 330                 335

Asp Asn Ser Leu His Gly Pro Ala Ala Thr Val Asp Ala Ala Thr Pro
            340                 345                 350

Glu Asn Met Ala Glu Leu Leu Arg Ile Gly Glu Arg Met Leu Ala Gln
        355                 360                 365

Arg Val Ser Arg Val Asn Val Glu Thr Gly Arg Tyr Glu Glu Ile Arg
    370                 375                 380

Gly Ala Gly Ser Asn Ala Asp Ala Leu Ala Gly Phe Ala Lys Gln Leu
385                 390                 395                 400

Ser Asp Glu Arg Arg Thr Arg Leu Gly Arg Arg Arg Val Gly Ala Gly
                405                 410                 415

Arg Leu Lys Ser Arg Arg
            420

<210>  76
<211>  423
<212>  PRT
<213>  Brachypodium distachyon

<400>  76

Met Ala Ser Tyr Ala Cys Arg Arg Pro Cys Glu Ser Cys Arg Thr Arg
1               5                   10                  15

Ala Met Ala Gly Gly Val Val Gly Glu Pro Thr Thr Pro Gly Gln Arg
            20                  25                  30

Val Thr Val Leu Thr Ile Asp Gly Gly Gly Ile Arg Gly Leu Ile Pro
        35                  40                  45

Gly Thr Ile Leu Ala Phe Leu Glu Asp Arg Leu Gln Glu Leu Asp Gly
    50                  55                  60

Pro Asp Ala Arg Leu Ala Asp Tyr Phe Asp Cys Ile Ala Gly Thr Ser
65                  70                  75                  80

Thr Gly Gly Leu Ile Thr Ala Met Ile Thr Ala Pro Gly Glu Glu Gly
                85                  90                  95

Arg Pro Leu Phe Ala Ala Glu Asp Ile Asn Arg Phe Tyr Leu Asp Asn
            100                 105                 110

Gly Pro Gln Ile Phe Pro Gln Lys Arg Ser Ser Leu Met Ser Val Leu
        115                 120                 125

Ala Ser Leu Thr Arg Pro Arg Tyr Asn Gly Lys Phe Leu His Gly Lys
    130                 135                 140

Ile Arg Ser Met Leu Gly Glu Thr Arg Val Cys Asp Thr Leu Thr Asp
145                 150                 155                 160

Val Val Ile Pro Thr Phe Asp Val Arg Leu Leu Gln Pro Ile Ile Phe
                165                 170                 175

Ser Thr Tyr Asp Ala Lys Ser Met Pro Leu Lys Asn Ala Leu Leu Ser
            180                 185                 190

Asp Val Cys Ile Ser Thr Ser Ala Ala Pro Thr Phe Leu Pro Ala His
        195                 200                 205

Tyr Phe Gln Thr Glu Asp Asp Asn Gly Lys Val Arg Glu Tyr Asn Leu
    210                 215                 220

Ile Asp Gly Gly Val Ala Ala Asn Asn Pro Thr Met Val Ala Met Thr
225                 230                 235                 240

Gln Ile Thr Lys Lys Ile Met Ala Lys Asp Lys Glu Glu Leu Tyr Pro
                245                 250                 255

Val Lys Pro Ser Asp Cys Gly Lys Phe Leu Val Leu Ser Ile Gly Thr
            260                 265                 270

Gly Ser Thr Ser Asp Gln Gly Leu Tyr Thr Ala Lys Gln Cys Ser Arg
        275                 280                 285

Trp Gly Ile Ile Arg Trp Leu Arg Asn Lys Gly Met Ala Pro Ile Ile
    290                 295                 300

Asp Ile Phe Met Ala Ala Ser Ser Asp Leu Val Asp Ile His Ala Ala
305                 310                 315                 320

Val Leu Phe Gln Ser Leu His Ser Asp Gly Asp Cys Tyr Leu Arg Ile
                325                 330                 335

Gln Asp Asn Ser Leu Arg Gly Ala Ala Ala Thr Val Asp Thr Ala Thr
            340                 345                 350

Pro Asp Asn Met Arg Glu Leu Val Arg Ile Gly Glu Arg Met Leu Ala
        355                 360                 365

Gln Arg Val Ser Lys Val Asn Val Glu Thr Gly Arg Tyr Glu Glu Met
    370                 375                 380

Gln Gly Ala Gly Thr Asn Ala Asp Ala Leu Ala Gly Phe Ala Arg Gln
385                 390                 395                 400

Leu Ser Asp Glu Arg Arg Ala Arg Phe Gly Pro Arg Asp Gly Ala Pro
                405                 410                 415

Ala Asn Gly Lys Ser Arg Cys
            420

<210>  77
<211>  414
<212>  PRT
<213>  Oryza sativa

<400>  77

Met Ala Gly Cys Val Val Gly Glu Pro Ala Ser Ala Pro Gly Gln Arg
1               5                   10                  15

Val Thr Leu Leu Ala Ile Asp Gly Gly Gly Ile Arg Gly Leu Ile Pro
            20                  25                  30

Gly Thr Ile Leu Ala Phe Leu Glu Ala Arg Leu Gln Glu Leu Asp Gly
        35                  40                  45

Pro Asp Ala Arg Leu Ala Asp Tyr Phe Asp Cys Ile Ala Gly Thr Ser
    50                  55                  60

Thr Gly Gly Leu Ile Thr Ala Met Leu Ala Ala Pro Gly Asp His Gly
65                  70                  75                  80

Arg Pro Leu Phe Ala Ala Ser Asp Ile Asn Arg Phe Tyr Leu Asp Asn
                85                  90                  95

Gly Pro Arg Ile Phe Pro Gln Lys Arg Cys Gly Met Ala Ala Ala Met
            100                 105                 110

Ala Ala Leu Thr Arg Pro Arg Tyr Asn Gly Lys Tyr Leu Gln Gly Lys
        115                 120                 125

Ile Arg Lys Met Leu Gly Glu Thr Arg Val Arg Asp Thr Leu Thr Asn
    130                 135                 140

Val Val Ile Pro Thr Phe Asp Val Arg Leu Leu Gln Pro Thr Ile Phe
145                 150                 155                 160

Ser Thr Tyr Asp Ala Lys Ser Met Pro Leu Lys Asn Ala Leu Leu Ser
                165                 170                 175

Asp Ile Cys Ile Ser Thr Ser Ala Ala Pro Thr Tyr Leu Pro Ala His
            180                 185                 190

Cys Phe Gln Thr Thr Asp Asp Ala Thr Gly Lys Val Arg Glu Phe Asp
        195                 200                 205

Leu Ile Asp Gly Gly Val Ala Ala Asn Asn Pro Thr Met Val Ala Met
    210                 215                 220

Thr Gln Ile Thr Lys Lys Ile Met Val Lys Asp Lys Glu Glu Leu Tyr
225                 230                 235                 240

Pro Val Lys Pro Ser Asp Cys Gly Lys Phe Leu Val Leu Ser Leu Gly
                245                 250                 255

Thr Gly Ser Thr Ser Asp Gln Gly Met Tyr Thr Ala Arg Gln Cys Ser
            260                 265                 270

Arg Trp Gly Ile Val Arg Trp Leu Arg Asn Lys Gly Met Ala Pro Ile
        275                 280                 285

Ile Asp Ile Phe Met Ala Ala Ser Ser Asp Leu Val Asp Ile His Ala
    290                 295                 300

Ala Val Met Phe Gln Ser Leu His Ser Asp Gly Asp Tyr Leu Arg Ile

305                 310                 315                 320

Gln Asp Asn Thr Leu His Gly Asp Ala Ala Thr Val Asp Ala Ala Thr
                325                 330                 335

Arg Asp Asn Met Arg Ala Leu Val Gly Ile Gly Glu Arg Met Leu Ala
            340                 345                 350

Gln Arg Val Ser Arg Val Asn Val Glu Thr Gly Arg Tyr Val Glu Val
        355                 360                 365

Pro Gly Ala Gly Ser Asn Ala Asp Ala Leu Arg Gly Phe Ala Arg Gln
    370                 375                 380

Leu Ser Glu Glu Arg Arg Ala Arg Leu Gly Arg Arg Asn Ala Cys Gly
385                 390                 395                 400

Gly Gly Gly Glu Gly Glu Pro Ser Gly Val Ala Cys Lys Arg
                405                 410

<210>  78
<211>  275
<212>  PRT
<213>  Triticum aestivum

<400>  78

Met Leu Gly Glu Thr Arg Leu Ser Asp Ala Leu Thr Asp Val Val Ile
1               5                   10                  15

Pro Thr Phe Asp Val Lys Leu Leu Gln Pro Ile Ile Phe Ser Thr Tyr
            20                  25                  30

Asp Ala Lys Ser Met Pro Leu Lys Asn Ala Arg Leu Ala Asp Val Cys
        35                  40                  45

Ile Gly Thr Ser Ala Ala Pro Thr Tyr Leu Pro Ala His His Phe His
    50                  55                  60

Thr His Asp Gly Asn Gly Lys Glu Arg Glu Tyr Asn Leu Ile Asp Gly
65                  70                  75                  80

Gly Val Ala Ala Asn Asn Pro Thr Met Val Ala Met Thr Gln Ile Thr
                85                  90                  95

Lys Lys Met Met Gly Lys Asp Arg Glu Glu Leu Tyr Pro Val Glu Pro
            100                 105                 110

Ser Asp Cys Gly Lys Phe Leu Val Leu Ser Val Gly Thr Gly Ser Thr

115                 120                 125

Ser Asp Gln Gly Leu Tyr Thr Ala Lys Gln Cys Ser Gln Trp Gly Ile
    130                 135                 140

Ile Ser Trp Leu Arg Asn Lys Gly Met Ala Pro Ile Ile Asp Ile Phe
145                 150                 155                 160

Met Ala Ala Ser Ser Asp Leu Val Asp Ile His Ala Ala Val Leu Phe
                165                 170                 175

Gln Ser Leu His Ser Asp Ala Asn Tyr Leu Arg Ile Gln Asp Asn Ser
            180                 185                 190

Leu His Gly Pro Ala Ala Thr Val Asp Ala Ala Thr Pro Glu Asn Met
        195                 200                 205

Ala Glu Leu Leu Arg Ile Gly Glu Arg Met Leu Ala Gln Arg Val Ser
    210                 215                 220

Arg Val Asn Val Glu Thr Gly Arg Tyr Glu Glu Val Lys Gly Ala Gly
225                 230                 235                 240

Asn Asn Ala Asp Ala Leu Ala Gly Phe Ala Arg Gln Leu Ser Asp Glu
                245                 250                 255

Arg Arg Thr Arg Leu Gly Ser Arg Arg Gly Gly Ala Gly Arg Leu Lys
            260                 265                 270

Ser Ser Arg
        275

<210>  79
<211>  416
<212>  PRT
<213>  Musa acuminata

<400>  79

Met Ala Ser Asp Gln Thr Pro Pro Ala Thr Ala Asp Ala Pro Ile Ser
1               5                   10                  15

Thr Pro Pro Pro Ser Phe Gly Lys Arg Val Thr Val Leu Cys Ile Asp
            20                  25                  30

Gly Gly Gly Val Arg Gly Leu Ile Pro Ala Thr Ile Ile Ala Phe Leu
        35                  40                  45

Glu Ala Glu Leu Gln Lys Leu Asp Gly Pro Glu Ala Arg Ile Ala Asp

50                  55                  60

Tyr Phe Asp Val Ile Ala Gly Thr Ser Thr Gly Gly Leu Val Thr Ala
65                  70                  75                  80

Met Leu Thr Ala Pro Asn Lys Glu Lys Arg Pro Leu Phe Ala Ala Lys
                85                  90                  95

Asp Ile Val Gln Phe Tyr Leu Asp Asn Ser Pro Lys Ile Phe Pro Gln
            100                 105                 110

Lys Asn Ala Gly Leu Phe Asn Ser Ala Leu Asn Leu Val Gly Ala Val
        115                 120                 125

Ser Gly Pro Lys Tyr Asp Gly Lys Tyr Leu His Ala Ile Ile Arg Gln
    130                 135                 140

Leu Leu Gly Asp Thr Arg Leu Ser Gln Thr Leu Thr Asn Val Val Ile
145                 150                 155                 160

Pro Thr Phe Asp Ile Lys Phe Leu Gln Pro Thr Ile Phe Ser Thr Tyr
                165                 170                 175

Gln Thr Lys Ser Thr Pro Leu Lys Asp Ala Leu Leu Ser Asp Ile Cys
            180                 185                 190

Ile Gly Thr Ser Ala Ala Pro Thr Tyr Leu Pro Gly His Tyr Phe Glu
        195                 200                 205

Thr Lys Asp Asp Asn Gly Asn Lys Arg Ser Phe Asn Leu Val Asp Gly
    210                 215                 220

Gly Val Ala Ala Asn Asn Pro Thr Leu Thr Ala Met Thr Glu Val Ser
225                 230                 235                 240

Lys Glu Ile Leu Leu Ser Asn Pro Asp Phe Phe Ser Tyr Gln Pro Val
                245                 250                 255

Glu Tyr Asp Arg Phe Leu Val Ile Ser Leu Gly Thr Gly Ala Pro Lys
            260                 265                 270

Gln Glu Glu Lys Phe Thr Ala Gln Glu Ser Ala Lys Trp Gly Val Leu
        275                 280                 285

Gly Trp Leu Leu Asn Lys Gly Thr Thr Pro Leu Ile Asp Ile Phe Thr
    290                 295                 300

Gln Ala Ser Ala Asp Met Val Asp Ile His Ala Ser Val Leu Phe Gln
305                 310                 315                 320

Ala Leu Asn Lys Gly Lys Asn Tyr Leu Arg Ile Glu Asp Asp Thr Leu
                325                 330                 335

Thr Gly Gln Thr Ser Ser Val Asp Val Ser Thr Lys Lys Asn Leu Gln
            340                 345                 350

Asp Leu Val Asp Ile Gly Asn Ser Leu Leu Lys Lys Pro Val Ser Arg
        355                 360                 365

Val Asn Ile Glu Thr Gly His Ser Glu Ala Val Asp Gly Glu Gly Thr
    370                 375                 380

Asn Glu Ala Ala Leu Thr Gly Phe Ala Lys Lys Leu Ser Asp Glu Lys
385                 390                 395                 400

Arg Arg Arg Gln Ser Lys Gln Leu Thr Ser Ser Asp Ala Thr Gln His
                405                 410                 415

<210>  80
<211>  402
<212>  PRT
<213>  Elaeis guineensis

<400>  80

Met Gly Ser Asn Glu Ser Pro Val Ala Asn Pro Pro Pro Cys Lys Gly
1               5                   10                  15

Lys Val Val Thr Ile Leu Ser Ile Asp Gly Gly Gly Val Arg Gly Ile
            20                  25                  30

Ile Pro Gly Thr Ile Leu Glu Phe Leu Glu Ala Lys Leu Gln Glu Leu
        35                  40                  45

Asp Gly Pro Asp Ala Arg Ile Ala Asp Tyr Phe Asp Ile Ile Ala Gly
    50                  55                  60

Thr Ser Thr Gly Gly Leu Val Thr Thr Met Leu Thr Ala Pro Asn Lys
65                  70                  75                  80

Asp Asn Arg Pro Leu Phe Ser Ala Lys Asp Ile Ile Gln Phe Tyr Leu
                85                  90                  95

Glu Asn Cys Pro Lys Ile Phe Pro Gln Arg Thr Gly Leu Leu Ala Gly
            100                 105                 110

Ala Leu Asn Leu Phe Gly Ala Val Ser Gly Pro Lys Tyr Asp Gly Lys
        115                 120                 125

Phe Leu His Ser Lys Val Lys Glu Leu Leu Gly Asp Thr Lys Leu His
    130                 135                 140

Gln Thr Leu Thr Asn Ile Val Ile Pro Ala Phe Asp Ile Lys Leu Leu
145                 150                 155                 160

Gln Pro Val Ile Phe Ser Thr Phe Glu Thr Lys Thr Asp Pro Ser Lys
                165                 170                 175

Asp Ala Leu Leu Ser Asp Ile Cys Ile Ser Thr Ser Ala Ala Pro Thr
            180                 185                 190

Tyr Leu Pro Ala His Tyr Phe Glu Thr Lys Asp Ser Gln Gly Lys Thr
        195                 200                 205

Arg Ser Phe Asn Leu Val Asp Gly Gly Val Ala Ala Asn Asn Pro Met
    210                 215                 220

Leu Ile Ala Thr Ser Gln Ile Thr Lys Gln Ile Phe Trp Asn Asn Glu
225                 230                 235                 240

Asp Phe Ser Lys Phe Lys Pro Thr Asp Phe Ala Lys Phe Leu Val Val
                245                 250                 255

Ser Leu Gly Thr Gly Ser Pro Lys Gln Glu Gln Lys Phe Thr Ala Pro
            260                 265                 270

Glu Ser Ala Lys Trp Gly Leu Leu Gly Trp Leu Gln Asn Lys Gly Ser
        275                 280                 285

Thr Pro Ile Ile Asp Ile Phe Ser Gln Ser Ser Ala Asp Met Val Asp
    290                 295                 300

Ile His Ala Ser Ile Leu Phe Gln Ala Leu Arg Ser Glu Lys Asn Tyr
305                 310                 315                 320

Leu Arg Ile Gln Asp Asp Thr Leu Glu Gly Asp Thr Ala Ser Val Asp
                325                 330                 335

Val Ser Thr Ser Glu Asn Leu Arg Lys Leu Val Gln Val Gly Gln Asp
            340                 345                 350

Leu Leu Lys Lys Pro Val Ser Arg Val Asn Leu Glu Thr Gly Val Ser
        355                 360                 365

Glu Ala Cys Asp Val Glu Gly Thr Asn Glu Asp Ala Leu Ile Arg Phe
    370                 375                 380

Ala Lys Met Leu Ser Asn Glu Arg Lys Ser Arg Asn Ala Lys Met Ser
385                 390                 395                 400

Ala Ala

<210>  81
<211>  407
<212>  PRT
<213>  Arabidopsis thaliana

<400>  81

Met Gln Met Asp Ser Pro Lys Ser Pro Leu Gln Pro Pro Thr Tyr Gly
1               5                   10                  15

Asn Leu Val Thr Ile Leu Ser Ile Asp Gly Gly Gly Ile Arg Gly Leu
            20                  25                  30

Ile Pro Ala Val Ile Leu Gly Phe Leu Glu Ser Glu Leu Gln Lys Leu
        35                  40                  45

Asp Gly Glu Glu Ala Arg Leu Ala Asp Tyr Phe Asp Val Ile Ala Gly
    50                  55                  60

Thr Ser Thr Gly Gly Leu Val Thr Ala Met Leu Thr Ala Pro Asn Lys
65                  70                  75                  80

Glu Gly Arg Pro Leu Phe Ala Ala Ser Glu Ile Lys Asp Phe Tyr Leu
                85                  90                  95

Glu Gln Cys Pro Lys Ile Phe Pro Gln Asp His Phe Pro Phe Ser Ala
            100                 105                 110

Ala Lys Lys Leu Val Lys Ser Leu Thr Gly Pro Lys Tyr Asp Gly Lys
        115                 120                 125

Tyr Leu His Gln Leu Ile His Ala Lys Leu Gly Asp Thr Lys Leu Ser
    130                 135                 140

Gln Thr Leu Thr Asn Val Val Ile Pro Thr Phe Asp Ile Lys His Leu
145                 150                 155                 160

Gln Pro Thr Ile Phe Ser Ser Tyr Glu Val Lys Asn His Pro Leu Lys
                165                 170                 175

Asp Ala Ala Leu Ala Asp Ile Ala Ile Ser Thr Ser Ala Ala Pro Thr
            180                 185                 190

Tyr Leu Pro Ala His Phe Phe Lys Val Glu Asp Leu Asn Gly Asn Ala
        195                 200                 205

Lys Glu Tyr Asn Leu Ile Asp Gly Gly Val Ala Ala Asn Asn Pro Ala
    210                 215                 220

Leu Leu Ala Ile Gly Glu Val Thr Asn Glu Ile Ser Gly Gly Ser Ser
225                 230                 235                 240

Asp Phe Phe Pro Ile Arg Pro Asn Asp Tyr Gly Arg Phe Leu Val Leu
                245                 250                 255

Ser Leu Gly Thr Gly Asn His Lys Ala Glu Glu Lys Phe Asn Ala Lys
            260                 265                 270

Glu Val Ala Gly Trp Gly Leu Leu Asn Trp Leu Thr His Asp Asn Ser
        275                 280                 285

Thr Pro Ile Ile Asp Ala Phe Ser Gln Ala Ser Ser Asp Met Val Asp
    290                 295                 300

Phe His Leu Ser Ala Val Phe Arg Ala Leu His Ser Glu Ala Asn Tyr
305                 310                 315                 320

Ile Arg Ile Gln Asp Asp Thr Leu Thr Gly Asp Ala Ala Ser Val Asp
                325                 330                 335

Ile Ala Thr Val Glu Asn Leu Asp Ile Leu Ala Lys Thr Gly Asp Glu
            340                 345                 350

Leu Leu Lys Lys Pro Val Ala Arg Val Asn Leu Asp Ser Gly Cys Asn
        355                 360                 365

Glu Asn Ala Tyr Glu Thr Thr Asn Glu His Ala Leu Ile Lys Leu Ala
    370                 375                 380

Gly Ile Leu Ser Lys Glu Lys Lys Ile Arg Asp Ile Arg Ser Pro His
385                 390                 395                 400

Ala Lys Ala Pro Ile Arg Ile
                405

## Description

### CLAIM FOR PRIORITY

This application claims the benefit under 35 U.S.C. § 120 of U.S. Provisional Application No. 62/256,902, filed on Nov. 18, 2015, and U.S. Provisional Application No. 62/300,507, filed on Feb. 26, 2016, the contents of which are incorporated herein by reference

### SEQUENCE LISING

This application is accompanied by a sequence listing entitled 80906-PCT_ST25.txt, created November 16, 2016, which is approximately 392 kilobytes in size. This sequence listing is incorporated herein by reference in its entirety. This sequence listing is submitted herewith via EFS-Web, and is in compliance with 37 C.F.R. § 1.824(a)(2)-(6) and (b).

### FIELD OF THE INVENTION

The presently disclosed subject matter relates to the diagnostic detection of haploid induction ("HI") or its absence and/or presence in plants which are or are not haploid inducers. More particularly, the presently disclosed subject matter relates to nucleic acids that can be employed for inducing HI in plants and/or the biological activities which can be modified in order to produce or prevent HI in either a plant that would otherwise exhibit HI or in a plant that would otherwise not exhibit HI. Even more particularly, the presently disclosed subject matter relates to a nucleic acid molecule that encodes a biologically active molecule as well as methods for using the same to regulate HI in plants.

Provided here are a series of independent human-induced mutations found in at least one patatin-like phospholipase A2a ("PLA") gene of maize; maize plants having these mutations in at least one of their PLA genes; and a method of creating and identifying similar and/or additional mutations in the PLA gene by screening pooled and/or individual maize plants. The maize plants of the present invention induce haploidy as a result of non-transgenic mutations in at least one of their PLA genes. Also provided are methods of inducing de novo haploid induction by chemical application to the reproductive tissues of plants. Also provided are methods of increasing the seed setting rate and haploid induction rate ("HIR", defined herein as the number of surviving haploid kernels over the total number of kernels after an ear is pollinated with haploid inducer pollen), by chemical application to reproductive plant tissues during the pollination.

### BACKGROUND

Pollination is a complex process. Angiosperm pollen grains consist of a large vegetative cell and two male gametes (sperm cells). After landing on the stigma, the grain germinates a pollen tube that exhibits rapid tip-growth as it navigates down the female transmitting tract, guided by chemo-attractants secreted by the two synergid cells at the micropylar end of the embryo sac. During transmittance down the tube, the sperm are connected to each other and the vegetative nucleus by a stringy cytoplasm called the male germ unit. Shortly after contact with one of the two synergids, the pollen tube bursts and the two sperm are propelled across the dying synergid cell cytoplasm to independently fuse with the egg and central cells of the embryo sac, completing double fertilization. Even after initial contact, fertilization failure events can be rescued by a second pollen tube that fertilizes the embryo sac via interaction with the persistent synergid cell.

Breeders cross inbred parent lines, one acting as a male and one as a female, in order to form hybrid seed. The process of developing inbred parent lines which are substantially homozygous usually requires a hybrid cross to be selected and self-pollinated (selfed) for numerous generations to become nearly homozygous. This process is time consuming and expensive. To shorten the time to develop homozygous inbreds in maize, rice, wheat, barley, and other crops, breeders may opt to use a haploid inducer line to induce haploid seed production on a hybrid parent. The chromosomes of the haploid plants are then doubled, for example by a chromosome doubling agent such as colchicine, to form doubled haploid homozygous inbred lines.

Haploid induction ("HI") is a class of plant phenomena characterized by loss of the inducer chromosomes during embryo development. WO2012/030893, incorporated herein by reference, describes a region of maize chromosome 1 that is may be responsible for haploid induction. The identified markers in that region increased haploid induction are described as being between 48,249,509-51,199,249, which is associated with a public marker umc1169 that has the physical position of (60,213,661). This region does not seem to align with the Haploid Induction region in Stock 6. Dong et al., (2013) Theor. Appl. Genet. 126: 1713-1720, describe a QTL located in bin 1.04 which explains up to 66% of the genotypic variance for HIR.

Haploid induction has been observed in numerous plant species, such as sorghum, barley, wheat, and other grasses. In maize, HI appears to be a result of rearrangements of, mutations in, and/or recombinations, insertion, or deletions within a region of chromosome 1 (with the notable exception of the *ig* type haploid induction, which is a result of a mutation in the *INDETERMINATE GAMETOPHYTE1* gene on chromosome 3). Purported HI lines have been studied and roughly identified. However, experimental evidence demonstrating a causative genetic agent of HI in maize has not been presented. Nor have the markers listed herein that associate with this trait been previously identified.

In maize haploid seed or embryos are specifically produced by making crosses between a haploid inducer male (i.e., "haploid inducer pollen") and virtually any ear that one chooses - the ear could be of any inbred, hybrid, or other germplasm. Haploids are produced when the haploid inducer pollen DNA is not fully transmitted and/or maintained through the first cell divisions of the embryos. The resulting phenotype is not fully penetrant, with some ovules containing haploid embryos, and others containing diploid embryos, aneuploid embryos, chimeric embryos, or aborted embryos. The haploid kernels have embryos that contain only the maternal DNA plus normal triploid endosperm. After haploid induction, haploid embryos or seed are typically segregated from diploid and aneuploid siblings using a phenotypic or genetic marker screen and grown or cultured into haploid plants. These plants are then converted either naturally or via chemical manipulation (i.e., colchicine) into doubled haploid (DH) plants which then produce inbred seed.

HI lines contain a quantitative trait locus ("QTL") on Chromosome 1 responsible for at least 66% of the variation in haploid induction. The QTL causes haploid induction at different rates when it is introgressed into various backgrounds. All haploid inducer lines used in the seed industry are derivatives of the founding HI line, known as Stock6, and all have the haploid inducer chromosome 1 QTL mutation. Here, we uncover the key mutation in that QTL which, when complemented, rescues normal reproduction. While the origins of this mutation are unclear, it is in all inducer lines, including Stock6.

Plant breeding is facilitated by the use of doubled haploid (DH) plants. The production of DH plants enables plant breeders to obtain inbred lines without multigenerational inbreeding, thus decreasing the time required to produce homozygous plants. DH plants provide an invaluable tool to plant breeders, particularly for generating inbred lines, QTL mapping, cytoplasmic conversions, trait introgression, and F2 screening for high throughput trait improvement. A great deal of time is spared as homozygous lines are essentially generated on one generation, negating the need for multigenerational conventional inbreeding. In particular, because DH plants are entirely homozygous, they are very amenable to quantitative genetics studies. The production of haploid seed is critical for the doubled haploid breeding process. Haploid seed are produced on maternal germplasm when fertilized with pollen from a gynogenetic inducer, such as Stock 6.

Maize haploid inducer plants produce pollen which, when crossed onto non-inducer germplasm, results in the gynogenic development of haploid seeds. Unfortunately, this process often yields a low frequency of haploid kernels. Inefficient haploid induction frequency is a limiting factor in maize doubled haploid breeding programs.

### SUMMARY

A high HIR allows a higher frequency of haploid seeds to be formed on the parent plant of interest. The parent plants can be pre-screened with genetic markers associated with desired traits or phenotypically-observed traits to enrich the genetic potential of the parent plants. When these desired parent plants are pollinated by a haploid inducer that has a higher HIR, a higher potential of desired doubled haploids is obtained with the desired genotype and phenotype.

Although the doubled haploid process resulted in faster production of homozygous inbreds, the volume of doubled haploid inbreds that are produced may be limited. Known inducer lines, including but not limited to: Stock 6, MHI (Moldovian Haploid Inducer), indeterminate gametophyte ("*ig*") mutation, KEMS, RWK, ZEM, ZMS, and KMS. All have a relatively low HIR. Stock 6, for example, only induces 1-3% haploid seeds. As such, the induction of haploids has been a rate-limiting step in the process of producing doubled haploid lines.

We have invented a way to induce haploid production and/or increase the haploid induction rate in plants by treating the plants with a lipid compound, a phospholipase inhibitor, and a fatty acid desaturase inhibitor. One such set of methods includes applying specific chemicals to reproductive tissues when crossing with wild-type (non-haploid inducer) pollen. For the first time, we have triggered *de novo* haploid induction chemically. This is accomplished by administering a concentration of the phospholipase inhibitor methyl alpha linolenyl fluorophosphonate (MALFP) to the flower during pollination, which leads to a high rate of haploid induction: up to 9% HIR. Separately, we have triggered *de novo* haploid induction by administering a concentration of arachidonyl fluorophosphonate (MAFP) to the flower during pollination. Separately, we have triggered *de novo* haploid induction by administering a concentration of 1,2-distearoyl-sn-glycero-3-phosphatidyl choline (also known as distearyl-phosphatidyl choline; "DSPC") to the flower during pollination. We have also triggered *de novo* haploid induction by administering a concentration of alpha linolenic acid to the flower during pollination. We have also triggered de novo haploid induction by administering readily available compounds, including corn oil and linseed oil, as well as chemically-synthesized linoleic acid, oleic acid ethyl ester (OAEE), arachidonic acid methyl ester, (AAME) and the phospholipase inhibitor manoalide. We have also increased the rate of haploid induction when crossing plants with a haploid inducer line (e.g., RWK, Stock 6, or ZMS) by administering a concentration of the phospholipase inhibitor methyl alpha linolenyl fluorophosphonate (MALFP). At concentrations of 2% MALFP+surfactant blend 91 emulsified with a buffered DML solution, we have doubled the haploid induction rate in pollinations using RWK as the pollinator. The typical induction rate in RWK is about 10 - 18%. With MALFP applied the induction rate increases to 20-35%. We have also boosted the haploid induction rate by applying concentrations of linoleic acid (LLE), linoleic acid ethyl ester (LLAEE), and the phospholipase inhibitor called methyl arachidonyl fluorophosphonate (MAFP) to the flower during pollination. Furthermore, doing so in this manner also reduces the rate of embryo abortion and increases the rate of kernel formation for MALFP, LLAEE, MAFP, and LLE. Together these effects lead to an increase in the total number of haploid embryos recovered on the ear after pollination.

We have also cloned and characterized the gene responsible for haploid induction in maize. The gene is PLA2 and it has pollen-specific expression. The PLA2 protein appears to localize to the sperm-cell cytoplasm, perhaps the endoplasmic reticulum or golgi bodies. The identification of the gene has led to inventions of several new techniques to improve the haploid induction process, defined as the act of producing haploid embryos, kernels, seed, or plants by crossing any ear with haploid inducer pollen. The identification of the gene has also led to the inventions of new methods to induce haploids. Another set includes methods to create new haploid inducer lines by changing the sequence of the causative gene, either through targeted mutagenesis, TILLING, or CRISPR/Cas9. Expression of the PLA2 protein may be downregulated using RNAi or by using targeting mutagenesis in the promoter, 3' UTR, 5' UTR, or the splice sites.

Based on the identification of this mutation, we invent ways to modify and improve the haploid induction process and, for the first time, we disclose methods to produce haploids *de novo* via chemical treatment during pollination. We show methods to increase the haploid induction rate ("HIR," i.e., the percentage of haploid embryos found on a given haploid induced ear) and also methods to raise the kernel survival rate during haploid induction. We also discuss methods to create new haploid inducer lines using genetic modification ("GM") or targeted mutagenesis strategies.

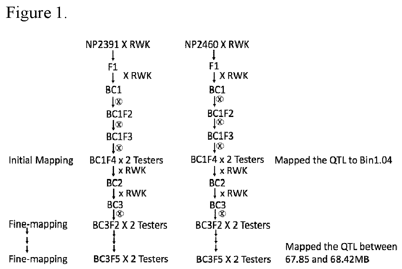

The haploid induction process can be improved through a variety of methods. First, one can strive to improve the average HIR. The HIR is rate-limiting in large-scale doubled haploid ("DH") plant production because the HIR is relatively low. See Figure 1. With rare exceptions, the HIR is usually less than 25% and most frequently is in the 10-20% range, meaning 75-90% of kernels are diploid. With some low frequency, aneuploid embryos are also produced during haploid induction. Examples of compounds that have been shown to increase the number of haploids formed and/or the rate of haploid formation during haploid inducer crosses without increasing the rate of embryo abortion include methyl alpha linolenyl fluorophosphonate (MALFP), methyl arachidonyl fluorophosphonate (MAFP), linoleic acid (LLA), and linoleic acid ethyl ester (LLAEE). Second, one can decrease the rate of kernel abortion and fertilization failure during haploid induction, leading to the formation of more kernels per ear. Kernel abortion occurs after a successful fertilization occurs but when a functional embryo or endosperm fails to develop, and as a result a small, colorless kernel grows lacking a mature embryo inside. The kernel abortion rate is high in haploid induction processes-between 10-50% of fertilized ovules on an ear. This limits the number of haploid kernels one can recover per ear. Fertilization failure results when an ovule fails to be fertilized by a pollen grain, and is characterized by the absence of any kernel development post-pollination. This rate tends to be high during haploid induction-between 10-70% of kernels on some haploid induced ears fail to be fertilized, depending on the alignment of the male and female flower maturity, the type of cross made, and the male and female genetics. Examples of compounds that have shown to increase the rate of viable kernel formation by reducing fertilization failure and embryo abortion include the same four mentioned above: MALFP, MAFP, LLA, and LLAEE. These four molecules, when applied to pollen, tassels or other flower parts during haploid induction crosses, increase the number of haploids formed by increasing both the haploid induction rate and the kernel count.

Finally, one could improve the haploid inducer process by negating the need for haploid inducer males, by enabling *de novo* haploid induction to occur on an ear or in an ovule when it is self-crossed or crossed by pollen from a non-haploid inducer line. These *de novo* haploid induction methods, including applications of phospholipids such as DSPC, fatty acids such as linolenic acid (LNA), common mixtures of triglycerides such as corn oil and linseed oil, or phospholipase inhibitors such as MALFP, could be applied in any outcross or self-pollination to induce haploids in corn. Here we describe examples that fall into one or more of these categories, constituting improvements to or new inventions in the haploid induction process.

The present invention is directed to a method for inducing haploid embryos in a cross between two parent plants. This is done by altering the expression of a phospholipase in one of the parent plants. This altering may be accomplished in several ways: either by causing one of the parent plants to express a mutated phospholipase; or by administering a small interfering RNA to one or both of the parent plants, which causes suppression of the phospholipase; or by transforming one of the parent plants with a mutated phospholipase; or by editing one of the parent plants' phospholipase, for example by site-directed mutagenesis such as CRISPR- or TALEN-based technologies. When the phospholipase's expression in one of the parent plants is altered by one of these techniques, then when that parent plant is used in a cross, at least one haploid embryo is produced.

In one embodiment of the method, the phospholipase is a patatin-like phospholipase. In another embodiment, the patatin-like phospholipase is an orthologue of pPLAIIa, which is encoded by a nucleotide sequence comprising SEQ ID NO: 1 or a sequence at least 70% identical to SEQ ID NO: 1. The nucleotide sequence encoding the patatin-like phospholipase may be mutated, and in one embodiment the nucleotide sequence has a frameshift mutation which creates an artificial stop codon. The frameshift mutation sequence comprises SEQ ID NO: 3 or a sequence at least 70% identical to SEQ ID NO: 3.

Other mutations are possible and are within the scope of the invention. Using site-directed mutagenesis can be used to create more mutations of a phospholipase. CRISPR/Cas9, TALENs, zinc fingers, and meganucleases are methods of accomplishing site-directed mutagenesis in accordance with embodiments of the invention.

The present invention is useful in many types of crosses between plants. In one embodiment, the parent plants used in the cross are monocot plants, such as maize, rice, barley, and wheat. The parent plants may be of the same monocot species, or they may different species. In another embodiment, the parent plants used in the cross are dicot plants, such as soybean, sunflower, tomato, pepper, sugar beet, or Brussels sprouts. In a preferred embodiment, the parent plants are maize or rice plants. Within the scope of this invention are the haploid embryo produced by the method, the haploid seed comprising the haploid embryo, and the haploid plant grown from the haploid seed. Also within the scope of this invention is a doubled haploid produced by exposing the haploid embryo to a chromosome doubling agent, such a colchicine or trifluralin.

The present invention is directed to a cDNA comprising SEQ ID NO: 3, or a sequence orthologous to SEQ ID NO: 3, or a sequence 70% identical to SEQ ID NO: 3. In preferred embodiments, the sequence orthologous to SEQ ID NO: 3 encompasses patatin-like phospholipases from maize, rice, wheat, soybean, and sunflower. In particular, a sequence orthologous to SEQ ID NO: 3 includes the rice gene Os03g27610. In a more preferred embodiment, the sequence orthologous to SEQ ID NO: 3 encompasses SEQ ID NOs: 23 and 73-81.

The present invention is directed to a plant containing a human-induced, non-transgenic mutation within its patatin-like phospholipase gene. In a preferred embodiment, the patatin-like phospholipase gene is a pPLAIIa. In another embodiment, the mutation causes a premature stop codon to be encoded in the gene. In a more preferred embodiment, the plant is any monocot or any dicot, but especially preferred is maize or rice.

The present invention is also directed to a method of inducing haploid embryos and seed production by treating plant reproductive tissues with a compound comprising a lipid or a phospholipase inhibitor. In one embodiment, the treatment occurs before, during, or immediately after pollination. The plants treated may be any monocot or dicot, but in preferred embodiments the plants are maize, rice, wheat, soybean, sunflower, and sugar beet. In another embodiment, the lipid or phospholipase inhibitor is selected from the group found in Table 7. In preferred embodiments, the treatment compound comprises methyl alpha-linolenoyl fluorophosphonate ("MALFP"), linoleic acid ethyl ester ("LLAEE"), linoleic acid ("LLA"), corn oil, distearyl-phosphatidyl choline ("DSPC"), or methyl arachidonyl fluorophosphonate ("MAFP").

In another embodiment, the lipid or phospholipase inhibitor matches the following formula (I): 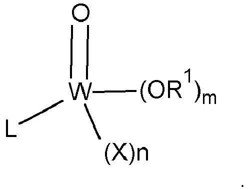 Within the scope of the invention, W of formula (I) is carbon ("C"), phosphorus ("P"), or sulfur ("S"); m may be 0 or 1; n may be 0 or 1; X is selected from the group consisting of OH, CN, O(C₁-C₄ alkyl), halogen, C₁-C₄ alkyl, and C₁-C₄ alkyl substituted by one, two, or three halogen or carbonyl; R¹ is selected from the group consisting of H, C₁-C₆ alkyl, and C₁-C₆ alkyl substituted by one or more hydroxyl groups wherein optionally one or more of said hydroxyl groups is esterified with a radical independently selected from the group consisting of: 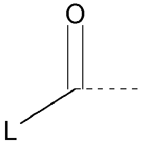 and 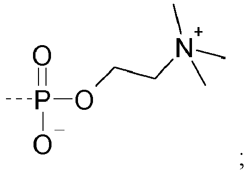 or R¹ is a bond to W when W is S; each L is independently a C₂-C₃₀ carbon chain, said carbon chain optionally comprising one or more groups independently selected from alkenyl, alkynyl, phenyl, and heteroaryl, and said carbon chain optionally interrupted by 1-6 oxygen atoms. When W is C and m is 1, then n is 0; however, when W is C and m is 0, then nis 1.

In a preferred embodiment, X is F, Cl, CF₃, CCl₃, CF₂H, CCl₂H, CF₂CF₃, CCl₂CCl₃, CF₂Cl, CF₂CH₃, C(O)CH₃ or CN. In a more preferred embodiment, the halogen is F or Cl.

It is important to note that in R¹, C₁-C₆ alkyl includes linear, branched, and cyclic alkyl groups. In a preferred embodiment, R¹ is C₁-C₆ alkyl substituted by one to six hydroxyl groups.

In one embodiment, each L of formula (I) is independently a C₂-C₃₀ carbon chain, including branched chains, which may be saturated, unsaturated, or polyunsaturated. In a preferred embodiment, the carbon chain of L comprises one to four groups independently selected from alkenyl, alkynyl, phenyl, and heteroaryl. Unsaturation is in the form of double or triple bonds. The alkenyl or alkynyl can be within the carbon chain, or terminal with respect to the carbon chain. Phenyl and/or heteroaryl rings can be joined in the carbon chain at the ortho, meta, or para position, or can be terminal to the carbon chain. Aryl rings may optionally be substituted. In a preferred embodiment, the carbon chain is interrupted by one to six oxygen atoms. As used herein, "interrupted by" means that the carbon chain comprises at least two carbons in sequence, followed by an oxygen atom. For example, -CH₂- CH₂-O- CH₂-CH₂-CH₃ is a carbon chain interrupted by an oxygen atom. In a preferred embodiment, the carbon chain is interrupted by one to two oxygen atoms.

Examples of suitable carbon chains compliant with the requirements of L include: (CH₂)₈-(CH)₂-CH₂-(CH)₂-CH₂-(CH)₂-CH₂-CH₃; (CH₂)₃-(CH)₂-CH₂-(CH)₂-CH₂-(CH)₂-CH₂-(CH)₂-(CH₂)₄-CH₃; (CH₂)₇-(CH)₂-(CH₂)₇-CH₃; (CH₂)₈-(CH)₂-CH₂-phenyl-CH₂-(CH)₂-CH₂-CH₃; (CH₂)₈-(CH)₂-(CH₂)₂-O-CH₂-(CH)₂-CH₂-CH₃; and (CH₂)₈-(CH)₂-CH₂-phenyl-O-(CH₂)₃-CH₃.

The treatment of these compounds is accomplished by applying the compound by any of the following techniques: dipping, injection, spray-based topical application, nebulizer, pipette-based topical application, and brush-based topical application, and any other topical application. Preferred embodiments use a spray or a nebulizer.

The present invention is further directed to a method of increasing seed set and reducing embryo abortion in plants during haploid induction, comprising treating plant reproductive tissues, such as silks, tassels, pollen, ears, kernels, or other flowering tissues, with a suitable concentration of compound prior to, during, or following pollination. In one embodiment, the compound is selected from the group consisting of the members of Table 7. In another embodiment, the compound is methyl alpha-linolenyl fluorophosphonate (MALFP). In another embodiment, the compound is linoleic acid (LLA), linoleic acid ethyl ester (LLAEE), linolenic acid (LNA), distearoyl-phosphatidylcholine (DSPC), or methyl arachidonyl fluorophosphonate (MAFP).

The present invention is further directed to a method of increasing the rate of haploid induction in a plant, comprising applying a lipid composition to tissues of the plant immediately preceding, during, or immediately following pollination. In one embodiment, the plant is a monocot or a dicot; or the plant is a maize plant or a rice plant. In another embodiment, the lipid acts as a phospholipase inhibitor and/or a fatty acid desaturase inhibitor. In another embodiment, the lipid is a fatty acid (e.g., LLA) or fatty acid ester (e.g., LLAEE) of a particular chain length and degree of saturation (eighteen carbons, and two double bonds), which is a class of fatty acid chain length that is lacking in haploid inducer pollen. By way of illustration and not limitation, the lipid is, for example, the phospholipase inhibitor methyl alpha linolenyl fluorophosphonate (MALFP), dissolved in a buffered DMSO solution at concentrations of MALFP between 0.0001 mg/mL and lg/mL, or dissolved in a surfactant formulation and then emulsified in a buffered dimethylactamide (DML) solution at concentrations of MALF between 0.0001 mg/mL and 1 g/mL. By way of further illustration and not limitation, the lipid composition is applied by dipping, injection, spray, mist, nebulization, pouring, brush, or any other method of application on the reproductive tissues of the plant. In one embodiment, the lipid composition is combined with pollen in a mixture, which mixture is then applied to the tissues of the plant. In another embodiment, the mixture is applied to the reproductive tissues of the plant, for example, the pollen or silks of a maize plant.

The present invention is directed to a method of inducing de novo haploid induction in a plant, comprising administering a lipid compound to at least a reproductive tissue of the plant during pollination, preceding pollination, or following pollination. In one embodiment, the plant is selected from the group consisting of monocots and dicots. In another embodiment, the plant is selected from the group consisting of rice, maize, wheat, sorghum, tomato, sugar beet, millet, barley, soybean, sunflower, cotton, oats, tobacco, vegetables, fruits, and any other crop plant.

In accordance with one exemplary embodiment, this invention includes a maize or a rice plant capable of inducing haploidy due to a human-induced mutation in the patatin-like phospholipase AIIα ("PLA") gene, as well as seeds, pollen, plant parts and progeny of that plant.

In accordance with yet another exemplary embodiment, this invention includes a maize or a rice plant capable of inducing haploids created by the steps of obtaining plant material from a parent maize or rice plant, inducing at least one mutation in at least one copy of a PLA gene of the plant material by treating the plant material with a mutagen to create mutagenized plant material, culturing the mutagenized plant material to produce progeny rice or maize plants, analyzing progeny rice or maize plants to detect at least one mutation in at least one copy of a PLA gene, selecting progeny rice or maize plants that have capability to induce haploids compared to the parent rice or maize plant; and repeating the cycle of culturing the progeny rice or maize plants to produce additional progeny plants having capability to induce haploids.

### BRIEF DESCRIPTION OF THE SEQUENCES IN THE SEQUENCE LISTING

SEQ ID NO: 1 is the cDNA sequence of an unmutated phospholipase found in GRMZM2G471240-NIL. The unmutated phospholipase allele is herein renamed *MATRILINEAL.*
SEQ ID NO: 2 is the amino acid sequence encoded by SEQ ID NO: 1.
SEQ ID NO: 3 is the cDNA nucleotide sequence of a mutated phospholipase found in GRMZM2G471240-mtl, comprising a 4 base pair insertion. The mutated phospholipase allele is herein renamed *matrilineal.*
SEQ ID NO: 4 is the amino acid sequence encoded by SEQ ID NO: 3.
SEQ ID NO: 5 is the GRMZM2G471240_nil.F1 primer.
SEQ ID NO: 6 is the GRMZM2G471240-nil.R1 primer.
SEQ ID NO: 7 is the GRMZM2G471240_rwk.F1 primer.
SEQ ID NO: 8 is the GRMZM2G471240_rwk.R1 primer.
SEQ ID NO: 9 is the nucleotide sequence for the TALEN-induced MTL mutation in Event 39A ID T1 individual 22808-3954 allele 1.
SEQ ID NO: 10 is the nucleotide sequence for the TALEN-induced MTL mutation in Event 23A T1 individual ID 22808-3924 allele 1.
SEQ ID NO: 11 is the nucleotide sequence for the TALEN-induced MTL mutation in Event 81A T1 individual ID 22808-3932, Event 81A individual ID 22808-3317, and Event 81A individual ID 22808-3303.
SEQ ID NO: 12 is the nucleotide sequence for the TALEN-induced MTL mutation in Event 39A ID 22808-3954 allele 2.
SEQ ID NO: 13 is the nucleotide sequence for the TALEN-induced MTL mutation in Event 23A ID 22808-3924 allele 2.
SEQ ID NO: 14 is the nucleotide sequence for the TALEN-induced MTL mutation in Event 38A T1 individual ID 22808-4108 allele 1.
SEQ ID NO: 15 is the nucleotide sequence for the CRISPR-induced MTL mutation in Event 18A T1 individual ID 22807-4016.
SEQ ID NO: 16 is the nucleotide sequence for the CRISPR-induced MTL mutation in Event 27A T1 individual ID 22807-4073 allele 1.
SEQ ID NO: 17 is the nucleotide sequence for the CRISPR-induced MTL mutation in Event 27A T1 individual ID 22807-4081 allele 1.
SEQ ID NO: 18 is the nucleotide sequence for the CRISPR-induced MTL mutation in Event 76A T1 individual ID 22873-3999.
SEQ ID NO: 19 is the nucleotide sequence for the CRISPR-induced MTL mutation in Event 32A T1 individual ID 22873-3991.
SEQ ID NO: 20 is the nucleotide sequence for a CRISPR guide RNA.
SEQ ID NO: 21 is the genomic nucleotide sequence for Os03g27610, the rice PLA2 ortholog.
SEQ ID NO: 22 is the cDNA sequence for SEQ ID NO: 21.
SEQ ID NO: 23 is the amino acid sequence encoded by SEQ ID NO: 22.
SEQ ID NO: 24 is the nucleotide sequence of unmutated GRMZM2G471240-B73.
SEQ ID NO: 25 is the nucleotide sequence of unmutated GRMZM2G471240-RWK.
SEQ ID NO: 26 is the nucleotide sequence of unmutated GRMZM2G471240-ST6.
SEQ ID NO: 27 is the amino acid sequence encoded by SEQ ID NO: 24.
SEQ ID NO: 28 is the amino acid sequence encoded by SEQ ID NO: 25.
SEQ ID NO: 29 is the amino acid sequence encoded by SEQ ID NO: 26.
SEQ ID NO: 30 is the nucleotide sequence for the expression cassette of construct 22466, comprising wildtype *MATRILINEAL.*
SEQ ID NO: 31 is the nucleotide sequence for the expression cassette of construct 22467, comprising wildtype *PHOSPHOGLYCERATEMUTASE.*
SEQ ID NO: 32 is the nucleotide sequence for the expression cassette of construct 22503, comprising a sequence encoding a stem-loop structure targeting exon 2 of *MATRILINEAL.*
SEQ ID NO: 33 is the nucleotide sequence for the expression cassette of construct 22513, comprising a sequence encoding a stem-loop structure targeting exon 4 of *MATRILINEAL.*
SEQ ID NO: 34 is the nucleotide sequence for the expression cassette of construct 22807, comprising sequences encoding CRISPR/Cas9 editing machinery targeting *MATRILINEAL* in NP2222.
SEQ ID NO: 35 is the nucleotide sequence for the expression cassette of construct 22808, comprising sequences encoding CRISPR/Cas9 editing machinery targeting *MA TRILINEAL* in NP2222.
SEQ ID NO: 36 is the nucleotide sequence for the expression cassette of construct 22873, comprising sequences encoding CRISPR/Cas9 editing machinery targeting *MATRILINEAL* in NP2222.
SEQ ID NO: 37 is the nucleotide sequence for the expression cassette of construct 23123, comprising sequences encoding TALEN editing machinery targeting *MATRILINEAL* in NP2222.
SEQ ID NO: 38 is the nucleotide sequence for the expression cassette of construct 23501, rice gRNA targeting exon 4 with dual guides.
SEQ ID NO: 39 is the nucleotide sequence for the expression cassette of construct 23501, rice gRNA targeting exon 4 single guide.
SEQ ID NO: 40 is the nucleotide sequence for the expression cassette of construct 23501, rice gRNA targeting exon 1 with dual guides.
SEQ ID NO: 41 is the nucleotide sequence for the expression cassette of construct 23501, rice gRNA targeting exon 1 with single guide.
SEQ ID NO: 42 is the nucleotide sequence for the TALEN-induced MTL mutation in Event 38A ID 22808-4108 allele 2.
SEQ ID NO: 43 is the nucleotide sequence for the CRISPR-induced MTL mutation in Event 27A ID 22807-4073 allele 2.
SEQ ID NO: 44 is the nucleotide sequence for the CRISPR-induced MTL mutation in Event 27A ID 22807-4081 allele 2.
SEQ ID NO: 45 is the nucleotide sequence for TILLING line 1139.
SEQ ID NO: 46 is the nucleotide sequence for TILLING line 3594.
SEQ ID NO: 47 is the nucleotide sequence for TILLING line 0505.
SEQ ID NO: 48 is the nucleotide sequence for TILLING line 2658.
SEQ ID NO: 49 is the nucleotide sequence for TILLING line 1983.
SEQ ID NO: 50 is the nucleotide sequence for TILLING line 2732.
SEQ ID NO: 51 is the nucleotide sequence for TILLING line 2414.
SEQ ID NO: 52 is the amino acid sequence encoded by SEQ ID NO: 45.
SEQ ID NO: 53 is the amino acid sequence encoded by SEQ ID NO: 46.
SEQ ID NO: 54 is the amino acid sequence encoded by SEQ ID NO: 47.
SEQ ID NO: 55 is the amino acid sequence encoded by SEQ ID NO: 48.
SEQ ID NO: 56 is the amino acid sequence encoded by SEQ ID NO: 49.
SEQ ID NO: 57 is the amino acid sequence encoded by SEQ ID NO: 50.
SEQ ID NO: 58 is the amino acid sequence encoded by SEQ ID NO: 51.
SEQ ID NO: 59 is the amino acid sequence encoded by SEQ ID NO: 9.
SEQ ID NO: 60 is the amino acid sequence encoded by SEQ ID NO: 10.
SEQ ID NO: 61 is the amino acid sequence encoded by SEQ ID NO: 11.
SEQ ID NO: 62 is the amino acid sequence encoded by SEQ ID NO: 12.
SEQ ID NO: 63 is the amino acid sequence encoded by SEQ ID NO: 13.
SEQ ID NO: 64 is the amino acid sequence encoded by SEQ ID NO: 14.
SEQ ID NO: 65 is the amino acid sequence encoded by SEQ ID NO: 15.
SEQ ID NO: 66 is the amino acid sequence encoded by SEQ ID NO: 16.
SEQ ID NO: 67 is the amino acid sequence encoded by SEQ ID NO: 17.
SEQ ID NO: 68 is the amino acid sequence encoded by SEQ ID NO: 18.
SEQ ID NO: 69 is the amino acid sequence encoded by SEQ ID NO: 19.
SEQ ID NO: 70 is the amino acid sequence encoded by SEQ ID NO: 42.
SEQ ID NO: 71 is the amino acid sequence encoded by SEQ ID NO: 43.
SEQ ID NO: 72 is the amino acid sequence encoded by SEQ ID NO: 44.
SEQ ID NO: 73 is the amino acid sequence for *MTL* ortholog found in *Sorghum bicolor.*
SEQ ID NO: 74 is the amino acid sequence for *MTL* ortholog found in *Setaria italica.*
SEQ ID NO: 75 is the amino acid sequence for *MTL* ortholog found in *Hordeum vulgare.*
SEQ ID NO: 76 is the amino acid sequence for *MTL* ortholog found in *Brachypodium distachyon.*
SEQ ID NO: 77 is the amino acid sequence for *MTL* ortholog found in *Oryza sativa* v. *indica.*
SEQ ID NO: 78 is the amino acid sequence for *MTL* ortholog found in *Triticum aestivum.*
SEQ ID NO: 79 is the amino acid sequence for *MTL* ortholog found *in Musa acuminata.*
SEQ ID NO: 80 is the amino acid sequence for *MTL* ortholog found in *Elaeis guineensis.*
SEQ ID NO: 81 is the amino acid sequence for *MTL* ortholog found in *Arabidopsis thaliana.*

### BRIEF DESCRIPTION OF THE FIGURES

Figure 1 is a mapping scheme used to map the haploid induction trait in RWK.
Figure 2 shows fine mapping narrowed the major QTL to a very small interval in bin 1.04, between 67.85 Mb and 68.42 Mb. This region has seven annotated genes. We sequenced and assembled the genes in this interval in several lines. The two genes with the most dramatic mutations in the haploid inducer lines are shown on the bottom right (GRMZM2G471240 and GRMZM2G062320).
Figure 3 shows the difference in expression of GRMZM2G471240 in haploid inducer and non-inducer pollen and post-anthesis anther sacs (sporophytic tissue with the pollen grains removed). This gene is specifically expressed in the male gametophyte.

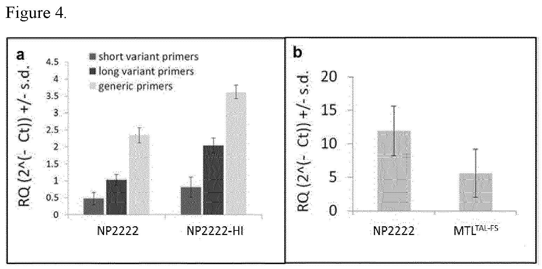

Figure 4a shows splice-specific qRT-PCR results for GRMZM2G471240. Three biological replicates of R1-staged anthers were tested in technical triplicate, and the average Ct and standard deviation was calculated for each reaction. The relative quantity of each transcript type was compared to the endogenous control using a log₂ regression of the delta Ct. Two sets of primers were used to assess the relative abundance of each of the two annotated splice variants compared to a primer set that is agnostic with respect to the splice variants. The shorter transcript variant had relatively low abundance compared to the long transcript in both NP2222 (wild type) and NP2222-HI (haploid inducer) genotypes. Expression of the mutant copies of the gene in NP2222-HI was significantly higher for all three primer pairs tested.
Figure 4b shows five biological replicates of fresh pollen from NP2222 and *MTL^{TAL-FS}* plants (T1 plants that are homozygous for edited *mtl-like* alleles) were tested in technical triplicate on the generic primer, and the average Ct and standard deviation was calculated for each reaction. The relative quantity of each transcript type was compared to the endogenous control using a log₂ regression of the delta Ct. *MTL^{TAL-FS}* pollen has lower transcript abundance than NP2222 (wild type) pollen.

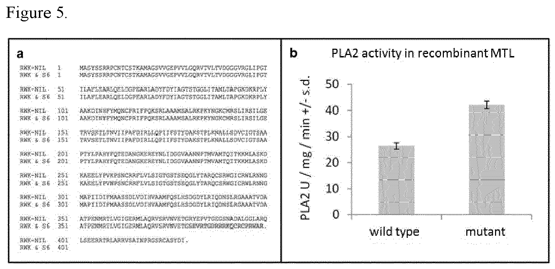

Figure 5a shows an amino acid alignment of the B73 predicted protein sequence of the long splice variant of the GRMZM2G471240 gene in B73 and RWK-NIL, with the predicted sequence of the *mtl* allele found in RWK and Stock 6 (S6). Amino acids that differ are in red; amino acids that match are indicated in normal grey text, and stop codons are indicated with a full stop. Two point mutations result in amino acid substitutions, a histidine (H) to a tyrosine (Y), and a lysine (K) to an arginine (N). These changes are not conservative; it is possible that one or both of these modifies the haploid induction phenotype - suggesting that an allelic series could be uncovered with further investigation of variants.
Figure 5b shows wild type MTL and mutant (truncated) MTL encoded by the *mtl* allele have *in vitro* phospholipase activity. PLA2 phospholipase activity as measured by fluorescent liposome assay on recombinant, purified protein produced using the *MTL* and *mtl* cDNAs. Error bars indicate standard error based on the average of four replicates.

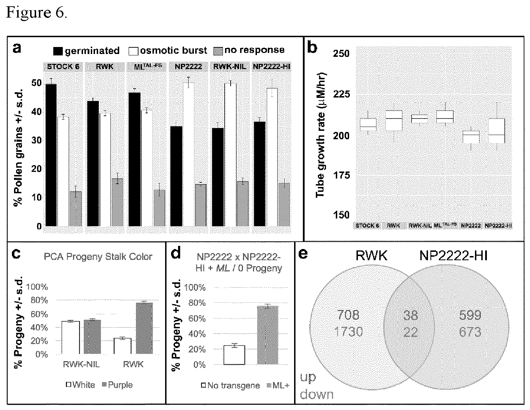

Figure 6 shows *mtl* is responsible for pleiotropic phenotypes associated with haploid induction. **6A:** Pollen tube germination rate was similar in inducers and non-inducers (n=200). **6B:** Initial pollen tube elongation was also similar (n=25). **6C:** RWK but not RWK-NIL is subject to segregation distortion (SD) based on low (25%) trait transmission in germinated progeny (n=300). **6D:** MTL/0 complementation lines also exhibit SD against mtl in germinated progeny (n=400). **6E:** Venn diagram showing RNA-seq profiling results of two haploid inducer-near isogenic pairs (left, RWK versus RWK-NIL; right, NP2222-HI versus NP2222; red text, up-regulated; green text, down-regulated). Only 60 genes were found significantly changed in the same direction.

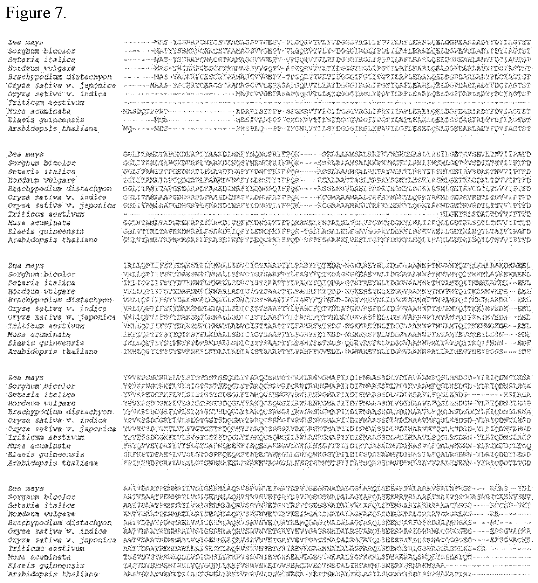

Figure 7 shows an amino acid alignment of the maize MTL gene to publically available MTL orthologs in eight grasses, two non-grass monocots, and *Arabidopsis* (thale cress). This alignment includes maize (*Zea mays),* sorghum *(Sorghum bicolor,* 92% sequence identity to MTL), foxtail millet *(Setaria italica,* 85% identity), barley *(Hordeum vulgare,* 78% identity) , *Brachypodium distachyon* (78% identity), Indica and Japonica variety rice *(Oryza sativa* v. *indica and japonica,* Os3g27610, 78 and 79% identity, respectively), bread wheat *(Triticum aestivum,* 55% identity), banana *(Musa acuminata,* 57% identity), oil palm (*Elaeis guineesnsis,* 56% identity), and *Arabidopsis thaliana* (52% identity).
Figure 8. Expression profile of rice phospholipases (adapted from Singh, A., et al., Rice phospholipase A superfamily: organization, phylogenetic and expression analysis during abiotic stresses and development, PLOS ONE 7: e30947 (2012)). The closest homolog to *MTL* is the rice gene *OspPLAIIϕ* (Os3g27610).
Figure 9. Diagram showing a route to editing Os3g27610 in order to make haploid inducer lines. One could target any part of the gene (shown here - targeting the first and fourth exons) and expect to create frame-shift mutations that would lead to knockout and loss of function of the gene, and that will lead to haploid induction.
Figure 10 shows the atomic structure of methyl alpha-linolenoyl fluorophosphonate (MALFP).

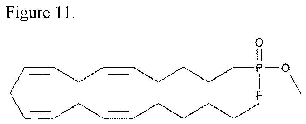

Figure 11 shows the atomic structure of methyl arachidonyl fluorophosphonate (MAFP).

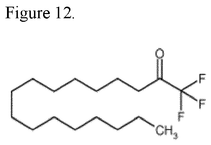

Figure 12 shows the atomic structure of palmityl trifluoromethylketone (PACOCF3).

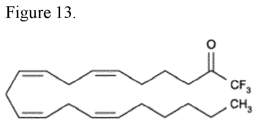

Figure 13 shows the atomic structure of arachidonyl trifluoromethylketone (AACOCF3).

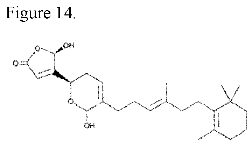

Figure 14 shows the atomic structure of manoalide.

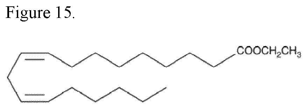

Figure 15 shows the atomic structure of linoleic acid ethyl ester (LLAEE).

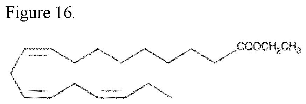

Figure 16 shows the atomic structure of linolenic acid ethyl ester (LNAEE).

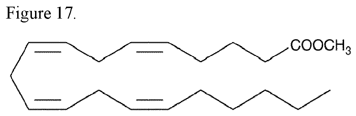

Figure 17 shows the atomic structure of arachidonic acid methyl ester (AAME).

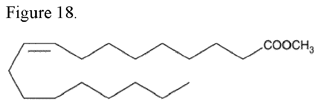

Figure 18 shows the atomic structure of oleic acid methy ester (OAME).

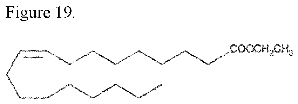

Figure 19 shows the atomic structure of oleic acid ethyl ester (OAEE).

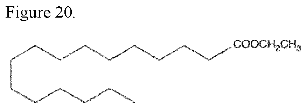

Figure 20 shows the atomic structure of palmitic acid ethyl ester (PAEE).

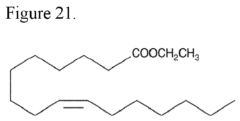

Figure 21 shows the atomic structure of palmitoleic acid ethyl ester (PLAEE).

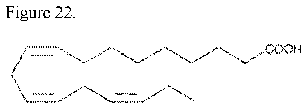

Figure 22 shows the atomic structure of alpha-linolenic acid (aLNA).

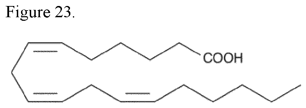

Figure 23 shows the atomic structure of gamma-linolenic acid (gLNA)

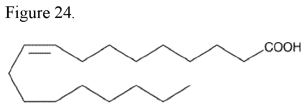

Figure 24 shows the atomic structure of oleic acid.

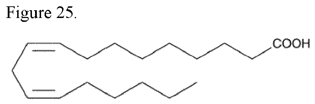

Figure 25 shows the atomic structure of Linoleic acid.

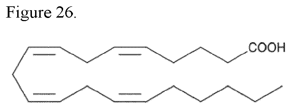

Figure 26 shows the atomic structure of Arachidonic acid.

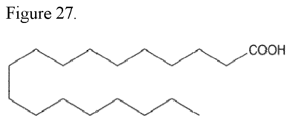

Figure 27 shows the atomic structure of Stearic Acid.

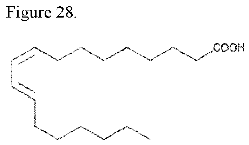

Figure 28 shows the atomic structure of 9(Z)-11(E)-conjugated Linoleic acid.

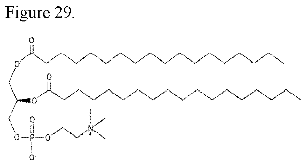

Figure 29 shows the atomic structure of Distearoyl phosphatidylcholine (DSPC).

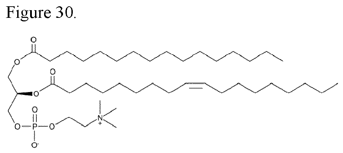

Figure 30 shows the atomic structure of 2-oleoyl-1-palmitoyl-sn-glycero-3-phospho-ethanolamine.

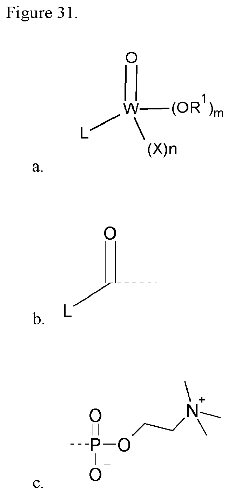

Figure 31 shows the generic atomic structure for molecules operable in the claimed invention.

### DEFINITIONS

While the following terms are believed to be well understood by one of ordinary skill in the art, the following definitions are set forth to facilitate explanation of the presently disclosed subject matter.

All technical and scientific terms used herein, unless otherwise defined below, are intended to have the same meaning as commonly understood by one of ordinary skill in the art. References to techniques employed herein are intended to refer to the techniques as commonly understood in the art, including variations on those techniques and/or substitutions of equivalent techniques that would be apparent to one of skill in the art. While the following terms are believed to be well understood by one of ordinary skill in the art, the following definitions are set forth to facilitate explanation of the presently disclosed subject matter.

Following long-standing patent law convention, the terms "a", "an", and "the" refer to "one or more" when used in this application, including the claims. For example, the phrase "a cell" refers to one or more cells, and in some embodiments can refer to a tissue and/or an organ. Similarly, the phrase "at least one", when employed herein to refer to an entity, refers to, for example, 1, 2, 3, 4, 5, 6, 7, 8, 9, 10, 15, 20, 25, 30, 35, 40, 45, 50, 75, 100, or more of that entity, including but not limited to all whole number values between 1 and 100 as well as whole numbers greater than 100.

Unless otherwise indicated, all numbers expressing quantities of ingredients, reaction conditions, and so forth used in the specification and claims are to be understood as being modified in all instances by the term "about." The term "about," as used herein when referring to a measurable value such as an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1 % from the specified amount, as such variations are appropriate to perform the disclosed methods and/or employ the discloses compositions, nucleic acids, polypeptides, etc. Accordingly, unless indicated to the contrary, the numerical parameters set forth in this specification and attached claims are approximations that can vary depending upon the desired properties sought to be obtained by the presently disclosed subject matter.

As used herein, the term "allele" refers to a variant or an alternative sequence form at a genetic locus. In diploids, a single allele is inherited by a progeny individual separately from each parent at each locus. The two alleles of a given locus present in a diploid organism occupy corresponding places on a pair of homologous chromosomes, although one of ordinary skill in the art understands that the alleles in any particular individual do not necessarily represent all of the alleles that are present in the species.

As used herein, the term "and/or" when used in the context of a list of entities, refers to the entities being present singly or in combination. Thus, for example, the phrase "A, B, C, and/or D" includes A, B, C, and D individually, but also includes any and all combinations and subcombinations of A, B, C, and D (e.g., AB, AC, AD, BC, BD, CD, ABC, ABD, and BCD). In some embodiments, one of more of the elements to which the "and/or" refers can also individually be present in single or multiple occurrences in the combinations(s) and/or subcombination(s).

As used herein, the phrase "associated with" refers to a recognizable and/or assayable relationship between two entities. For example, the phrase "associated with HI" refers to a trait, locus, gene, allele, marker, phenotype, etc., or the expression thereof, the presence or absence of which can influence an extent and/or degree at which a plant or its progeny exhibits HI. As such, a marker is "associated with" a trait when it is linked to it and when the presence of the marker is an indicator of whether and/or to what extent the desired trait or trait form will occur in a plant/germplasm comprising the marker. Similarly, a marker is "associated with" an allele when it is linked to it and when the presence of the marker is an indicator of whether the allele is present in a plant/germplasm comprising the marker. For example, "a marker associated with HI" refers to a marker whose presence or absence can be used to predict whether and/or to what extent a plant will display haploid induction.

The term "comprising," which is synonymous with "including," "containing," and "characterized by," is inclusive or open-ended and does not exclude additional, unrecited elements and/or method steps. "Comprising" is a term of art that means that the named elements and/or steps are present, but that other elements and/or steps can be added and still fall within the scope of the relevant subject matter.

As used herein, the phrase "consisting of" excludes any element, step, or ingredient not specifically recited. When the phrase "consists of" appears in a clause of the body of a claim, rather than immediately following the preamble, it limits only the element set forth in that clause; other elements are not excluded from the claim as a whole.

As used herein, the phrase "consisting essentially of" limits the scope of the related disclosure or claim to the specified materials and/or steps, plus those that do not materially affect the basic and novel characteristic(s) of the disclosed and/or claimed subject matter.

With respect to the terms "comprising," "consisting essentially of," and "consisting of," where one of these three terms is used herein, the presently disclosed and claimed subject matter can include in some embodiments the use of either of the other two terms. For example, if a subject matter relates in some embodiments to nucleic acids that encode polypeptides comprising amino acid sequences that are at least 95% identical to a SEQ ID NO: 2 or 3. It is understood that the disclosed subject matter thus also encompasses nucleic acids that encode polypeptides that in some embodiments consist essentially of amino acid sequences that are at least 95% identical to that SEQ ID NO: 2 or 3 as well as nucleic acids that encode polypeptides that in some embodiments consist of amino acid sequences that are at least 95% identical to that SEQ ID NO: 2 or 3. Similarly, it is also understood that in some embodiments the methods for the disclosed subject matter comprise the steps that are disclosed herein, in some embodiments the methods for the presently disclosed subject matter consist essentially of the steps that are disclosed, and in some embodiments the methods for the presently disclosed subject matter consist of the steps that are disclosed herein.

As used herein, the term "de novo haploid induction" refers to the triggering of haploid induction by the introduction of a spontaneous-haploid inducing agent. Such introduction can be achieved by topical spray, hand-pollination, mutagenesis, or transgenic methods. The terms "de novo haploid induction," "de novo HI," and "haploid induction de novo" are used interchangeably throughout this specification.

As used herein, the term "gene" refers to a hereditary unit including a sequence of DNA that occupies a specific location on a chromosome and that contains the genetic instruction for a particular characteristic or trait in an organism.

A "genetic map" is a description of genetic linkage relationships among loci on one or more chromosomes within a given species, generally depicted in a diagrammatic or tabular form.

As used herein, a plant referred to as "haploid" has a single set (genome) of chromosomes and the reduced number of chromosomes (n) in the haploid plant is equal to that of the gamete. As used herein, a plant referred to as "doubled haploid" is developed by doubling the haploid set of chromosomes. A plant or seed that is obtained from a doubled haploid plant that is selfed to any number of generations may still be identified as a doubled haploid plant. A doubled haploid plant is considered a homozygous plant. A plant is considered to be doubled haploid if it is fertile, even if the entire vegetative part of the plant does not consist of the cells with the doubled set of chromosomes; that is, a plant will be considered doubled haploid if it contains viable gametes, even if it is chimeric.

As used herein, the term "human-induced mutation" refers to any mutation that occurs as a result of either direct or indirect human action. This term includes, but is not limited to, mutations obtained by any method of targeted mutagenesis.

As used herein, the terms "marker probe" and "probe" refer to a nucleotide sequence or nucleic acid molecule that can be used to detect the presence or absence of a sequence within a larger sequence, e.g., a nucleic acid probe that is complementary to all of or a portion of the marker or marker locus, through nucleic acid hybridization. Marker probes comprising about 8, 10, 15, 20, 30, 40, 50, 60, 70, 80, 90, 100 or more contiguous nucleotides can be used for nucleic acid hybridization.

As used herein, the term "molecular marker" can be used to refer to a genetic marker, as defined above, or an encoded product thereof (e.g., a protein) used as a point of reference when identifying the presence/absence of a HI-associated locus. A molecular marker can be derived from genomic nucleotide sequences or from expressed nucleotide sequences (e.g., from an RNA, a cDNA, etc.). The term also refers to nucleotide sequences complementary to or flanking the marker sequences, such as nucleotide sequences used as probes and/or primers capable of amplifying the marker sequence. Nucleotide sequences are "complementary" when they specifically hybridize in solution (e.g., according to Watson-Crick base pairing rules). This term also refers to the genetic markers that indicate a trait by the absence of the nucleotide sequences complementary to or flanking the marker sequences, such as nucleotide sequences used as probes and/or primers capable of amplifying the marker sequence.

As used herein, the terms "nucleotide sequence," "polynucleotide," "nucleic acid sequence," "nucleic acid molecule," and "nucleic acid fragment" refer to a polymer of RNA or DNA that is single- or double-stranded, optionally containing synthetic, non-natural, and/or altered nucleotide bases. A "nucleotide" is a monomeric unit from which DNA or RNA polymers are constructed and consists of a purine or pyrimidine base, a pentose, and a phosphoric acid group. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

As used herein, the term "nucleotide sequence identity" refers to the presence of identical nucleotides at corresponding positions of two polynucleotides. Polynucleotides have "identical" sequences if the sequence of nucleotides in the two polynucleotides is the same when aligned for maximum correspondence (e.g., in a comparison window). Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence identity" for polynucleotides, such as about 50, 55, 60, 65, 70, 75, 80, 85, 90, 95, 98, 99 or 100 percent sequence identity, can be determined by comparing two optimally aligned sequences over a comparison window, wherein the portion of the polynucleotide sequence in the comparison window can include additions or deletions (i.e., gaps) as compared to the reference sequence for optimal alignment of the two sequences. In some embodiments, the percentage is calculated by: (a) determining the number of positions at which the identical nucleic acid base occurs in both sequences; (b) dividing the number of matched positions by the total number of positions in the window of comparison; and (c) multiplying the result by 100. Optimal alignment of sequences for comparison can also be conducted by computerized implementations of known algorithms, or by visual inspection. Readily available sequence comparison and multiple sequence alignment algorithms are, respectively, the Basic Local Alignment Search Tool (BLAST) and ClustalW/ClustalW2/Clustal Omega programs available on the Internet (*e.g.,* the website of the EMBL-EBI). Other suitable programs include, but are not limited to, GAP, BestFit, Plot Similarity, and FASTA, which are part of the Accelrys GCG Package available from Accelrys, Inc. of San Diego, California, United States of America. *See also* Smith & Waterman, 1981; Needleman & Wunsch, 1970; Pearson & Lipman, 1988; Ausubel *et al.,* 1988; and Sambrook & Russell, 2001.

One example of an algorithm that is suitable for determining percent sequence identity and sequence similarity is the BLAST algorithm, which is described in Altschul *et al.,* 1990. In some embodiments, a percentage of sequence identity refers to sequence identity over the full length of one of the gDNA, cDNA, or the predicted protein sequences in the largest ORF of SEQ ID No: 1 being compared. In some embodiments, a calculation to determine a percentage of nucleic acid sequence identity does not include in the calculation any nucleotide positions in which either of the compared nucleic acids includes an "N" (i.e., where any nucleotide could be present at that position).

The term "open reading frame" (ORF) refers to a nucleic acid sequence that encodes a polypeptide. In some embodiments, an ORF comprises a translation initiation codon, a translation termination (i.e., stop) codon, and the nucleic acid sequence there between that encodes the amino acids present in the polypeptide. The terms "initiation codon" and "termination codon" refer to a unit of three adjacent nucleotides (i.e., a codon) in a coding sequence that specifies initiation and chain termination, respectively, of protein synthesis (mRNA translation).

Patatin-like phospholipase A2a may also be known as PLA, pPLA, pPLAIIA pPLAIIa, PLA2alpha, or PLA2, or other similar variation. Patatin-like phospholipase AIIα is also referred to as *MATRILINEAL.* These terms are used interchangeably throughout. *AMATRILINEAL* gene comprising a four basepair frameshift mutation is hereby named *matrilineal.*

As used herein, the terms "phenotype," "phenotypic trait" or "trait" refer to one or more traits of a plant or plant cell. The phenotype can be observable to the naked eye, or by any other means of evaluation known in the art, e.g., microscopy, biochemical analysis, or an electromechanical assay. In some cases, a phenotype is directly controlled by a single gene or genetic locus (i.e., corresponds to a "single gene trait"). In the case of haploid induction use of color markers, such as R Navajo, and other markers including transgenes visualized by the presences or absences of color within the seed evidence if the seed is an induced haploid seed. The use of R Navajo as a color marker and the use of transgenes is well known in the art as means to detect induction of haploid seed on the female plant. In other cases, a phenotype is the result of interactions among several genes, which in some embodiments also results from an interaction of the plant and/or plant cell with its environment.

As used herein, the term "plant" can refer to a whole plant, any part thereof, or a cell or tissue culture derived from a plant. Thus, the term "plant" can refer to any of: whole plants, plant components or organs (e.g., leaves, stems, roots, etc.), plant tissues, seeds and/or plant cells.

A plant cell is a cell of a plant, taken from a plant, or derived through culture from a cell taken from a plant. Thus, the term "plant cell" includes without limitation cells within seeds, suspension cultures, embryos, meristematic regions, callus tissue, leaves, shoots, gametophytes, sporophytes, pollen, and microspores. The phrase "plant part" refers to a part of a plant, including single cells and cell tissues such as plant cells that are intact in plants, cell clumps, and tissue cultures from which plants can be regenerated. Examples of plant parts include, but are not limited to, single cells and tissues from pollen, ovules, leaves, embryos, roots, root tips, anthers, flowers, fruits, stems, shoots, and seeds; as well as scions, rootstocks, protoplasts, calli, and the like.

As used herein, the term "primer" refers to an oligonucleotide which is capable of annealing to a nucleic acid target (in some embodiments, annealing specifically to a nucleic acid target) allowing a DNA polymerase and/or reverse transcriptase to attach thereto, thereby serving as a point of initiation of DNA synthesis when placed under conditions in which synthesis of a primer extension product is induced (e.g., in the presence of nucleotides and an agent for polymerization such as DNA polymerase and at a suitable temperature and pH). In some embodiments, one or more pluralities of primers are employed to amplify plant nucleic acids (e.g., using the polymerase chain reaction; PCR).

As used herein, the term "probe" refers to a nucleic acid (e.g., a single stranded nucleic acid or a strand of a double stranded or higher order nucleic acid, or a subsequence thereof) that can form a hydrogen-bonded duplex with a complementary sequence in a target nucleic acid sequence. Typically, a probe is of sufficient length to form a stable and sequence-specific duplex molecule with its complement, and as such can be employed in some embodiments to detect a sequence of interest present in a plurality of nucleic acids.

As used herein, the terms "progeny" and "progeny plant" refer to a plant generated from a vegetative or sexual reproduction from one or more parent plants. In haploid induction the seed on the female parent is haploid, thus not a progeny of the inducing haploid line. The progeny of the haploid seed is not the only desired progeny. There is also the HI seed and subsequent plant and seed progeny of the haploid inducing plant. Both the haploid seed and the HI seed can be progeny. A progeny plant can be obtained by cloning or selfing a single parent plant, or by crossing two or more parental plants. For instance, a progeny plant can be obtained by cloning or selfing of a parent plant or by crossing two parental plants and include selfings as well as the F₁ or F₂ or still further generations. An F₁ is a first-generation progeny produced from parents at least one of which is used for the first time as donor of a trait, while progeny of second generation (F₂) or subsequent generations (F₃, F₄, and the like) are specimens produced from selfings, intercrosses, backcrosses, and/or other crosses of F₁s, F₂S, and the like. An F₁ can thus be (and in some embodiments is) a hybrid resulting from a cross between two true breeding parents *(i.e.,* parents that are true-breeding are each homozygous for a trait of interest or an allele thereof), while an F₂ can be (and in some embodiments is) a progeny resulting from self-pollination of the F₁ hybrids.

As used herein, the phrase "recombination" refers to an exchange of DNA fragments between two DNA molecules or chromatids of paired chromosomes (a "crossover") over in a region of similar or identical nucleotide sequences. A "recombination event" is herein understood to refer in some embodiments to a meiotic crossover.

As used herein, the term "reference sequence" refers to a defined nucleotide sequence used as a basis for nucleotide sequence comparison. In some embodiments, any of SEQ ID NOs: 1-4, 22-23, or 73-81 can serve as a reference sequence for comparing to other sequences obtained from plants.

As used herein, the term "regenerate," and grammatical variants thereof, refers to the production of a plant from tissue culture.

As used herein, the phrase "stringent hybridization conditions" refers to conditions under which a polynucleotide hybridizes to its target subsequence, typically in a complex mixture of nucleic acids, but to essentially no other sequences. Stringent conditions are sequence-dependent and can be different under different circumstances.

Longer sequences typically hybridize specifically at higher temperatures. An extensive guide to the hybridization of nucleic acids is found in Sambrook & Russell, 2001. Generally, stringent conditions are selected to be about 5-10°C lower than the thermal melting point (Tₘ) for the specific sequence at a defined ionic strength pH. The Tm is the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Exemplary stringent conditions are those in which the salt concentration is less than about 1.0 M sodium ion, typically about 0.01 to 1.0 M sodium ion concentration (or other salts) at pH 7.0 to 8.3 and the temperature is at least about 30°C for short probes *(e.g.,* 10 to 50 nucleotides) and at least about 60°C for long probes *(e.g.,* greater than 50 nucleotides).

Stringent conditions can also be achieved with the addition of destabilizing agents such as formamide. Additional exemplary stringent hybridization conditions include 50% formamide, 5x SSC, and 1 % SDS incubating at 42°C; or SSC, 1 % SDS, incubating at 65°C; with one or more washes in 0.2x SSC and 0.1% SDS at 65°C. For PCR, a temperature of about 36°C is typical for low stringency amplification, although annealing temperatures can vary between about 32°C and 48°C (or higher) depending on primer length. Additional guidelines for determining hybridization parameters are provided in numerous references (*see e.g.,* Ausubel *et al.,* 1999).

As used herein, the term "trait" refers to a phenotype of interest, a gene that contributes to a phenotype of interest, as well as a nucleic acid sequence associated with a gene that contributes to a phenotype of interest. For example, a "HI trait" refers to a haploid induction phenotype as well as a gene that contributes to a haploid induction and a nucleic acid sequence (*e.g.,* a HI-associated gene product) that is associated with the presence or absence of the haploid induction phenotype.

As used herein, the term "transgene" refers to a nucleic acid molecule introduced into an organism or one or more of its ancestors by some form of artificial transfer technique. The artificial transfer technique thus creates a "transgenic organism" or a "transgenic cell." It is understood that the artificial transfer technique can occur in an ancestor organism (or a cell therein and/or that can develop into the ancestor organism) and yet any progeny individual that has the artificially transferred nucleic acid molecule or a fragment thereof is still considered transgenic even if one or more natural and/or assisted breedings result in the artificially transferred nucleic acid molecule being present in the progeny individual.

As used herein, the term "targeted mutagenesis" or "mutagenesis strategy" refers to any method of mutagenesis that results in the intentional mutagenesis of a chosen gene. Targeted mutagenesis includes the methods CRISPR, TILLING, TALEN, and other methods not yet discovered but which may be used to achieve the same outcome.

As used herein, haploid induction rate ("HIR") means the number of surviving haploid kernels over the total number of kernels after an ear is pollinated with haploid inducer pollen.

Particular problems plague that haploid induction: increased embryo abortion rates and increased fertilization failure rates (reduced seed set rates). For these reasons, there exists a need to successfully determine the cause of HI, and to use that knowledge to determine methods of stably or increasingly creating haploid plants while simultaneously reducing fertilization failure and embryo abortions.

It is specifically contemplated that one could mutagenize a promoter to potentially improve the utility of the elements for the expression of transgenes in plants. The mutagenesis of these elements can be carried out at random and the mutagenized promoter sequences screened for activity in a trial-by-error procedure. Alternatively, particular sequences which provide the promoter with desirable expression characteristics, or the promoter with expression enhancement activity, could be identified and these or similar sequences introduced into the promoter via mutation. It is further contemplated that one could mutagenize these sequences in order to enhance their expression of transgenes in a particular species. The means for mutagenizing a DNA segment encoding a promoter sequence of the current invention are well-known to those of skill in the art. As indicated, modifications to promoter or other regulatory element may be made by random, or site-specific mutagenesis procedures. The promoter and other regulatory element may be modified by altering their structure through the addition or deletion of one or more nucleotides from the sequence which encodes the corresponding unmodified sequences.

Mutagenesis may be performed in accordance with any of the techniques known in the art, such as, and not limited to, synthesizing an oligonucleotide having one or more mutations within the sequence of a particular regulatory sequence. In particular, site-specific mutagenesis is a technique useful in the preparation of promoter mutants, through specific mutagenesis of the underlying DNA. RNA-guided endonucleases ("RGEN," e.g., CRISPR/Cas9) may also be used. The technique further provides a ready ability to prepare and test sequence variants, for example, incorporating one or more of the foregoing considerations, by introducing one or more nucleotide sequence changes into the DNA. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered.

Where a clone comprising a promoter has been isolated in accordance with the instant invention, one may wish to delimit the essential promoter regions within the clone. One efficient, targeted means for preparing mutagenized promoters relies upon the identification of putative regulatory elements within the promoter sequence. This can be initiated by comparison with promoter sequences known to be expressed in similar tissue specific or developmentally unique patterns. Sequences which are shared among promoters with similar expression patterns are likely candidates for the binding of transcription factors and are thus likely elements which confer expression patterns. Confirmation of these putative regulatory elements can be achieved by deletion analysis of each putative regulatory sequence followed by functional analysis of each deletion construct by assay of a reporter gene which is functionally attached to each construct. As such, once a starting promoter sequence is provided, any of a number of different deletion mutants of the starting promoter could be readily prepared.

The invention disclosed herein provides polynucleotide molecules comprising regulatory element fragments that may be used in constructing novel chimeric regulatory elements. Novel combinations comprising fragments of these polynucleotide molecules and at least one other regulatory element or fragment can be constructed and tested in plants and are considered to be within the scope of this invention. Thus the design, construction, and use of chimeric regulatory elements is one embodiment of this invention. Promoters of the present invention include homologues of cis elements known to affect gene regulation that show homology with the promoter sequences of the present invention.

Functional equivalent fragments of one of the transcription regulating nucleic acids described herein comprise at least 50, 100, 150, 200, 250, 300, 350, 400, 450, 500, 550, 600, 650, 700, 750, 800, 850, 900, 950, or 1000 base pairs of a transcription regulating nucleic acid. Equivalent fragments of transcription regulating nucleic acids, which are obtained by deleting the region encoding the 5'-untranslated region of the mRNA, would then only provide the (untranscribed) promoter region. The 5'-untranslated region can be easily determined by methods known in the art (such as 5'-RACE analysis). Accordingly, some of the transcription regulating nucleic acids, described herein, are equivalent fragments of other sequences.

As indicated above, deletion mutants of the promoter of the invention also could be randomly prepared and then assayed. Following this strategy, a series of constructs are prepared, each containing a different portion of the promoter (a subclone), and these constructs are then screened for activity. A suitable means for screening for activity is to attach a deleted promoter or intron construct which contains a deleted segment to a selectable or screenable marker, and to isolate only those cells expressing the marker gene. In this way, a number of different, deleted promoter constructs are identified which still retain the desired, or even enhanced, activity. The smallest segment which is required for activity is thereby identified through comparison of the selected constructs. This segment may then be used for the construction of vectors for the expression of exogenous genes.

An expression cassette as described herein may comprise further regulatory elements. The term in this context is to be understood in the broad meaning comprising all sequences which may influence construction or function of the expression cassette. Regulatory elements may, for example, modify transcription and/or translation in prokaryotic or eukaryotic organisms. The expression cassette described herein may be downstream (in 3' direction) of the nucleic acid sequence to be expressed and optionally contain additional regulatory elements, such as transcriptional or translational enhancers. Each additional regulatory element may be operably liked to the nucleic acid sequence to be expressed (or the transcription regulating nucleotide sequence). Additional regulatory elements may comprise additional promoters, minimal promoters, promoter elements, or transposon elements which may modify or enhance the expression regulating properties. The expression cassette may also contain one or more introns, one or more exons and one or more terminators.

Furthermore, it is contemplated that promoters combining elements from more than one promoter may be useful. For example, U.S. Pat. No. 5,491,288 discloses combining a Cauliflower Mosaic Virus promoter with a histone promoter. Thus, the elements from the promoters disclosed herein may be combined with elements from other promoters. Promoters which are useful for plant transgene expression include those that are inducible, viral, synthetic, constitutive (Odell Nature 313: 810-812 (1985)), temporally regulated, spatially regulated, tissue specific, and spatial temporally regulated. Using the regulatory elements described herein, numerous agronomic genes can be expressed in transformed plants. More particularly, plants can be genetically engineered to express various phenotypes of agronomic interest.

The compounds of the present invention may exist in different geometric or optical isomers (diastereoisomers and enantiomers) or tautomeric forms. This invention covers all such isomers and tautomers and mixtures thereof in all proportions as well as isotopic forms such as deuterated compounds. The invention also covers all salts, N-oxides, and metalloidic complexes of the compounds of the present invention.

Each alkyl moiety either alone or as part of a larger group (such as alkoxy, alkoxycarbonyl, alkylcarbonyl, alkylaminocarbonyl, dialkylaminocarbonyl) is a straight or branched chain and is, for example, methyl, ethyl, n-propyl, n-butyl, n-pentyl, n-hexyl, iso-propyl, n-butyl, sec-butyl, iso-butyl, tert-butyl or neo-pentyl. The alkyl groups include C₁-C₆ alkyl, C₁-C₄ alkyl, and C1-C3 alkyl.

The term "alkenyl," as used herein, is an alkyl moiety having at least one carbon-carbon double bond, for example C2-C6 alkenyl. Specific examples include vinyl and allyl. The alkenyl moiety may be part of a larger group (such as alkenoxy, alkenoxycarbonyl, alkenylcarbonyl, alkyenlaminocarbonyl, dialkenylaminocarbonyl).

The term "acetoxy" refers to -OC(=O)CH3.

The term "alkynyl," as used herein, is an alkyl moiety having at least one carbon-carbon triple bond, for example C2-C6 alkynyl. Specific examples include ethynyl and propargyl. The alkynyl moiety may be part of a larger group (such as alkynoxy, alkynoxycarbonyl, alkynylcarbonyl, alkynylaminocarbonyl, dialkynylaminocarbonyl).

Halogen is fluorine (F), chlorine (Cl), bromine (Br) or iodine (I).

Haloalkyl groups (either alone or as part of a larger group, such as haloalkoxy or haloalkylthio) are alkyl groups which are substituted with one or more of the same or different halogen atoms and are, for example, -CF3, -CF2C1, -CH2CF3, or - CH2CHF2.

Hydroxyalkyl groups are alkyl groups which are substituted with one or more hydroxyl group and are, for example, -CH2OH, -CH2CH2OH or -CH(OH)CH3.

Alkoxyalkyl groups are an alkoxy group bonded to an alkyl (R-O-R'), for example -(CH2)rO(CH2)ₛCH3, wherein r is 1 to 6 and s is 1 to 5.

In the context of the present specification, the term "aryl" refers to a ring system which may be mono, bi or tricyclic. Examples of such rings include phenyl, naphthalenyl, anthracenyl, indenyl or phenanthrenyl.

Unless otherwise indicated, alkenyl and alkynyl, on their own or as part of another substituent, may be straight or branched chain and may contain 2 to 6 carbon atoms, and where appropriate, may be in either the (E) or (Z) configuration. Examples include vinyl, allyl, ethynyl and propargyl.

Unless otherwise indicated, cycloalkyl may be mono- or bi-cyclic, may be optionally substituted by one or more C1-C6 alkyl groups, and contain 3 to 7 carbon atoms. Examples of cycloalkyl include cyclopropyl, 1-methylcyclopropyl, 2-methylcyclopropyl, cyclobutyl, cyclopentyl, and cyclohexyl.

The term "heterocyclyl" refers to a ring system containing from one to four heteroatoms selected from N, O and S, wherein the nitrogen and sulphur atoms are optionally oxidized, and the nitrogen atom(s) are optionally quaternized. Heterocyclyl includes heteroaryl, saturated analogs, and in addition their unsaturated or partially unsaturated analogues such as 4,5,6,7-tetrahydro-benzothiophenyl, 9H-fluorenyl, 3,4-dihydro-2H-benzo-1,4-dioxepinyl, 2,3-dihydro-benzo-furanyl, piperidinyl, 1,3-dioxolanyl, 1,3-dioxanyl, 4,5-dihydro-isoxazolyl, tetrahydrofuranyl and morpholinyl. In addition, the term "heterocyclyl" includes heterocycloalkyl, a non-aromatic monocyclic or polycyclic ring comprising carbon and hydrogen atoms and at least one heteroatom selected from nitrogen, oxygen, and sulfur such asoxetanyl or thietanyl. A monocyclic heterocycloalkyl may contain 3 to 7 members.

The term "heteroaryl" refers to an aromatic ring system containing from one to four heteroatoms selected from N, O, and S, wherein the nitrogen and sulfur atoms are optionally oxidized, for example having 5, 6, 9, or 10 members, and consisting either of a single ring or of two or more fused rings. Single rings may contain up to three heteroatoms, and bicyclic systems up to four heteroatoms, which will preferably be chosen from nitrogen, oxygen, and sulfur. Examples of such groups include pyridyl, pyridazinyl, pyrimidinyl, pyrazinyl, furanyl, thienyl, oxazolyl, isoxazolyl, oxadiazolyl, thiazolyl, isothiazolyl, thiadiazolyl, pyrrolyl, pyrazolyl, imidazolyl, triazolyl, and tetrazolyl.

### ADDITIONAL EMBODIMENTS

Embodiment 1. A method for inducing haploid seed production, the method comprising treating reproductive tissues of a plant with a composition comprising a compound according to formula (I): 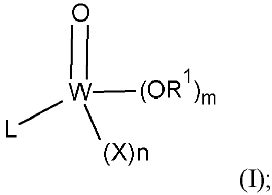 wherein
   a. W is C, P, or S;
   b. m and n are each independently 0 or 1;
   c. X is selected from the group consisting of OH, CN, O(C₁-C₄ alkyl), halogen, C₁-C₄ alkyl, and C₁-C₄ alkyl substituted by one, two, or three halogen or carbonyl;
   d. R¹ is:
      i. selected from the group consisting of H, C₁-C₆ alkyl, and C₁-C₆ alkyl substituted by one or more hydroxyl groups wherein optionally one or more of said hydroxyl groups is esterified with a radical independently selected from the group consisting of: 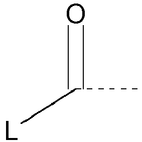 and 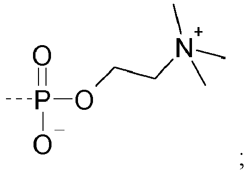 or
      ii. a bond to W when W is S;
   e. each L is independently a C₂-C₃₀ carbon chain, said carbon chain optionally comprising one or more groups independently selected from alkenyl, alkynyl, phenyl, and heteroaryl, and said carbon chain optionally interrupted by 1-6 oxygen atoms;
   f. provided that n is 0 when W is C and m is 1; and
   g. provided that n is 1 when W is C and m is 0;
   wherein the treatment occurs immediately preceding, during or immediately following pollination, and wherein at least one haploid seed is produced.
Embodiment 2. A method for inducing haploid seed production, the method comprising treating reproductive tissues of a plant with a composition comprising a compound according to formula (I): 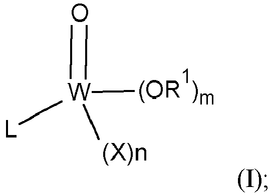 wherein
   a. W is C or P;
   b. m and n are each independently 0 or 1;
   c. X is selected from the group consisting of OH, CN, O(C₁-C₄ alkyl), halogen, C₁-C₄ alkyl, and C₁-C₄ alkyl substituted by one, two, or three halogen or carbonyl;
   d. R¹ is selected from the group consisting of H, C₁-C₄ alkyl, and C₁-C₄ alkyl substituted by one or more hydroxyl groups;
   e. L is a C₂-C₃₀ carbon chain, said carbon chain optionally comprising one or more groups independently selected from alkenyl and alkynyl, and said carbon chain optionally interrupted by 1-6 oxygen atoms;
   f. provided that n is 0 when W is C and m is 1; and
   g. provided that n is 1 when W is C and m is 0;
   wherein the treatment occurs immediately preceding, during or immediately following pollination, and wherein at least one haploid seed is produced.
Embodiment 3. The method of embodiments 1-2, wherein L of formula (I) is selected from the group consisting of:
   a. (CH₂)₈-(CH)₂-CH₂-(CH)₂-CH₂-(CH)₂-CH₂-CH₃;
   b. (CH₂)₃-(CH)₂-CH₂-(CH)₂-CH₂-(CH)₂-CH₂-(CH)₂-(CH₂)₄-CH₃;
   c. (CH₂)₇-(CH)₂-(CH₂)₇-CH₃;
   d. (CH₂)₈-(CH)₂-CH₂-phenyl-CH₂-(CH)₂-CH₂-CH₃;
   e. (CH₂)₈-(CH)₂-(CH₂)₂-O-CH₂-(CH)₂-CH₂-CH₃; and
   f. (CH₂)₈-(CH)₂-CH₂-phenyl-O-(CH₂)₃-CH₃.
Embodiment 4. A method for inducing haploid seed production in a cross between two plants, the method comprising treating at least one plant's reproductive tissues with a composition comprising a lipid or a phospholipase inhibitor, wherein the treatment occurs immediately preceding, during or immediately following pollination, and wherein at least one haploid seed is produced from the cross.
Embodiment 5. A method for inducing haploid seed production, the method comprising treating reproductive tissues of a plant with a composition comprising a lipid or a phospholipase inhibitor, wherein the treatment occurs immediately preceding, during or immediately following pollination, and wherein at least one haploid seed is produced.
Embodiment 6. The method of embodiments 4 or 5, wherein the haploid seeds are induced at an increased rate.
Embodiment 7. The method of embodiments 4 or 5, wherein the lipid or the phospholipase inhibitor is selected from the group consisting of compounds listed in Table 7.
Embodiment 8. The method of embodiment 7, wherein the lipid or the phospholipase inhibitor is selected from the group consisting of methyl alpha-linolenoyl fluorophosphonate ("MALFP"), linoleic acid ethyl ester ("LLAEE"), linoleic acid ("LLA"), corn oil, distearyl-phosphatidyl choline ("DSPC"), or methyl arachidonyl fluorophosphonate ("MAFP").
Embodiment 9. The method of embodiments 4 or 5, wherein the composition of a lipid or a phospholipase inhibitor comprises a compound selected from Table 7 dissolved or emulsified in a suitable formulation at a concentration of between 0.0001 mg/mL to 100 mg/mL, or between 0.1 mg/mL to 50 mg/mL, or between 1 mg/mL to 10 mg/mL, or is approximately 5 mg/mL.
Embodiment 10. The method of embodiment 9, wherein the suitable formulation is selected from the group consisting of water, alcohol, DMSO, Tris-EDTA, EDTA, acetic acid, benzene, DME, glycerin, hexane, ethanol, isopropanol, propanol, buffered saline, glycine-HCl, sodium acetate, cacodylate buffer, citrate buffer, Sørensen's phosphate buffer, and phosphate-citrate buffer, barbital buffer, Tris buffer, Glycine-NaOH buffer, Formulation 91, Formulation 92, phosphate buffered saline ("PBS"), PBS plus Tween and DMSO, and any other available solvent or buffer in which a fatty acid may be diluted or emulsified.
Embodiment 11. The method of embodiment 10, wherein the suitable compound is phosphate buffered saline ("PBS").
Embodiment 12. The method of embodiments 4 or 5, wherein the plant is a dicot plant or a monocot plant.
Embodiment 13. The method of embodiment 12, wherein the plant is rice or maize.
Embodiment 14. The method of embodiment 12, wherein the plant is sunflower or soybean.
Embodiment 15. The method of embodiments 4 or 5, wherein the lipid is applied by a technique selected from the group consisting of: dipping, injection, spray-based topical application, nebulizer, pipette-based topical application, and brush-based topical application, and any other topical application.

### EXAMPLES

### I. Identifying the Frameshift Mutation in PLA

The present invention identifies a series of independent human-induced mutations found in at least one patatin-like phospholipase AIIα (pPLAIIa) gene of maize; maize plants having these mutations in at least one of their PLA genes; and a method of creating and identifying similar and/or additional mutations in the PLA gene by screening pooled and/or individual rice and maize plants. The rice and maize plants of the present invention induce haploidy as a result of non-transgenic mutations in at least one of their PLA genes.

More specifically, the present invention produces new maize haploid-inducing lines. A number of known haploid-inducing maize lines exist including but not limited to: Stock 6, MHI (Moldovian Haploid Inducer), indeterminate gametophyte ("*ig*") mutation, KEMS, ZEM, ZMS, KMS, RWS, and RWK. The present invention relates to a method of identifying, and/or selecting germplasm which can or cannot induce haploids. The present invention also relates to increasing and further development of the selected haploid inducing germplasm. The invention further relates to a method of improving haploid inducing germplasm to increase the induction of haploids on the seed producing parent.

The initial step in the production of haploid seeds from a hybrid or segregating maternal parent plant derives from the pollination with pollen from a haploid inducer onto the ear from a seed producing plant. A result of this hybridization process is the production of diploid and maternal haploid (In) kernels. The induced haploid (In) kernels are often distinguished from the diploid seed by the use of color markers which indicate embryo ploidy. The diploid seeds are generally discarded, while haploid kernels or embryos are often subjected to chromosome doubling processes to produce doubled haploid plants. More specifically, the haploid genetic material is treated with one or more mitotic arrest agents to allow the haploid (In) chromosome complement in one or more cells to produce homolog-pairs. After the chemical treatment procedure, the chromosome doubling chemical(s) are removed. The now-doubled haploid maize is allowed to mature and the resulting doubled haploid seeds when planted will produce homozygous plants (also called inbred plant or lines). These inbred lines are the materials that breeders utilize to pursue their hybrid development programs.

The locus for the haploid induction trait was fine mapped. Although a major QTL on chromosome 1 responsible for haploid induction has been mapped and published, Dong et al. Theor. Appl. Genet (2013) 126: 1713-1720, the exact gene/genetic element responsible for the induction process has not been identified until now. To clarify the developmental genetics underlying haploid induction, the Stock 6 derivative RWK (~ 13% HIR) was obtained from the University of Hohenheim in 2006, crossed to inbreds NP2460 and NP2391, and subsequently backcrossed to RWK to generate mapping populations. See Figure 1.

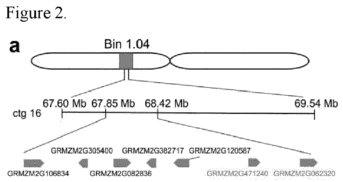

Elevated HIR in both populations co-segregated with marker SM020SDQ in bin 1.04, consistent with recent reports on a QTL called qhir1. *See* Prigge, et al., New Insights into the Genetics of in Vivo Induction of Maternal Haploids, the Backbone of Doubled Haploid Technology in Maize, GENETICS (2012) 190:781-793 (discussing major QTL for HI in Bin 1.04 [qhir1] and minor QTLs for HI in Bins 3.02 [qhir2], 3.06 [qhir3], 4.03 [qhir4], 5.01 [qhir5], 5.04 [qhir6], 7.01 [qhir7], and 9.01 [qhir8]); Liu, et al., Fine mapping of qhir8 affecting in vivo haploid induction in maize, THEOR. APPL. GENET. (2015) 128:2507-2515 (fine mapping thirty-five genes to qhir8); Hu, et al., The Genetic Basis of Haploid Induction in Maize Identified with a Novel Genome-Wide Association Method, GENETICS (2016) 202:1267-1276 (asserting that qhir1 is two QTLs: qhir1 1 and qhir12, and fine mapping qhir6 to a 1.1Mb region). We did several rounds of fine mapping and narrowed the QTL to an approximately 0.57 Mb region between 67.85 Mb and 68.42 Mb that lies within *qhir11.* This region has seven annotated genes (Figure 2).

Using the Illumina HiSeq2000, we sequenced RWK, Stock 6, and a BC3F5 non-inducer "RWK-NIL" that is near-isogenic to RWK but has NP2391 haplotypes in the qhir1 1 interval. By comparing inducer and non-inducer germplasm, it was determined that a four nucleotide insertion present in haploid inducers which shifts the frame for amino acid coding of GRMZM2G471240 is not present in non-inducer germplasm. Therefore, the present invention has identified a gene with a frameshift mutation in inducer germplasm as being responsible for maize haploid induction. The candidate gene corresponding to gene model GRMZM2G471240 encodes patatin-like phospholipase AIIα (pPLAIIa), which we have renamed *MATRILINEAL (MTL)* to represent the wildtype allele and the frameshift allele is referred to as *matrilineal (mtl).*

DNA sequence was generated for each candidate gene from the two inducer lines (Stock6 and RWK) and one non-inducer line (RWK-NIL). In addition, the public B73 genome data was used as a second non-inducer line. Gene model information was compared to EST/cDNA data to confirm the structure of each gene. The annotated sequence data were compared to catalog differences between the four alleles of each gene.

The sequence comparisons revealed that B73 and RWK-NIL alleles were similar to each other, and RWK and Stock 6 alleles were similar to each other. Most sequence differences were single nucleotide polymorphisms that do not alter protein coding sequence. There were some insertions and some deletions, most of which are in non-protein coding sequence.

Table 1 below lists gene identities in the interval shown. This information is from chromosome 1, *see, e.g.,* Patrick S. Schnable et al., The B73 Maize Genome: Complexity, Diversity, and Dynamics, 326 SCIENCE 1112-15 (2009), incorporated herein by reference in its entirety, and lists a short description of the other encoded proteins from the genes within the haploid inducing locus.

**Table 1. Information on Chromosome 1 from Maize.**

| | **Tra nscript** | | | | | | | | |
|---|---|---|---|---|---|---|---|---|---|
| **Gene_id** | **Start** | **End** | **Query length** | **Subject length** | **Score** | **Identity** | **Similarity** | **Align lengt h** | **Description** |
| GRMZM2G305400 | 67991172 | 67994092 | 308 | 362 | 385 | 33.3 | 53.33752 | 314 | Cyclin D2; 1 |
| GRMZM2G082836 | 66107606 | 68110989 | 202 | 205 | 729 | 71.2 | 83.33333 | 198 | GTP-binding protein 1 |
| GRMZM2G382717 | 68113455 | 68115168 | 396 | 464 | 489 | 38 .77 | 53.17371 | 314 | Chaperone DnaJ-domain superfamily protein |
| GRMZM2G120587 | 68133178 | 68136953 | 458 | 461 | 1329 | 55 | 71.23894 | 452 | serine carboxypeptidase-like 51 |
| GRMZM2G471240 | 68240862 | 68242656 | 428 | 407 | 1049 | 51.5 | 72.36181 | 398 | phospholipase A 2A |
| GRMZM2G471240 | 68240862 | 68242656 | 401 | 407 | 961 | 50.15 | 70.0938 | 395 | phospholipase A 2A |
| GRMZM2GO62320 | 68318898 | 66321409 | 335 | 334 | 1064 | 73.3 | 84.21053 | 285 | Phosphoglycerate mutase family protein |
| GRMZM5G866758 | 68430654 | 68436197 | 401 | 403 | 1678 | 80.4 | 90.45226 | 398 | acetoacetyl-CoA thiolase 2 |
| GRMZM5G866758 | 68430654 | 68436197 | 303 | 403 | 1248 | 78.4 | 89.40397 | 302 | acetoacetyl-CoA thiolase 2 |
| GRMZM2G003530 | 68435670 | 68439997 | 360 | 344 | 1063 | 60.5 | 76.41791 | 335 | P-loop containing nucleoside triphosphate hydrolases superfamily protein |
| GRMZM2G077991 | 68543246 | 68546264 | 94 | 95 | 424 | 79.7 | 91.48936 | 94 | Zinc-binding ribosomal protein family protein |
| GRMZM2G077991 | 68543694 | 68546264 | 94 | 95 | 424 | 79.7 | 91.48936 | 94 | Zinc-binding ribosomal protein family protein |
| GRMZM2G077991 | 68543805 | 68546269 | 147 | 95 | 419 | 79.5 | 91.39785 | 93 | Zinc-binding ribosomal protein family protein |
| GRMZM2G077960 | 68554980 | 68559182 | 438 | 428 | 1422 | 65.3 | 79.80998 | 421 | Protein phosphatase 2C family protein |
| GRMZM2G077897 | 68561209 | 68565155 | 784 | 807 | 1561 | 48.1 | 65.69848 | 723 | Plant protein of unknown function (DUF827) |
| GRMZM2G347583 | 68660278 | 68665995 | 1651 | 2156 | 1201 | 41.37 | 55.70954 | 1375 | |
| GRMZM2G173030 | 68668900 | 68671460 | 626 | 2156 | 858 | 35.6 | 48.30299 | 586 | |
| GRMZM2G022061 | 68876150 | 68662226 | 203 | 556 | 618 | 64.9 | 79.89691 | 194 | |
| GRMZM2G022061 | 68876150 | 68882226 | 322 | 556 | 1004 | 66 | 77.47748 | 333 | |
| GRMZM2G022061 | 68876150 | 68882226 | 142 | 556 | 547 | 79.6 | 89.84375 | 128 | |
| GRMZM2G022061 | 68876150 | 68882226 | 322 | 556 | 1004 | 66 | 77.47748 | 333 | |
| GRMZM2G022061 | 68876150 | 68882226 | 534 | 556 | 1802 | 67.7 | 79.81651 | 545 | |
| GRMZM2G340286 | 68928213 | 68929600 | 378 | 403 | 570 | 37.83 | 55.75713 | 407 | |
| GRMZM2G340279 | 68934652 | 68937080 | 746 | 937 | 3095 | 29.34 | 50.31745 | 2517 | Tetratricopeptide repeat (TPR)-like superfamily protein |
| GRMZM2G347808 | 69005208 | 69012612 | 589 | 455 | 1115 | 50.4 | 66.60178 | 423 | S-adenosyl-L-methionine-dependent methyltransferases superfamily protein |

Having completed fine mapping of the haploid inducer trait to an interval containing only seven genes, we focused on those in the sequence assembly and analysis. The sequences for the seven genes were nearly identical between B73 and RWK-NIL, but RWK and Stock 6 lacked GRMZM2G062320, a PHOSPHOGLYCERATE MUTASE (PGM), and had a 4 basepair ("bp") insertion in the fourth exon of GRMZM2G471240, a PATATIN-LIKE PHOSPHOLIPASE AIIα (pPLAIIα) (Figure 2). We found that RWK and Stock6 both have the same 4 bp insertion in the fourth exon of pPLAIIa, and that this gene is specifically expressed in pollen (see maizegdb.org/gene_center/gene?id=GRMZM2G471240 incorporated herein by reference). The unmutated GRMZM2G471240 is represented by SEQ ID NO: 1. GRMZM2G471240, comprising the 4 bp insertion in the fourth exon, is represented by SEQ ID NO: 3.

Most of the haploids that were identified were found using a taqman marker test. This marker test takes advantage of a difference in the pPLAIIα gene between RWK x NP2222. In crosses where we use RWK as the female, and NP2222 as the male, the RWK parent is homozygous for the mtl allele, while NP2222 is homozygous for the *MTL* allele. Diploid progeny *are MTL*/*mtl* and haploid gynogenetic haploid progeny are *mtl*/0*.* Therefore when this test is done the taqman results show 1 copy of the *mtl* allele and one copy of *MTL* allele in the diploid progeny, and 1 copy of the *mtl* but no copies of *MTL* in the haploid progeny. When this type of cross is performed, ears are harvested between 12-21 days following pollination, the embryos are extracted and a small sample of the embryos are taken for taqman marker analysis. Alternatively the embryos are plated on solid media and germinated in the dark so that a larger sample of the extended shoot or root can be taken between 2-10 days later for marker analysis. At the same time some of the tissue is saved for ploidy analysis. In this latter case after the molecular test is used, the larger samples of the haploids can be run on a CyFlow Space ploidy analyzer and confirmed as haploids. In most cases this results in the positive identification of haploids. In a few rarer cases this results in the overturning of the false positive marker results and correction of the call as a diploid.

Another way we test for haploids is via dominant marker assay. In this case, an X26 male line is used. This line is homozygous for a marker that acts in a dominant fashion. In such a cross any line can be used as a female as long as it doesn't have a marker or any genes or alleles that work to inhibit the marker phenotype. The X26 line is a non-inducer and is homozygous for MTL. Using such a line, the progeny are dissected between 12 - 21 days after pollination and evaluated for the presence of the marker, or they are examined directly on the ear, or the dried kernels are harvested and evaluated for the presence of the marker. Diploid progeny show the marker phenotype because they have a single copy of the marker gene from the X26 male parent, whereas gynogenic haploid progeny do not show the marker phenotype. The penetrance of the marker and the spontaneous haploid induction rate of X26 was tested in numerous control crosses. Using this system we screen for haploids and then test them on the ploidy analyzer to confirm that they are truly haploids.

We developed PCR tests to specifically detect the "wild-type" and "mutant" alleles for screening of nineteen Stock 6-derived inducers, including NP2222-Haploid Inducer (NP2222-HI), a BC3 introgression of RWK into Syngenta's standard transformable inbred line NP2222. We also screened nine non-inducer control lines.

To develop a PCR test that would distinguish between RWK/Stock6 and RWK-NIL haplotypes, two primer pairs were designed: one pair should amplify the RWK/Stock6 frame-shift allele, while the other should amplify the B73/RWK-NIL allele. These pairs worked as expected on RWK-NIL, RWK, and Stock6 DNA: RWK-NIL gDNA only amplified the RWK-NIL primer pair. RWK and Stock6 gDNA only amplified the RWK/Stock6 primer pair, which specifically detects the frame-shift allele. The PCR products were sequenced and the sequences were identical to that from whole genome sequencing. SNPs that were identified in the whole genome sequencing were confirmed in the PCR products. Below, in Figure 2, the DNA used in each reaction is in capital letters. The primers are "nil.F1/R1" and "rwk.F1/R1." GRMZM2G471240_ml.F1: GTACGCCGTGCGCTAACA (SEQ ID NO: 5). GRMZM2G471240_nil.R1: TCGTACCTCCCTGTCTCCAC (SEQ ID NO: 6). GRMZM2G471240_rwk.F1: TACGCCGTGCGCTAACATA (SEQ ID NO: 7). GRMZM2G471240_rwk.R1: GTACCTCGCTCCCTGTCTCC (SEQ ID NO: 8).

The "rwkF1/R1" and "nil.F1/R1" primer pairs were used to genotype the panel of high, low, and non-inducers. We found that all 19 haploid inducer lines had the 4 bp insertion, including Stock6 (3% haploid induction rate ["HIR"]), RWK (line derived from the University of Honheim stocks, 10-15% HIR), RWS, and Z22, among others. In contrast, the wild-type allele was found in all nine non-haploid inducer lines (average HIR of 0.1%). The data indicates that homozygosity for the frame-shift allele correlates with induction capacity: 12/12 high and 7/7 low inducers amplified the frame-shift assay, but not the wild type assay, while 9/9 non-inducers amplified the wild type but not frame-shift assay. This indicates that induction capacity correlates with the GRMZM2G471240 mutation, and that *pPLAIIα* underlies *qhir11* and is the primary mutation responsible for haploid induction in these lines.

**Table 2. GRMZM2G471240 PCR test results.**

| | **Induction Rate** | **RWK amplicon** | **RWK-NIL amplicon** |
|---|---|---|---|
| **Controls:** | | | |
| Stock 6 (low inducer) | 2.50% | + | - |
| RWK (high inducer) | 12% | + | - |
| RWK-NIL (non-inducer) | <1% | - | + |

| **Good Inducers:** | | | |
|---|---|---|---|
| ZMS | 7% | + | |
| Z19-PR | 7% | + | - |
| RWS-Z86 | 10% | + | - |
| K13 | 9% | + | - |
| (ID3002/Z22)B>29-5>2-5-1-B- | 7% | + | - |
| Z-19-//AF4031PR//Z-19-)1-1-2-3-1-3-B- | 9.50% | + | - |
| ZR86 | 12% | + | - |
| ZR53 | 12% | + | - |
| ZR75 | 13% | + | - |
| (Z21/RWS)B(GS)-75-1-2-3-B- | -8% | + | - |
| NP2222 inducer-good | -9% | + | - |

| **Poor Inducers:** | | | |
|---|---|---|---|
| Stock6 R1-nj | 2.50% | + | - |
| (Z21/RWS//[RWS]B$)33-5- | <2% | + | |
| (K-13-/(ZMS/SEW-PR)B>2>B-9//K-13-)2-4-1- | <2% | + | |
| (K-13-/(ZMS/SEW-PR)B>2>B-9//K-13-)6-1-2- | <2% | + | - |
| (ZMS/SEW-PR)B>2>B-7-2-1-2- | <2% | + | |
| NP2222 inducer-low | ~3% | + | |

| **Non-inducer Lines and Donors:** | | | |
|---|---|---|---|
| Stock6 R1-nj B1P11 | < 0.1% | - | + |
| (Z-21-/AF4031PR//Z-21-1-B-)1-1-1-1-B- | < 0.1% | - | + |
| FF6096 | < 0.1% | - | + |
| ID5829 | < 0.1% | - | + |
| XO5744 | < 0.1% | - | + |
| ID3002 | < 0.1% | - | + |
| AF4031PR | < 0.1% | - | + |
| NP2222 | < 0.1% | - | + |

We also identified a number of single nucleotide polymorphisms ("SNPs") between the frame-shift allele and that of RWK-NIL. For many of these SNPs, the STOCK6 and RWK sequences agreed with other inbreds we have sequenced, and thus likely represent natural variation. Indeed most of these SNPs did not alter the amino acid sequence and thus likely do not contribute to the haploid induction phenotype. Two SNPs did result in amino acid changes (H107Y; K232N) and these are not highly conservative changes, so they may have a small contribution to the phenotype, but mostly like they do not impact the phenotype because the frame-shift causes a loss of function.

We renamed *pPLAIIα "MATRILINEAL"* (*MTL;* i.e., SEQ ID NO: 1) and the native 4 bp insertion allele *"matrilineal" (mtl;* i.e., SEQ ID NO: 3). According to the predicted protein sequence, the 4 bp insertion causes a shift in the open reading frame of the protein at amino acid ("AA") 352 out of 401. The frame-shift leads to a premature stop codon.

After finding the frame-shift knock-out mutation we directly tested the effect it had on haploid induction by complementing a haploid inducer line with a wild-type *pPLAIIα* transgene. Heterologous complementation of NP2222-HI (10.2% HIR) with a wild-type copy of *MATRILINEAL* virtually eliminated haploid induction and kernel abortion. Compared to controls the HIR decreased 50-fold, from 10% to 0.23%. It also decreases the embryo abortion rate to 0.65%. Full length functional reporter lines were also made using transgenic fusions of the wild *type MTL* gene to GFP as well as the mutant allele *mtl* to GFP, in order to both visualize subcellular localization of wild-type *MTL,* but also to see if the mutant version of the protein localizes correctly or is produced at all. These lines also served as additional material to test for complementation. Haploid inducer material (NP2222-HI) that was homozygous for the *MTL-GFP* transgene also did not exhibit the haploid inducer phenotype. The induction rate of NP2222-HI falls to to 0.60% when it is homozygous for *MTL-GFP.* Additionally, the *MTL-GFP* transgene also knocked down embryo abortion to 4.86%. Finally we tested whether the mutant *mtl* allele fused to GFP complements the haploid induction phenotype, and it does not. Haploid induction and embryo abortion rates were very similar in NP2222-HI compared to NP2222-HI that was homozygous for the mutant fusion transgene *mtl-GFP.* See Table 3. This represents conclusive evidence that the MATRILINEAL frame-shift is responsible and required for haploid induction. To apply this knowledge, we demonstrate that mutating or modulating the expression of pPLAIIα in a wild type line leads to the creation of new haploid induction lines.

**Table 3. Reproductive characteristics of haploid inducer, complementation and edited lines. This table shows the haploid induction and kernel abortion rates of inducer lines in the NP2222 background. The number testcrosses is listed first ("ears") and then the kernel and embryo statistics are listed. Embryo abortion and haploid induction generally comes together on the same ear. That is why the embryo abortion rate is so high in an ear crossed by the NP2222-HI male, which has a 10.17% haploid induction rate (HIR). Both the WT transgenes, including one without GFP and one fused to GFP, complemented the haploid induction phenotype. Meanwhile, the mutant mtl fused to GFP did not complement.**

| **Complementation Assays** | | | | Kernel characteristics | | Embryos tested | | |
|---|---|---|---|---|---|---|---|---|
| Male parent | ears | viable | aborted | **% aborted** | embryos | haploids | diploids | **HIR** |
| NP2222-HI | 4 | 548 | 498 | **47.61%** | 531 | 54 | 477 | **10.17%** |
| NP2222-HI + *MTL* / *MTL* | 17 | 4403 | 29 | **0.65%** | 4321 | 11 | 4310 | **0.25%** |
| NP2222-HI + *mtl-GFPlmtl-GFP* | 3 | 371 | 298 | **44.54%** | 360 | 34 | 326 | **9.44%** |
| NP2222-HI + *MTL-GFPIMTL-GFP* | 3 | 1019 | 52 | **4.86%** | 836 | 5 | 831 | **0.60%** |

Several *mtl-like* alleles were generated in the inbred NP2222 by introducing small deletions *in MTL* close to the 4 bp insertion site in *mtl,* using transcription activator-like effector nucleases (TALEN) (Boch, J. et al., Breaking the code of DNA binding specificity of TAL-type III effectors, SCIENCE 326:1509-1512 (2009), incorporated herein by reference). Several mutant events were self-pollinated and T1 plants lacking the TALEN T-DNA insert but homozygous for small deletions in *MTL* were outcrossed onto NP2222. Edited lines homozygous for frame-shift deletions in *MTL* (hereafter called *MTL^{TAL-FS})* exhibited an HIR of 4.0 - 12.5% (average 6.65%) (Table 4). The ploidy status of 118/127 putative haploids was confirmed by Flow Cytometry, and phenotypic evaluations indicated these plants were haploids. These results prove that a frame-shift in *MTL* is sufficient to induce high rates of haploid induction. Other contributors to the phenotype have been mapped including the neighboring qhir12 (see Liu, 2016)), which may account for the difference between the HIR of *MTL^{TAL-FS}* and NP2222-HI. It reasonable to infer that seed set, HIR and kernel abortion rates are set through *mtl* by paternal and maternal genotype-specific interactions.

**Table 4. Reproductive characteristics of MTL edited lines (generally referred to as MTL^{TAL-FS} lines). This table shows the haploid induction and kernel abortion rates of inducer lines in the NP2222 background. The transgenic events tested are given on the left followed by the number of testcrosses made ("ears") and the progeny statistics.**

| | | | Kernel Characteristics | | | | Ploidy Analysis Data | | | |
|---|---|---|---|---|---|---|---|---|---|---|
| Event | ID | Mutation(s) | Ears | Avg. viable | Avg. aborted | % aborted | Total Embryos | Putative Haploids | Confirmed Haploids | HIR |
| 39A | 3954 | Biallelic (13 bp & 28 bp dels) | 4 | 162 | 128 | 44.10% | 579 | 37 | 35 | 6.04% |
| 23A | 3924 | Biallelic (8 bp & 5 bp dels) | 2 | 114 | 116 | 50.40% | 128 | 18 | 16 | 12.50% |
| 81A | 3932 | Homozygous (13 bp del) | 2 | 165 | 129 | 43.90% | 169 | 18 | 15 | 8.88% |
| 81A | 3317 | Homozygous (13 bp del) | 2 | 183 | 108 | 37.10% | 343 | 19 | 19 | 5.54% |
| 81A | 3303 | Homozygous (13 bp del) | 1 | 189 | 100 | 34.60% | 176 | 7 | 7 | 3.98% |
| 38A | 4108 | Biallelic (11 bp & 5 bp dels) | 4 | 147 | 102 | 40.10% | 379 | 28 | 26 | 6.86% |
| 18A | 22807-4016 | Homozygous (8 bp del) | 8 | 144 | 97 | 40.20% | 1025 | 47 | 44 | 4.29% |
| 27A | 22807-4073 | Biallelic (1 bp insert & 5 bp del) | 2 | 161 | 92 | 36.40% | 180 | 18 | 18 | 10.00% |
| 27A | 22807-4081 | Biallelic (1 bp insert & 8 bp del) | 6 | 176 | 116 | 39.80% | 931 | 45 | 44 | 4.73% |
| 76A | 22873-3999 | Homozygous (2 bp insert) | 2 | 175 | 95 | 35.20% | 117 | 17 | 16 | 13.68% |
| 32A | 22873-3991 | Homozygous (1 bp del) | 2 | 140 | 105 | 42.90% | 260 | 14 | 14 | 5.38% |
| Total | | Totals | 15 | 160 | 108 | 40% | 390 | 24 | 23 | 7% |

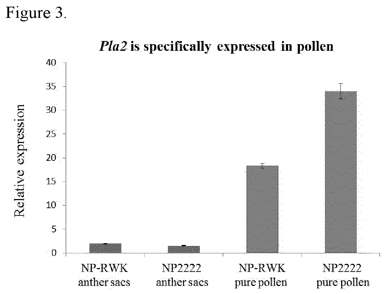

Haploid seed formation in maize is a post-zygotic character triggered by a defective male gametophyte. This fact is reflected in *MTL* expression data. Public RNA-seq profiles indicate the wild-type *MTL* transcript is specific to anthesis-staged anthers (see Sekhon, R. S., et al. Genome-wide atlas of transcription during maize development, PLANT JOURNAL, 66, 553-563 (2011), incorporated herein by reference), in agreement with a developmental profile that found it exclusively in pre-dehiscent anthers (see Zhai, J., et al. Spatiotemporally dynamic, cell-type-dependent premeiotic and meiotic phasiRNAs in maize anthers, PNAS 112, 3146-3151 (2015), incorporated herein by reference). We found that wild-type pollen had 18x more *MTL* transcript than post-anthesis anther sacs, indicating the gene is male gametophyte-specific (Figure 3). Attempts to knockdown *MTL* by RNAi led to elevated rates of haploid formation for *MTL^{RNAi}* only (Table 5). There are two annotated splice variants of *MTL*, reflecting an alternative splice site 81 nucleotides prior to the 3' end of exon 2. Compared to NP2222, Mtl was elevated in NP2222-HI but not MTL^{TAL-FS} pollen (Figure 4), while the abundance of the two annotated splice variants was consistent.

**Table 5. RNAi construct 22503 (SEQ ID NO: 32) to knockdown Mtl led to haploid induction.**

| GRMZM2G471240 RNAi | Event ID | | Kernel Characteristics | | | Embryos tested for ploidy | | | |
|---|---|---|---|---|---|---|---|---|---|
| Individual ID | | ears | viable | aborted | % aborted | embryos | haploids | diploids | HIR |
| 5148 | 001 | 2 | 701 | 43 | 5.78% | 369 | 3 | 366 | 0.81% |
| 5149 | 001 | 2 | 186 | 22 | 10.58% | 166 | 1 | 165 | 0.60% |
| 5153 | 001 | 2 | 625 | 61 | 8.89% | 323 | 7 | 316 | 2.17% |
| 5161 | 001 | 3 | 1116 | 87 | 7.23% | 485 | 4 | 481 | 0.82% |
| 5170 | 028 | 2 | 629 | 23 | 3.53% | 324 | 1 | 323 | 0.31% |
| 5173 | 028 | 2 | 551 | 33 | 5.65% | 322 | 0 | 322 | 0.00% |
| 5187 | 028 | 3 | 379 | 27 | 6.65% | 333 | 9 | 324 | 2.70% |
| 3731 | 014 | 2 | 894 | 23 | 2.51% | 263 | 4 | 259 | 1.52% |
| 3732 | 014 | 2 | 648 | 49 | 7.03% | 351 | 0 | 351 | 0.00% |
| 3736 | 007 | 1 | 277 | 21 | 7.05% | 277 | 0 | 277 | 0.00% |
| 3737 | 007 | 1 | 223 | 49 | 18.01% | 175 | 3 | 172 | 1.71% |
| 3751 | 005 | 1 | 133 | 6 | 4.32% | 118 | 0 | 118 | 0.00% |
| | 5 | | | | | | | | |
| TOTALS | events | 23 | 6362 | 444 | 6.52% | 3506 | 32 | 3474 | 0.91% |

The frame-shift in *mtl* occurs at amino acid 380, leading to 20 altered amino acids followed by a premature stop codon which truncates the protein by 29 amino acids (Figure 5a). The wild-type *MTL* protein was found in LS-MS profiles of RWK-NIL and NP2222 pollen, but was below the detection limit in three out of three RWK and 3 out of 3 NP2222-HI samples (Table 6). This demonstrates that even though there is mutant m*tl* transcript produced in pollen, the protein is not detected, confirming this is a loss of function mutation. Both mutant and wild type recombinant *MTL* proteins exhibited phospholipase activity in vitro in pPLAIIα-like fluorescent liposome cleavage assays (Figure 5b). This demonstrates that the functional annotation of the *MTL* gene (i.e. that it codes for a phospholipase protein) is correct.

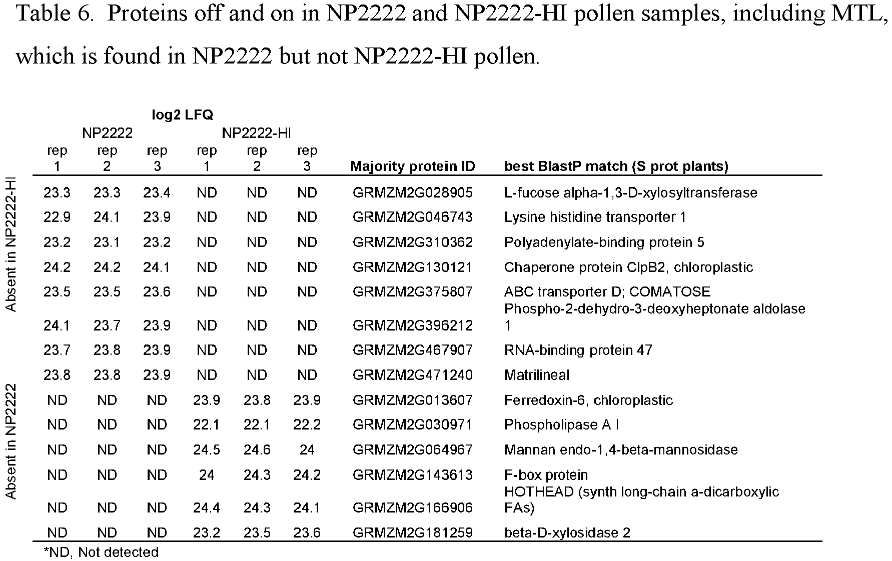

### II. Pollen Germination and Localization Experiments

Full length functional reporter lines were used to characterize *MTL* localization. No signal was found in the pollen of NP2222 or NP2222 + *mtl-GFP*/*mtl-GFP.* In contrast, NP2222 + *MTL-GFP*/*MTL-GFP* pollen exhibited a strong signal in the cytoplasm of the two sperm cells. This signal was found in the stringy gamete cytoplasm within germinated pollen tubes. NP2222 embryo sacs fixed 18 hours after pollination with MTL-GFP pollen had signal in the area of the degenerating synergid consistent with that of SCs delivered during fertilization. This indicates *MTL* is part of the male germ unit that is deposited in the embryo sac after pollen tube burst. *MTL-GFP* but not *mtl-GFP* eliminated haploid induction in NP2222-HI (Table 3). Collectively these data indicate that *MTL* is a phospholipase specific to the SC cytoplasm, and that the frame-shift in *mtl* compromises *MTL* localization or stability in haploid inducer pollen..

The identification of *MTL* as the causative gene in maize haploid induction permitted dissection of the pleiotropic phenotypes historically associated with the trait. Phospholipase mutations are associated with delayed pollen germination and tube growth (see Kim, H. J., et al. Endoplasmic reticulum- and golgi-localizedphospholipase A2 plays critical roles in Arabidopsis pollen development and germination, PLANT CELL 23, 94-110 (2011)), but these were normal in RWK, Stock 6 and MTL^{TAL-FS} lines (Figure 6A, B). Ears pollinated with NP2222-HI and *MTL^{TAL-FS}* pollen exhibit ∼10-25% fertilization failure, and a pollen competition assay showed that RWK is subject to segregation distortion (SD) (Figure 6C), consistent with prior reports (see Xu, X., et al. Gametophytic and zygotic selection leads to segregation distortion through in vivo induction of a maternal haploid in maize, J. EXP. BOT. 64, 1083-1096 (2013)). Crosses with hemizygous NP2222-HI + *MTL*/*0* pollen produced a proportional bias towards *MTL*+ progeny (Figure 6D), indicating that inducer SD is attributable to *mtl.* Embryo abortion, a persistent byproduct of haploid induction linked to endosperm proliferation failure, occurred at similar rates in native and *MTL^{TAL-FS}* inducers (Table 4). Collectively these data implicate *mtl* in every reproductive defect associated with haploid induction. The two mechanisms typically proffered to explain haploid formation are single fertilization and post-zygotic genome elimination (*see* Sarkar, K. R. & Coe, E. H, A genetic analysis of the origin of maternal haploids in maize, GENETICS 54, 453-464 (1966); Zhang, Z., et al., Chromosome elimination and in vivo haploid production induced by Stock 6-derived inducer line in maize (Zea mays L.), PLANT CELL REP. 27, 1851-1860 (2008); and Barret, P., Brinkmann, M., & Beckert, M., A major locus expressed in the male gametophyte with incomplete penetrance is responsible for in situ gynogenesis in maize, THEOR. APPL. GENET. 117, 581-594 (2008)). In the former, haploids result from fertilization of the central cell but not the egg, which subsequently develops via parthenogenesis. In the latter, double fertilization precedes male chromosome elimination. Clarifying the precise mechanism will require careful embryology after *MTL^{TAL-FS}* pollinations, along with quantitative data tracking the rare persistence of male DNA in maize haploids.

Haploid induction was recently engineered in *Arabidopsis* via manipulation of *CENTROMERIC HISTONE3,* which causes uniparental genome elimination through post-zygotic centromere imbalance between hybridized genomes. An attempt to replicate this in maize was successful *(see* Ravi, M. & Chan, S. W. L. Haploid plants produced by centromere-mediated genome elimination, NATURE 464, 615-618 (2010)), but this filing is the first instance of a haploid inducer system triggered by a cytoplasmic protein that does not bind chromatin. Thus, this work highlights the importance of non-nuclear sperm components in reproductive success and faithful genome transmittance. The conservation of *MTL* in the grasses (see Fig. 7), especially in rice where the closest homolog is pollen-specific and also found in sperm, suggests these findings will lead to the development of novel intra-specific haploid inducer lines in important crop plants.

### III. Chemical Sprays to Induce de novo HI

After discovering that the *MTL* phospholipase (and specifically, the *mtl* loss-of-function frameshift mutation) triggers haploid induction, we tested whether phospholipase inhibitors, fatty acids, or other lipid compounds might act as chemical haploid inducers.

We have successfully achieved *de novo* haploid induction through application of various chemistries to flowers, silks, ears, tassels, and pollen. These chemistries are all in the class of lipids or phospholipase inhibitors. Table 7 outlines those chemistries and the haploid induction rates that resulted from their application. There is also formulation information and mode of application information in Table 7. From this table it is clear that a variety of lipid compounds induce haploids *de novo* when applied before, during, or after pollination to silks, which is stigmatic tissue specific to the maize plant, pollen, tassels (which contain male flowers containing pollen prior to pollen shed), and ears. Such compounds include mixed oils, fatty acids, fatty acid esters, phospholipids, and phospholipase inhibitors.

We tested many phospholipase inhibitors, including manoalide, a phospholipase inhibitor without any fatty acid chains. This chemistry was able to induce haploid formation *de novo.* With that result in hand, we started testing more compounds. We found that the most active compound for de novo induction is methyl alpha linolenyl fluorophosphonate (MALFP), a potent phospholipase inhibitor and inhibitor of other lipid modifying enzymes. This compound contains three linolenate fatty acid chains (eighteen carbons with three triple bonds) and a methyol group and fluorine atom at the head group position. Another highly active *de novo* haploid inducer compound is methyl arachidonyl fluorophosphonate (MAFP), another phospholipase inhibitor with a slightly different fatty acid chain structure (20 carbons and four double bonds). Without wishing to be bound by theory, MALFP and MAFP inhibit phospholipases by sitting in the fatty acid chain binding pocket, and in some cases catalyzing irreversible phosphorylation of the serine amino acid in the active site (*see* Lio Y.C., Reynolds L.J., Balsinde J., and Dennis E.A. Irreversible inhibition of Ca(2+)-independent phospholipase A2 by methyl arachidonyl fluorophosphonate. BIOCHIMBIOPHYS ACTA 1302:55-60 (1996)). Some fatty acids such as linoleic acid and linolenic acid can also non-competitively inhibit phospholipases by sitting in the fatty acid chain binding pocket without covalently modifying the active site, though the effect of this inhibition is weaker. Ballou, L.R., and Cheung, W.Y. Inhibition of human platelet phospholipase A2 activity by unsaturated fatty acids PROC NATL ACAD SCI 82 (2): 371-375 (1985)). These references may help to explain the reason for our findings that *de novo* haploid induction is not only triggered by strong inhibitors of phospholipases like MALFP and MAFP, but also by fatty acids esters including arachidonic acid methyl ester (AAME), which has 20 carbons and four double bonds (poly unsaturated 20 carbon fatty acid ester), as well as linoleic acid (LLE), which has 18 carbons and two double bonds (18:2), and linolenic acid (LNA), which has 18 carbons and three double bonds (18:3), and oleic acid ethyl ester (OAEE), and arachidonic acid (which has 20 carbons and four double bonds). Again it is thought that these unsaturated fatty acid chains, whether in their free fatty acid form, their ester form (which are synthesized in the lab and may be able to penetrate tissues) or in some other form, can act as inhibitors of phospholipases. While it is generally not recognized that the ester form of these compounds can inhibit phospholipases (*see* Ballou, *supra*), we find that such fatty acid methyl and ethyl esters can indeed lead to de novo haploid function. See Table 7. Of note, saturated fatty acids and derivatives, including stearic acid and palmitic acid ethyl ester, were tested. The prior art states that molecules that contain saturated fatty acid side chains do not or would not be able competitively inhibit a phospholipase by residing in its active site. *See* Ballou, *supra.* Such compounds were believed to lack the ability to inhibit phospholipases. However, we were surprisingly able to use molecules containing saturated fatty acid side chains to induce formation of haploids *de novo.* For example, palmitic acid ethyl ester (PAEE) induces haploids. See Table 7.

We also decided to test whether certain oils and phospholipids could trigger haploid induction *de novo,* including common plant oils. We found that one highly active *de novo* haploid inducer lipid mixture is corn oil, a natural plant extract that can be purchased from a grocery store. Another highly active lipid mixture is linseed oil that can be purchased from a hardware store. Canola oil, vegetable oil, peanut oil, sesame oil, or any other oil, when applied in the correct fashion, will work as well. Another highly active compound is 1,2-distearyl-sn-glycero-phosphatidylcholine (DSPC) (also known as 18:0 18:0 PC), a phospholipid with a phosphatidylcholine head group and a two 18-carbon saturated fatty acid chains. This common phospholipid is present in many biological materials because it is one of the core and most abundant components of phospholipid bilayers, which comprise the cellular membranes of all living things on the planet. Another haploid inducing compound is phosphatidylethanolamine with one stearyl and one oleoyl chain (also known as 1-stearyl-2-oleoyl-sn-phosphatidylethanolamine), another common phospholipid. Other phospholipids, as well as lyso-phospholipids and other triacylglycerides, diacylglycerols, lysophospholipids, triterpenoid esters, or glycerolipids will act as *de novo* haploid inducers. These oils and phospholipids are not commonly known to inhibit phospholipases, but they may be the source material for the generation of the byproducts of phospholipase activity, including the very fatty acids that inhibit phospholipases. The fact that these compounds act as *de novo* haploid inducer chemistries thus indicates these oils and phospholipids may inhibit phospholipases indirectly, or perhaps they are causing an imbalance or alteration in lipid or membrane composition of pollen leading to haploid induction. Either way, it is clear from this work that an enormous range of lipid-containing compounds, in addition to non-lipidic phospholipase inhibitors, when applied to flowers, can act as chemical inducers of haploid seed.

We also tested whether the buffers we used to apply these compounds to flowers were able to induce haploids by themselves. These buffers variably included certain surfactant blends and/or salt and/or DMSO (dimethyl sulfoxide) and/or other related additions. These buffers were not able to induce haploids, as can be seen in lines 121-123of Table7. Also, de novo haploid induction without the addition of any compounds is extremely rare in maize, as can be seen from line 1. Two haploids were found among the 3,073 progeny tested (Kelliher, et al. Maternal haploids are preferentially induced by CENH3-tailswap transgenic complementation in maize, FRONTIERS IN PLANT SCIENCE 7: 414 (2016)). Thus, the control induction rate was found to be approximately 0.065%, which falls in the reported background range of gynogenic haploid induction (0.05 - 0.1%) (Chang, M.-T.,and Coe, E. "Doubled haploids,"in Molecular Genetic Approaches to Maize Improvement, eds A.L.Kriz and B.A. Larkins (Heidelberg: Springer), 127-142).

The mode of application of these various lipid and lipase inhibitor compounds can be quite variable and still produce haploids. One such mode of application is to dissolve the compounds in a salt solvent with additions of 1% DMSO. Another such solvent is a surfactant blend (Table 8). The tissue of application can also vary, as is evident from Tables 7, where application to both pollen and silks during pollination, as well as to tassels hours or days before pollination, can result in the formation of haploids. Furthermore, a wide variety of concentrations of the compounds can induce haploids, from 20 uM to 100 mM, or from 0.2 mg/mL up to 50 mg/mL. In fact, a much wider range than this is able to induce haploids, as can be seen in Table 7.

In two cases, we have also developed novel surfactant blends to help specific classes of lipids be able to emulsify and form a microemulsion (an aqueous - organic mixture with lipid droplet sizes smaller than a certain size - at least smaller than 10 microns in diameter and in many cases smaller than 1 micron in diameter). See Table 8 for the composition of the two surfactant blends. Blend "91" is ideally used with fatty acids at a concentration of 6.5 parts surfactant blend to 1 part fatty acid. However, multiple related concentrations and related surfactant blends could also work. Surfactant Blend "92" is ideally used with fatty acid esters, as well as oils, either in a pure form or as a triglyceride mixture. This is ideally blended at a ratio of 10 parts surfactant blend to 1 part ester or oil. Either of these blends or related blends of compounds may be suitable for proper dissolving or microemulsion construction with related lipid molecular classes outside of fatty acids, esters, and oils. These may include phospholipids, diacylglycerols, lysophospholipids, triterpenoid esters, or glycerolipids.

These surfactant blends are typically blended with the active ingredient and then mixed at a certain percentage with an aqueous buffer to make an emulsion and ideally a microemulsion. When making the emulsion, the percentage of the surfactant blend + Active ingredient in the aqueous buffer can be anywhere from 0.01 - 50%. The aqueous buffer can consist of any number of things. We have used 1x PBS + 1% DML and PBS + 50% DML, but other formulations work as well. "PBS" stands for phosphate-buffered saline; "DML" stands for dimethyl lactamide.

The use of these buffers alone was not able to lead to de novo haploid induction, as can be seen in Table 7, lines 121-123.

The mode of application of the compounds can take any number of forms including floral dip, floral injection, microinjection, pollen soaking, pollen misting, pollen spraying, floral misting, floral spraying, silk dousing, silk spraying, etc. We have most often used tassel, pollen, and silk spraying before, during, or after pollination. We have achieved *de novo* haploid formation with DSPC and linolenic acid when tassels were sprayed two days prior to pollination. We have also achieved de novo haploid formation with DSPC when tassels were sprayed just a few minutes prior to pollen collection and subsequent pollination. Similarly, we have achieved *de novo* haploid formation with DSPC when pollen was sprayed just before or during pollination, and also when silks were sprayed before, during, or after pollination, including up to two days before pollination and up to six hours after pollination.

We have also demonstrated *de novo* haploid formation when different applicators were used, including a cheap, plastic spray bottle that one can purchase at a convenience store, and a nebulizer, which is a medical device used to deliver medicines orally in the form of very small aqueous droplets. While the spray bottle typically produced droplet sizes of 50 - 150 microns in diameter, the nebulizer is able to generate droplet sizes of less than 10 microns. This is beneficial for application to pollen because pollen sizes range from 20 - 200 microns in diameter in most plants (approximately 70 microns in maize; approximately 50 microns in rice) and if one of the droplets from a typical spray bottle hits a grain, the droplet would be bigger than the grain. Pollen is extremely sensitive to moisture and osmotic shock. If the pollen grain comes into contact with a droplet of too large a size, that grain fails to germinate a successful, growing pollen tube. The same might be the case if the grain lands on a stigma or silk that is too wet - for instance, if that silk or stigma received too much of the lipid application (be it a microemulsion, or simply lipid droplets or micelles formed in an aqueous solution). If we applied more than 3 mL of lipid spray to the pollen or silks via spray bottle or nebulizer, we would often get very low seed set.

Therefore the mode of application is critical and in particular it is most important to try to apply as little volume of spray as possible to the pollen or stigma or silks or to the flower generally, and the best way to distribute the active ingredient, especially a lipid, with as little volume as possible is to make a microemulsion of that lipid in an aqueous buffer where the lipid droplet sizes is at the sub-micron level, and then to dispense that microemulsion with a nebulizer or similar device capable of making droplet sizes in the 1-2 micron range. This results in delivery of millions of droplets of active ingredient in "vapor" form to the relevant tissues, with the droplet size being >100,000 x smaller volume than the pollen grain itself (if the droplet is 1 micron, and the pollen grain is 50 microns, then the diameter of the droplet is 50x smaller than the grain. And 50³ = 125,000, so the volume of the droplet is >105,000 times smaller than the pollen grain. With microemulsions, the lipid droplet size can range from approximately 20-1000 nm in diameter, so depending on the concentration of the surfactant blend plus active ingredient in the aqueous solution, one might have one or more lipid packets in each droplet delivered to each pollen grain or silk. Furthermore, considering some lipid type molecules or phospholipase inhibitors can be dissolved in an aqueous solution instead of being delivered as a microemulsion, we find that a wide variety of concentrations of the compounds can induce haploids, from 20 µg/mL to up to 50 mg/mL. Possibly higher concentrations will induce haploids *de novo.*

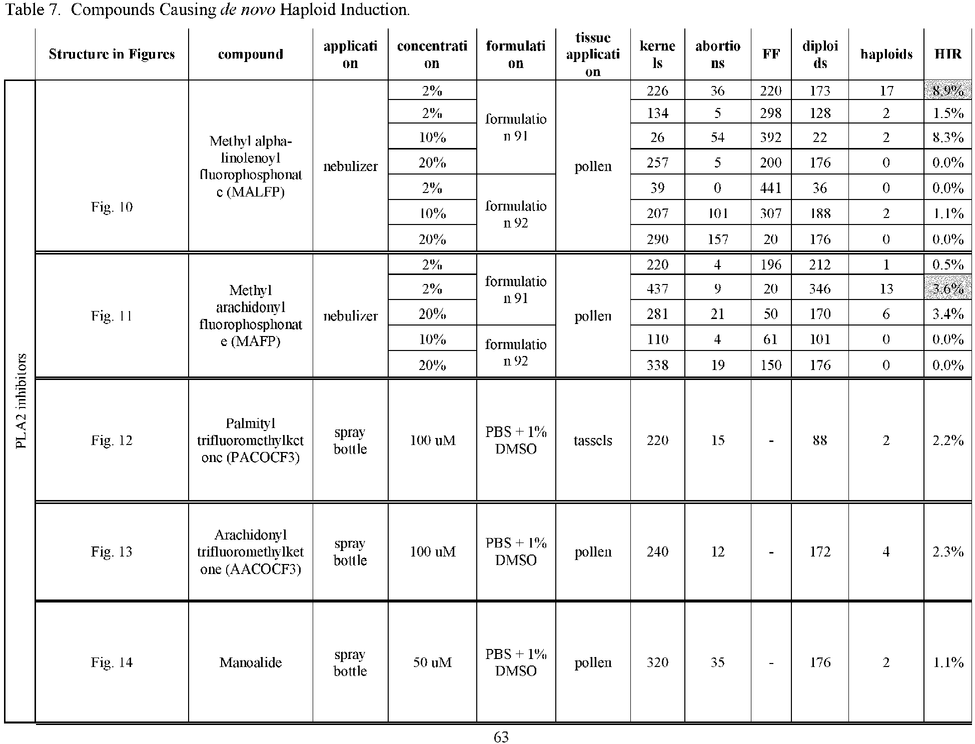

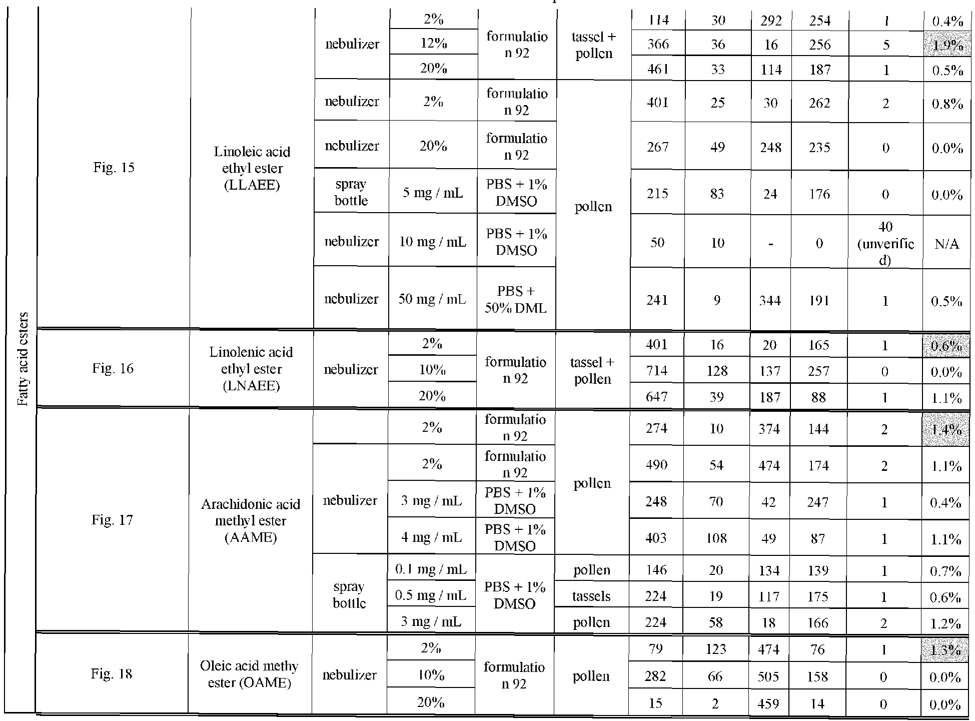

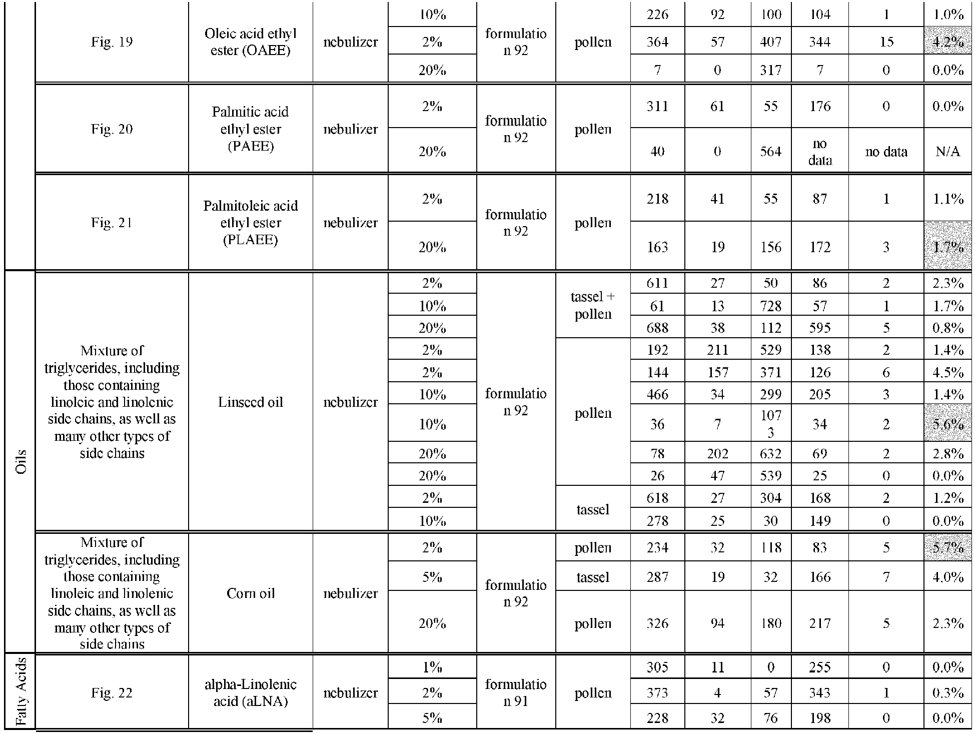

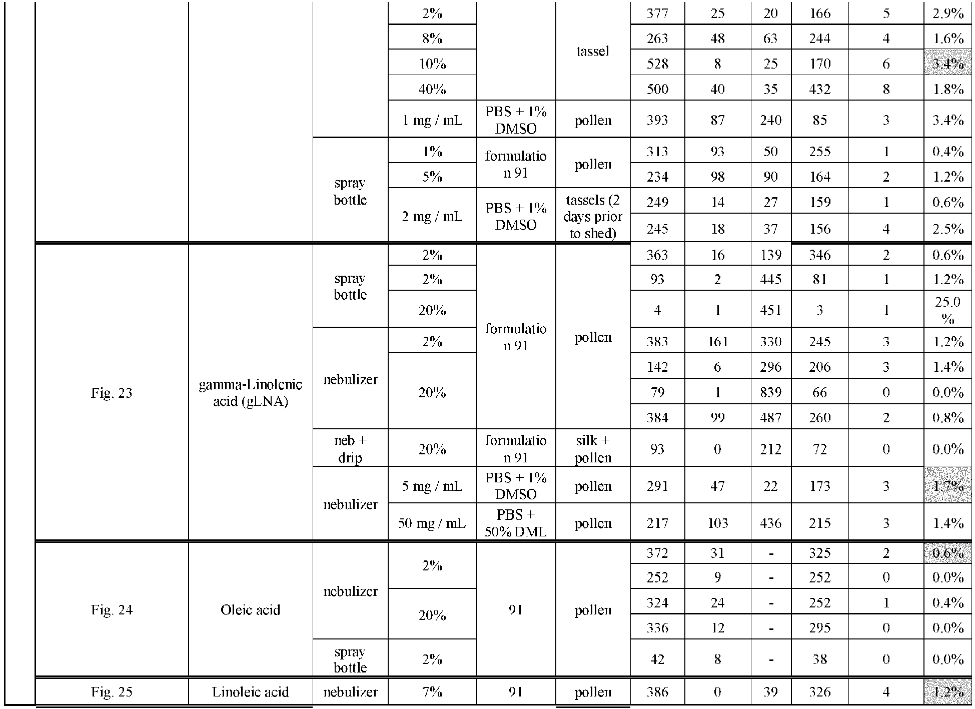

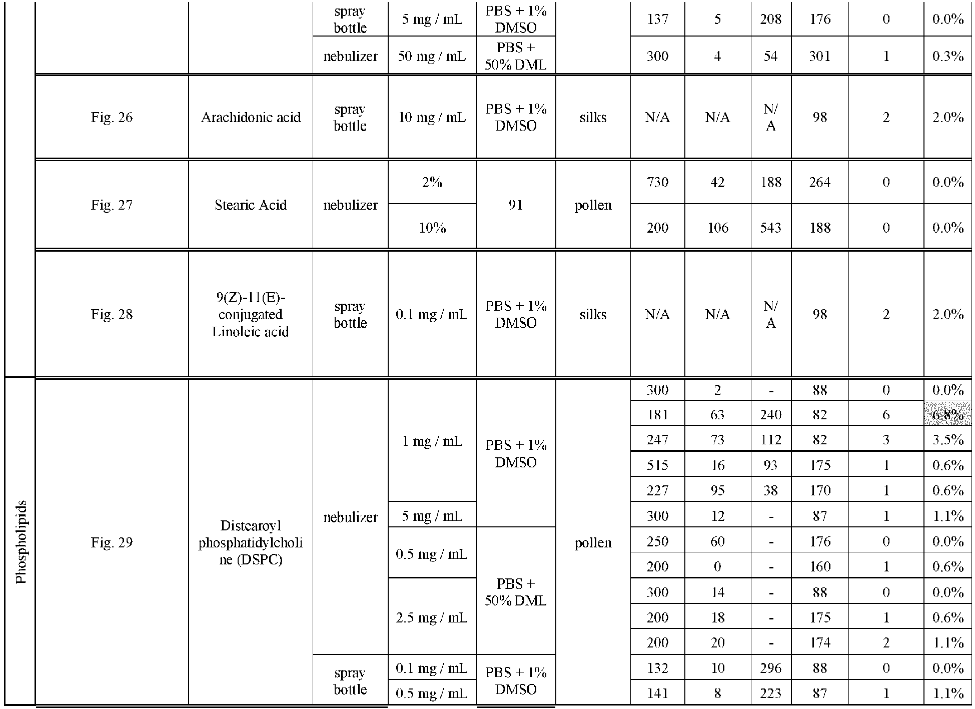

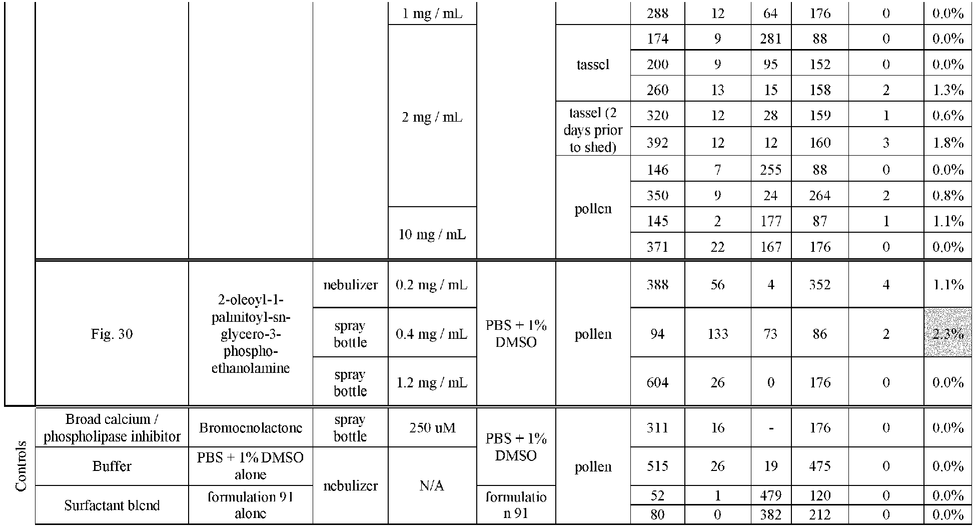

**TABLE 8. Recipes for formulation 91 ("F91") and formulation 92 ("F92"), which help active ingredients such as fatty acids, oils, esters, and phospholipids to be emulsified in very small lipid droplets. Formulation 91 is a surfactant blend to be mixed with the fatty acid optimally at 1 part fatty acid to six and a half parts formulation 91. The mixture is meant for creating emulsions with an aqueous buffer at concentrations of 0.001 - 50%. Formulation 92 is also a surfactant blend which can be mixed with esters or oils optimally at 1 part active ingredient to 10 parts formulation 92, and then emulsified in an aqueous buffer at concentrations of 0.001 - 50%.**

| **Formulation 91** | Amount (grams) | Chemical identity |
|---|---|---|
| Jeffsol AG1560 | 95.99 | butylene carbonate solvent |
| Emulpon CO360 | 24.02 | castor oil ethoxylate (36 mole EO) |
| Witconate P1220EH | 24.08 | dodecyl (branched) benzene sulfonate, Ca salt. 60% solution in ethyl-hexanol |
| Agrimer AL-22 | 12.03 | poly(alkyl-pyrrolidone). Alkyl mainly C16 |
| | 156.12 | |
| | | |
| **Formulation 92** | Amount (grams) | Chemical identity |
| Agnique AMD 3-L | 104 | dimethyl lactamide solvent |
| Witconic 1298 soft | 15.67 | dodecyl (linear) benzene sulfonic acid |
| Surfonic T-5 | 12.2 | tallowamine ethoxylate (5 mole EO) |
| Surfonic T-15 | 24.21 | tallowamine ethoxylate (15 mole EO) |
| | 156.08 | |

### IV. Increasing the HIR and Restoring Seed Set by Application of a Lipid Compound

Molecular profiling, including metabolomics and lipomics profiling, of haploid inducer and non-inducer pollen (comparatively) show that haploid inducer pollen is particularly deficient in certain types of lipid classes and overabundant in others. This deficiency is particularly pronounced in the 18 carbon chain class of lipid molecules, although it is also seen in 20 carbon chain classes. The deficiency is particularly pronounced with 18 carbon chain lipids with one and two double bonds (the so-called oleates and linoleates) and the overabundance was found particularly with the 18 carbon chain lipids with three double bonds (the so called linolenates). With respect to the types of lipids that are altered in haploid inducer pollen, it is very broad, and includes triglycerides, diacylglycerides, free fatty acids, lyso-phospholipids, and phospholipids. The changes in lipid content in haploid inducer pollen are also variable across different levels of fatty acid saturation. After seeing this data, we decided to apply lipid compounds flowers during pollination with haploid inducer pollen to see if simply adding certain types of lipids might impact the rate of haploid formation or the kernel count. We were particularly interested in applying compounds that had, in their molecular structure, some 18 or 20 carbon saturated fatty acid chains that were among those that were seen to be lower in abundance in haploid inducer pollen (e.g. oleate and linoleates).

Lipid applications can also lead to increases in the rate of haploid formation, seed set, and reductions in the frequency of kernel abortion, as is indicated in Tables 10-13. Table 10 shows nine separate, controlled experiments using the compound methyl alpha linolenyl fluorophosphonate (MALFP). Each experiment involved two ears crossed by the same haploid inducer male parent plant, on the same day, at nearly the exact same time (within 5 minutes of each other) from the exact same pollen population collected from the same male parent. These two ears either received a control buffer application, or a buffer plus active ingredient. Both the male and female test plants were grown next to each other under the same conditions in the same greenhouse. On average, and compared to control applications of buffer without the MALFP, when MALFP was applied the haploid induction rate increased by 3.6%, the number of kernels increased by nearly 42 per ear, and the number of total haploids recovered per ear increased by 11.8 haploids per ear, and the embryo abortion frequency decreased by 1.1% (Table 9). These all represent improvements for a haploid induction production scheme. Improvements were particularly apparent when formulation 91 was used as the surfactant blend to dissolve the MALFP. The mechanism by which these inhibitors are having an additive effect with *mtl* leading to higher induction rates could be that they are disrupting other phospholipases or lipid-modifying proteins.

The same type of data was collected for the fatty acid ethyl ester LLAEE, leading to an increase in the haploid induction rate of 2.4%, an increase of over 43 kernels formed per ear, an increase over 8 haploids per ear, and a decrease in the frequency of embryo abortion of 3.7%. Though many of these treatments involved different amounts of the active ingredient, on the majority of these experiments LLAEE led to a boost in these haploid formation and seed setting statistics. See Table 10.

The same type of data was collected for the fatty acid linoleic acid (LLA) (Table 11), leading to an increase in the haploid induction rate of 1.5%, an increase of 41 viable kernels per ear and 7.6 haploids per ear, and a decrease in the embryo abortion rate of 2.9%. This is the result of 22 separate experiments, again with each experiment consisting of one ear pollinated by the control buffer and one ear pollinated by the buffer plus active ingredient (LLE). It is interesting that the abundance of 18:2 is down in haploid inducer pollen (as shown by the lipomics data). Without wishing to be bound by theory, it is reasonable to hypothesize that this may cause or contribute to fertilization issues and embryo abortion. When we add these back via spray application of LLA and LLAEE, these molecules could be inhibiting phospholipases leading to higher HIR, and supplementing 18:2 leading to more kernels.

Finally, similar data was collected for MAFP, and similar results were seen, including a boost to the haploid induction rate of 1.9%, an increase in the number of haploids recovered per ear of 6.6 and the number of total kernels of 28, well as a decrease in the embryo abortion rate of 2.2% (Table 12). These compounds, as well as other lipids, can be used to boost the frequency of haploid embryo formation in haploid induction nurseries, and can also be used to increase seed set in the context of self-pollinating haploid inducer lines in order to increase more stock seed of those lines.

**able 9. MALFP effect on HIR, kernel set, kernel abortion, and overall haploids recovered.**

| Experiment No. | MALFP Concentration | Buffer | *Comparing MALFP* to *the control* | | | |
|---|---|---|---|---|---|---|
| | | | Δ Haploids | Δ HIR | Δ kernels | Δ k. a. rate |
| 1 | 2% | formulation 91 | + 37 | + 16.3% | - 52 | + 16.3% |
| 2 | 2% | formulation 91 | + 25 | + 9.4% | + 101 | - 4.4% |
| 3 | 2% | formulation 92 | - 9 | - 6.2% | + 68 | - 3.4% |
| 4 | 2% | formulation 92 | + 71 | + 29.8% | + 86 | - 21.0% |
| 5 | 10% | formulation 92 | - 20 | - 7.9% | - 38 | - 2.4% |
| 6 | 14% | formulation 91 | + 2 | - 0.5% | - 18 | + 19.9% |
| 7 | 14% | formulation 91 | + 11 | + 3.5% | + 70 | - 19.2% |
| 8 | 20% | formulation 92 | - 9 | - 6.0% | + 89 | - 4.0% |
| 9 | 20% | formulation 92 | - 9 | - 7.6% | + 69 | -4.3% |

| | | ***AVERAGE*** / ***EAR*** | haploids | HIR | kernels | k. a. rate |
|---|---|---|---|---|---|---|
| | | MAFP | 34.6 | 17.1% | 238.8 | 32.4% |
| | | control | 22.8 | 13.5% | 197.3 | 33.5% |
| | | MAFP vs control | + 11.8 | + 3.6% | + 41.5 | - 1.1% |

**Table 10. LLAEE effect on HIR, kernel set, kernel abortion, and overall haploids recovered.**

| Experiment No. | LLAEE Concentration | Buffer | *Comparing LLAEE to the control* | | | |
|---|---|---|---|---|---|---|
| | | | Δ Haploids | Δ HIR | Δ kernels | Δ k. a. rate |
| 1 | 2% | formulation 92 | + 7 | + 11.3% | - 29 | - 2.4% |
| 2 | 5% | formulation 92 | + 3 | + 1.7% | + 12 | + 1.8% |
| 3 | 20% | formulation 92 | + 10 | + 1.7% | + 117 | - 14.5% |
| 4 | 0.3 mg/mL | PBS + 1%DMSO | + 19 | + 6.5% | + 97 | + 11.1% |
| 5 | 0.3 mg/mL | PBS + 1%DMSO | + 4 | + 1.8% | + 14 | - 8.2% |
| 6 | 0.3 mg/mL | PBS + 1%DMSO | - 5 | + 11.7% | - 117 | - 11.0% |
| 7 | 1 mg/mL | PBS + 1%DMSO | + 20 | + 6.8% | + 44 | - 5.3% |
| 8 | 1 mg/mL | PBS + 1%DMSO | + 14 | + 7.7% | + 67 | + 0.2% |
| 9 | 1 mg/mL | PBS + 1%DMSO | + 8 | - 1.7% | + 87 | + 5.6% |
| 10 | 3 mg/mL | PBS + 1%DMSO | = | - 6.7% | + 99 | - 17.0 % |
| 11 | 3 mg/mL | PBS + 1%DMSO | + 13 | + 8.8% | - 29 | - 0.6% |
| 12 | 3 mg/mL | PBS + 1%DMSO | - 1 | - 5.2% | + 110 | - 9.8% |
| 13 | 3 mg/mL | PBS + 1%DMSO | + 20 | + 6.7% | + 17 | - 0.4% |
| 14 | 3 mg/mL | PBS + 1%DMSO | - 7 | + 3.0% | + 24 | - 15.3% |
| 15 | 10 mg/mL | PBS + 1%DMSO | + 2 | - 0.6% | + 31 | - 1.5% |
| 16 | 10 mg/mL | PBS + 1%DMSO | + 9 | = | + 51 | - 14.2% |
| 17 | 10 mg/mL | PBS + 1%DMSO | + 4 | + 2% | - 2 | - 1.0% |
| 18 | 20 mg/mL | PBS + 1%DMSO | + 4 | + 0.9% | + 21 | + 4.7% |
| 19 | 30 mg/mL | PBS + 1%DMSO | + 8 | - 1.1% | + 90 | - 6.5% |
| 20 | 30 mg/mL | PBS + 1%DMSO | + 5 | + 6.6% | - 64 | + 10.7% |
| 21 | 30 mg/mL | PBS + 1%DMSO | + 22 | + 0.8% | + 165 | - 6.2% |

| | | ***AVERAGE*** / ***EAR*** | haploids | HIR | kernels | k. a. rate |
|---|---|---|---|---|---|---|
| | | LLAEE | 23.1 | 13.0% | 194.1 | 29.9% |
| | | control | 14.9 | 10.6% | 150.8 | 33.6% |
| | | LLAEE vs control | + 8.2 | + 2.4% | + 43.3 | - 3.7% |

**Table 11. LLA effect on HIR, kernel set, kernel abortion, and overall haploids recovered.**

| | | | *Comparing Linoleic acid (LLA) to the control* | | | |
|---|---|---|---|---|---|---|
| Experiment No. | LLA Concentration | Buffer | Δ Haploids | Δ HIR | Δ kernels | Δ k. a. rate |
| 1 | 7% | Formulation 91 | - 9 | - 2.7% | - 9 | + 7.6% |
| 2 | 0.1 mg/mL | PBS + 1%DMSO | + 26 | + 2.2% | + 134 | + 14.3% |
| 3 | 0.1 mg/mL | PBS + 1%DMSO | - 18 | + 10.2% | - 122 | + 9.2% |
| 4 | 0.1 mg/mL | PBS + 1%DMSO | + 16 | + 8.0% | + 8 | + 15.4% |
| 5 | 0.5 mg/mL | PBS + 1%DMSO | + 12 | + 0.2% | + 108 | - 9.4% |
| 6 | 0.5 mg/mL | PBS + 1%DMSO | + 9 | + 3.2% | + 19 | - 3.4% |
| 7 | 0.5 mg/mL | PBS + 1%DMSO | - 3 | - 0.5% | - 14 | + 2.3% |
| 8 | 1 mg/mL | PBS + 1%DMSO | +14 | + 2.2% | + 132 | - 15.6% |
| 9 | 1 mg/mL | PBS + 1%DMSO | + 9 | + 1.7% | + 87 | - 0.7% |
| 10 | 5 mg/mL | PBS + 1%DMSO | + 23 | + 4.9% | + 112 | - 6.9% |
| 11 | 5 mg/mL | PBS + 1%DMSO | - 1 | - 3.1% | + 53 | - 5.5% |
| 12 | 5 mg/mL | PBS + 1%DMSO | - 4 | - 0.8% | - 18 | + 3.7% |
| 13 | 5 mg/mL | PBS + 1%DMSO | + 10 | + 1.6% | + 61 | - 3.0% |
| 14 | 10 mg/mL | PBS + 30% DML | + 24 | + 3.2% | + 80 | - 7.5% |
| 15 | 10 mg/mL | PBS + 30% DML | + 9 | + 1.4% | + 44 | + 2.2% |
| 16 | 10 mg/mL | PBS + 30% DML | - 2 | - 3.1% | + 32 | - 0.8% |
| 17 | 10 mg/mL | PBS + 30% DML | - 5 | - 2.6% | - 49 | - 0.2% |
| 18 | 10 mg/mL | PBS + 30% DML | + 12 | + 9.1% | + 87 | - 6.0% |
| 19 | 25 mg/mL | PBS + 1%DMSO | - 15 | + 3.7% | - 92 | - 5.1% |
| 20 | 25 mg/mL | PBS + 1%DMSO | + 3 | + 6.0% | - 59 | + 0.7% |
| 21 | 25 mg/mL | PBS + 1%DMSO | + 21 | + 2.5% | + 97 | - 11.8% |
| 22 | 25 mg/mL | PBS + 50% DML | + 20 | + 10.7% | + 107 | - 11.7% |

| | | ***AVERAGE*** / ***EAR*** | haploids | HIR | kernels | k. a. rate |
|---|---|---|---|---|---|---|
| | | LLAEE | 27.9 | 15.4% | 194.6 | 33.0% |
| | | control | 20.3 | 13.9% | 156.3 | 35.9% |
| | | LLAEE vs control | + 7.6 | + 1.5% | + 41.7 | - 2.9% |

**Table 12. MAFP effect on HIR, kernel set, kernel abortion, and overall haploids recovered.**

| | | | *Comparing MAFP to the control* | | | |
|---|---|---|---|---|---|---|
| Experiment No. | MAFP Concentration | Buffer | Δ Haploids | Δ HIR | Δ kernels | Δ k. a. rate |
| 1 | 20 ug/mL | PBS + 1%DMSO | + 2 | + 9.3% | - 20 | + 7.4% |
| 2 | 20 ug/mL | PBS + 1%DMSO | - 4 | + 0.6% | - 27 | - 5.9% |
| 3 | 20 ug/mL | PBS + 1%DMSO | + 4 | - 1.9% | + 65 | + 1.7% |
| 4 | 20 ug/mL | PBS + 1%DMSO | | - 1.4% | + 33 | - 0.6% |
| 5 | 50 ug/mL | PBS + 1%DMSO | + 16 | + 6.8% | + 38 | - 4.4% |
| 6 | 50 ug/mL | PBS + 1%DMSO | + 12 | + 4.6% | + 60 | - 8.8% |
| 7 | 50 ug/mL | PBS + 1%DMSO | + 12 | + 4.8% | + 40 | - 2.0% |
| 8 | 50 ug/mL | PBS + 1%DMSO | + 17 | + 10.5% | + 18 | - 7.7% |
| 9 | 50 ug/mL | PBS + 1%DMSO | - 8 | - 1.6% | - 35 | + 6.4% |
| 10 | 50 ug/mL | PBS + 1%DMSO | | + 9.1% | - 86 | + 6.1% |
| 11 | 2% | formulation 92 | + 8 | = | + 109 | - 1.2% |
| 12 | 2% | formulation 92 | - 1 | - 2.4% | + 26 | - 10.9% |
| 13 | 10% | formulation 92 | + 8 | + 0.8% | + 44 | + 0.7% |
| 14 | 10% | formulation 92 | - 5 | - 6.9% | - 7 | - 2.1% |
| 15 | 20% | formulation 92 | - 10 | - 0.9% | + 90 | - 6.7% |
| 16 | 20% | formulation 92 | | - 0.8% | + 10 | - 5.8% |

| | | ***AVERAGE*** / ***EAR*** | haploids | HIR | kernels | k. a. rate |
|---|---|---|---|---|---|---|
| | | MAFP | 23.6 | 14.6% | 172.5 | 34.6% |
| | | control | 0 | 12.7% | 144.4 | 36.8% |
| MAFP vs control | + 6.6 | + 1.9% | + 28.1 | - 2.2% | | |

### V. Mutagenesis/Knockouts of PLA

In an effort to alter the haploid induction rate or decrease the embryo abortion rate during haploid induction crosses, we created or obtained several mutant lines by several methods, including GM RNAi lines, TILLING lines, CRISPR lines, and TALEN lines. First, we sought evidence that targeted mutagenesis of pPLAIIα is a viable strategy to create new haploid inducer lines. Therefore, we tested both CRISPR/CAS9 and TALEN maize targeted mutation strategy aimed at the same sequence that contains the frame-shift in the mutant haploid inducer allele. This led to the generation of lines with novel mutations, which we tested for haploid induction.

There are three key components to the CRISPR process. See U.S. Patent No. 8,697,359 B1, incorporated herein by reference in its entirety. The first key component is the target sequence. The second is the Cas9, which is the endonuclease. The third key component is the guide RNA ("gRNA"), which is complementary to the target sequence and is responsible for recruiting Cas9 to the desired location. The target sequence is 18 to 20bp long, and optimally should be sitting just 5' to a protospacer adjacent motif ("PAM") in the plant genome. For Cas9 from *Streptococcus pyogenes*, the PAM sequence should be 5'-NGG-3'. Transcription of the gRNA can be driven by the Pol III promoter U3 (RNA starts with an A) or U6 (RNA starts with a G). The gRNA should carry target sequence at the 5' end right after the A (U3) or G (U6). Cas9 will generate a double-stranded break ("DSB") at the target sequence three base pairs 5' to the PAM sequence. The amino acid sequence of Cas9 is the same as Cas9 from *Streptococcus pyogenes* strain SF370, with two amino acid changes, L1164V and I1179V in the PI domain (1099-1368) in NUC lobe. Cas9 activity has been demonstrated in transformation experiments to have approximately a 90% mutation frequency of tested target sequence in corn. Generally, it is advisable to identify multiple candidate PAMs and target sequences in the target region, then look for the best one by seeing which of the sequences is unique in the genome of the target. The target plant is maize, rice, or any monocot plant.

This strategy was followed to identify CRISPR target sequences that overlap with the existing frame-shift mutation. The precise cut site is just two base pairs away from the insertion point in the frame-shift. Constructs containing both the Cas9 and the gRNA were transformed into maize plants. Generally, biallelic or homozygous mutant plants are recoverable from the multiple events generated, but heterozygous mutant plants are also useful. The heterozygous plants were selfed, then the T1 seed was grown up, screened for homozygosity of the mutation, and outcrossed. Homozygous or biallelic mutant T0 transformants were simply selfed and outcrossed to untransformed NP2222. All outcrossed embryos were isolated for ploidy analysis to find haploids.

Three different targeted mutagenesis constructs created: CRISPR/CAS9 I, CRISPR/CAS9 II, and TALEN. The difference between CRISPR/CAS9 I and II is minor. The target site locus for all three constructs was the same region where the frame-shift was found in haploid inducer lines. For the CRISPR constructs, the guide RNA sequence starts at nucleotide +1560: -GTCAACGTGGAGAC*AGG*G*-* (i.e., SEQ ID NO: 20). The -AGG- PAM site of SEQ ID NO: 20 is underlined and italicized. The four basepair insertion in haploid inducer lines is at that exact site, at nucleotide +1576. After transformation, several different CRISPR I events (comprising the expression construct found in SEQ ID NO: 34), CRISPR II events (comprising the expression construct found in SEQ ID NO: 36), and TALEN events (comprising the expression construct found in SEQ ID NO: 35) were selected, grown to maturity, and set viable seed. In the T0 generation, we performed PCR at the target site and sequenced the PCR products after sub-cloning. We identified many unique mutations amongst those events (and many of the events were chimeras or had multiple alleles).

Many plants were chimeric, as evidenced by multiple different sequences appearing in the T1 generation. After T0 self-pollination, the T1 plants segregated 1:2:1 for the target mutagenesis construct, and many had novel mutations at the target locus in either a biallelic or homozygous state. We screened seedlings at the DNA level using TAQMAN markers, identified the biallelics that lacked the Cas9 or TALEN transgenes, and performed PCR sequencing to produce PCR product reading basepairs +1494 to +1691 in the GRMZM2G471240 gene sequence. We then tested homozygous mutants for haploid induction capacity. See SEQ ID NOs: 9-19 & 42-44 for the sequences of the new T1 plants at the *mtl* gene.

The HIR was measured for the putative new lines. See Table 4, above. This HIR data is from crosses where the male was a putative haploid inducer line and the female was our standard inbred transformation line NP2222. The putative haploid inducer lines were created using either TALEN- or CRISPR/CAS9-mediated targeted mutation of the pPLAIIa locus. Among those shown here, there are eleven different putative inducer plants comprising eight different events from three distinct transformation constructs. Event 39A was a TALEN event. Events 18A and 27A were CRISPR events. The latter was a chimera as a T0 plant, and after it was self-pollinated, multiple mutations were found in the T1 population, including "biallelic" plants (by biallelic, we mean that when we sequenced the region of pPLAIIα that was mutated, we found two different novel alleles - such that it is clear that both wild type copies of the gene had been mutated, but they were mutated differently, so there are two novel alleles). Each of these eleven individual plants thus had distinct combinations of mutations in pPLAIIα. What they all had in common is that none of the eleven plants had a wild type copy of pPLAIIα. Therefore, these are all "homozygous mutant" for the pPLAIIα gene. The mutations were all frameshifts in exon 4, mimicking the original mutation in the native haploid inducer lines. Using these five plants as males, we crossed onto either one or several female ears, generating thousands of embryos. We dissected and did ploidy analysis on those progeny and discovered that each of the progeny sets had at least 3.98% haploids with a maximum of 12.5% haploids. This demonstrates that generating mutations in pPLAIIa will lead to haploid induction. We think that other types of mutations, besides frameshifts, will also lead to haploid induction. Those mutations could be anywhere in the gene, and they could be point mutations or insertions or deletions or other types of mutations.

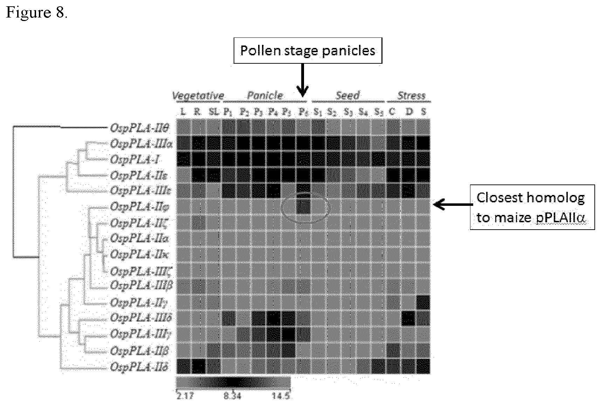

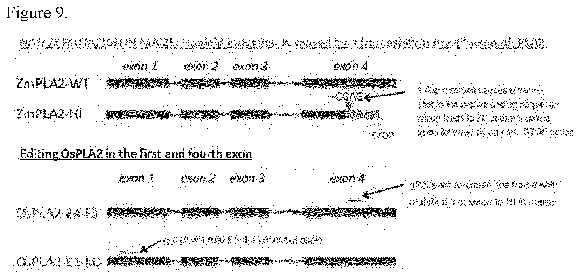

RNAi was also used to generate haploid inducer lines. For the RNAi, two hairpin constructs were made; one mapping to the border between exon 1 and 2, and the other mapping to exon 4 (Figures 8 and 9). The constructs were transformed into wild-type and the T0 plants were selfed. The T1 seed from three events per construct were grown, screened for homozygosity of the transgene, and outcrossed onto several ears as tests for haploid induction. After examining over 1500 kernels from these outcrosses, we found both events induce haploids at a rate of approximately 1% to 2%. The highest rate of haploid induction obtained on a single ear was 4.3%. That ear had about 300 kernels, so we can conclude that the embryo abortion rate was also lower than a typical high-inducer line. This work demonstrates than an RNAi + GM strategy can be used to create new haploid inducer lines in otherwise-typically wild-type lines by altering the expression of pPLAIIα.

The TILLING mutagenesis method was also used to create and identify the phospholipase mutations and maize of the present invention. Publications describing TILLING are available for crop plants such as rice: Till et al., BMC Plant Biology 7:19 (2007), tomato: Rigola et al. PLOS ONE March 13, 2009, and maize: Till et al. BMC Plant Biol. 2004 Jul 28;4:12 (2004), all of which are incorporated herein by reference. In the basic TILLING methodology, plant material, such as seed, is subjected to chemical mutagenesis, which creates a series of mutations within the genomes of the seeds' cells. The mutagenized seeds are grown into adult M1 plants and self-pollinated. DNA samples from the resulting M2 plants are pooled and are then screened for mutations in a gene of interest. Once a mutation is identified in a gene of interest, the seeds of the M2 plant carrying that mutation are grown into adult M3 plants and screened for the phenotypic characteristics associated with the gene of interest.

Any cultivar of maize having at least one phospholipase gene with substantial homology to SEQ ID NO: 1 may be used in accordance with the present invention. As used herein, "substantial homology" means that the DNA sequence of the gene is sufficiently similar to SEQ ID NO: 1 at the nucleotide level to code for the equivalent protein as SEQ ID NO: 1, allowing for allelic differences between cultivars. In accordance with one aspect of an exemplary embodiment of the invention, "substantial homology" may be present when the homology between the phospholipase gene and SEQ ID NO: 1 is as low as about 85%, provided that the homology in the conserved regions of the gene is higher (e.g., at least about 90%). Preferably, the percent identity in the coding region is 85-90%, more preferably 90-95%, and optimally, it is above 95%. One of skill in the art may prefer a maize cultivar having commercial popularity or one having specific desired characteristics in which to create the phospholipase-mutated maize. Alternatively, one of skill in the art may prefer a maize cultivar having few polymorphisms, such as an in-bred cultivar, in order to facilitate screening for mutations within the phospholipase loci.

In accordance with one aspect of an exemplary embodiment of the present invention, seeds from rice and maize were mutagenized and then grown into M1 plants. The M1 plants were then allowed to self-pollinate and seeds from the M1 plant were grown into M2 plants, which were then screened for mutations in their phospholipase locus. While M1 plants may be screened for mutations, an advantage of screening the M2 plants is that all somatic mutations correspond to the germline mutations. One of skill in the art would recognize that a variety of maize plant materials, including, but not limited to, seeds, pollen, plant tissue or plant cells, may be mutagenized in order to create the phospholipase-mutated maize of the present invention. However, the type of plant material mutagenized may affect when the plant DNA is screened for mutations. For example, when pollen is subjected to mutagenesis prior to pollination of a non-mutagenized plant, the seeds resulting from that pollination are grown into M1 plants. Every cell of the M1 plants will contain mutations created in the pollen, thus these M1 plants may then be screened for phospholipase mutations instead of waiting until the M2 generation.

Mutagens that create primarily point mutations and short deletions, insertions, transversions, and or transitions (about 1 to about 5 nucleotides), such as chemical mutagens or radiation, may be used to create the mutations of the present invention. Mutagens conforming with the method of the present invention include, but are not limited to, ethyl methanesulfonate (EMS), methylmethane sulfonate (MMS), N-ethyl-N-nitrosurea (ENU), triethylmelamine (TEM), N-methyl-N-nitrosourea (MNU), procarbazine, chlorambucil, cyclophosphamide, diethyl sulfate, acrylamide monomer, melphalan, nitrogen mustard, vincristine, dimethylnitosamine, N-methyl-N'-nitro-Nitrosoguanidine (MNNG), nitrosoguanidine, 2-aminopurine, 7,12 dimethyl-benz(a)anthracene (DMBA), ethylene oxide, hexamethylphosphoramide, bisulfan, diepoxyalkanes (diepoxyoctane (DEO), diepoxybutane (BEB), and the like), 2-methoxy-6-chloro-9[3-(ethyl-2-chloro-ethyl)aminopropylamino]acridine dihydrochloride (ICR-170), and formaldehyde. Spontaneous mutations in the nucleolar organizing region ("NOR") that may not have been directly caused by the mutagen can also be identified in accordance with various embodiments of the present invention.

Any suitable method of plant DNA preparation now known or hereafter devised may be used to prepare the maize plant DNA for phospholipase mutation screening. For example, see Chen and Ronald, Plant Molecular Biology Reporter 17:53-57, 1999; Stewart and Via, Bio Techniques 14:748-749, 1993. Additionally, several commercial kits are available, including kits from Qiagen (Valencia, Calif.) and Qbiogene (Carlsbad, Calif.).

In accordance with one aspect of an exemplary embodiment of the invention, DNA samples from individual maize plants are prepared and then pooled in order to expedite screening for mutations in phospholipase of the entire population of plants originating from the mutagenized plant tissue. The size of the pooled group may be dependent upon the sensitivity of the screening method used. In accordance with one aspect of an exemplary embodiment of the invention, groups of four or more individual maize plants are pooled.

In accordance with another aspect of an exemplary embodiment, after the DNA samples are pooled, the pools are subjected to phospholipase sequence-specific amplification techniques, such as Polymerase Chain Reaction (PCR). For a general overview of PCR, see PCR Protocols: A Guide to Methods and Applications (Innis, Gelfand, Sninsky, J., and White, eds.), Academic Press, San Diego, 1990, which is incorporated herein by reference. Any primer specific to the phospholipase locus or the sequences immediately adjacent to the phospholipase locus may be utilized to amplify the phospholipase sequences within the pooled DNA sample. Preferably, the primer is designed to amplify the regions of the phospholipase locus where useful mutations are most likely to arise. Most preferably, the primer is designed to detect mutations in the coding region of the phospholipase gene. Additionally, it is preferable for the primer to avoid known polymorphic sites in order to ease screening for point mutations. To facilitate detection of PCR products on a gel, the PCR primer may be labeled using any conventional or hereafter devised labeling method.

In accordance with one aspect of an exemplary embodiment of the invention, the PCR amplification products may be screened for phospholipase mutations using any method that identifies nucleotide differences between wild type and mutant sequences. These may include, without limitation, sequencing, denaturing high pressure liquid chromatography (dHPLC), constant denaturant capillary electrophoresis (CDCE), temperature gradient capillary electrophoresis (TGCE) (see Li et al., Electrophoresis 23(10): 1499-1511, 2002), or by fragmentation using enzymatic cleavage, such as used in the high throughput method described by Colbert et al., Plant Physiology 126:480-484, 2001. Preferably, the PCR amplification products are incubated with an endonuclease that preferentially cleaves mismatches in heteroduplexes between wild type and mutant sequences. In accordance with another aspect of an exemplary embodiment, cleavage products are electrophoresed using an automated sequencing gel apparatus, and gel images are analyzed with the aid of a standard commercial image-processing program.

The present inventors have determined that to achieve haploid induction in maize, mutations that alter phospholipase function are desirable. Preferred mutations include missense, nonsense and splice junction changes, including mutations that prematurely truncate the translation of the phospholipase protein from messenger RNA, such as those mutations that create a stop codon within the coding regions of the phospholipase gene. Such mutations include insertions, repeat sequences, modified open reading frames (ORFs) and, most preferably, point mutations.

In accordance with yet another aspect of an exemplary embodiment of the invention, once an M2 plant having a mutated phospholipase sequence is identified, the mutations are analyzed to determine its effect on the expression, translation, and/or activity of the protein. In accordance with one exemplary embodiment, the phospholipase fragment containing the mutation is sequenced, using standard sequencing techniques, in order to determine the exact location of the mutation in relation to the overall phospholipase sequence. Each mutation is evaluated in order to predict its impact on protein function (i.e., completely tolerated to loss-of-function) using bioinformatics tools such as SIFT (Sorting Intolerant from Tolerant; Ng et al., Nucleic Acids Research 31:3812-3814, 2003), PSSM (Position-Specific Scoring Matrix; Henikoff and Henikoff, Computer Applications in the Biosciences 12:135-143, 1996) and PARSESNP (Taylor and Greene, Nucleic Acids Research 31:3808-3811, 2003). For example, a SIFT score that is less than 0.05 and a large change in PSSM score (e.g., roughly 10 or above) indicate a mutation that is likely to have a deleterious effect on protein function.

In accordance with a further aspect of an exemplary embodiment, if the initial assessment of a mutation in an M2 plant indicates it to be of a useful nature and in a useful position within the phospholipase gene, then further phenotypic analysis of the maize plant containing that mutation is pursued. First, the M2 plant is backcrossed or outcrossed twice to create a BC1 plant in order to eliminate background mutations. Then the backcrossed or outcrossed BC1 plant is self-pollinated in order to create a BC1F2 plant that is homozygous for the phospholipase mutation.

Several physical characteristics of these homozygous phospholipase mutant plants are assessed to determine if the mutation results in a useful phenotypic change in the maize. Mutant phospholipase maize are evaluated for haploid induction compared to normal (e.g., wild type) parental maize or to wild type sibling control maize. Table 13 shows novel mutations obtained by TILLING.

**Table 13. Novel pPLAIIα Mutations Obtained by TILLING & their HIR. The nucleotide change column represents the position from the start of the cDNA sequence (SEQUENCE No. 1), and the changed nucleotide is capitalized within its codon context. The amino acid change is then indicated followed by the impact of that change (Tolerated or Not Tolerated). Of the two alleles that were not tolerated, one induced haploids at a rate of 1.04% (3/288).**

| Line | Nucleotide change | | Exon | AA change | Tolerated? | Diploids | Haploids | PA confirmed | HIR |
|---|---|---|---|---|---|---|---|---|---|
| 1139 | bp+128 | tGt / tAt | 1 | C13Y | Yes | 389 | 0 | 0 | 0.00% |
| 3594 | bp+167 | cCc / cTc | 1 | P26L | Yes | 381 | 0 | 0 | 0.00% |
| 0505 | bp+431 | cCg/ cTg | 1 | P114L | No | 235 | 0 | 0 | 0.00% |
| 2658 | bp+718 | Gcg / Acg | 4 | A237T | Yes | 379 | 0 | 0 | 0.00% |
| 1983 | bp+1077 | atG / atA | 4 | M356I | No | 285 | 3 | 3 | 1.04% |
| 2732 | bp+1163 | aCt / aTt | 4 | T385I | Yes | 383 | 0 | 0 | 0.00% |
| 2414 | bp+1226 | aGa / aAa | 4 | R406K | Yes | 392 | 0 | 0 | 0.00% |

The nomenclature used in the Table 13 indicates the wild type nucleotide or amino acid, followed by its position according to the referenced SEQ ID NO1, followed by the changed nucleotide or amino acid at that position using standard genetic code terminology.

For maize, TILLING the maize pPLAIIα gene generates new alleles which have low rates of haploid induction. This enables the creation of an allelic series, including knock-outs, of GRMZM2G471240. The sequence of two segments of this gene (maximum 1.5 kb, which equals 20 amplicons per gene) are screened for mutations. These sequences included the genomic sequence including introns, plus the predicted cDNA sequence and coding sequences for the two splice variants. elevant and unique amplicon sequences are designed based on those sequences, and mutation screening is performed in an existing bulked-M2 corn population. The identified mutants are characterized in terms of DNA sequence and consequences on translated protein sequence. The M3 seed is grown and selfed to generate M4 lines with putative mutant homozygous individuals segregating. These individuals are identified by PCR sequencing and outcrossed and selfed to test for these mutant lines' ability to induce haploids.

To execute the test crosses, the new lines are grown alongside a marker line which is homozygous recessive for a non-lethal color marker gene. Reciprocal crosses are used to test the specificity of induction to male vs. female transmission by evaluating the resulting plants for haploids, which exhibit the color phenotype. Positive hits are confirmed by the ploidy analysis as described above.

Individuals that are homozygous for the SNP mutations were crossed as males to the marker line female and led to the formation of a low rate of haploids in some instances (see Table 18). Positive hits are confirmed by the ploidy analysis as described above. In particular, a line that led to haploid formation had a G to A mutation at base pair 1077 of the cDNA sequence. This mutation causes an amino acid substitution of a methionine (M) to an isoleucine (I) at amino acid 356. This is a non-conservative amino acid change that may disrupt the protein's activity leading to the formation of low rate of haploids. Among 288 progeny tested, we found three haploids, for an induction rate of 1% (3/288).

### VI. Creating Haploid Inducing Lines in Rice

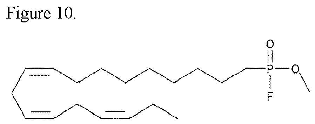

In rice, the closest homolog to the maize pPLAIIα is Os03g27610, a rice patatin-like phospholipase (*OspPLAIIϕ*) with a similar annotation, gene structure and expression pattern, i.e., expressed in pollen and absent elsewhere (Figure 10). SEQ ID NO: 21 comprises the genomic DNA sequence of Os03g27610, SEQ ID NO: 22 comprises the cDNA sequence, and SEQ ID NO: 23 comprises the amino acid sequence. The close agreement of these features, along with the short evolutionary distance between these two grasses, suggests that a mutation in the rice gene may also give rise to a haploid induction line. In a recent publication the rice protein was detected in sperm nuclei of pollen grains (Abiko et al., 2013), suggesting involvement of this protein in fertilization and/or zygote development.

To improve the haploid induction rate in maize and create the first haploid inducer lines in rice, a reverse genetics TILLING approach was used to obtain novel mutants in the maize GRMZM2G471240 gene and the rice Os03g27610 gene. *See* McCallum CM et al. (2000) Targeting induced local lesions IN genomes (TILLING) for plant functional genomics, PLANT PHYSIOL. 123: 439-42, incorporated herein by reference. TILLING provides an unbaised approach to generating new mutants as there is no control by the researcher of where the ethylmethanesulfonate (EMS) mutagen will create new mutations. A diversity and abundance of new alleles were generated and tested for haploid induction rate.

Thirteen different TILLING M3 lines were obtained. See Table 14. The PosGenomic column indicates the nucleotide position of the mutation and the change (e.g., G803A indicates that base pair G at position 803 was changed to an A). The effect is the amino acid change or other protein change that results from the mutation (e.g. A209T indicates that an Alanine at amino acid 209 was changed to a Threonine). The BLOSUM score is a prediction of the strength of the effect the amino acid change will have on the protein's conformation or fold (the more negative, the more severe the effect). The "Type" indicates the type of amino acid change ("NSM" means non-silent mutation; "PSM" means partially silent mutation; "silent" means silent mutation; "splice" means splice site mutation resulting in aberrant splicing; "intron" means mutation is in an intron). Finally the GSOR# is the line ID for the Genetic Stocks - Oryza collection at the USDA.

These thirteen lines were selfed to make the M4 and the M4 seed are grown and tested for homozygosity. Homozygous mutant individuals are selfed and outcrossed to test for haploid induction capacity. The resulting progeny are examined for DNA content per cell (ploidy) using the ploidy analyzer.

The non-conservative changes, such as the splice site changes and the changes with most negative BLOSUM scores have the greatest haploid induction potential. These should have the more destabilizing effects on the protein product, and so are the superior haploid induction TILLING alleles compared to the others, giving rise to more haploids per haploid induction cross and likely resulting in partially compromised seed set. Indeed, we have already started to see that in some of the T4 self-pollinations. The line with the lowest seed set was the splice site mutant G153A, with only 29 seeds being recovered per 12 homozygous mutant M4 plants crossed. The other lines had more than 100 recovered.

**Table 14. TILLING alleles in rice Os03g27610.**

| **Gene** | **PosGen** | **PosTIL** | **Effect** | **BLOSUM** | **Type** | **GSOR#** |
|---|---|---|---|---|---|---|
| Os03g27610 | G803A | G590A | A209T | 0 | NSM | 406317 |
| Os03g27610 | G761A | G548A | D195N | 1 | NSM | 405490 |
| Os03g27610 | G1163A | G950A | G293E | -2 | PSM | 403403 |
| Os03g27610 | G1189A | G976A | G302R | -2 | PSM | 406250 |
| Os03g27610 | T374C | T161C | intron | NA | intron | 403453 |
| Os03g27610 | G1026A | G813A | K247= | NA | silent | 406338 |
| Os03g27610 | C738T | C525T | P187L | -3 | PSM | 405205 |
| Os03g27610 | G1149A | G936A | Q288= | NA | silent | 405898 |
| Os03g27610 | G366A | G153A | splice | NA | splice | 403878 |
| Os03g27610 | G366A | G153A | splice | NA | splice | 405549 |
| Os03g27610 | C792T | C579T | T205I | -1 | PSM | 404794 |
| Os03g27610 | A1021G | A808G | T246A | 0 | NSM | 404534 |
| Os03g27610 | G558A | G345A | V156M | 1 | NSM | 404675 |

Alternately, the rice phospholipase gene found in Os03g27610 may be edited by CRISPR/Cas9 methods. As stated above, there are three key components to the CRISPR process. The first key component is the target sequence. The second is the Cas9, which is the endonuclease. The third key component is the guide RNA ("gRNA"), which is complementary to the target sequence and is responsible for recruiting Cas9 to the desired location. Guide RNAs can be in the form of single guide RNA (sgRNA) or double guide RNA (dgRNA). For rice, we created four constructs targeting the rice phospholipase gene. SEQ ID NO: 38 comprises an expression cassette that provides for dgRNA targeting Os03g27610, in exon 4 very near to where the native four base pair mutation is located in the maize homolog. In the rice gene, the guide RNA target site is GAGACCGGCAGGTACGTCGAGG. SEQ ID NO: 39 comprises an expression cassette that provides for sgRNA targeting Os03g27610, exon 4, at the same gRNA target site as is targeted in SEQ ID NO: 38. The frameshift mutations for both SEQ ID NO 38 and 39 are expected to occur where the vertical bar is placed between the G and the T in the sequence CAGGTACG | TCGAGG (at base pair +1150 of the gDNA sequence in the SEQ ID NO 22. Therefore, both of these constructs are expected to generate haploid inducer mutations that are only seven base pairs downstream from where the maize haploid inducer insertion is located. These mutations in most cases will be frame-shifting mutations that induce small insertions or deletions, for instance a deletion of a G or a T at the cut site, or any other similar mutation. SEQ ID NO: 40 comprises an expression cassette that provides for dgRNA targeting Os03g27610. SEQ ID NO: 41 comprises an expression cassette that provides for sgRNA targeting Os03g27610. Both of these harbor guide RNAs that target the sequence CCTCGCCGATTACTTCGACTGCA in Exon 1. This should generate a knockout of the majority of the coding sequence of the gene. The mutation that is generated should occur at the cut site where the vertical bar is placed between the C and the C in the sequence CCTCGC | CGATTAC (at base pair +215 of the cDNA sequence in SEQ ID NO 22). Therefore both construct 40 and 41 are expected to generate a high frequency of plants containing knockout mutations of the gene, which should also lead to high haploid induction rates in rice.

Rice plants are transformed with a transformation construct comprising a sequence selected from the group consisting of SEQ ID Nos: 38-41. Through the CRISPR/Cas9 machinery encoded in the transformation construct, new phospholipase alleles are generated in the transformants, i.e., the T0 rice plants. T0 rice plants, are grown and crossed (i.e., self-pollinated) to create T1 plants. The T1 rice plants are tested for homozygosity at the new phospholipase allele. Homozygous T1 rice plants are crossed with a rice line, and resulting progeny are tested for haploidy using a ploidy analyzer. Haploid embryos containing no detectable T1 DNA are identified and counted, and the HIR is measured. At least one haploid embryo is produced from the cross, and the HIR is elevated. Preferably, the HIR is at least 5%. The at least one haploid embryo is treated with a chromosome doubling agent, for example colchicine, and a doubled-haploid plant is grown therefrom.

## Claims

1. A method for inducing haploid embryos in a cross between two plants, the method comprising:
(a) expressing a mutated patatin-like phospholipase in a plant; or
(b) administering to a plant a small interfering RNA molecule comprising 18-21 nucleotides of a gene encoding a patatin-like phospholipase; or
(c) transforming a plant with a mutated patatin-like phospholipase; or
(d) mutating a patatin-like phospholipase sequence of a plant using gene editing;
wherein the plant is used as a parent plant in the cross between two plants, such that the cross produces at least one haploid embryo.

2. The method of claim 1, wherein the patatin-like phospholipase is encoded by (i) a nucleic acid sequence comprising SEQ ID NO: 1 or a sequence 70% identical to SEQ ID NO: 1; or (ii) a nucleic acid comprising SEQ ID NO: 3 or a sequence 70% identical to SEQ ID NO: 3.

3. The method of claim 1, wherein the gene editing of step (d) is accomplished by a site-directed mutagenesis technique selected from the group consisting of CRISPR, TALENs, zinc fingers, and meganucleases.

4. The method of claim 1, wherein the cross between two plants is between two monocot plants, wherein the two monocot plants are maize plants, rice plants, wheat plants, or barley plants.

5. The method of claim 1, wherein the plant used as a parent plant in the cross is a maize plant or a rice plant.

6. The method of claim 1, wherein the maize plant or the rice plant provides pollen used in the cross.

7. The method of claim 1, wherein the small interfering RNA molecule comprises 18-21 consecutive nucleotides of SEQ ID NO: 1.

8. A haploid embryo produced by the method of any of claims 1 to 7.

9. A haploid plant produced from the haploid embryo of claim 8.

10. A haploid seed comprising the haploid embryo of claim 8.

11. A doubled haploid plant produced from the haploid embryo of claim 8, wherein the haploid embryo is exposed to a chromosome doubling agent.

12. A cDNA comprising SEQ ID NO: 3, a sequence orthologous to SEQ ID NO: 3, or a sequence 70% identical to SEQ ID NO: 3.

13. The cDNA of claim 12, wherein the sequence orthologous to SEQ ID NO: 3 comprises a sequence selected from the group consisting of SEQ ID NO: 22 and a nucleotide sequence which encodes any of SEQ ID NOs: 73-81.

14. A plant containing a human-induced, non-transgenic mutation within a patatin-like phospholipase gene which creates a premature stop codon in the patatin-like phospholipase gene.

15. A seed, a pollen grain, a plant part, or the progeny of the plant of claim 14; wherein the seed, the pollen grain, the plant part, or the progeny comprises the mutation.

16. The plant of claim 14, wherein the plant is selected from the group consisting of maize (*Zea mays*), sorghum (*Sorghum bicolor*), millet (*Setaria italica*), barley (*Hordeum vulgare*), stiff brome (*Brachypodium distachyon*), rice (*Oryza sativa*), wheat (*Triticum aestivum*), banana (*Musa acuminata*)*,* palm (*Elaeis guineensis*), oats (*Avena sativa*), sunflower (*Helianthus annuus*), sugar beet (*Beta vulgaris*), *Arabidopsis thaliana,* and soybean (*Glycine max*).

17. The plant of claim 16, wherein the plant is a maize plant.

18. The plant of claim 16, wherein the plant is a rice plant.

19. The plant of claim 14, wherein the patatin-like phosphase gene comprises SEQ ID NO: 1, a sequence orthologous to SEQ ID NO: 1, or a sequence 70% identical to SEQ ID NO: 1.

20. The plant of claim 19, wherein the patatin-like phospholipase gene comprises SEQ ID NO: 3.
